# EUROPEAN PATENT SPECIFICATION

(11) **EP 2 561 037 B1**
(45) Date of publication and mention of the grant of the patent: **15.03.2017**
(21) Application number: 11772295.9
(22) Date of filing: 25.04.2011
(51) Int. Cl.: C09K 11/06, H01L 51/54

(54) **COMPOUND FOR OPTOELECTRONIC DEVICE, ORGANIC LIGHT EMITTING DIODE INCLUDING THE SAME AND DISPLAY INCLUDING THE ORGANIC LIGHT EMITTING DIODE**
VERBINDUNG FÜR EINE OPTOELEKTRONISCHE VORRICHTUNG, ORGANISCHE LICHTEMITTIERENDE DIODE DAMIT UND ANZEIGE MIT DER ORGANISCHEN LICHTEMITTIERENDEN DIODE
COMPOSÉ DESTINÉ À UN DISPOSITIF OPTOÉLECTRONIQUE, DIODE ÉLECTROLUMINESCENTE ORGANIQUE LE COMPRENANT ET AFFICHAGE COMPRENANT LA DIODE ÉLECTROLUMINESCENTE ORGANIQUE

(30) Priority: 22.07.2010 US 344433 P; 23.04.2010 KR 20100038169
(43) Date of publication of application: 27.02.2013
(73) Proprietor: Cheil Industries Inc., Kumi-city, Kyungsangbuk-do 730-030 (KR)
(72) Inventor: RYU, Dong-Wan, Uiwang-si, Gyeonggi-do 437-711 (KR); JUNG, Sung-Hyun, Uiwang-si, Gyeonggi-do 437-711 (KR); HUH, Dal-Ho, Uiwang-si, Gyeonggi-do 437-711 (KR); LEE, Kyoung-Mi, Uiwang-si, Gyeonggi-do 437-711 (KR); LEE, Nam-Heon, Uiwang-si, Gyeonggi-do 437-711 (KR); CHAE, Mi-Young, Uiwang-si, Gyeonggi-do 437-711 (KR)
(74) Representative: Michalski Hüttermann & Partner Patentanwälte mbB
(86) International application number: PCT/KR2011/003003
(87) International publication number: WO 2011/133007

(56) References cited:
- WO-A1-2007/125714
- WO-A1-2008/062636
- WO-A1-2009/020095

## Description

### BACKGROUND OF THE INVENTION

### (a) Field of the Invention

A compound for an optoelectronic device being capable of providing an optoelectronic device having excellent life-span, efficiency, electrochemical stability, and thermal stability, an organic light emitting doide including the same, and a display including the organic light emitting diode, are disclosed.

### (b) Description of the Related Art

A photoelectric device is, in a broad sense, a device for transforming photo-energy to electrical energy, or conversely, a device for transforming electrical energy to photo-energy.

An organic photoelectric device may be classified as follows in accordance with its driving principles. A first organic photoelectric device is an electron device driven as follows: excitons are generated in an organic material layer by photons from an external light source; the excitons are separated to electrons and holes; and the electrons and holes are transferred to different electrodes from each other as a current source (voltage source).

A second organic photoelectric device is an electron device driven as follows: a voltage or a current is applied to at least two electrodes to inject holes and/or electrons into an organic material semiconductor positioned at an interface of the electrodes; and then the device is driven by the injected electrons and holes.

As examples, the organic photoelectric device includes an organic light emitting diode (OLED), an organic solar cell, an organic photo-conductor drum, an organic transistor, an organic memory device, etc., and it requires a hole injecting or transporting material, an electron injecting or transporting material, or a light emitting material.

Particularly, an organic light emitting diode (OLED) has recently drawn attention due to an increase in demand for flat panel displays. In general, organic light emission refers to transformation of electrical energy to photo-energy.

The organic light emitting diode transforms electrical energy into light by applying current to an organic light emitting material. It has a structure in which a functional organic material layer is interposed between an anode and a cathode. The organic material layer includes multiple layers including different materials from each other, for example a hole injection layer (HIL), a hole transport layer (HTL), an emission layer, an electron transport layer (ETL), and an electron injection layer (EIL), in order to improve efficiency and stability of an organic light emitting diode.

In such an organic light emitting diode, when a voltage is applied between an anode and a cathode, holes from the anode and electrons from the cathode are injected to an organic material layer. The generated excitons generate light having certain wavelengths while shifting to a ground state.

Recently, it is has become known that a phosphorescent light emitting material can be used for a light emitting material of an organic light emitting diode in addition to the fluorescent light emitting material. Such a phosphorescent material emits lights by transiting the electrons from a ground state to an exited state, non-radiance transiting of a singlet exciton to a triplet exciton through intersystem crossing, and transiting a triplet exciton to a ground state to emit light.

As described above, in an organic light emitting diode, an organic material layer includes a light emitting material and a charge transport material, for example a hole injection material, a hole transport material, an electron transport material, an electron injection material, and so on.

The light emitting material is classified as blue, green, and red light emitting materials according to emitted colors, and yellow and orange light emitting materials to emit colors approaching natural colors.

When one material is used as a light emitting material, a maximum light emitting wavelength is shifted to a long wavelength or color purity decreases because of interactions between molecules, or device efficiency decreases because of a light emitting quenching effect. Therefore, a host/dopant system is included as a light emitting material in order to improve color purity and increase luminous efficiency and stability through energy transfer.

In order to implement the above excellent performance of an organic light emitting diode, a material constituting an organic material layer, for example a hole injection material, a hole transport material, a light emitting material, an electron transport material, an electron injection material, and a light emitting material such as a host and/or a dopant should be stable and have good efficiency. However, development of an organic material layer forming material for an organic light emitting diode has not been satisfactory up to now, and thus there is a need for a novel material. This material development is also required for other organic photoelectric devices.

The low molecular organic light emitting diode is manufactured as a thin film in a vacuum deposition method, and can have good efficiency and life-span performance. A polymer organic light emitting diode manufactured in an Inkjet or spin coating method has an advantage of low initial cost and being large-sized.

Both low molecular organic light emitting and polymer organic light emitting diodes have an advantage of self-light emitting, high speed response, wide viewing angle, ultrathinness, high image quality, durability, a large driving temperature range, and the like. In particular, they have good visibility due to the self-light emitting characteristic compared with a conventional LCD (liquid crystal display) and have an advantage of decreasing thickness and weight of an LCD up to a third, because they do not need a backlight.

In addition, since they have a response speed that is 1000 times faster than an LCD, they can realize a perfect motion picture without an after-image. Based on these advantages, they have been remarkably developed to have 80 times the efficiency and more than 100 times the life-span since they first came out in the late 1980s. Recently, they have kept becoming rapidly larger, such as development of a 40-inch organic light emitting diode panel.

They are simultaneously required to have improved luminous efficiency and life-span in order to be larger. Herein, their luminous efficiency needs a smooth combination between holes and electrons in an emission layer. However, since an organic material in general has slower electron mobility than hole mobility, it has a drawback of inefficient combination between holes and electrons. Accordingly, while increasing electron injection and mobility from a cathode, simultaneous prevention of movement of holes is required.

In order to improve the life-span, material crystallization caused by Joule heat generated during device operation must be prevented. Accordingly, there has been a strong need for an organic compound having excellent electron injection and mobility, and high electrochemical stability. One possible solution for improving the life-span is for instance disclosed in the patent document US 2009/0066235 A1.

### SUMMARY OF THE INVENTION

A compound for an optoelectronic device that may act as hole injection, hole transport, light emitting, or electron injection and/or transport material, and also act as a light emitting host along with an appropriate dopant, is provided.

An organic optoelectronic device having excellent life-span, efficiency, driving voltage, electrochemical stability, and thermal stability is provided.

According to one embodiment of the present invention, a compound for an optoelectronic device represented by the following Chemical Formula 1 is provided.

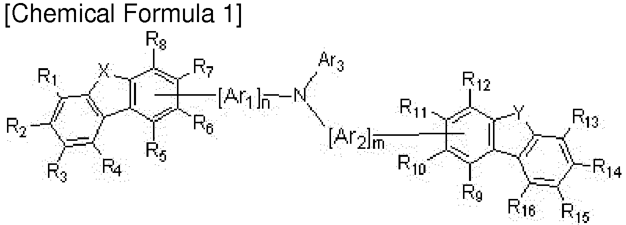

In Chemical Formula 1,
R₁ to R₁₆ are the same or different, and are independently selected from hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a

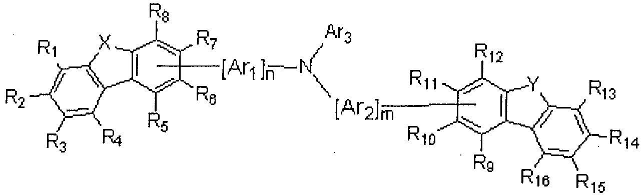

In Chemical Formula 1,
R₁ to R₁₆ are the same or different, and are independently selected from hydrogen, deuterium, a single bond, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonyl amino group, a substituted or unsubstituted C7 to C20 aryloxycarbonyl amino group, a substituted or unsubstituted C1 to C20 sulfamoyl amino group, a substituted or unsubstituted C1 to C20 sulfonyl group, a substituted or unsubstituted C1 to C20 alkylthiol group, a substituted or unsubstituted C6 to C20 arylthiol group, a substituted or unsubstituted C1 to C20 heterocyclothiol group, a substituted or unsubstituted C1 to C20 ureide group, and a substituted or unsubstituted C3 to C40 silyl group,
at least one of R₁ to R₈ represents a bond with Ar₁,
at least one of R₉ to R₁₆ represents a bond with Ar₂,
at least one of R₁ to R₈ is optionally bound to Ar₁ through a sigma bond, at least one of R₉ to R₁₆ is optionally bound to Ar₂ through a sigma bond,
X is selected from NR₁₇, O, S and SO₂(O=S=O), R₁₇ is a substituted or unsubstituted C1 to C20 alkyl group, a substituted or unsubstituted C6 to C30 aryl group and a substituted or unsubstituted C2 to C30 heteroaryl group,
Y is selected from O, S and SO₂ (O=S=O),
Ar₁ and Ar₂ are the same or different, and are independently a substituted or unsubstituted C6 to C30 aryl group or a substituted or unsubstituted C2 to C30 heteroaryl group,
n is an integer ranging from 1 to 4,
m is an integer ranging from 0 to 4, and
Ar₃ is a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthydinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, or a substituted or unsubstituted phenoxazinyl group.

X is selected from NR₁₇, O, S, and SO₂ (O=S=O), wherein R₁₇ is a substituted or unsubstituted C1 to C20 alkyl group, a substituted or unsubstituted C6 to C30 aryl group, or a substituted or unsubstituted C2 to C30 substituted or unsubstituted C1 to C20 acyl group, a substituted or unsubstituted C2 to C20 alkoxycarbonyl group, a substituted or unsubstituted

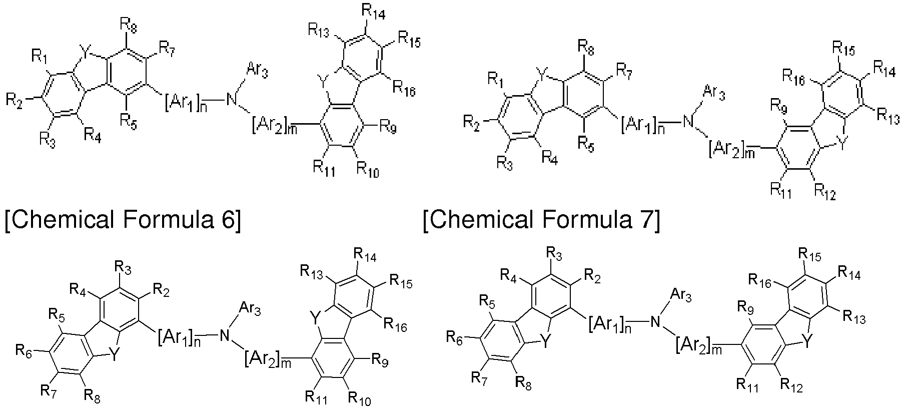

In Chemical Formulae 2 to 7,
R₁ to R₁₆ are the same or different, and are independently selected from hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonyl amino group, a substituted or unsubstituted C7 to C20 aryloxycarbonyl amino group, a substituted or unsubstituted C1 to C20 sulfamoyl amino group, a substituted or unsubstituted C1 to C20 sulfonyl group, a substituted or unsubstituted C1 to C20 alkylthiol group, a substituted or unsubstituted C6 to C20 arylthiol group, a substituted or unsubstituted C1 to C20 heterocyclothiol group, a substituted or unsubstituted C1 to C20 ureide group, and a substituted or unsubstituted C3 to C40 silyl group,
R₁₇ is a substituted or unsubstituted C1 to C20 alkyl group, a substituted or unsubstituted C6 to C30 aryl group, or a substituted or unsubstituted C2 to C30 heteroaryl group,
Y is selected from O, S, and SO₂ (O=S=O),
Ar₁ and Ar₂ are the same or different, and are independently a substituted or unsubstituted C6 to C30 aryl group or a substituted or unsubstituted C2 to C30 heteroaryl group,
n is an integer ranging from 1 to 4,
m is an integer ranging from 0 to 4, and
Ar₃ is a substituted or unsubstituted C6 to C30 aryl group or a substituted or unsubstituted C2 to C30 heteroaryl group,
provided that Ar₃ is not a substituted or unsubstituted carbazolyl group, a substituted or unsubstituted dibenzofuranyl group, or a substituted or unsubstituted dibenzothiophenyl group.

The compound for an optoelectronic device may include one of the compounds represented by the following Chemical Formula 8 or 9.

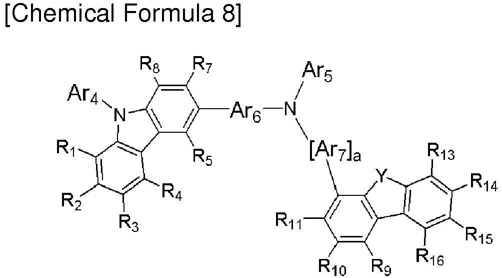

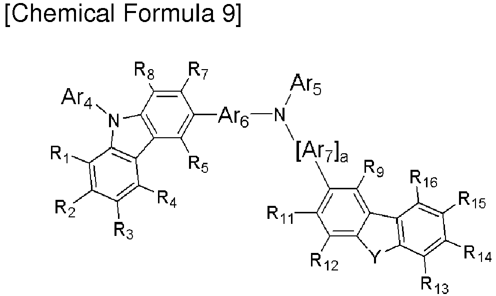

In Chemical Formulae 8 and 9, Ar₄ and Ar₅ are the same or different, and are independently a substituent selected from the following Chemical Formulae 10 to 18.

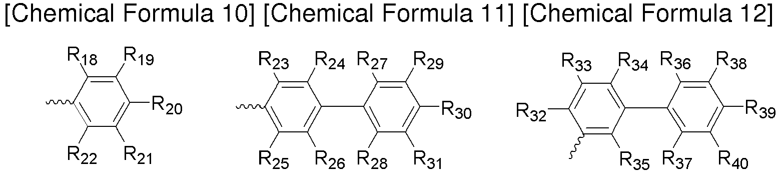

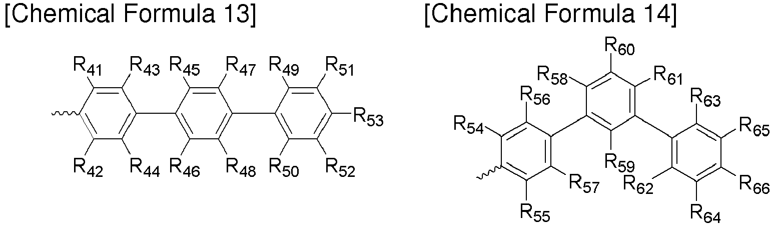

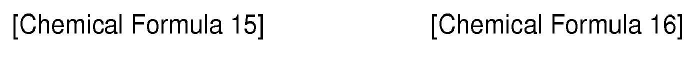

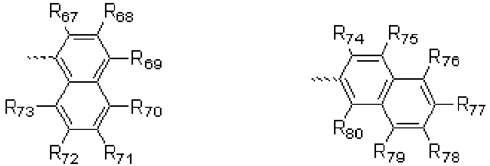

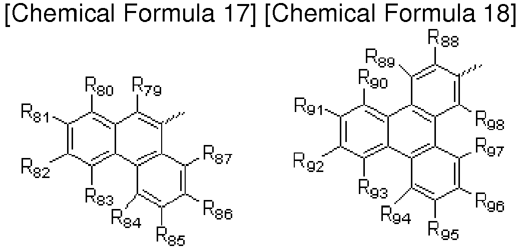

Herein, R₁ to R₅, R₇ to R₁₆, and R₁₈ to R₉₈ are the same or different, and are independently selected from hydrogen, deuterium, a halogen, a cyano group, a hydroxyl group, an amino group, a substituted or unsubstituted C1 to C20 amine group, a nitro group, a substituted or unsubstituted C1 to C20 alkyl group, a substituted or unsubstituted C1 to C20 alkoxy group, and a substituted or unsubstituted C3 to C40 silyl group.

Ar₆ and Ar₇ are the same or different, and are independently a substituent selected from the above Chemical Formulae 9 to 17, and at least one of R₁₈ to R₉₈ is bound to an adjacent atom, and
a is 0 or 1.

Ar₃ may be selected from a substituted or unsubstituted phenyl group, a substituted or unsubstituted naphthyl group, a substituted or unsubstituted anthracenyl group, a substituted or unsubstituted phenanthryl group, a substituted or unsubstituted naphthacenyl group, a substituted or unsubstituted pyrenyl group, a substituted or unsubstituted biphenylyl group, a substituted or unsubstituted p-terphenyl group, a substituted or unsubstituted m-terphenyl group, a substituted or unsubstituted chrysenyl group, a substituted or unsubstituted triperylenyl group, a substituted or unsubstituted perylenyl group, a substituted or unsubstituted indenyl group, a substituted or unsubstituted furanyl group, a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthydinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, or a substituted or unsubstituted phenoxazinyl group.

Ar₄ is selected from one of the substituents represented by the above Chemical Formulae 10 to 18 provided that at least one of R₁₈ to R₉₈ is not hydrogen, and for example it may be selected from deuterium, a halogen, a cyano group, a hydroxyl group, an amino group, a substituted or unsubstituted C1 to C20 amine group, a nitro group, a substituted or unsubstituted C1 to C20 alkyl group, a substituted or unsubstituted C1 to C20 alkoxy group, and a substituted or unsubstituted C3 to C40 silyl group.

The compound for an optoelectronic device may include at least one of the compounds represented by the following Chemical Formulae A-1 to A-305, A-414 to A-416, A-457, A-458, or A-469 to A-473.

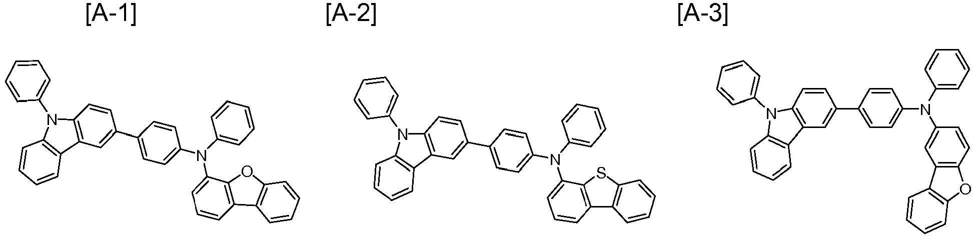

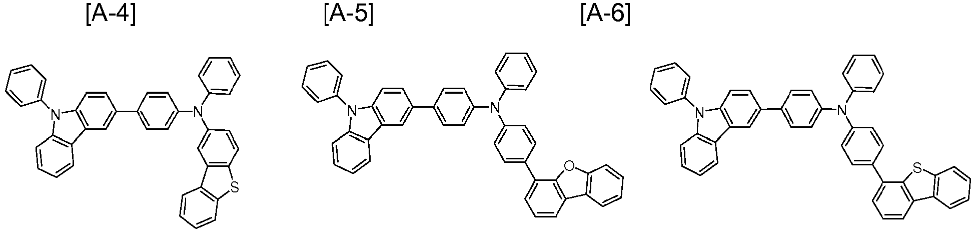

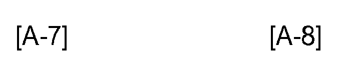

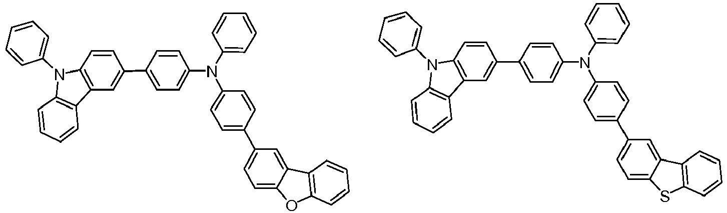

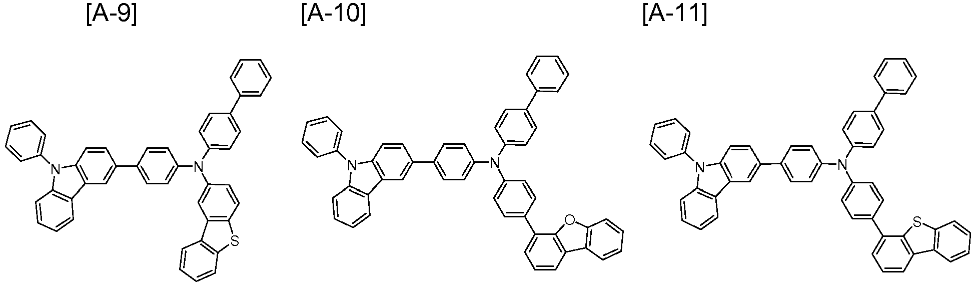

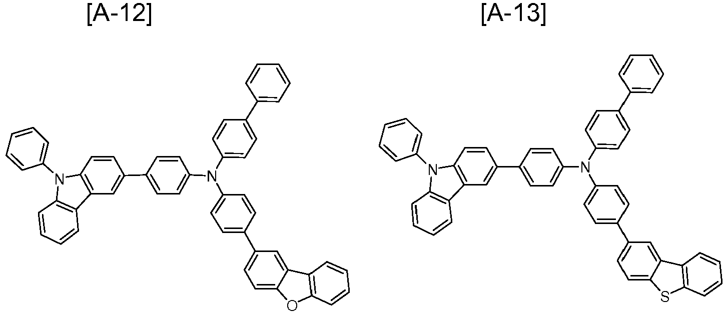

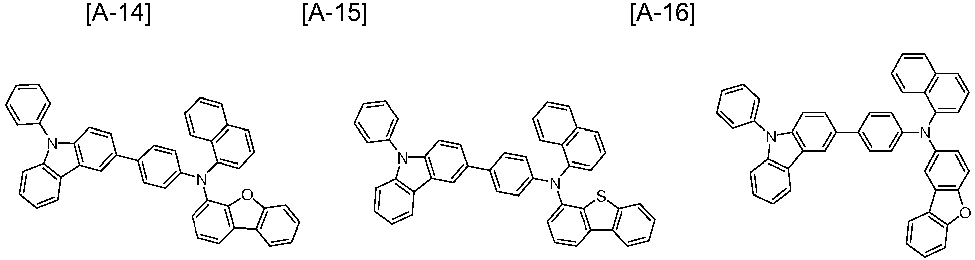

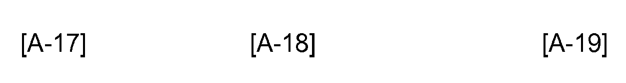

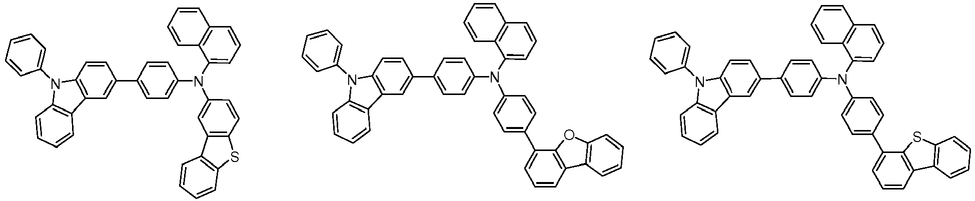

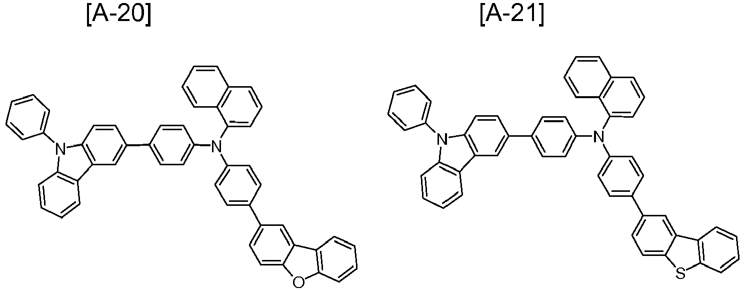

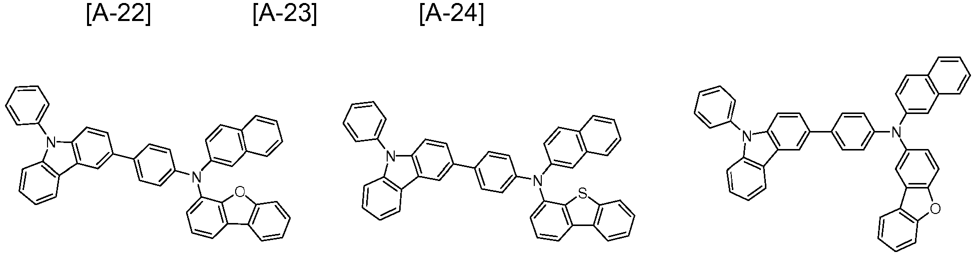

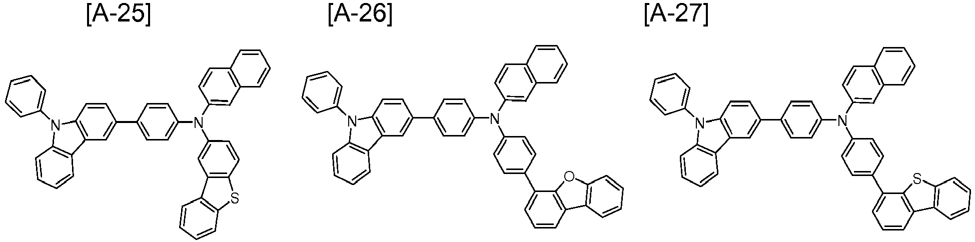

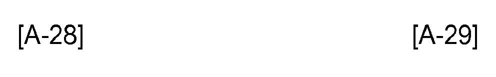

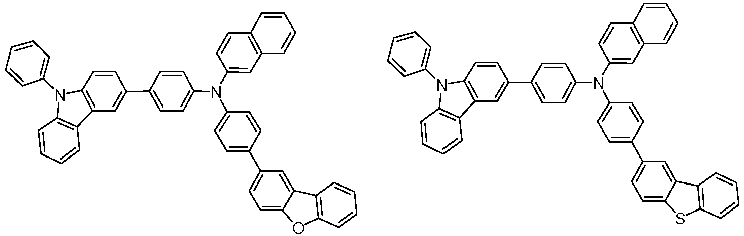

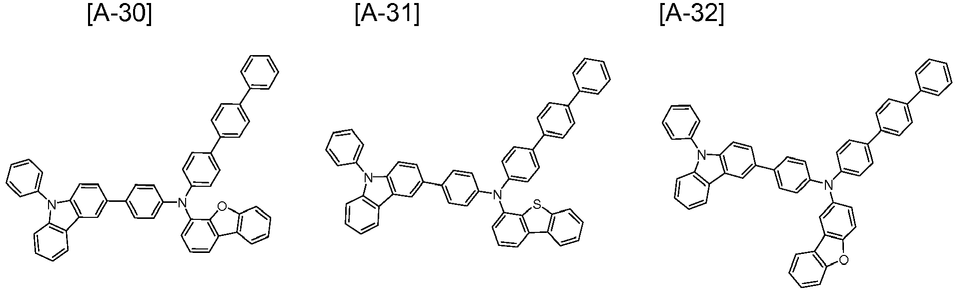

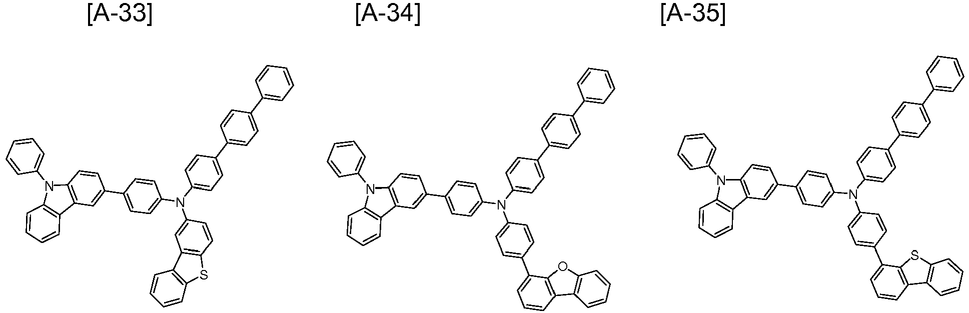

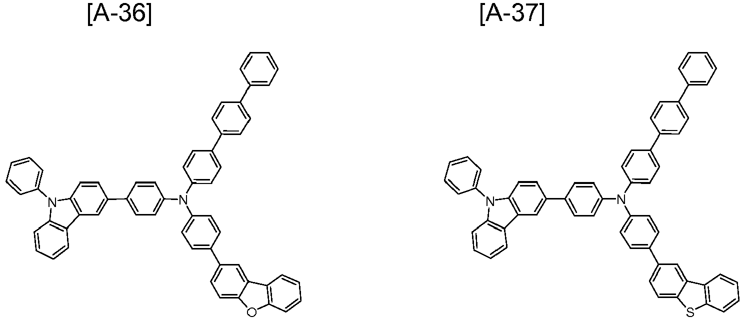

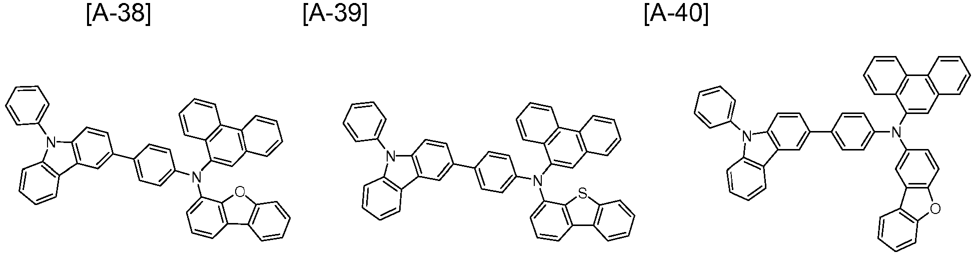

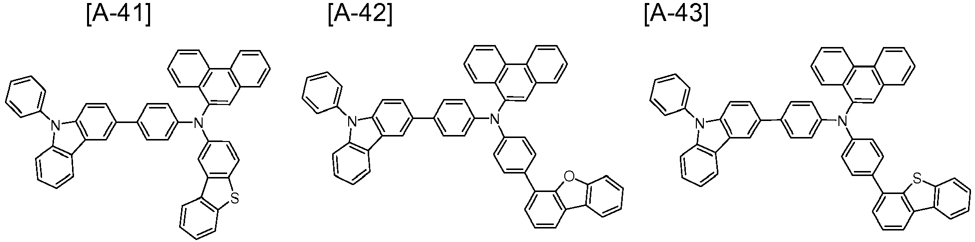

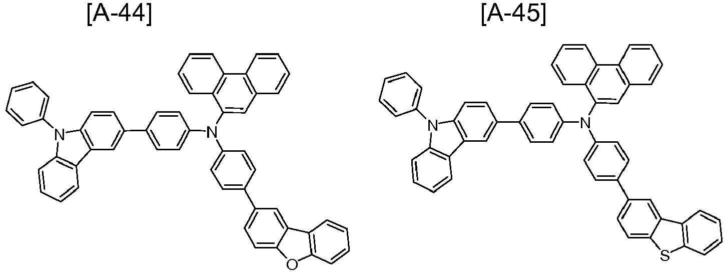

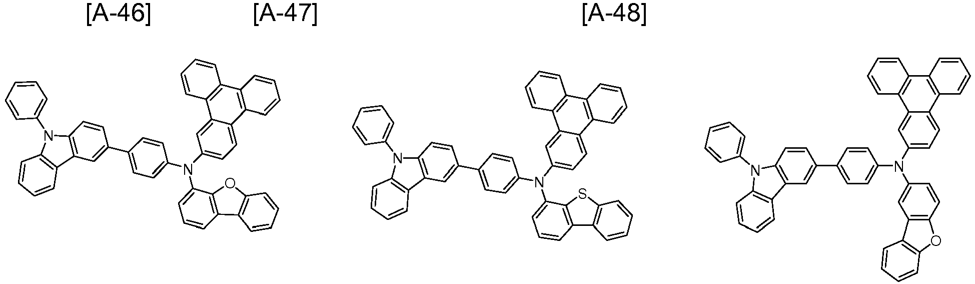

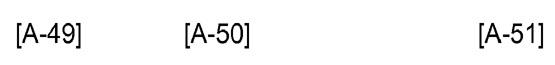

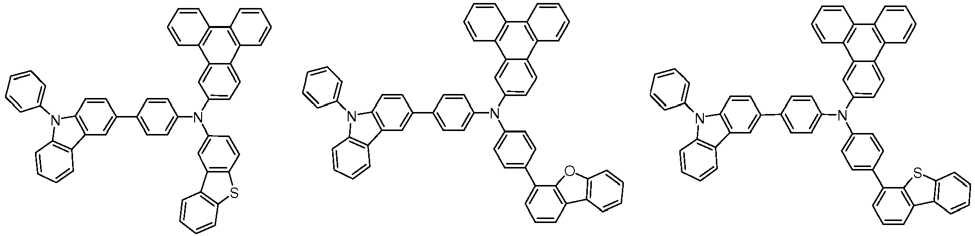

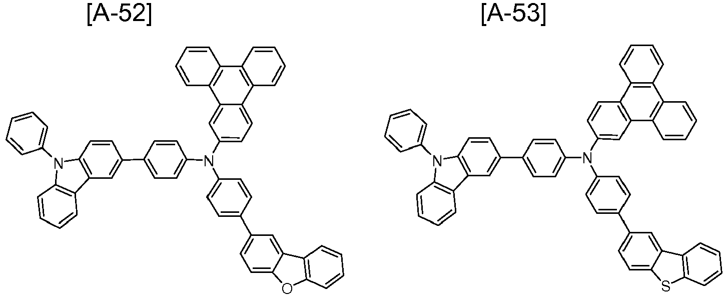

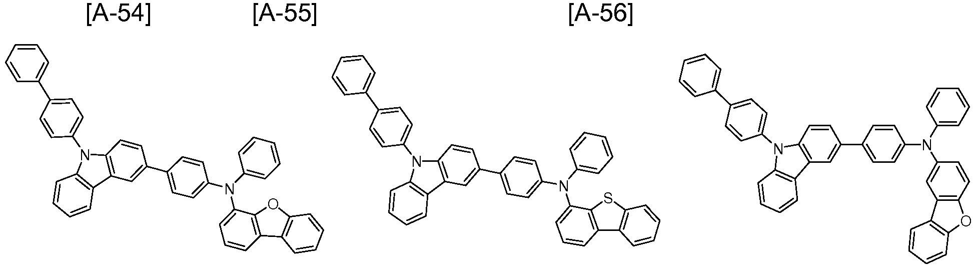

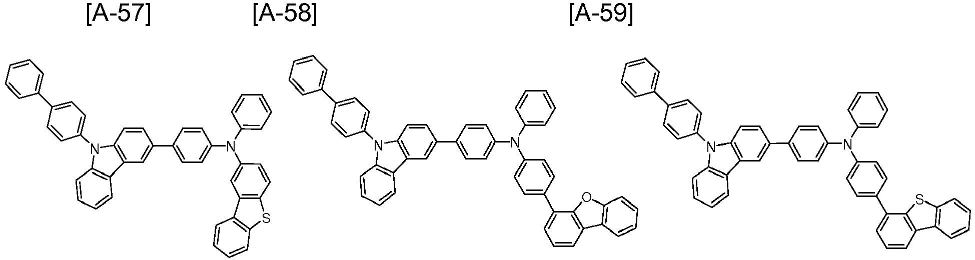

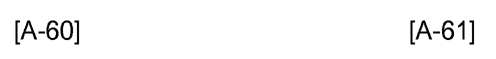

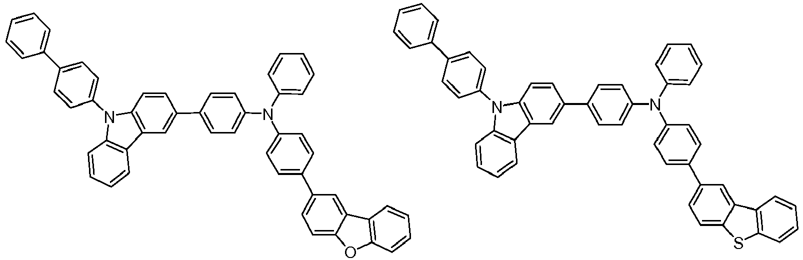

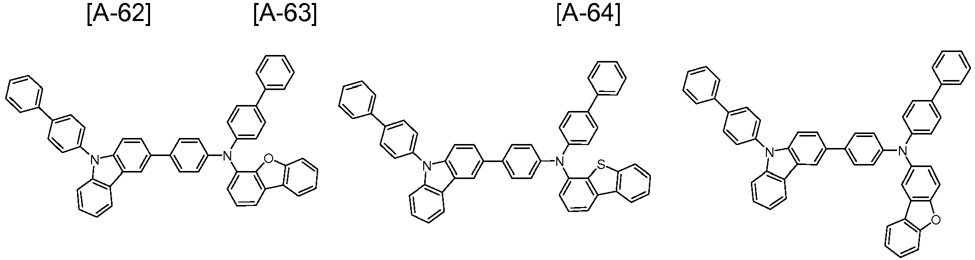

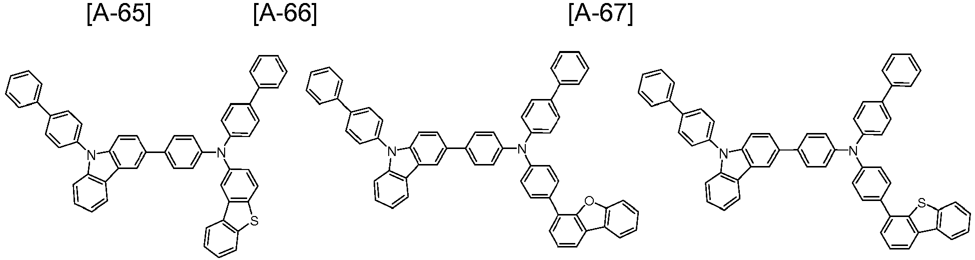

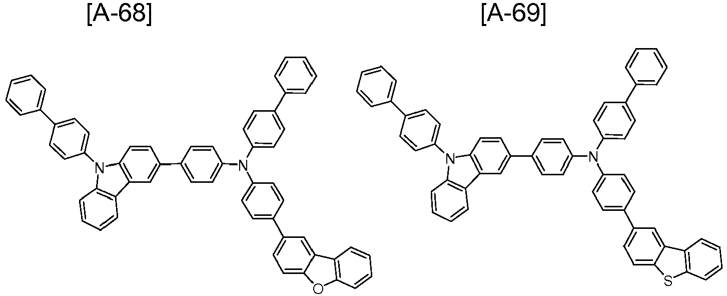

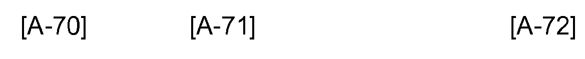

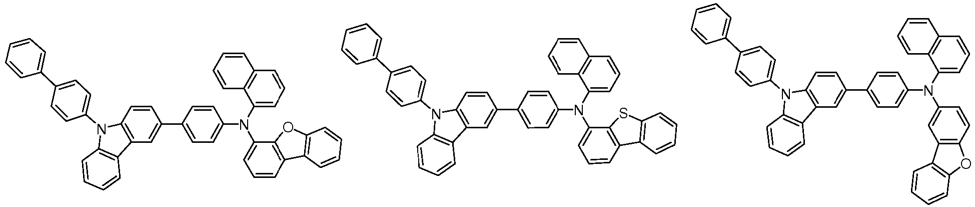

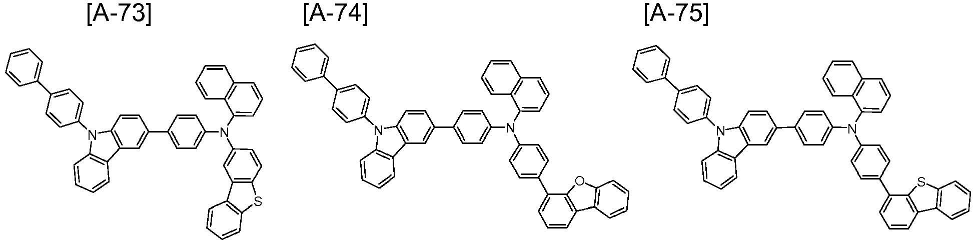

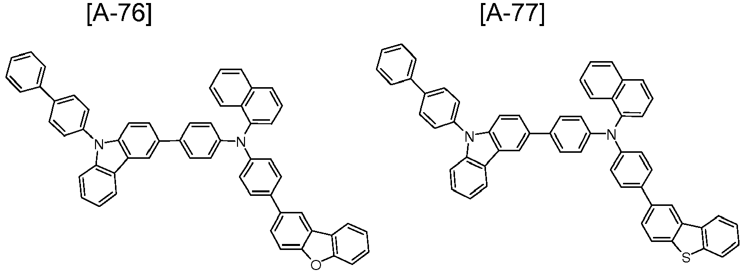

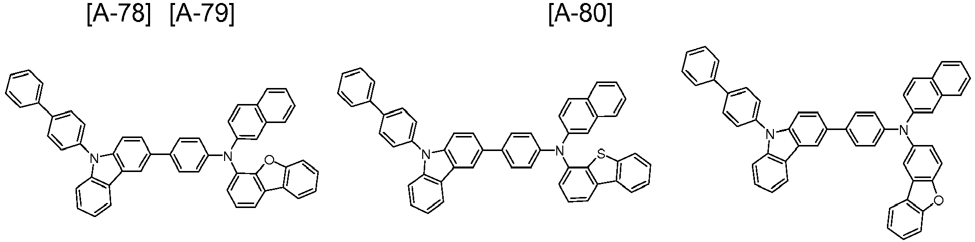

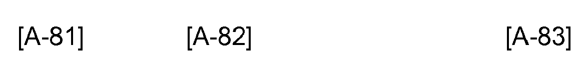

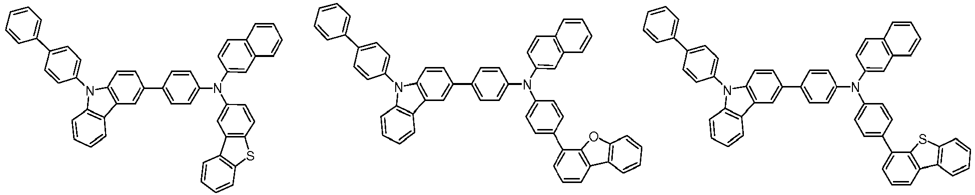

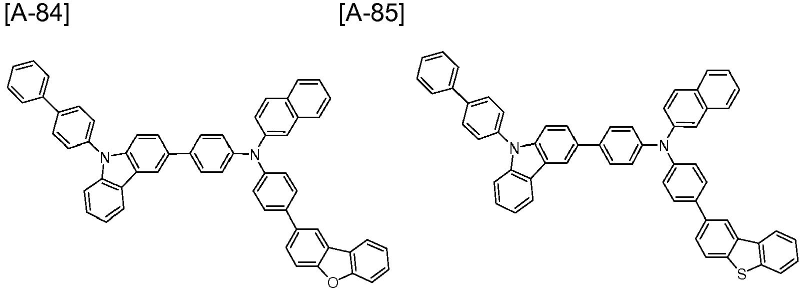

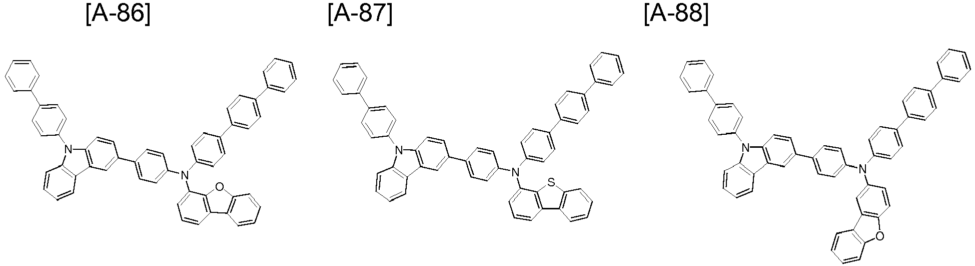

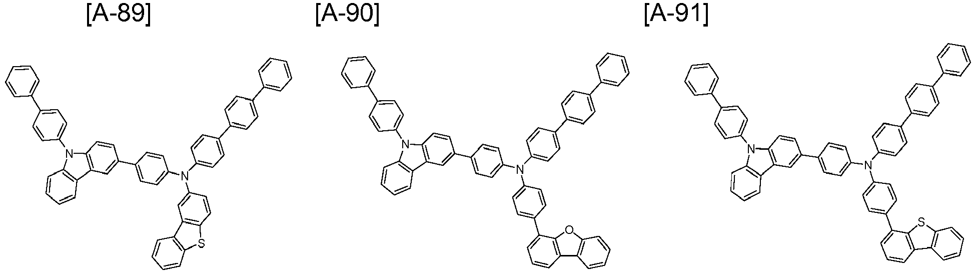

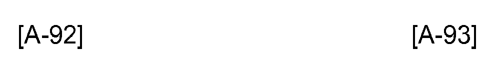

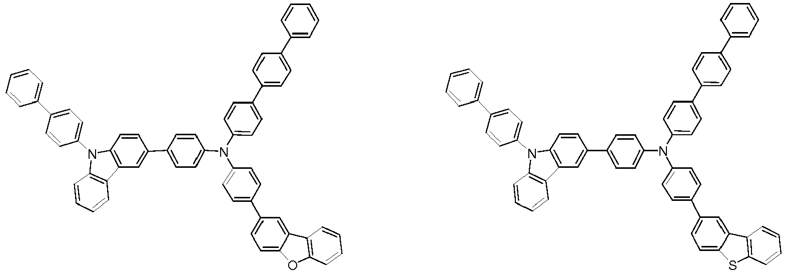

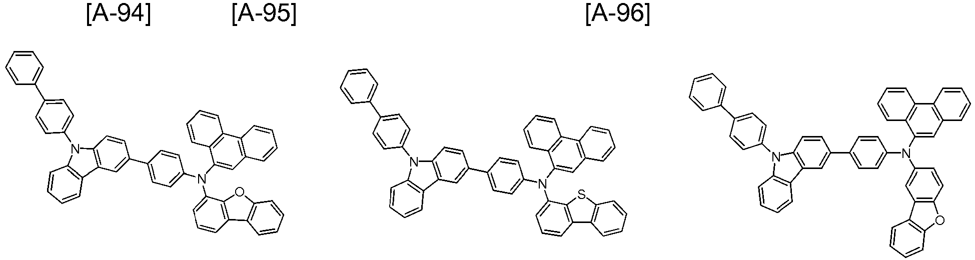

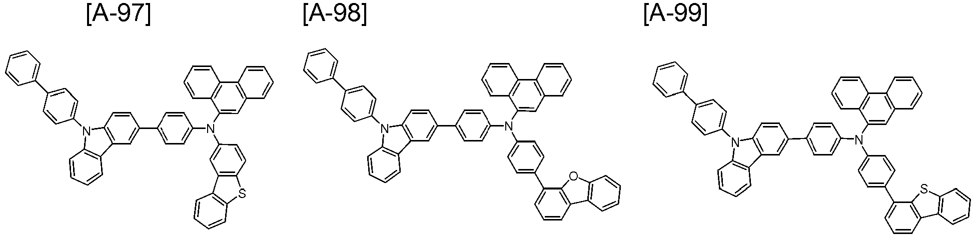

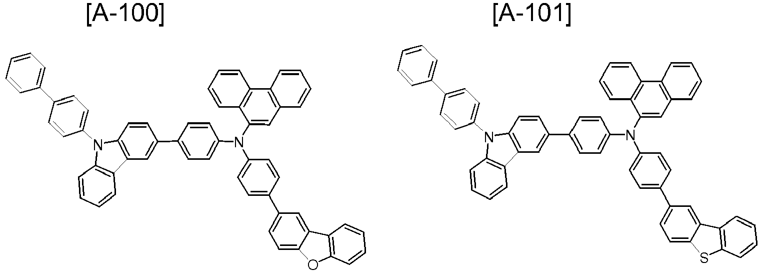

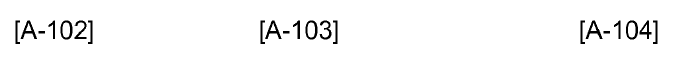

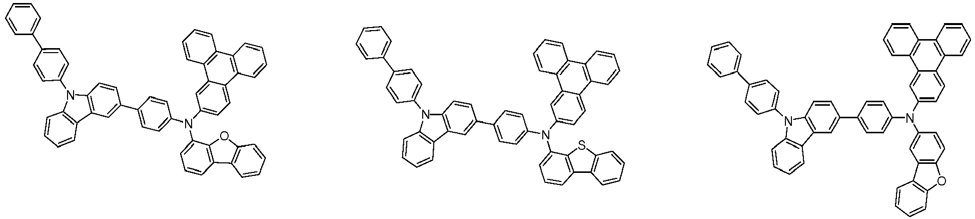

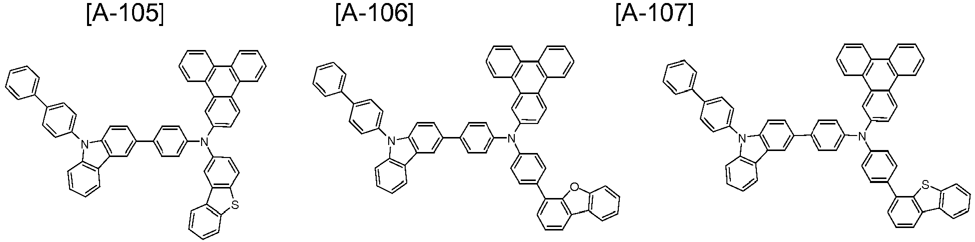

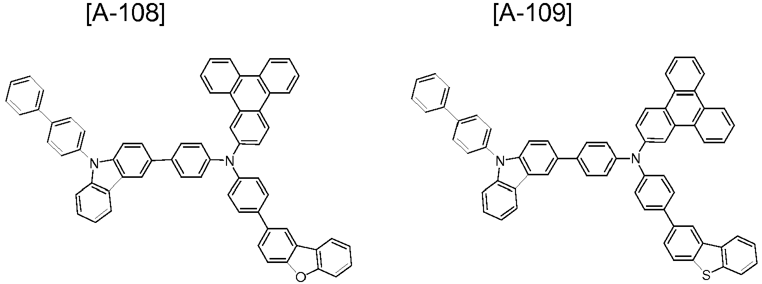

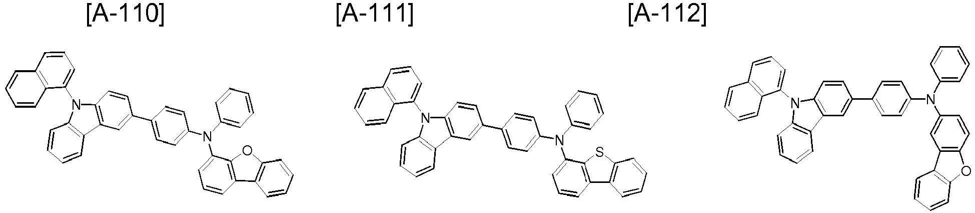

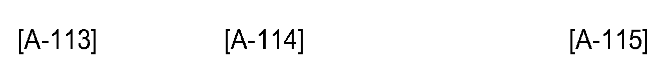

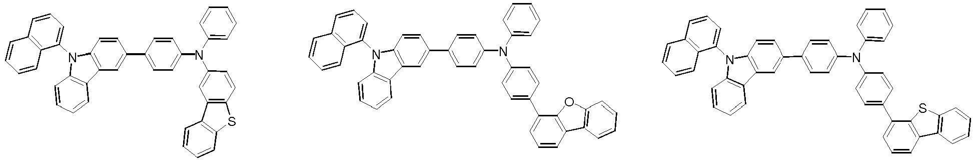

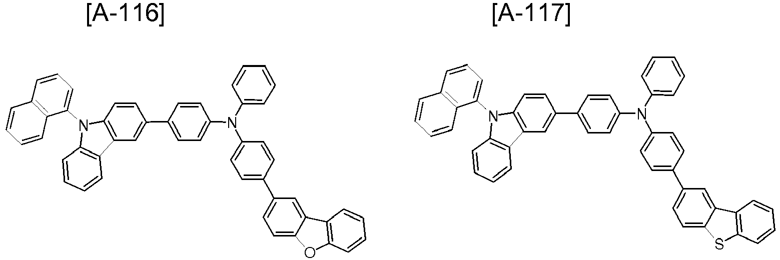

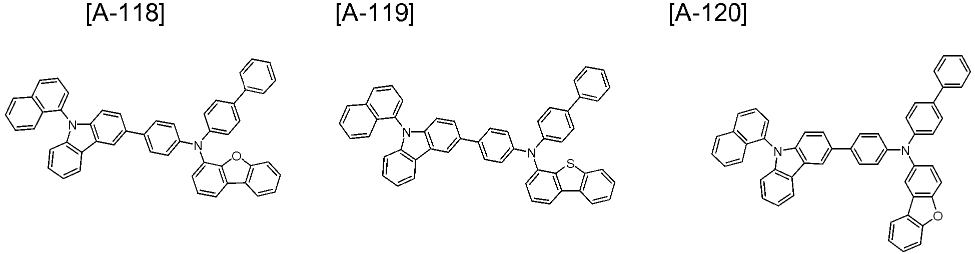

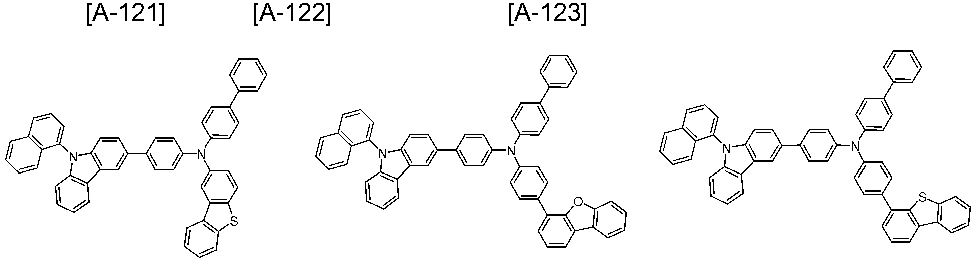

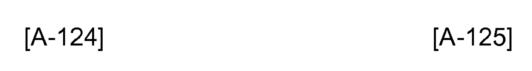

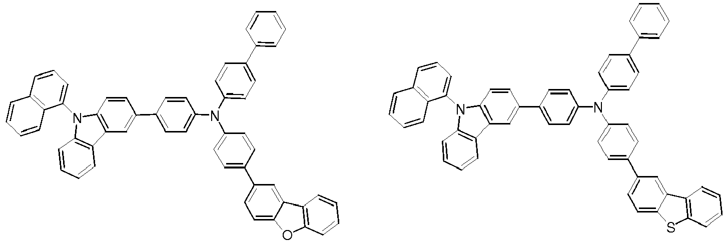

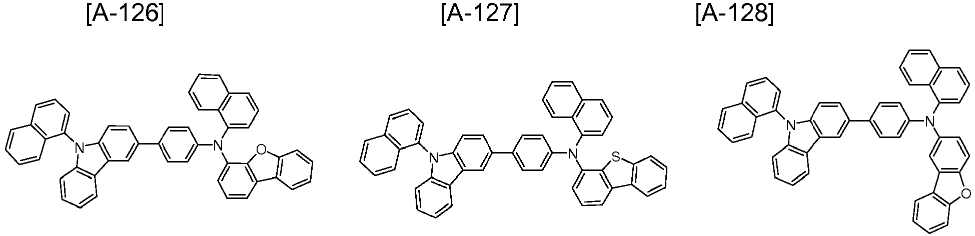

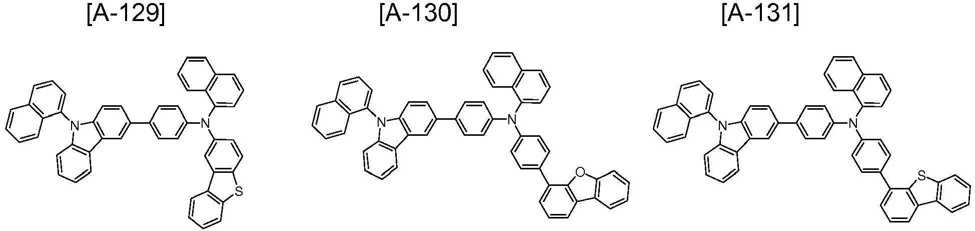

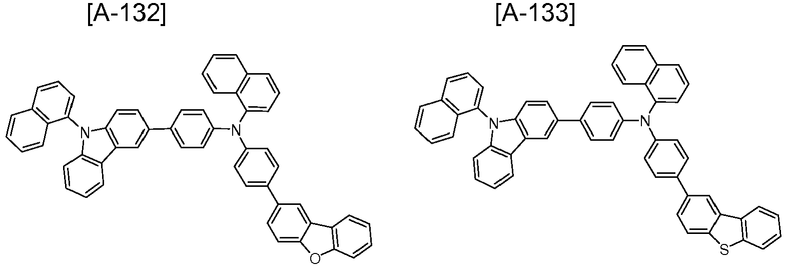

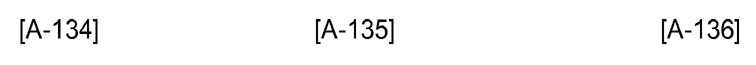

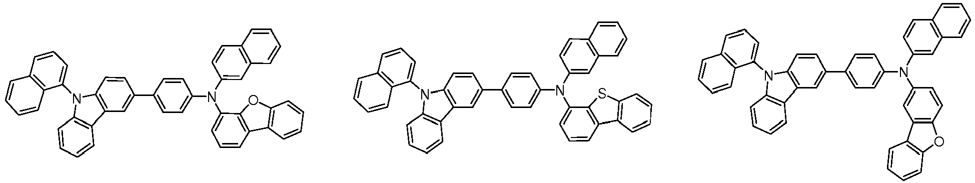

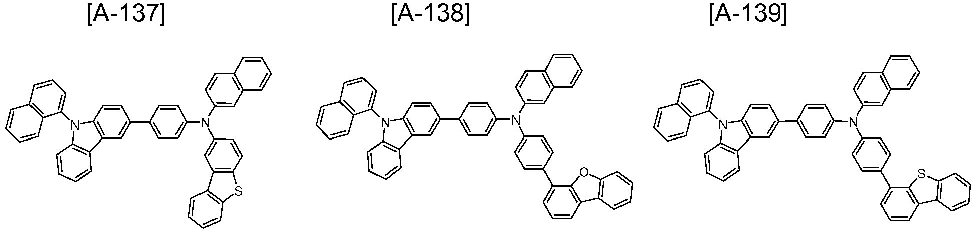

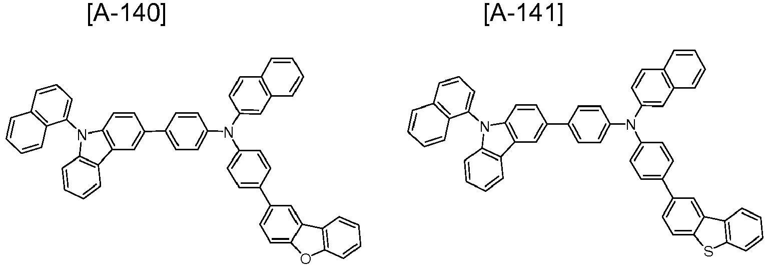

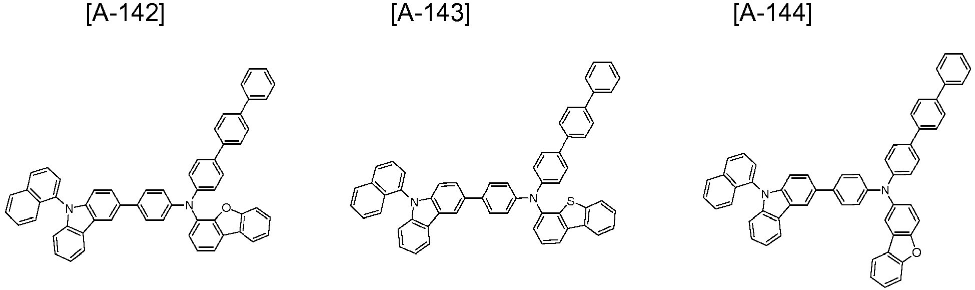

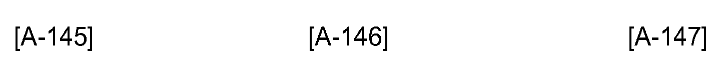

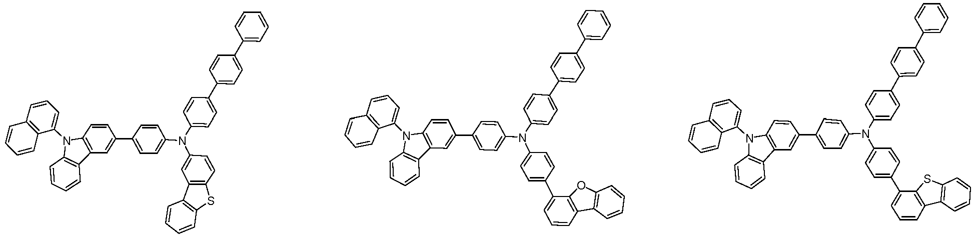

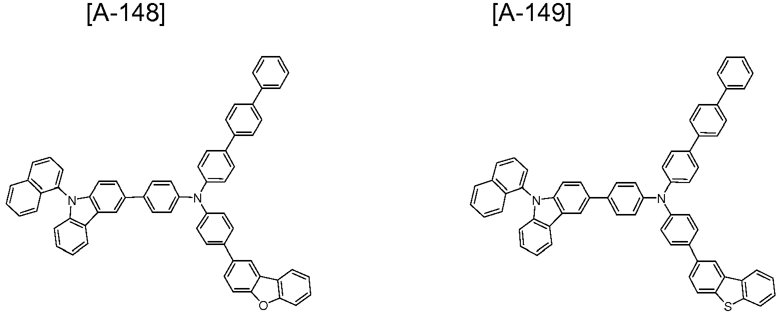

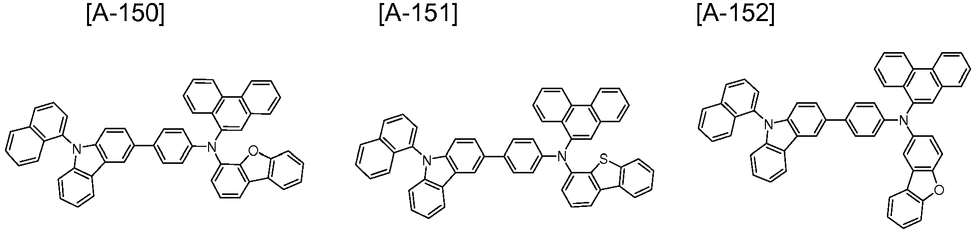

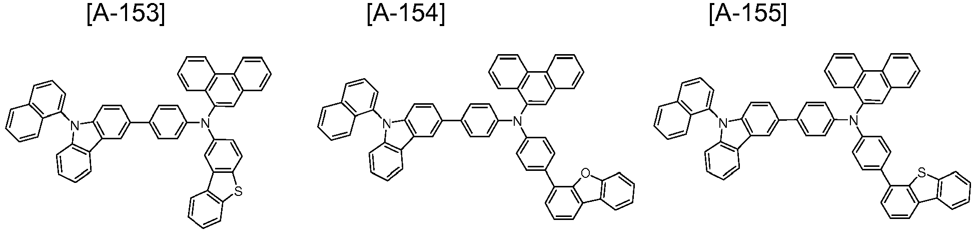

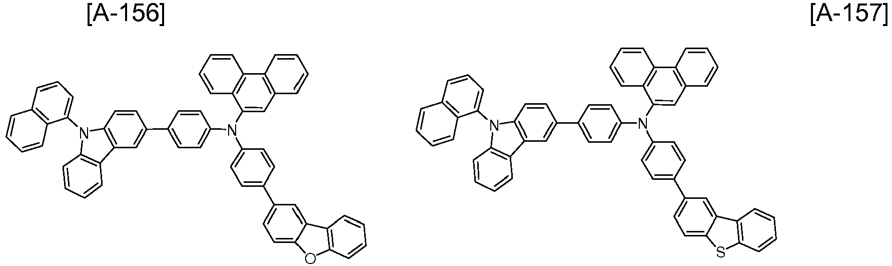

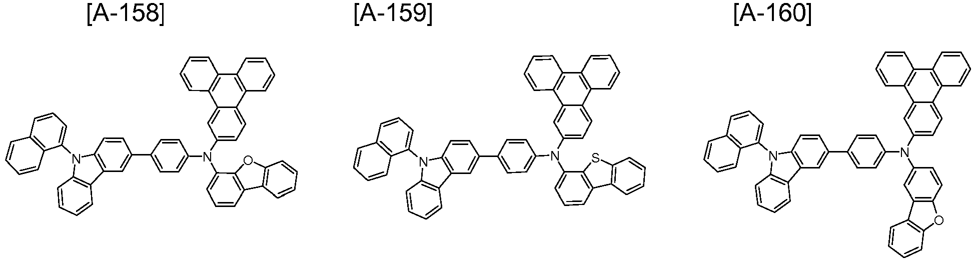

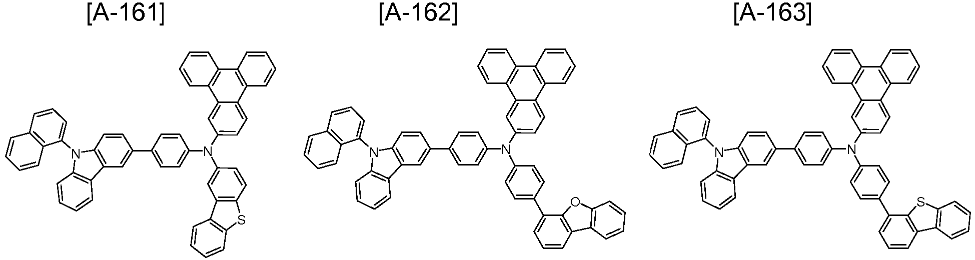

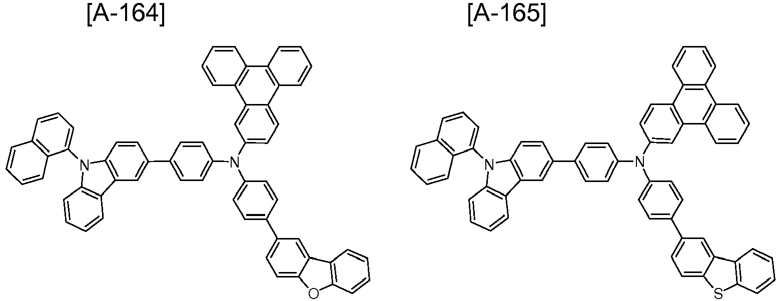

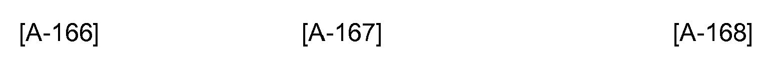

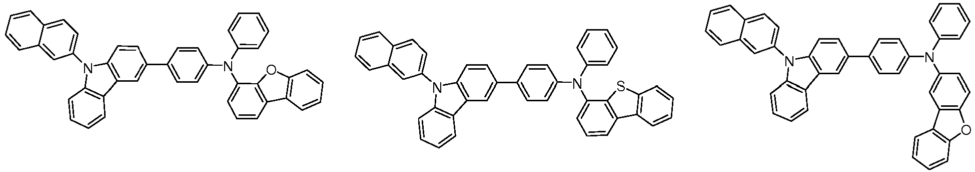

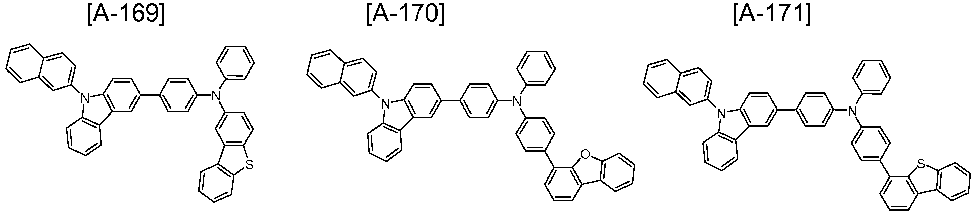

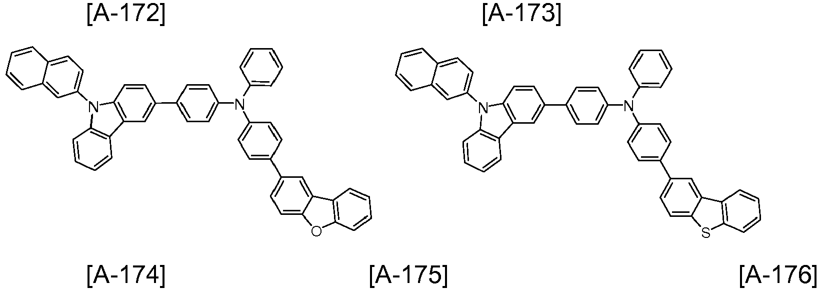

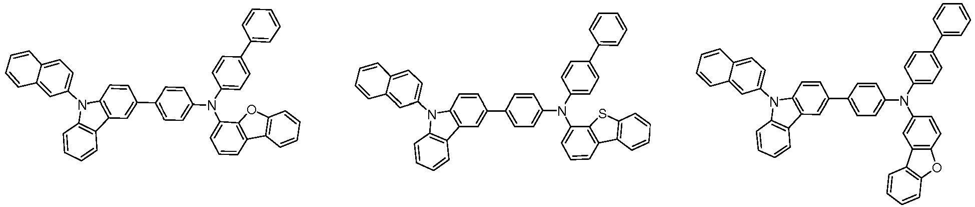

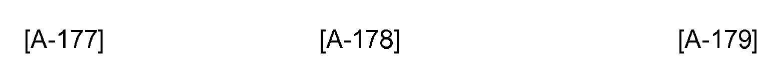

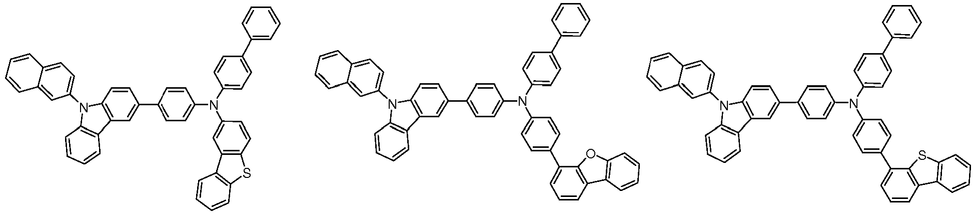

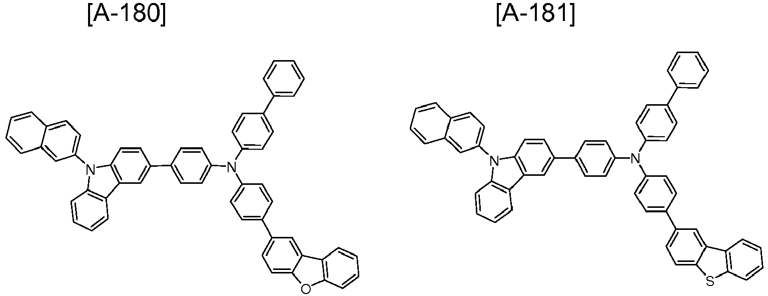

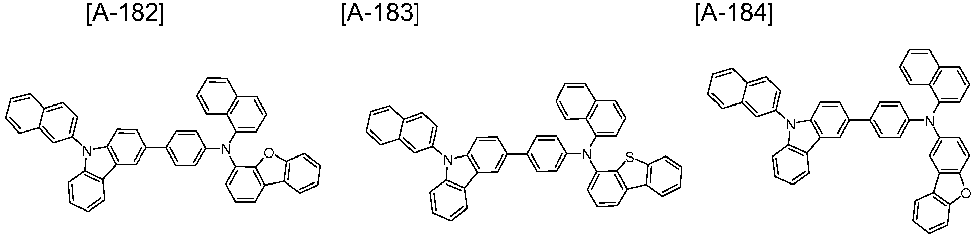

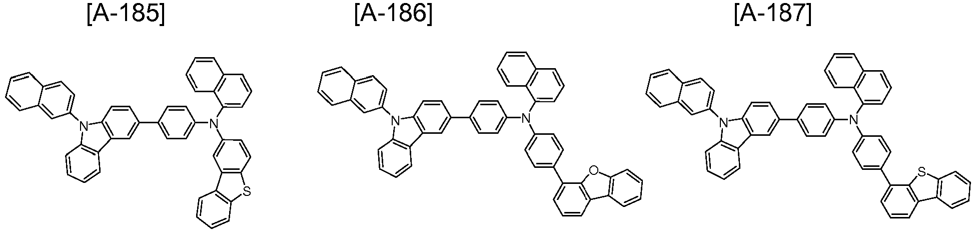

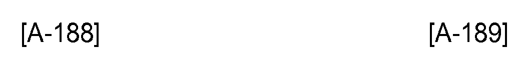

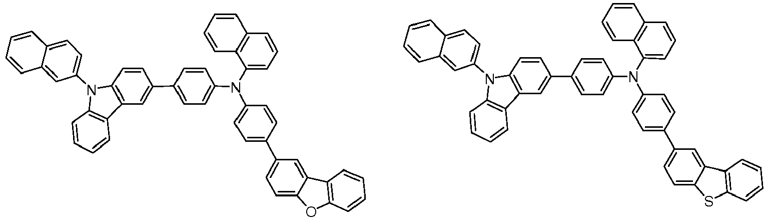

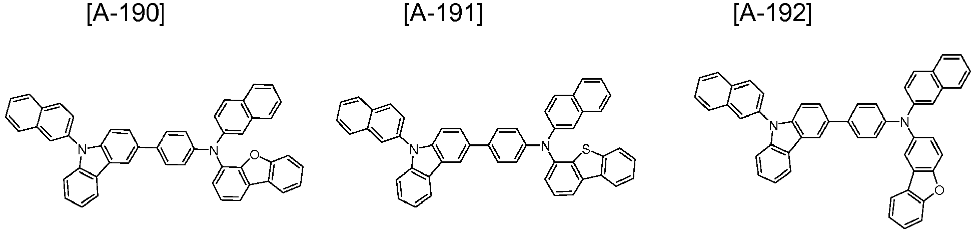

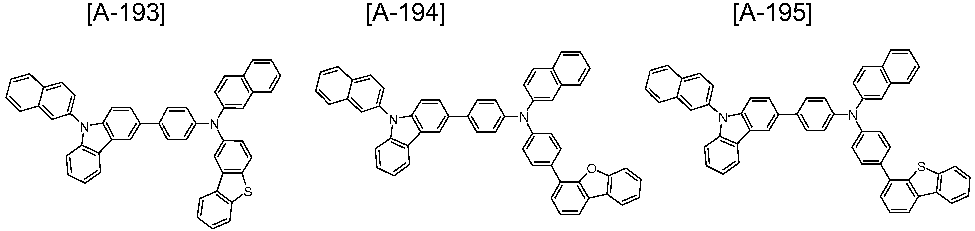

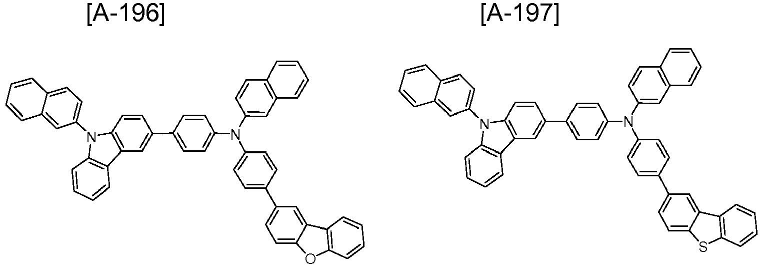

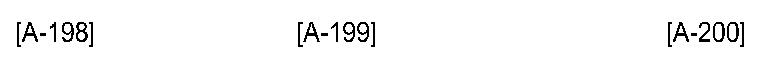

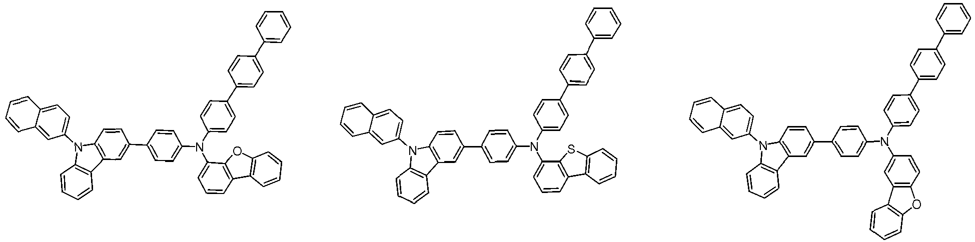

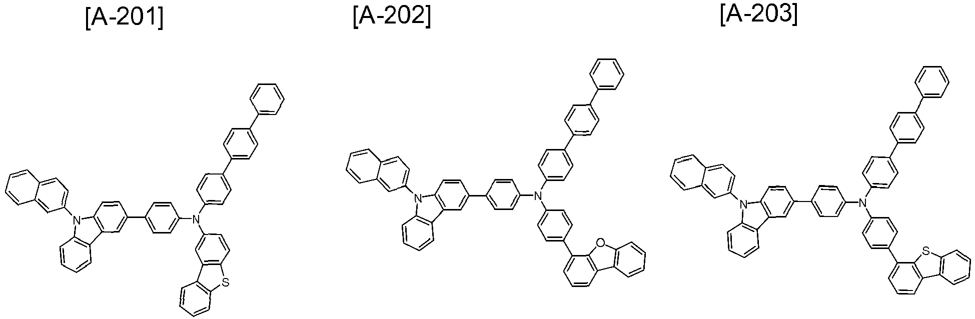

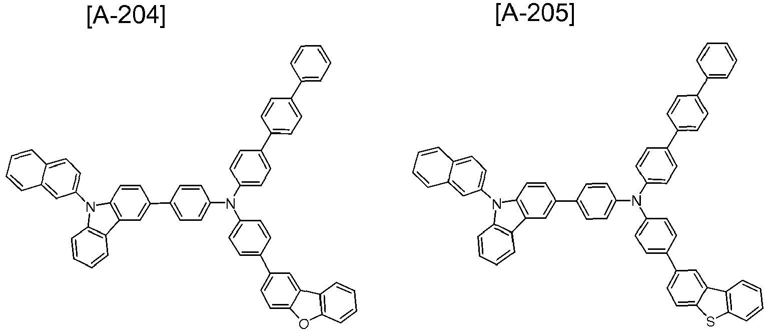

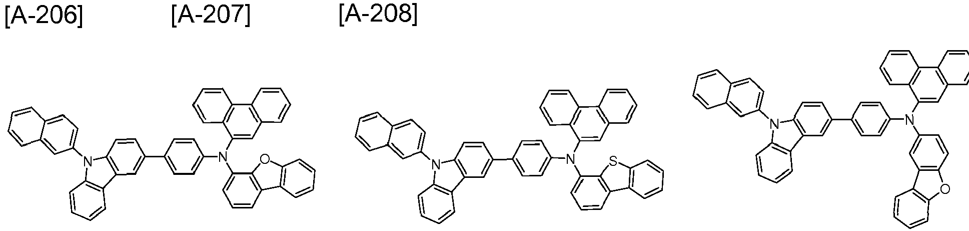

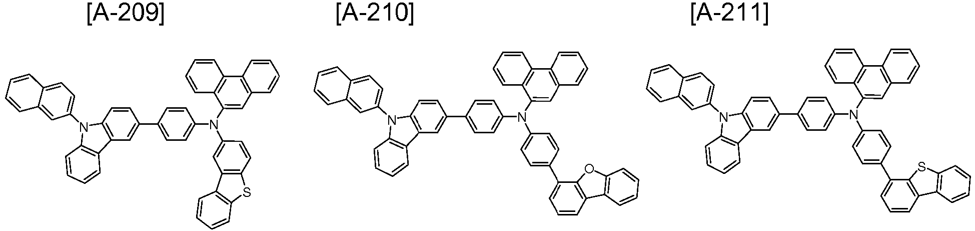

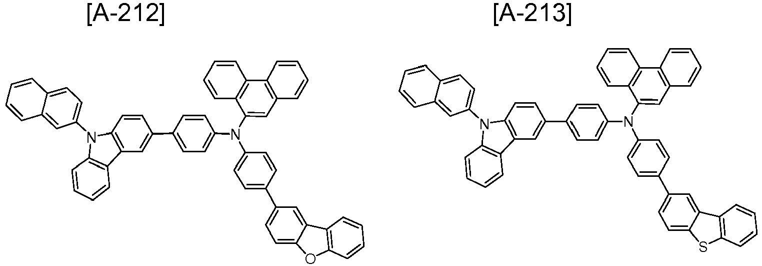

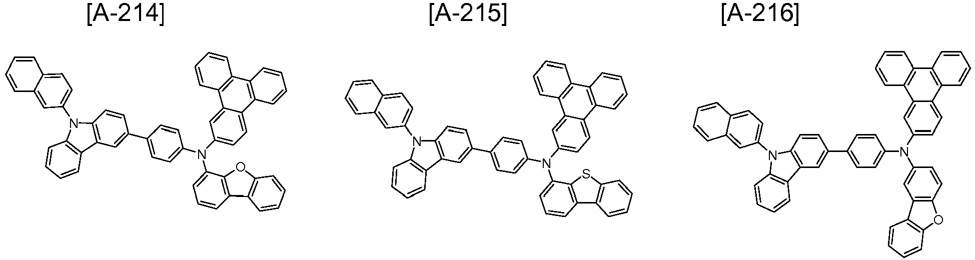

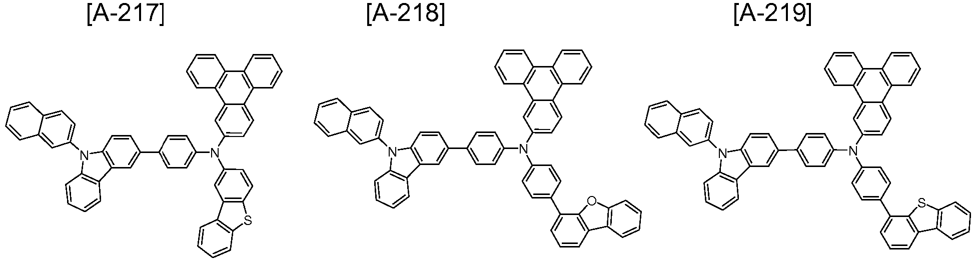

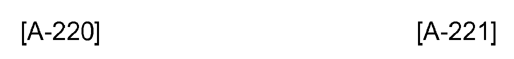

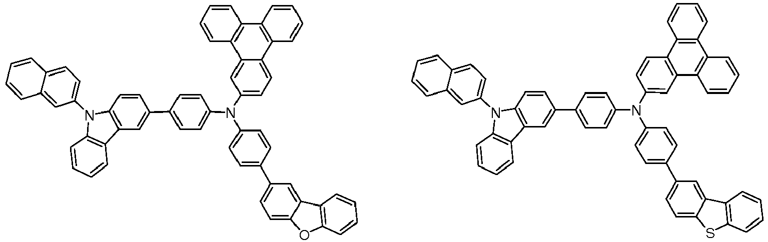

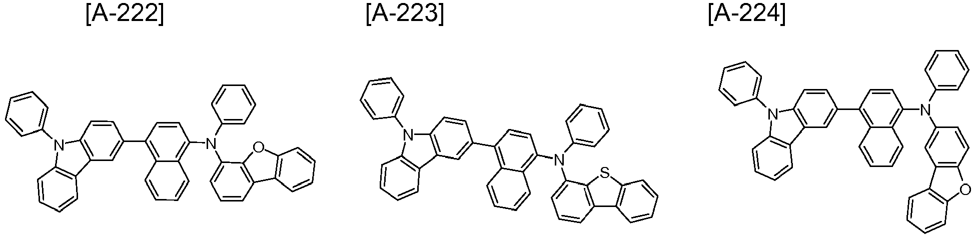

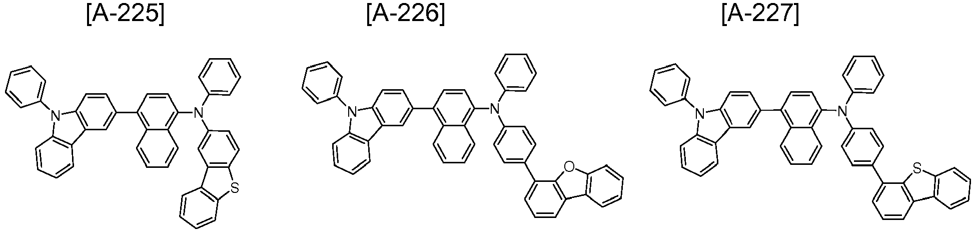

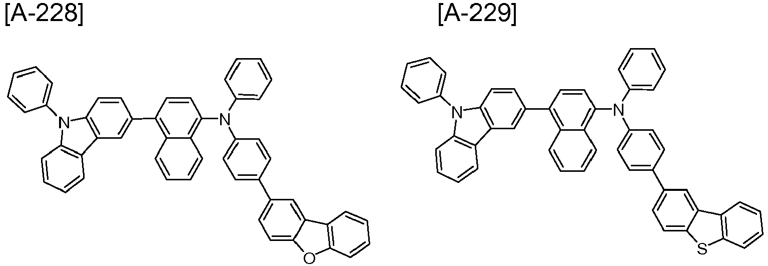

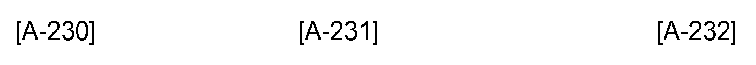

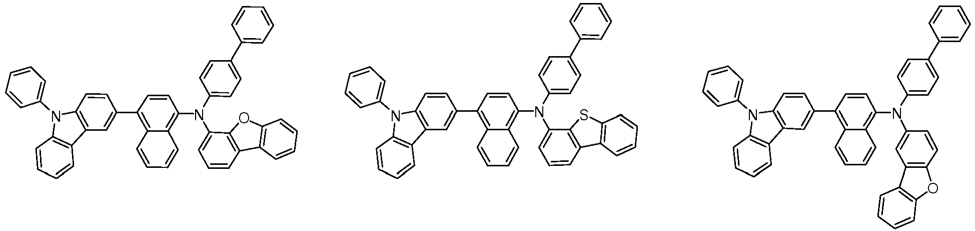

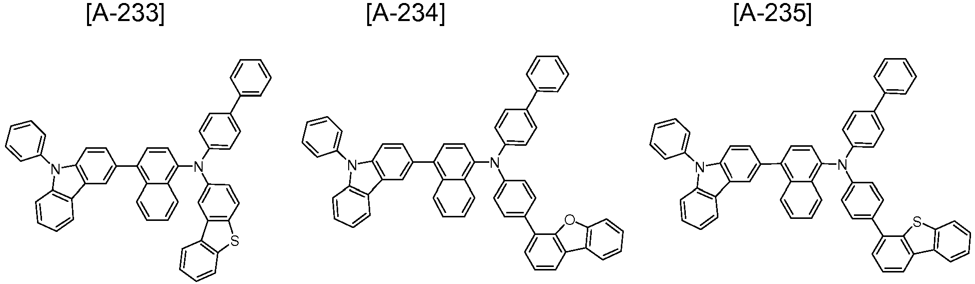

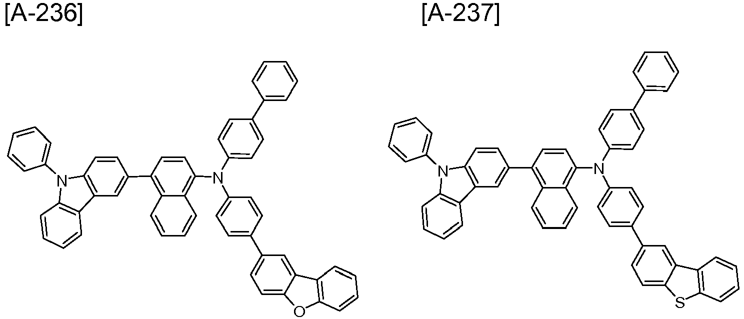

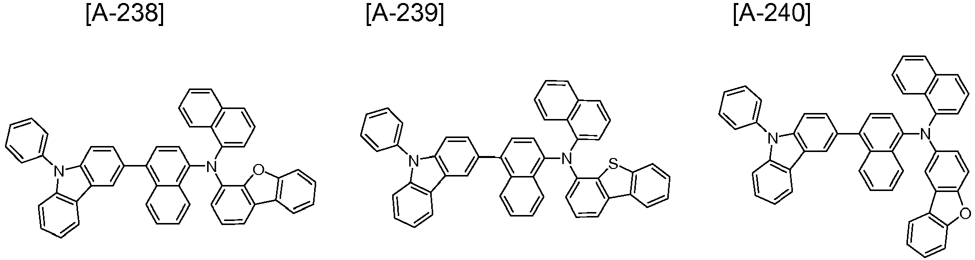

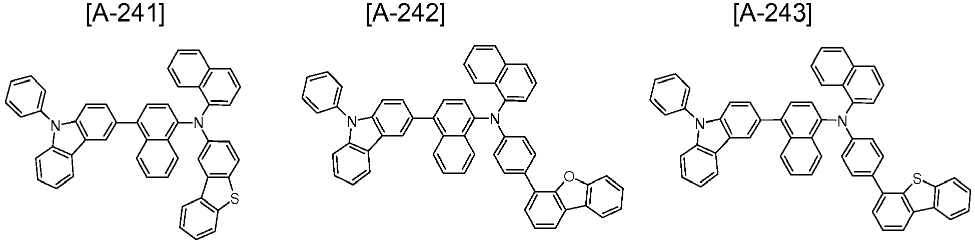

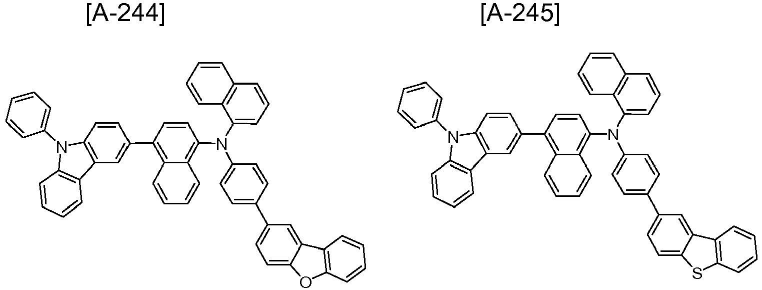

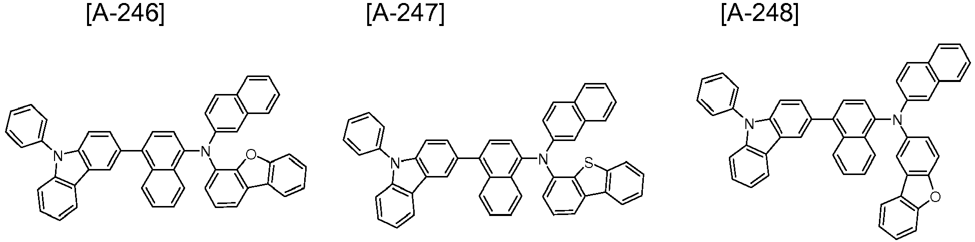

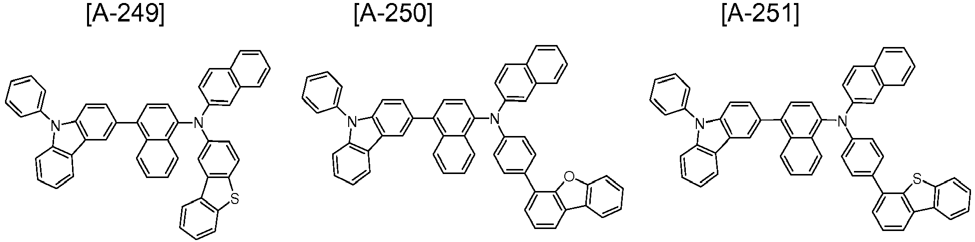

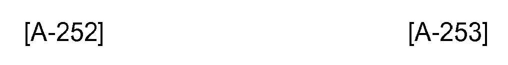

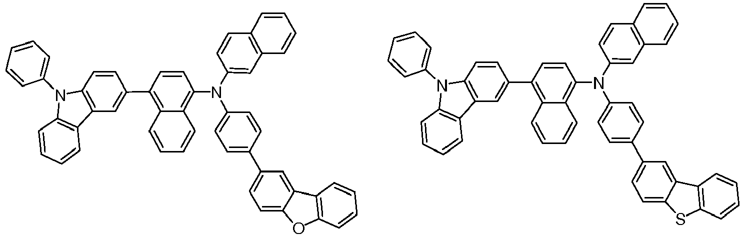

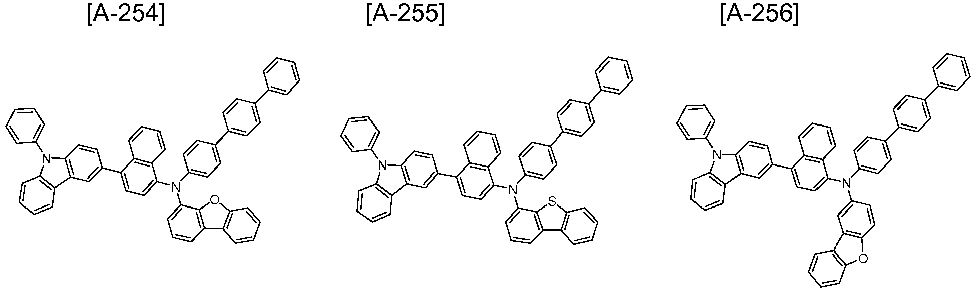

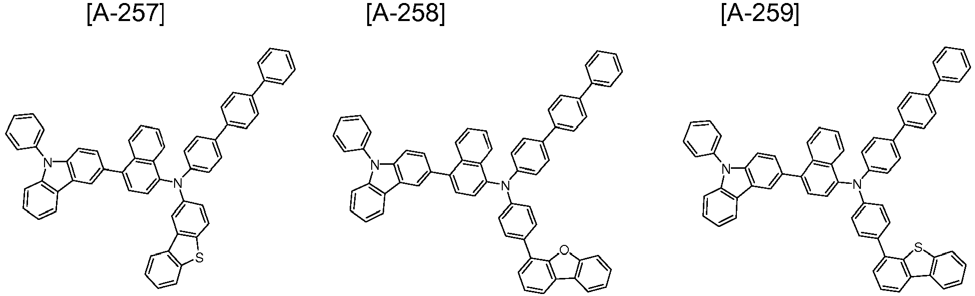

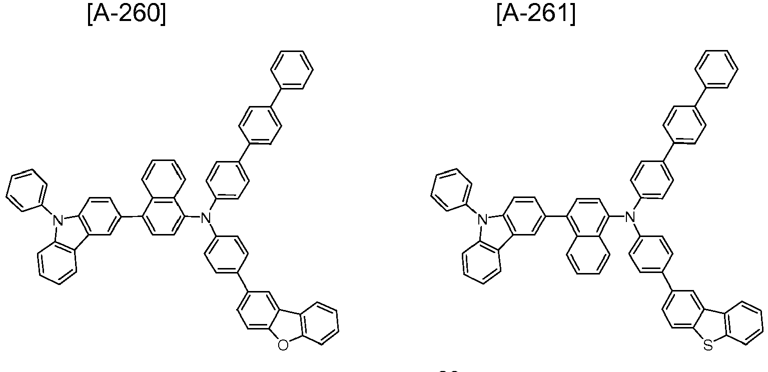

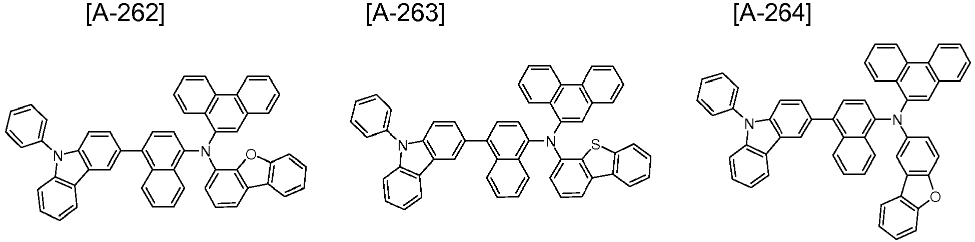

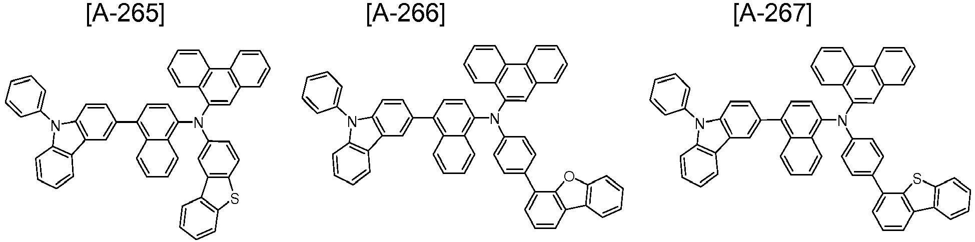

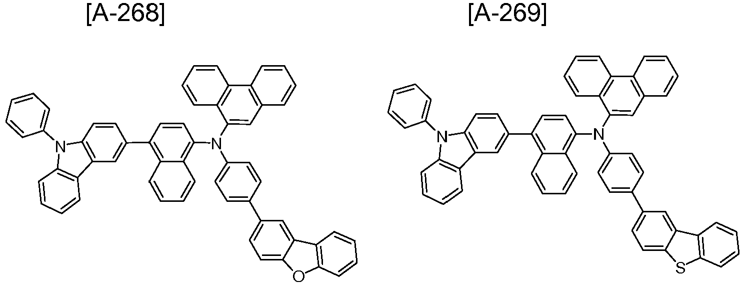

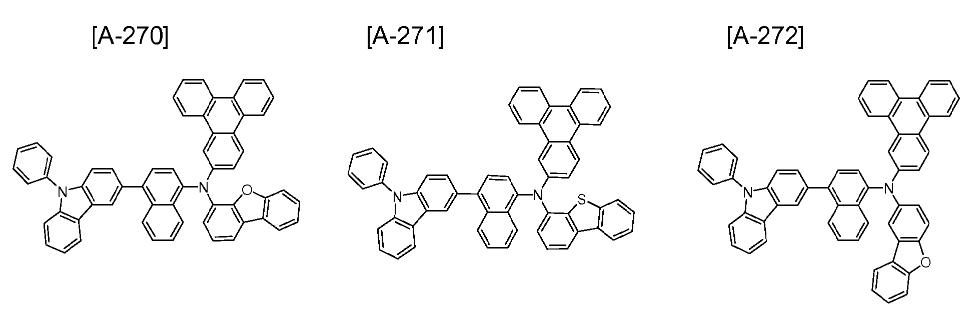

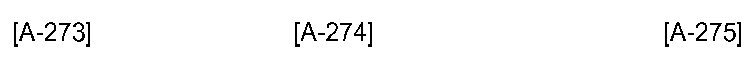

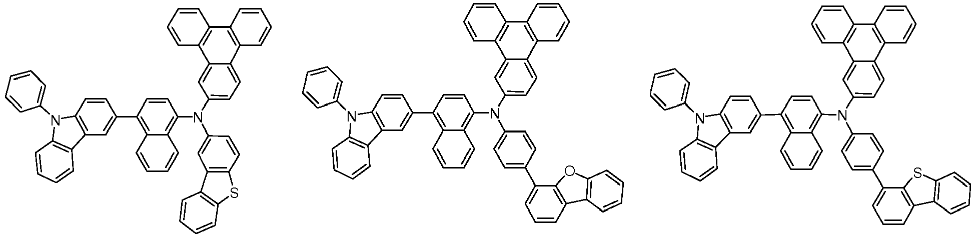

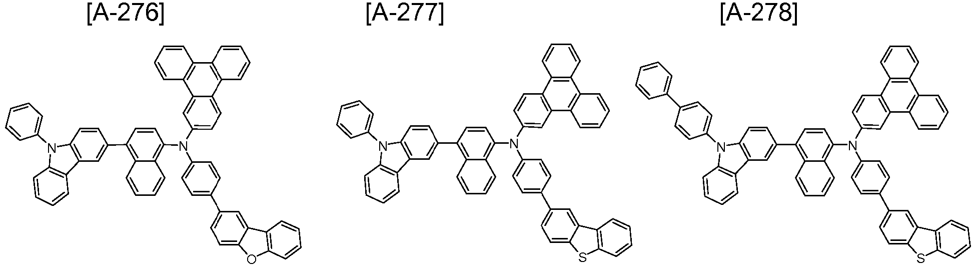

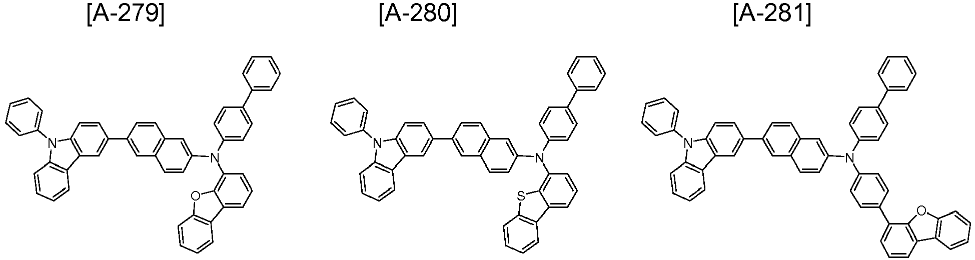

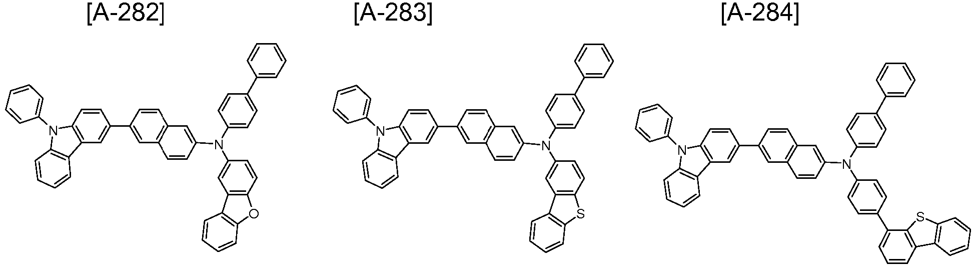

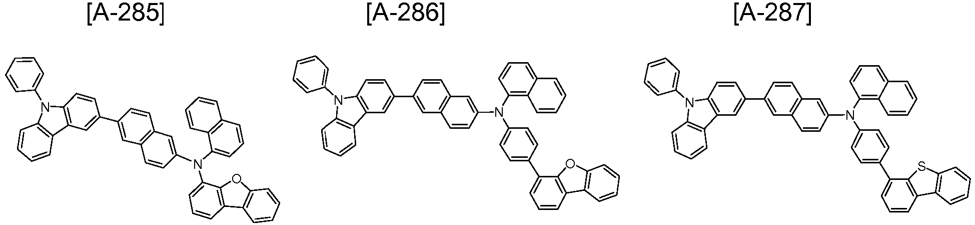

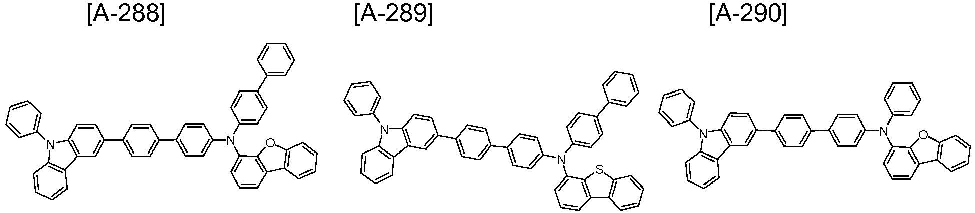

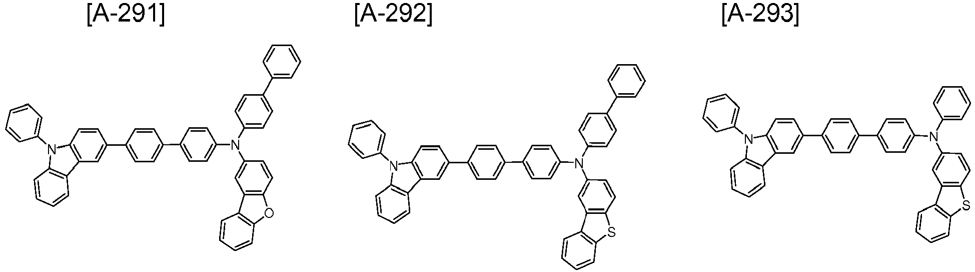

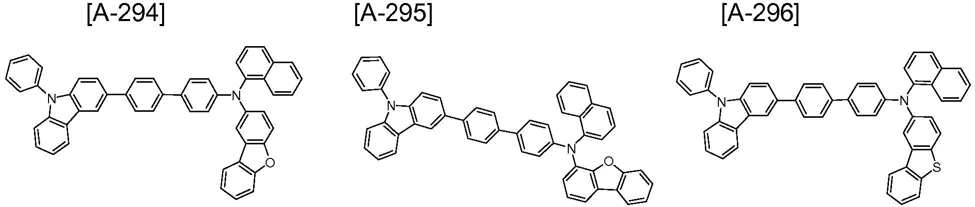

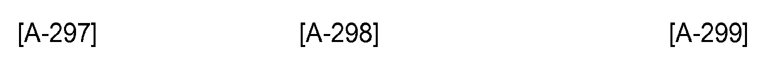

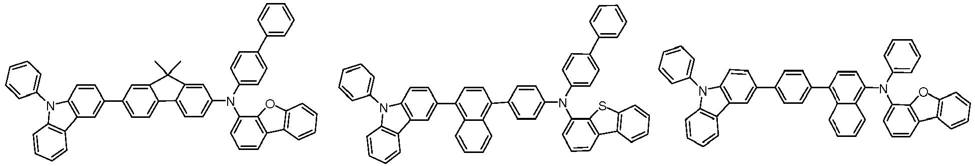

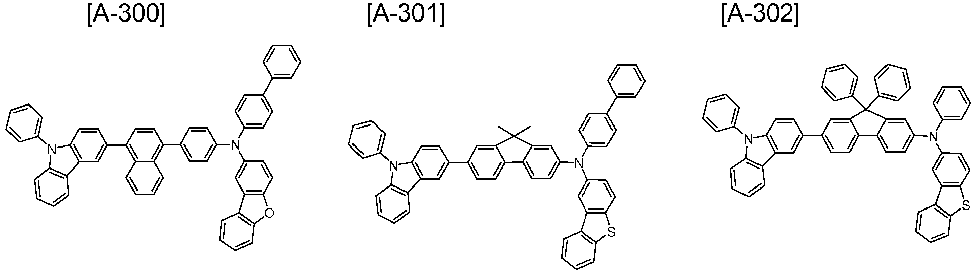

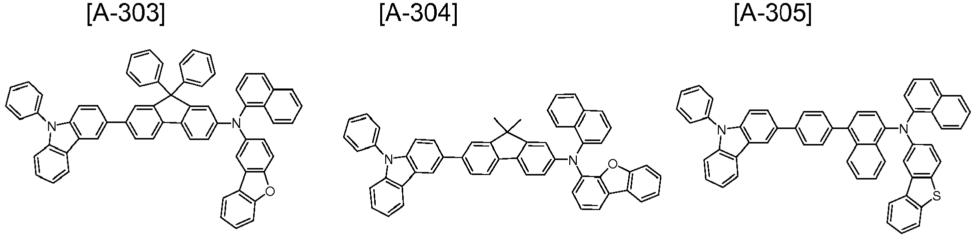

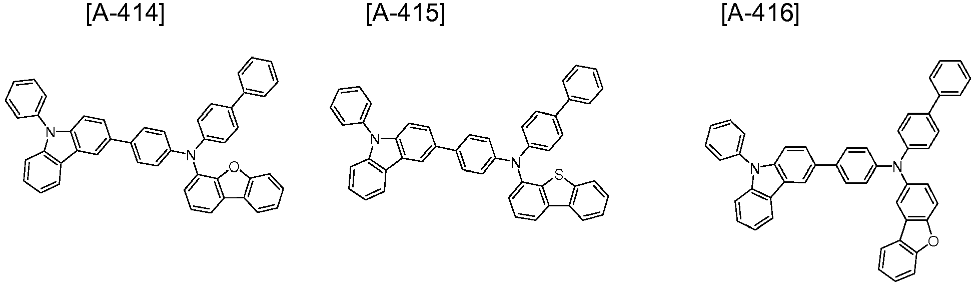

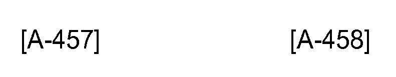

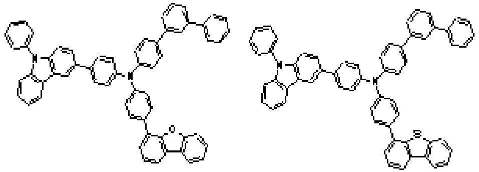

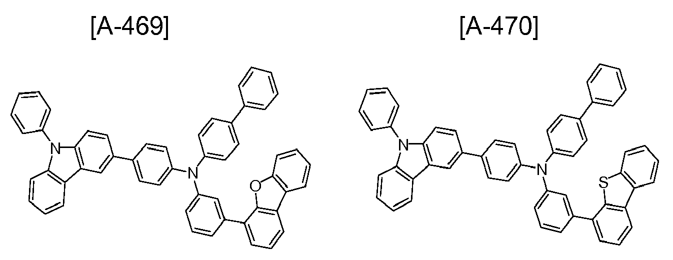

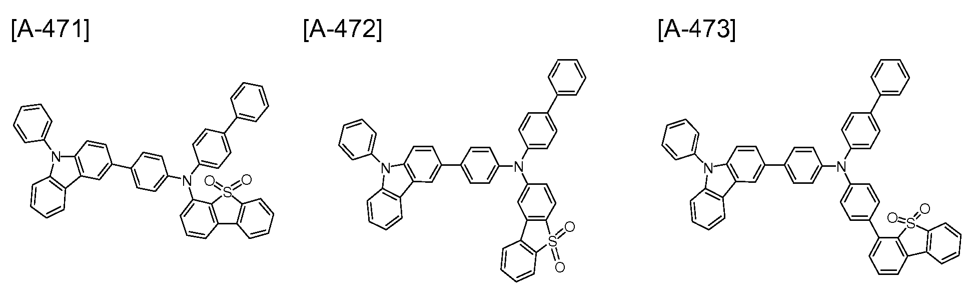

The compound for an optoelectronic device may include at least one of the compounds represented by the following Chemical Formulae A-417 to A-456 or A-459 to A-468.

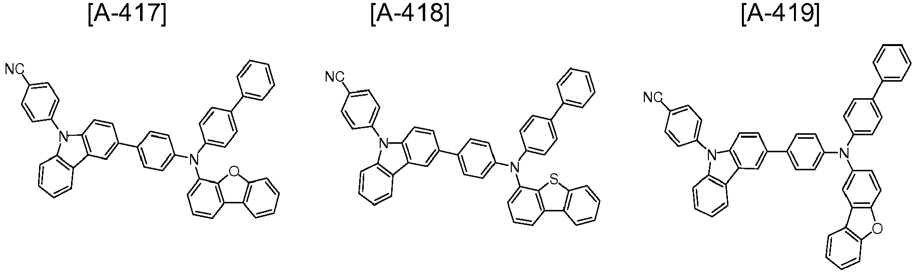

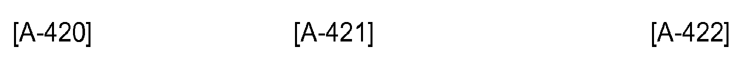

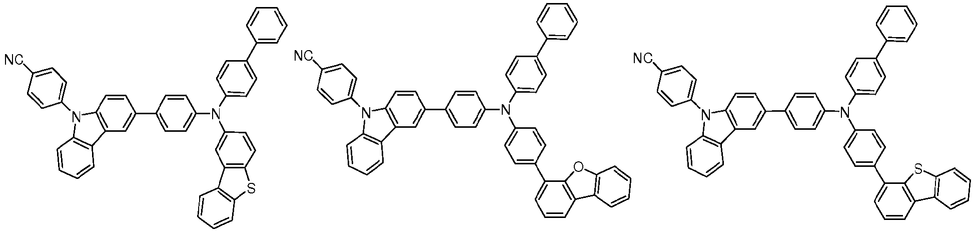

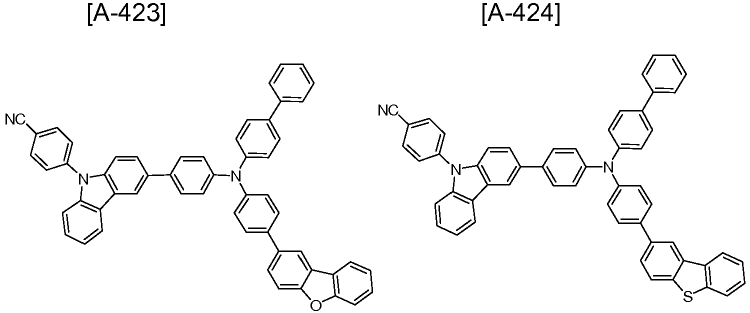

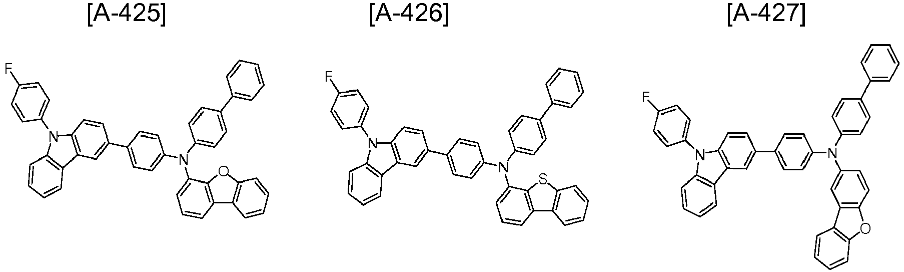

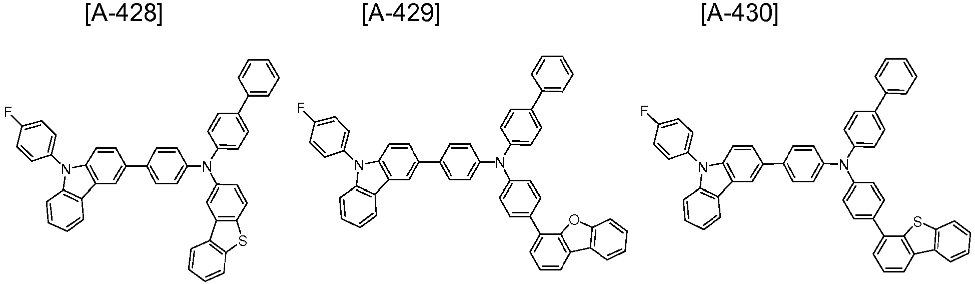

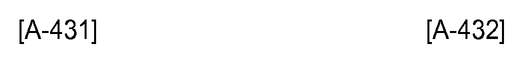

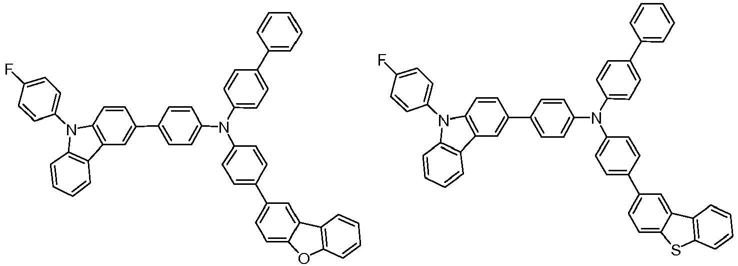

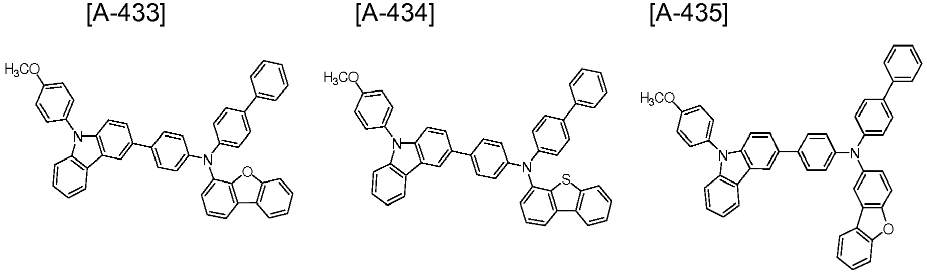

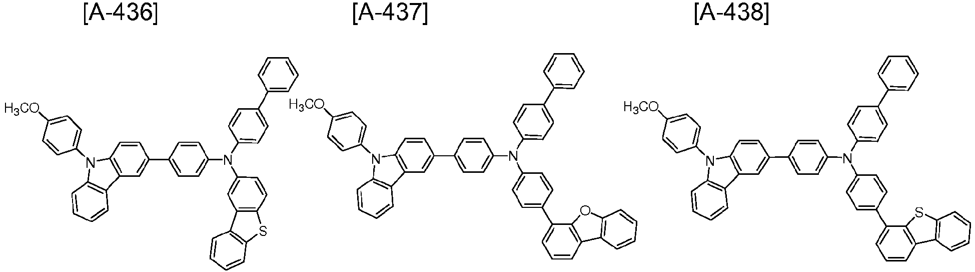

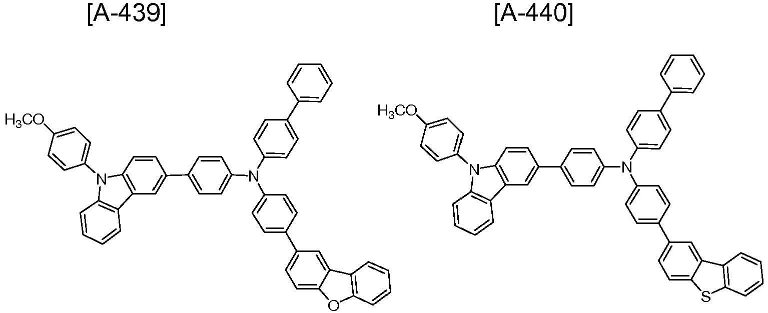

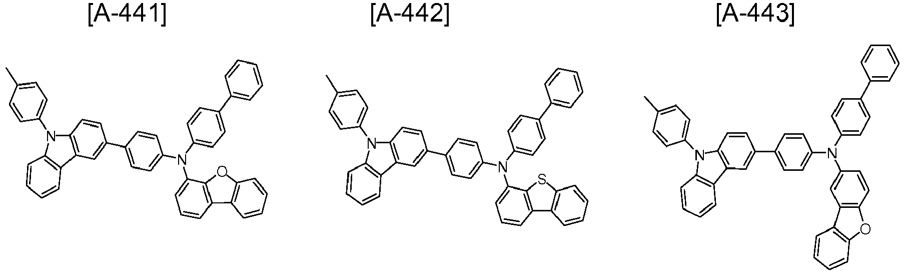

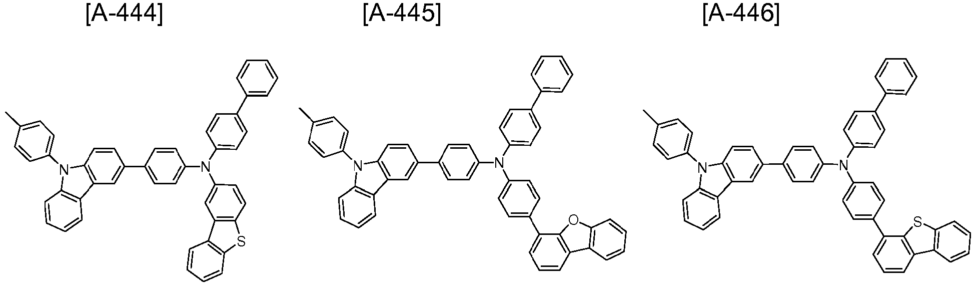

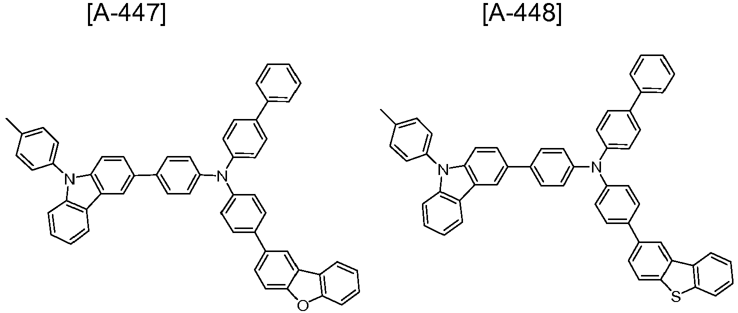

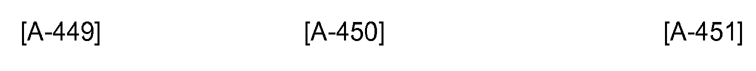

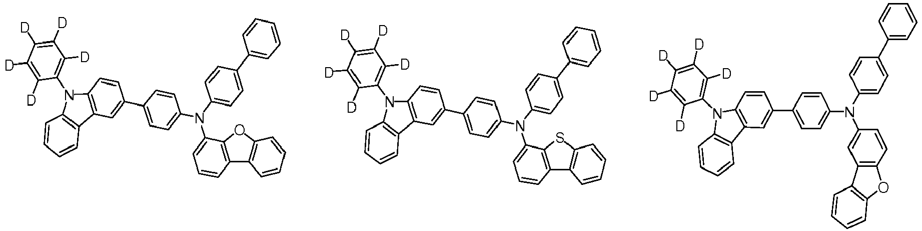

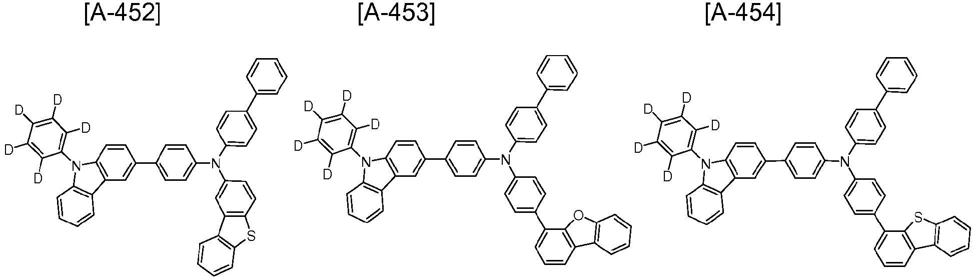

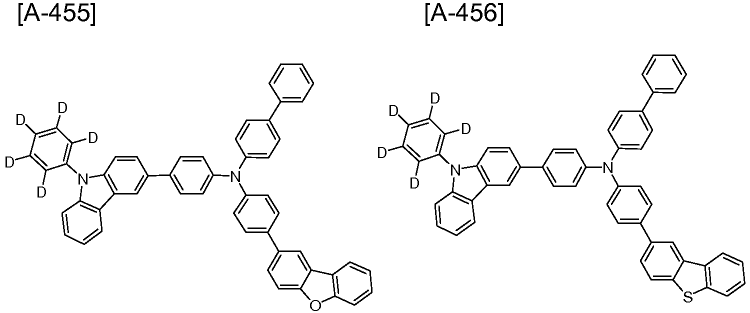

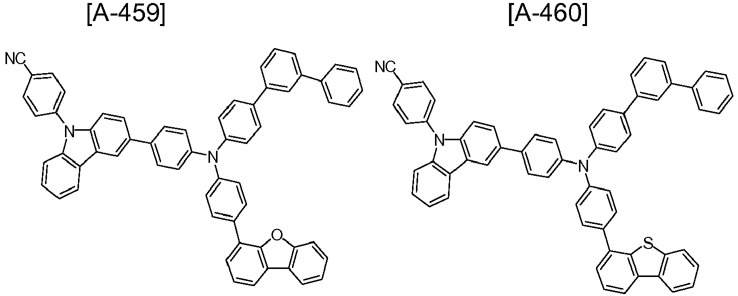

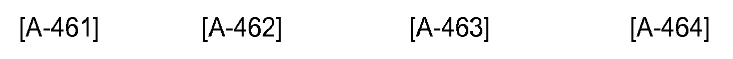

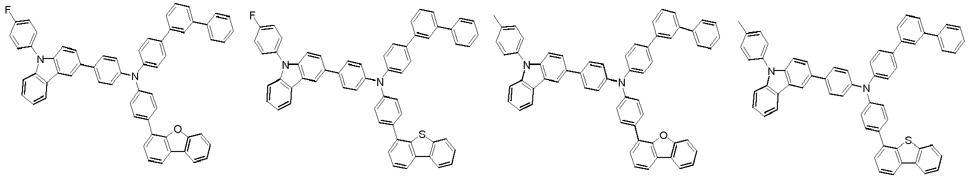

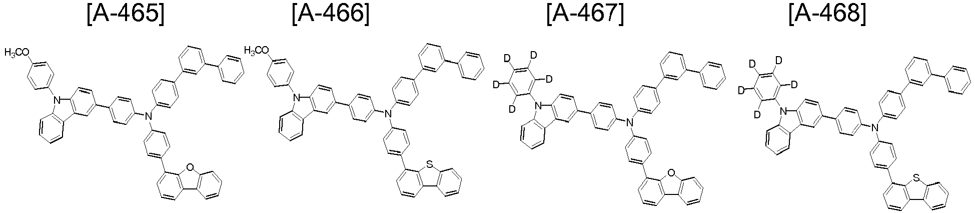

The compound for an optoelectronic device may include at least one of the compounds represented by the following Chemical Formulae A-324 to A-395.

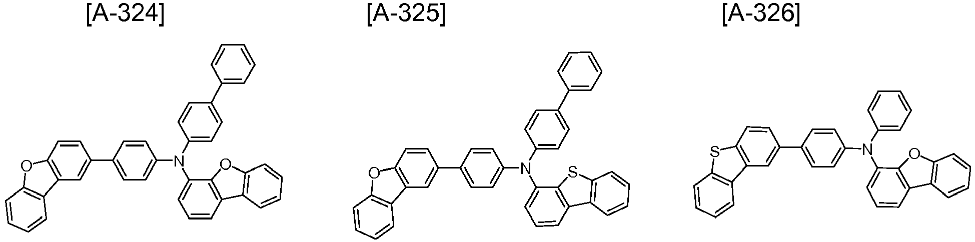

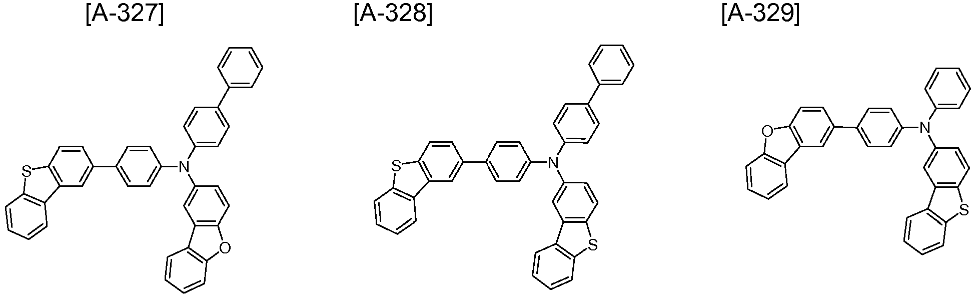

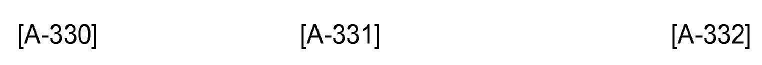

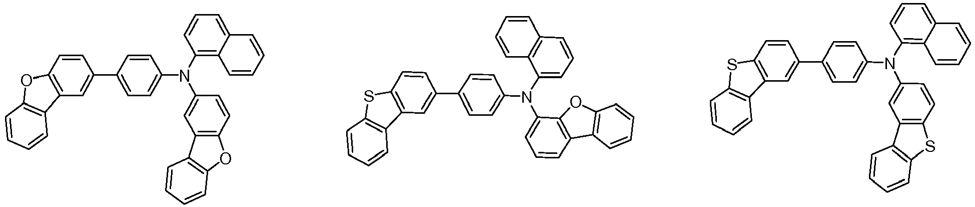

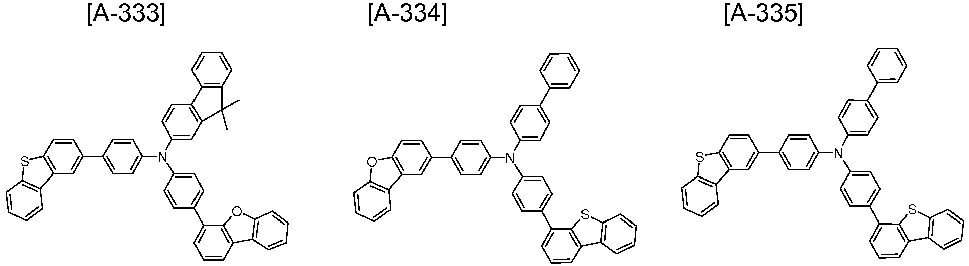

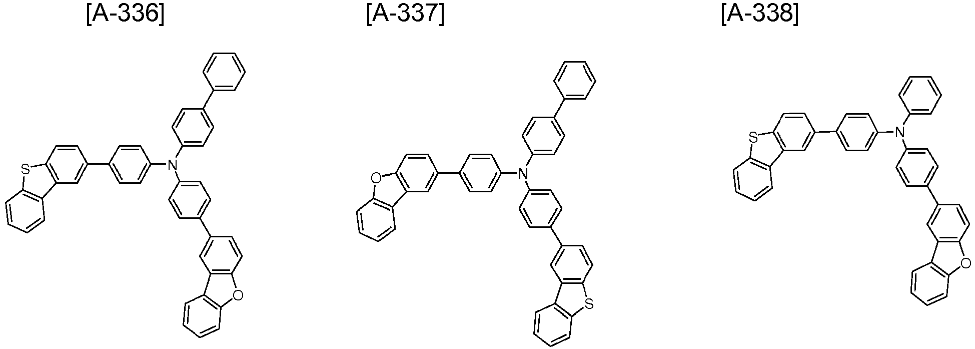

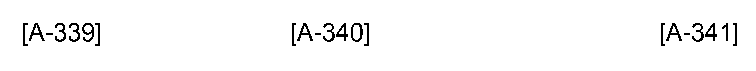

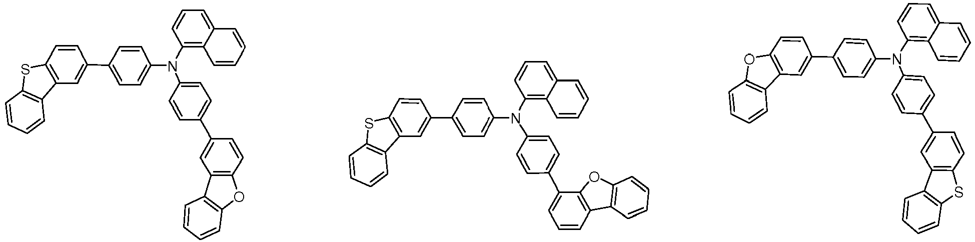

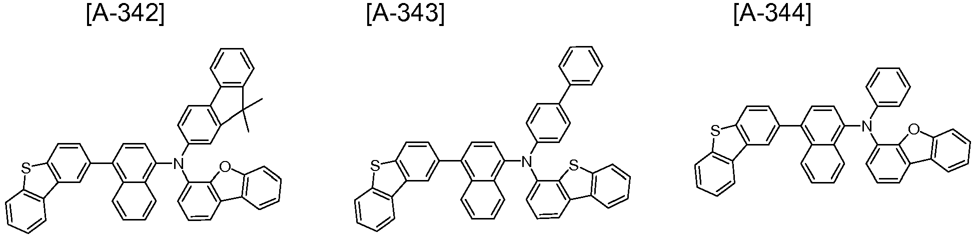

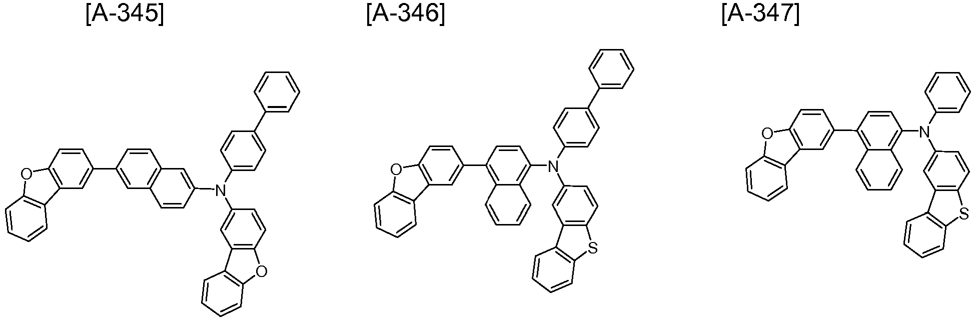

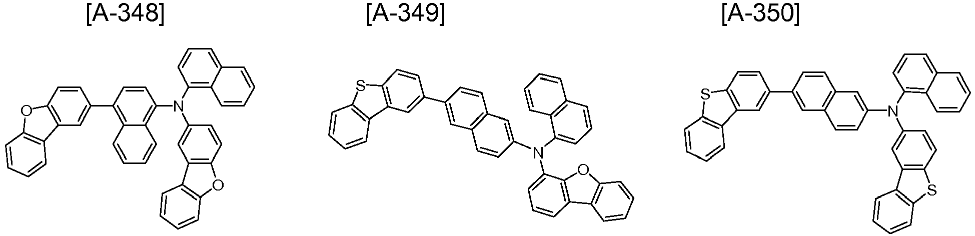

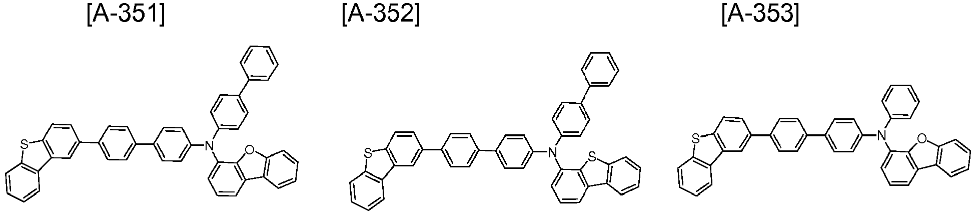

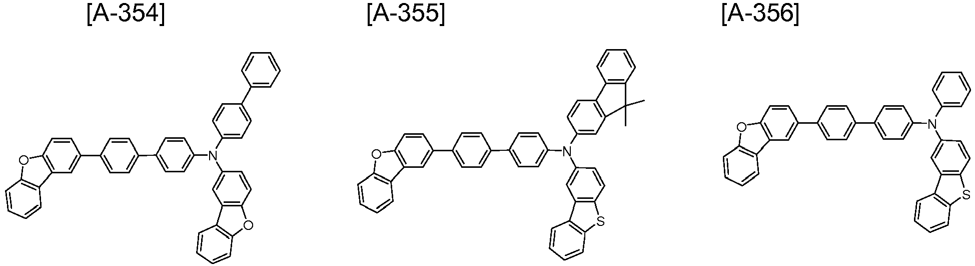

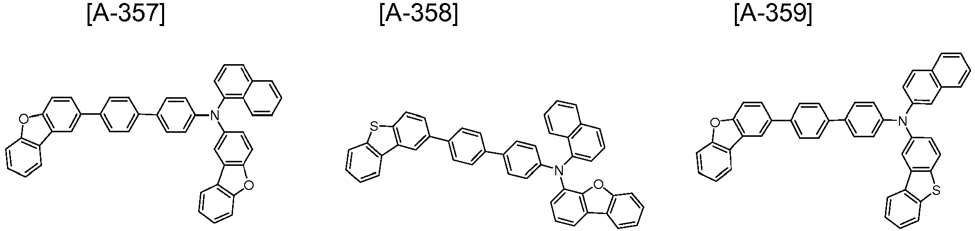

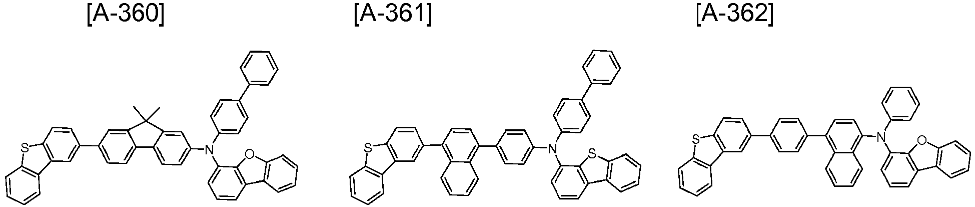

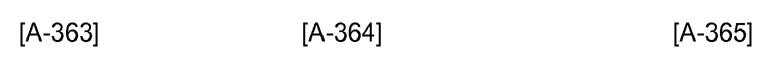

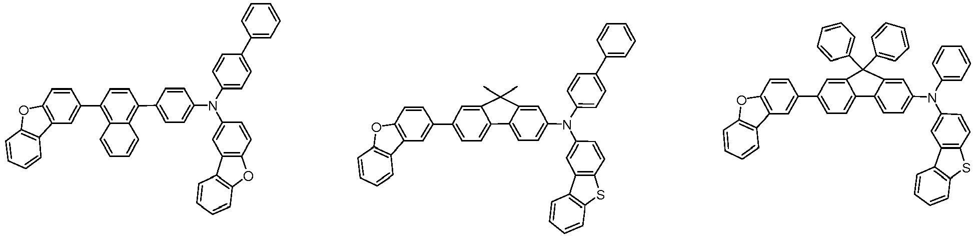

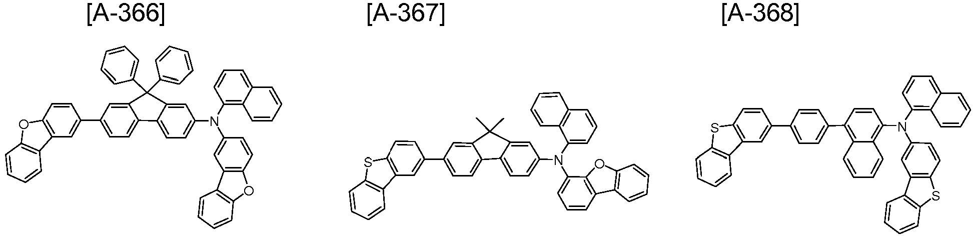

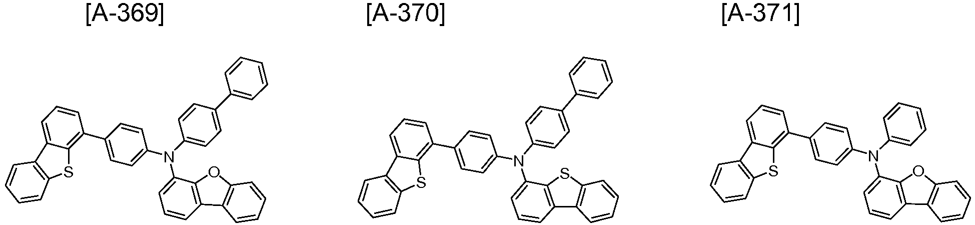

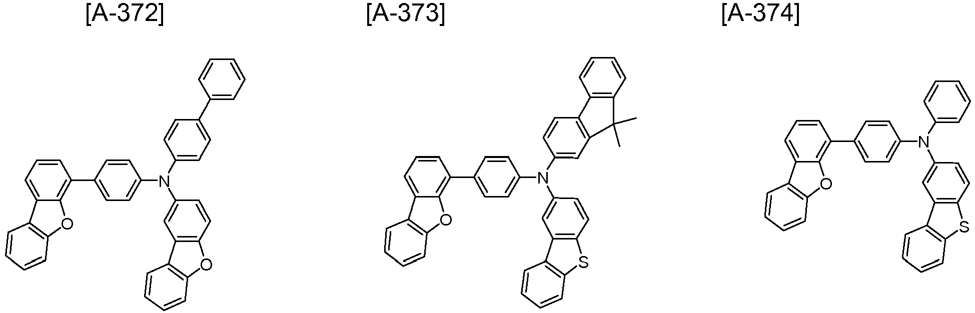

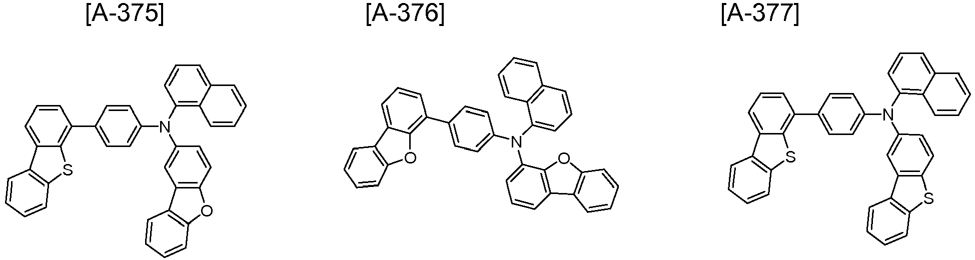

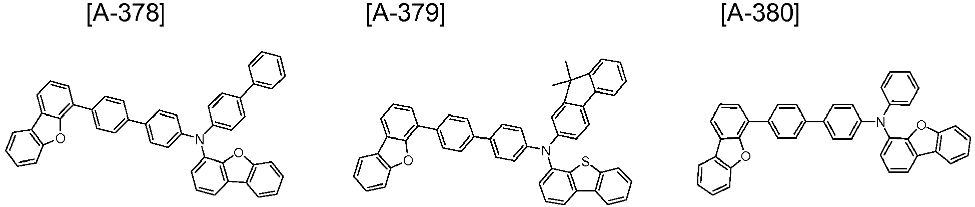

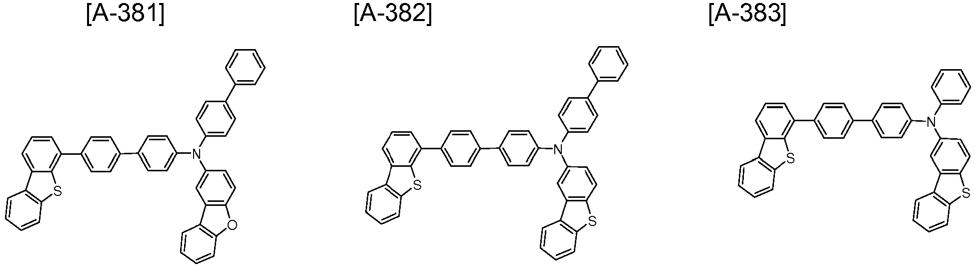

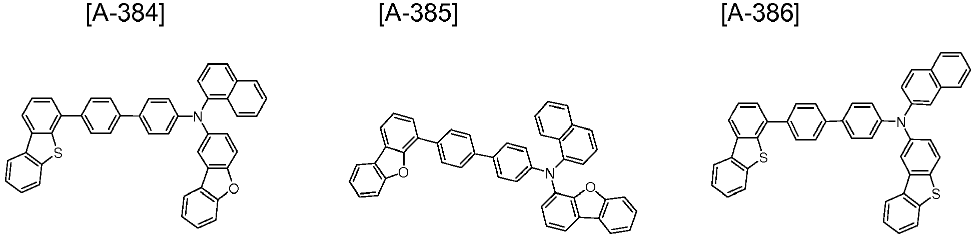

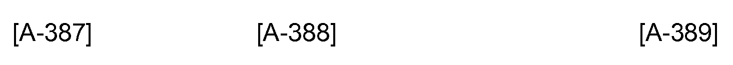

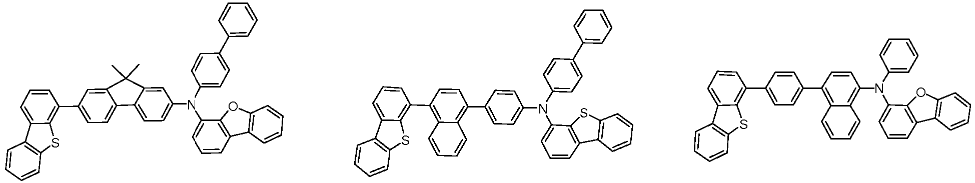

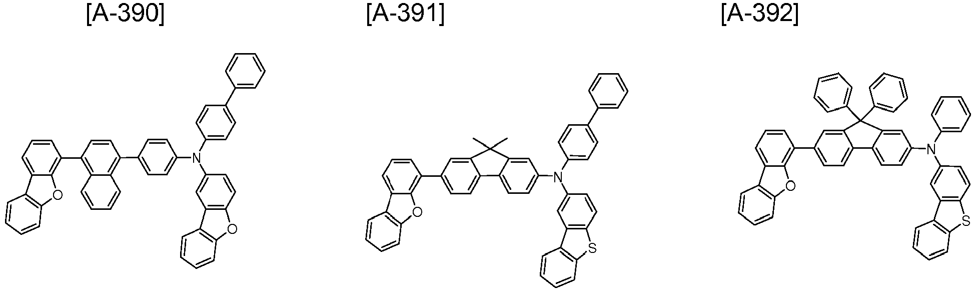

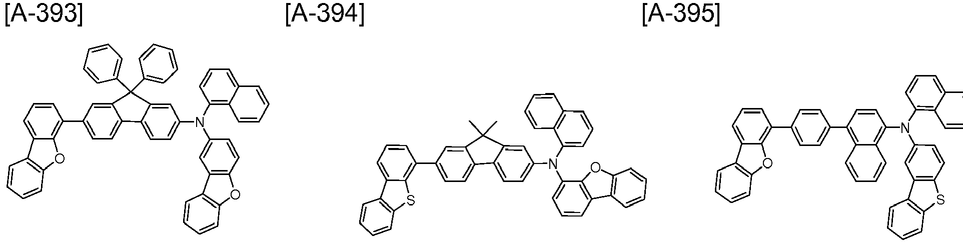

The compound for an optoelectronic device may include at least one of the compounds represented by the following Chemical Formulae A-306 to A-323.

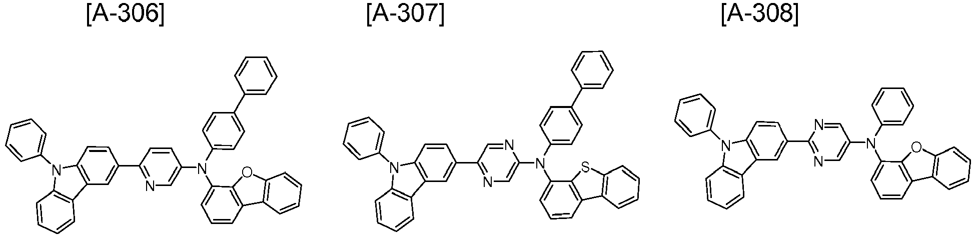

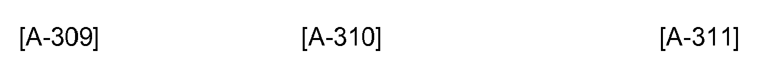

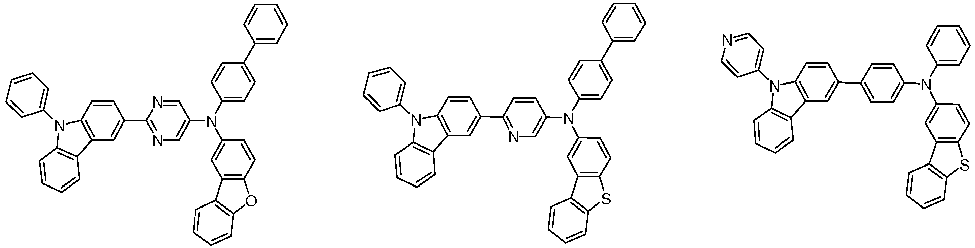

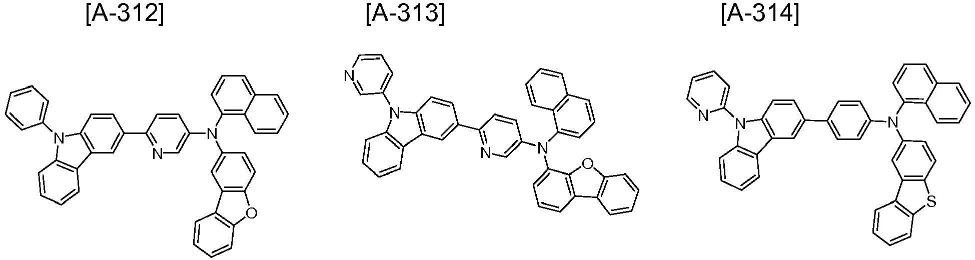

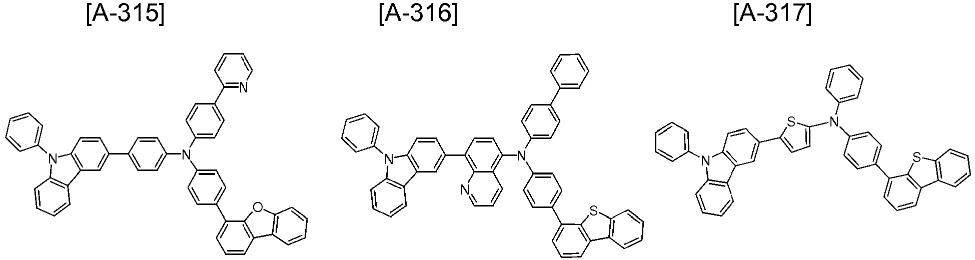

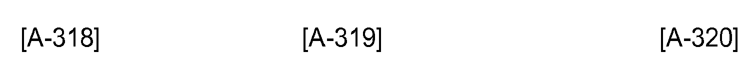

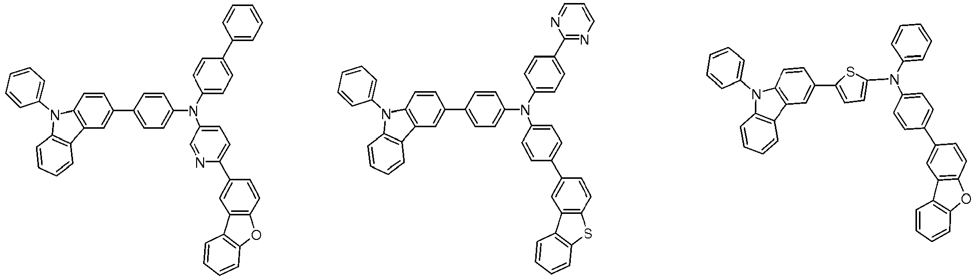

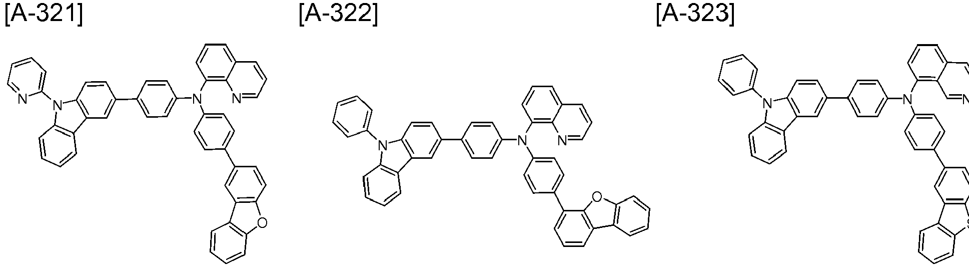

The compound for an optoelectronic device may include at least one of the compounds represented by the following Chemical Formulae A-396 to A-413.

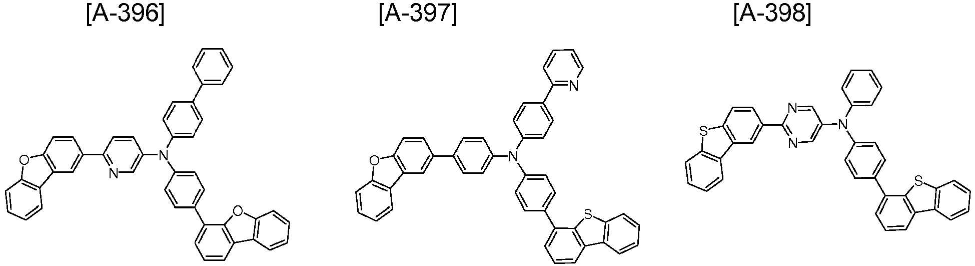

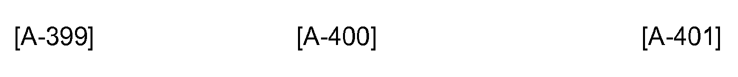

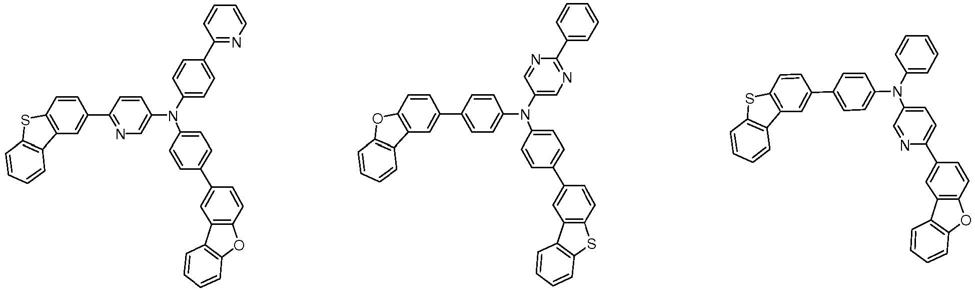

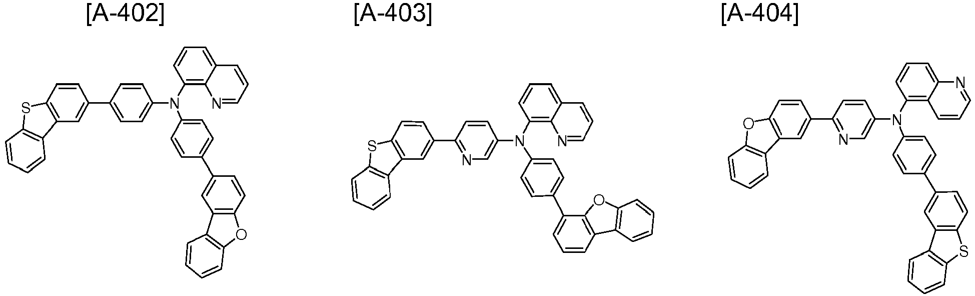

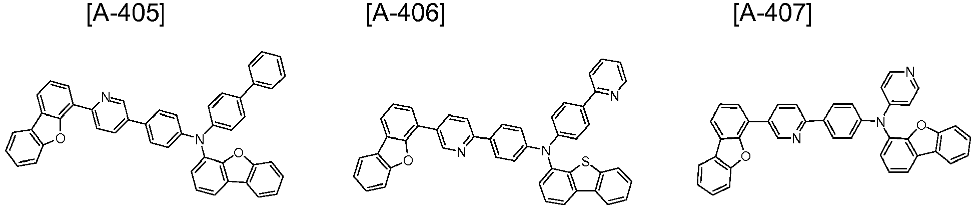

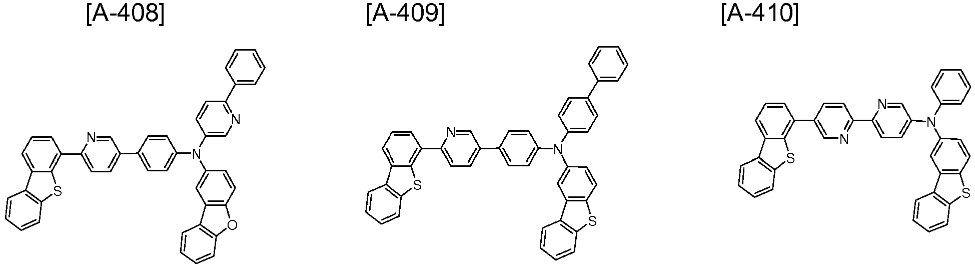

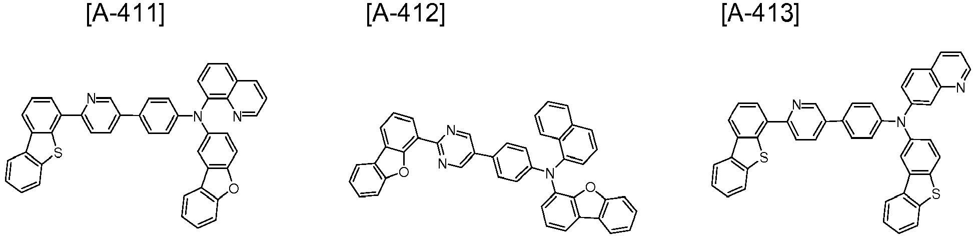

The compound for an optoelectronic device may be used as a hole transport material or a hole injection material for an organic light emitting diode.

The compound for an optoelectronic device may have triplet exciton energy (T1) of about 2.0eV or higher.

The optoelectronic device comprises an organic photoelectronic device, an organic light emitting diode, an organic solar cell, an organic transistor, an organic photo-conductor drum, an organic memory device, or the like.

According to another embodiment of the present invention, an organic light emitting device including an anode, a cathode, and at least one or more organic thin layers between the anode and the cathode is provided. At least one of the organic thin layers may include the compound for an optoelectronic device of the present invention.

The organic thin layer may include an emission layer, a hole transport layer (HTL), a hole injection layer (HIL), an electron transport layer (ETL), an electron injection layer (EIL), a hole blocking layer, or a combination thereof.

The compound for an optoelectronic device may be included in a hole transport layer (HTL), a hole injection layer (HIL), an electron transport layer (ETL), or an electron injection layer (EIL).

The compound for an optoelectronic device may be included in an emission layer.

The compound for an optoelectronic device may be used as a phosphorescent or fluorescent host material in an emission layer.

The compound for an optoelectronic device may be used as a fluorescent blue dopant material in an emission layer.

According to another embodiment of the present invention, a display device including the organic light emitting element is provided.

The compound for an optoelectronic device may include an excellent hole or electron transporting property, high film stability, good thermal stability, and good triplet exciton energy.

The compound may be used as a hole injection/ transport material of an emission layer, a host material, or an electron injection/ transport material. The organic photoelectric device may have excellent electrochemical and thermal stability, and therefore may have an excellent life-span characteristic and high luminous efficiency at a low driving voltage.

### BRIEF DESCRIPTION OF THE DRAWINGS

FIGS. 1 to 5 are cross-sectional views showing organic light emitting diodes including compounds according to various embodiments of the present invention.
FIG. 6 shows a ¹H-NMR spectrum of the A-414 compound according to Example 1.
FIG. 7 shows a ¹H-NMR spectrum of the A-415 compound according to Example 2.
FIG. 8 shows a ¹H-NMR spectrum of the A-9 compound according to Example 3.
FIG. 9 shows a ¹H-NMR spectrum of the A-10 compound according to Example 4.
FIG. 10 shows a ¹H-NMR spectrum of the A-11 compound according to Example 5.
FIG. 11 shows a ¹H-NMR spectrum of the A-18 compound according to Example 6.
FIG. 12 shows a ¹H-NMR spectrum of the A-19 compound according to Example 7.
FIG. 13 shows a ¹H-NMR spectrum of the A-469 compound according to Example 13.
FIG. 14 shows a ¹H-NMR spectrum of the A-470 compound according to Example 28.
FIG. shows a ¹H-NMR spectrum of the A-457 compound according to Example 29.
FIG. 16 shows a ¹H-NMR spectrum of the A-416 compound according to Example 37.
FIG. 17 shows a ¹H-NMR spectrum of the A-12 compound according to Example 38.
FIG. 18 shows a ¹H-NMR spectrum of the A-13 compound according to Example 39.
FIG. 19 is a graph showing photoluminescence (PL) of the A-9, A-10, and A-11 compounds according to Examples 3 to 5.

### DETAILED DESCRIPTION OF THE EMBODIMENTS

Exemplary embodiments of the present invention will hereinafter be described in detail. However, these embodiments are only exemplary, and the present invention is not limited thereto but rather is defined by the scope of the appended claims.

As used herein, when specific definition is not otherwise provided, the term "substituted" refers to one substituted with deuterium, a halogen, a hydroxy group, an amino group, a substituted or unsubstituted C1 to C20 amine group, a nitro group, a substituted or unsubstituted C3 to C40 silyl group, a C1 to C30 alkyl group, a C1 to C10 alkylsilyl group, a C3 to C30 cyclo alkyl group, a C6 to C30 aryl group, a C1 to C20 alkoxy group, a fluoro group, a C1 to C10 trifluoro alkyl group such as a trifluoromethyl group, or a cyano group, instead of hydrogen.

As used herein, when specific definition is not otherwise provided, the term "hetero" refers to one including 1 to 3 of N, O, S, or P, and remaining carbons in one ring.

As used herein, when a definition is not otherwise provided, the term "combination thereof" refers to at least two substituents bound to each other by a linker, or at least two substituents condensed to each other.

As used herein, when a definition is not otherwise provided, the term "alkyl" refers to an aliphatic hydrocarbon group. The alkyl may be a saturated alkyl group that does not include any alkene or alkyne. The alkyl may be branched, linear, or cyclic.

As used herein, when a definition is not otherwise provided, the term "alkene" refers to a group in which at least two carbon atoms are bound in at least one carbon-carbon double bond, and the term "alkyne" refers to a group in which at least two carbon atoms are bound in at least one carbon-carbon triple bond.

The alkyl group may have 1 to 20 carbon atoms. The alkyl group may be a medium-sized alkyl having 1 to 10 carbon atoms. The alkyl group may be a lower alkyl having 1 to 6 carbon atoms.

For example, a C1-C4 alkyl may have 1 to 4 carbon atoms and may be selected from the group consisting of methyl, ethyl, propyl, iso-propyl, n-butyl, iso-butyl, sec-butyl, and t-butyl.

Representative examples of an alkyl group may be selected from a methyl group, an ethyl group, a propyl group, an isopropyl group, a butyl group, an isobutyl group, a t-butyl group, a pentyl group, a hexyl group, an ethenyl group, a propenyl group, a butenyl group, a cyclopropyl group, a cyclobutyl group, a cyclopentyl group, a cyclohexyl group, or the like, which may be individually and independently substituted.

The term "aromatic group" may refer a functional group including a cyclic structure where all elements have p-orbitals that form conjugation. An aryl group and a heteroaryl group may be exemplified.

The term "aryl" may refer to a monocyclic or fused ring-containing polycyclic (i.e., rings sharing adjacent pairs of carbon atoms) group.

The "heteroaryl group" may refer to one including 1 to 3 heteroatoms selected from N, O, S, or P in an aryl group, and remaining carbons. When the heteroaryl group is a fused ring, each ring may include 1 to 3 hetero atoms.

The term "spiro structure" refers to a cyclic structure having a contact point of one carbon. Further, the spiro structure may be used as a compound including the spiro sturcture or a substituent including the spiro sturcture.

The compound for an optoelectronic device of one embodiment may have a core structure in which two carbazole-based derivatives are independently bound to a nitrogen atom. In this specification, the carbazole-based derivative refers to a structure in which a nitrogen atom of a substituted or unsubstituted carbazolyl group is substituted with another hetero atom instead of nitrogen. However, the structure including two carbazolyl groups bound to each other is not included in one embodiment.

Since the core structure includes at least two or more carbazole derivatives and has excellent hole characteristics, it may be used as a hole injection material or a hole transport material of an organic light emitting device.

At least one of the substituent that is bound to the core may be a substituent having excellent electron characteristics.
At least one of the substituent that is bound to the core may be a substituent having excellent electron characteristics.

Therefore, the compound may satisfy requirements of an emission layer by reinforcing electron characteristics to a carbazole structure having excellent hole characteristics. In one embodiment, it may be used as a host material of an emission layer.

In addition, the compound for an optoelectronic device may be a synthesis of a compound having various energy band gaps by introducing various substituents into the core of a carbazole and two carbazoles bound to the core of the carbazole.

As the organic photoelectric device includes the compound having the appropriate energy level depending upon the substituents, the electron transporting property is enforced to provide excellent efficiency and driving voltage, and the electrochemical and thermal stability are improved to enhance the life-span characteristic while driving the organic photoelectric device.

According to one embodiment of the present invention, a compound for an optoelectronic device represented by the following Chemical Formula 1 is provided.

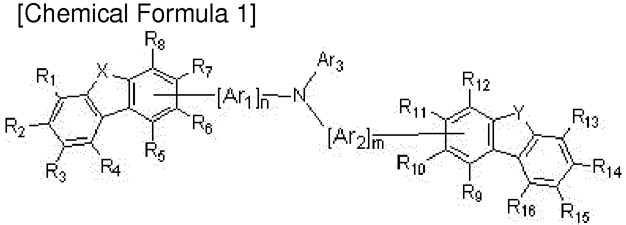

In Chemical Formula 1, R₁ to R₁₆ are the same or different, and are independently selected from hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonyl amino group, a substituted or unsubstituted C7 to C20 aryloxycarbonyl amino group, a substituted or unsubstituted C1 to C20 sulfamoyl amino group, a substituted or unsubstituted C1 to C20 sulfonyl group, a substituted or unsubstituted C1 to C20 alkylthiol group, a substituted or unsubstituted C6 to C20 arylthiol group, a substituted or unsubstituted C1 to C20 heterocyclothiol group, a substituted or unsubstituted C1 to C20 ureide group, and a substituted or unsubstituted C3 to C40 silyl group.

In addition, one of R₁ to R₈ is bound to Ar₁, and one of R₉ to R₁₆ is bound to Ar₂. At least one of R₁ to R₈ is bound to Ar₁ through a sigma bond, and at optoelectronic device having an excellent hole or electron transporting property, high film stability, thermal stability, and triplet exciton energy (T1) may be provided.

Also, a compound having improved thermal stability or oxidation resistance through a suitable combination of the substituents may be provided.

An asymmetrical bipolar structure may be proived through a suitable combination of substituents. Since the asymmetrical bipolar structure improves hole and electron transporting properties, it may improve luminous efficiency and performance of a device.

A structure of a compound may bulk up by controlling substituents, and therefore crystallinity may be decreased. When the crystallinity of a compound is decreased, the life-span of a device may be improved.

X is selected from NR₁₇, O, S, or SO₂(O=S=O), R₁₇ is a substituted or unsubstituted C1 to C20 alkyl group, a substituted or unsubstituted C6 to C30 aryl group, or a substituted or unsubstituted C2 to C30 heteroaryl group, and Y is O, S, or SO₂(O=S=O).

In the core structure of the above Chemical Formula 1, the hetero atom of the carbazole derivatives that are both substituents of a nitrogen atom may not simultaneously be N (i.e., carbazole). In other words, two or more carbazolyl groups may not exist as a substituent of nitrogen of a tertiary arylamine in the above Chemical Formula 1. The symmetric compound having the same substituents may increase crystallinity.

In addition, Ar₁ and Ar₂ are the same or different, and are independently a substituted or unsubstituted C6 to C30 aryl group, or a substituted or unsubstituted C2 to C30 heteroaryl group, n is an integer ranging from 1 to 4, and m is an integer ranging from 0 to 4. The π-conjugation length may be controlled by adjusting the length of Ar₁ and Ar₂. Thereby, the triplet exciton energy bandgap is controlled, and may be usefully applied as a phosphorescent host of the emission layer of an organic photoelectric device. In addition, when a heteroaryl group is introduced, a bipolar characteristic of a molecular structure is realized to provide a phosphorescent host of an organic photoelectric device having high efficiency.

Ar₃ is a substituted or unsubstituted C6 to C30 aryl group, or a substituted or unsubstituted C2 to C30 heteroaryl group. When X is NR₁₇, a fluorenyl group is not included.

Further, Ar₃ is a substituted or unsubstituted C6 to C30 aryl group, or a substituted or unsubstituted C2 to C30 heteroaryl group. Also, a substituted or unsubstituted carbazolyl group, a substituted or unsubstituted dibenzofuranyl group, or a substituted or unsubstituted dibenzothiophenyl group may be excluded for Ar₃. When X is NR₁₇, a fluorenyl group may be excluded for Ar₃. When the substituents described above are excluded, the crystallinity may be suppressed by decreasing a symmetric structure in the molecule, and thereby recrystallization may be inhibited in a device.

Examples of Ar₃ may include a substituted or unsubstituted phenyl group, a substituted or unsubstituted naphthyl group, a substituted or unsubstituted anthracenyl group, a substituted or unsubstituted phenanthryl group, a substituted or unsubstituted naphthacenyl group, a substituted or unsubstituted pyrenyl group, a substituted or unsubstituted biphenylyl group, a substituted or unsubstituted p-terphenyl group, a substituted or unsubstituted m-terphenyl group, a substituted or unsubstituted chrysenyl group, a substituted or unsubstituted triperylenyl group, a substituted or unsubstituted perylenyl group, a substituted or unsubstituted indenyl group, a substituted or unsubstituted furanyl group, a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthydinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, or a substituted or unsubstituted phenoxazinyl group.

X is selected from NR₁₇, O, S, or SO₂ (O=S=O), where R₁₇ is a substituted or unsubstituted C1 to C20 alkyl group, a substituted or unsubstituted C6 to C30 aryl group, or a substituted or unsubstituted C2 to C30 heteroaryl group, wherein the term "substituted" refers to at least one hydrogen of an aryl group or a heteroaryl group substituted with deuterium, a halogen, a cyano group, hydroxy group, an amino group, a substituted or unsubstituted C1 to C20 amine group, a nitro group, a substituted or unsubstituted C1 to C20 alkyl group, a substituted or unsubstituted C1 to C20 alkoxy group, a substituted or unsubstituted C3 to C40 silyl group, or a combination thereof.

As described above, when one of substituents of R₁₇ is the above substituent instead of hydrogen, electro-optical characteristics and thin film characteristics for maximizing the performance of the material for an optoelectronic device may be finely adjusted while maintaining basic characteristics of the compound.

The Chemical Formula 1 may be represented by one of the Chemical Formulae 2 to 7.

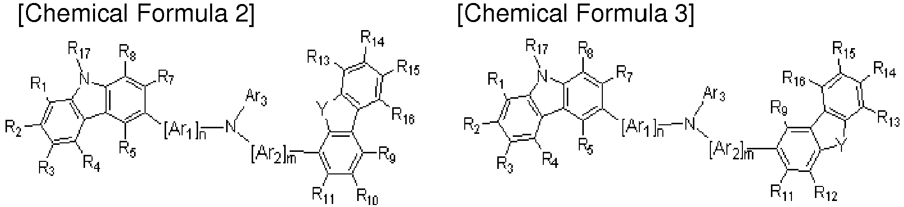

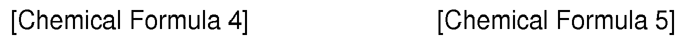

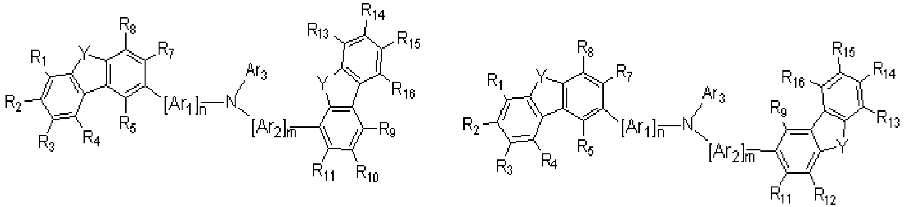

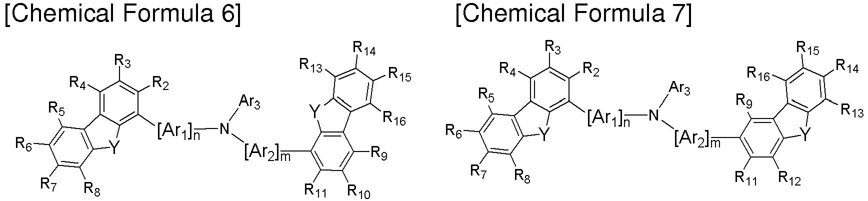

The Chemical Formulae 2 to 7 include fixed positions at which a substituent of a carbazole derivative such as a dibenzofuranyl group or a dibenzothiophenyl group is bound in Chemical Formula 1. When the substituent is bound at fixed positions, substantial synthesis may be advantageously performed.

The compound for an optoelectronic device may include at least one of the compounds represented by the following Chemical Formulae 8 or 9.

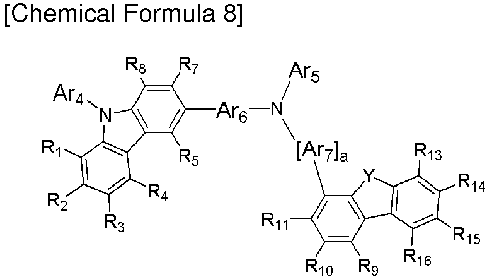

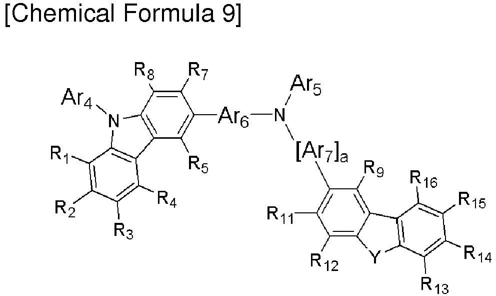

In Chemical Formulae 8 and 9, Ar₄ and Ar₅ are the same or different, and are independently selected from substituents represented by the following Chemical Formulae 10 to 18.

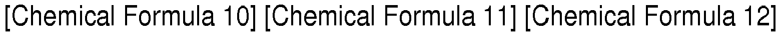

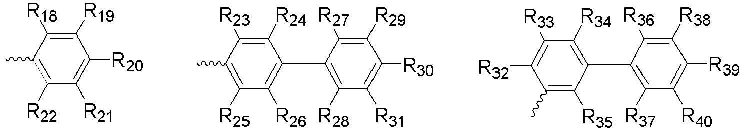

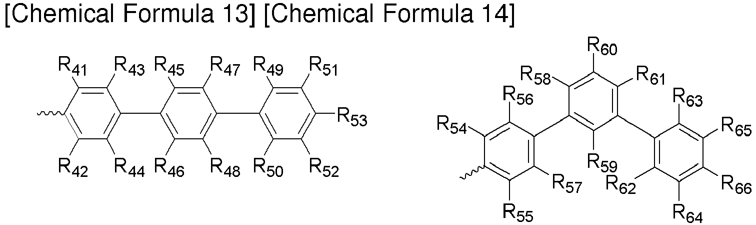

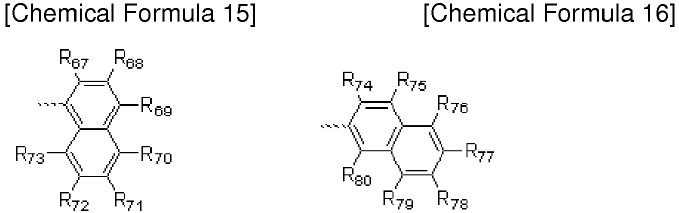

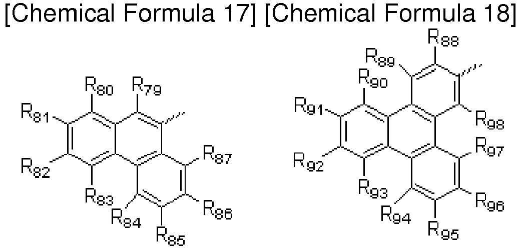

In Chemical Formulae 10 to 18, R₁ to R₅, R₇ to R₁₆, and R₁₈ to R₉₈ are the same or different, and are independently selected from hydrogen, deuterium, a halogen, a cyano group, a hydroxyl group, an amino group, a substituted or unsubstituted C1 to C20 amine group, a nitro group, a substituted or unsubstituted C1 to C20 alkyl group, a substituted or unsubstituted C1 to C20 alkoxy group, or a substituted or unsubstituted C3 to C40 silyl group, Ar₆ and Ar₇ are the same or different, and are independently selected from at least one of the substituents represented by the above Chemical Formulae 10 to 18, one of the selected substituents of R₁₈ to R₉₈ is bounded to an adjacent atom, and a is 0 or 1.

The compound represented by Chemical Formula 8 or 9 includes a substituted or unsubstituted aryl group that is substited with a substituent including nitrogen bound to a carbazolyl group and/or a substituent bound to an amine group. In this structure, it is hard to be recrystallized due to asymmetrical molecule structure as well as excellent hole transporting properties of a carbazolyl group. Therefore, when the compound is used for a hole injection and hole transport layer (HTL) of an organic light emitting diode, a long life-span and high efficiency can be realized.

Also, Ar₄ is selected from the substituents represented by Chemical Formulae 10 to 18. At least one of the substituents R₁₈ to R₉₈ for Ar₄ may not be hydrogen, and in one embodiment, may be selected from deuterium, a halogen, a cyano group, a hydroxyl group, an amino group, a substituted or unsubstituted C1 to C20 amine group, a nitro group, a substituted or unsubstituted C1 to C20 alkyl group, a substituted or unsubstituted C1 to C20 alkoxy group, or a substituted or unsubstituted C3 to C40 silyl group.

In other words, one of the substituents of Ar₄ may be substituted with one of the substituents described above. In this structure, electro-optical characteristics and thin film characteristics for maximizing the performance of the material for an optoelectronic device may be finely adjusted while maintaining basic characteristics of the compound.

The compound for an optoelectronic device according to one embodiment may include at least one of the compounds represented by the following Chemical Formulae A-1 to A-305, A-414 to A-416, A-457, A-458, or A-469 to A-473. The compounds of the following structures have an excellent hole transport property due to carbazolyl, excellent thin film characteristics due to an asymmetrical molecule, and thermal stability. Therefore when they are used for a hole injection layer and a hole transport layer (HTL) of an organic light emitting diode, a long life-span and high efficiency may be realized.

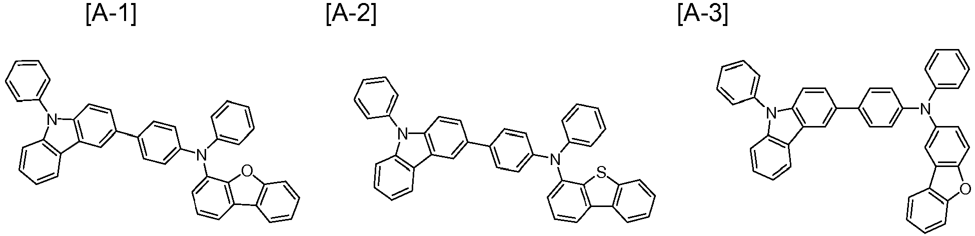

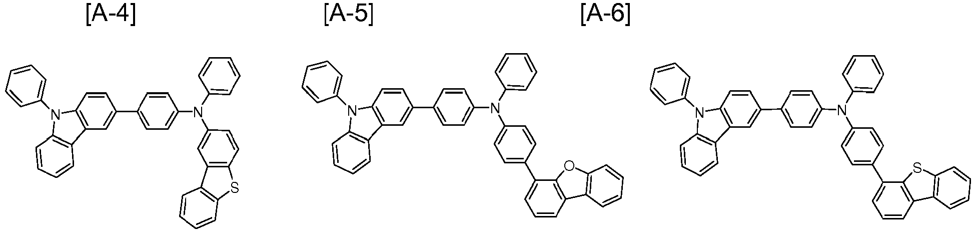

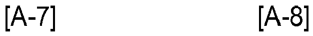

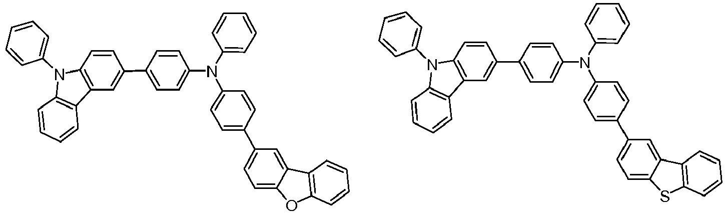

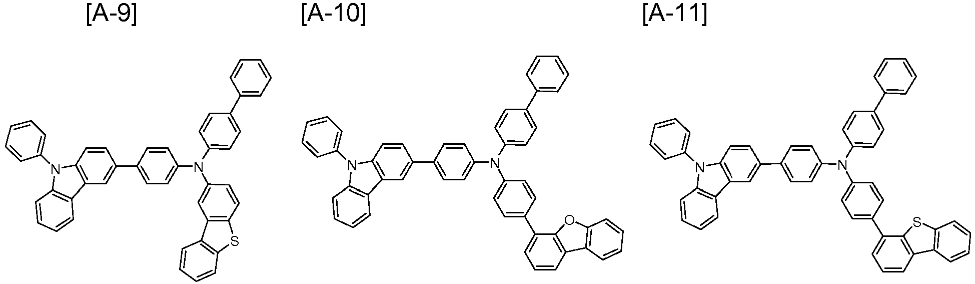

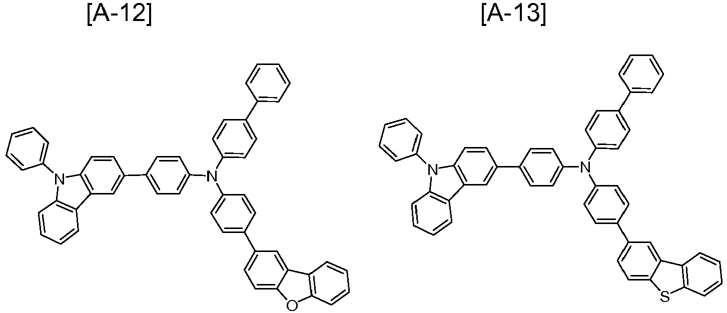

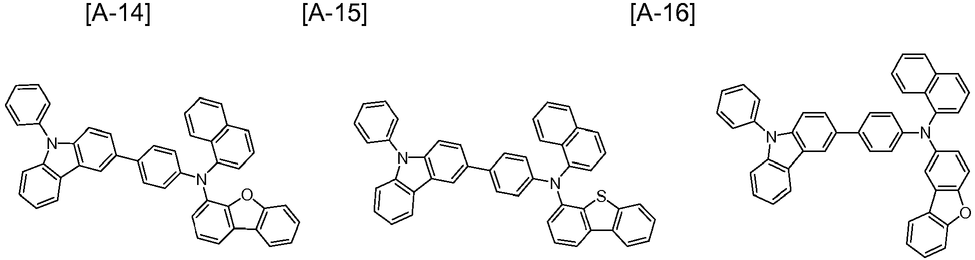

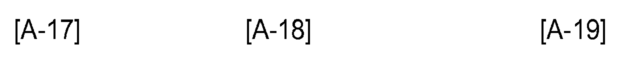

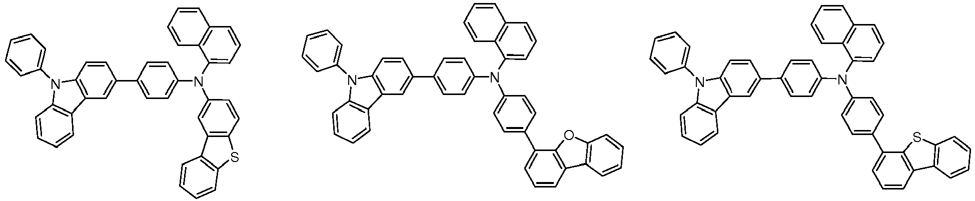

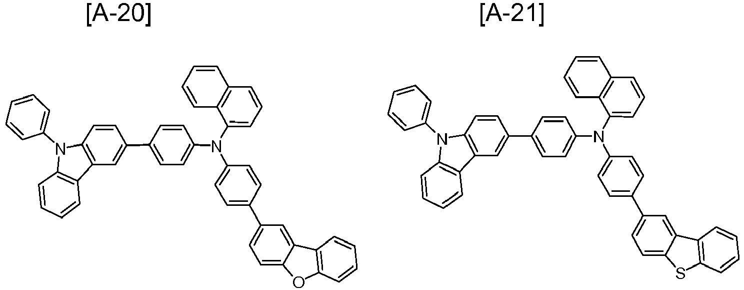

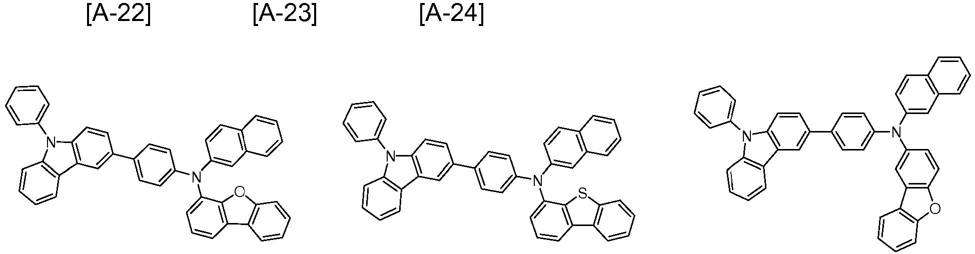

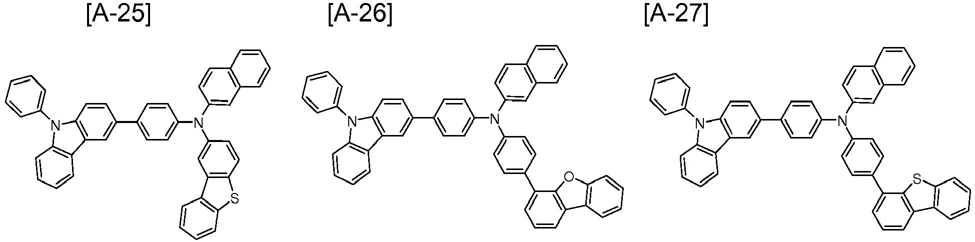

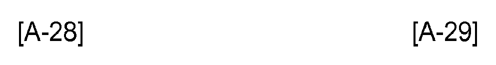

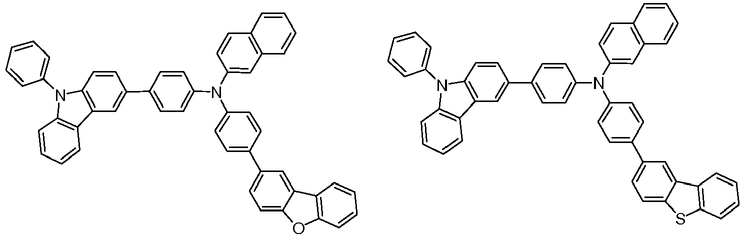

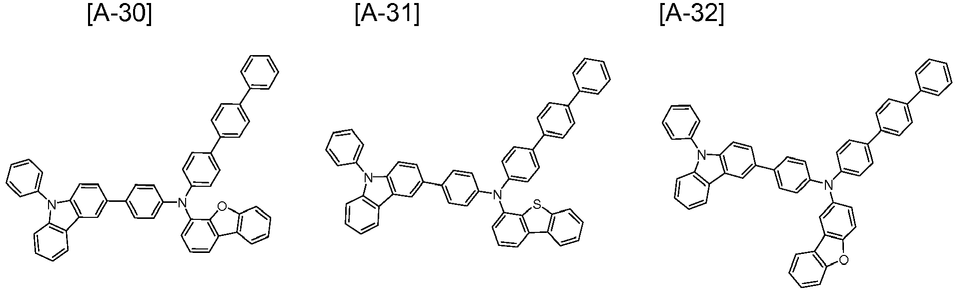

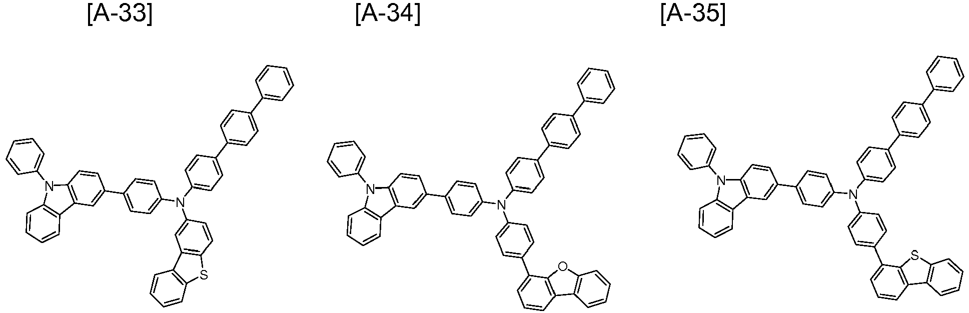

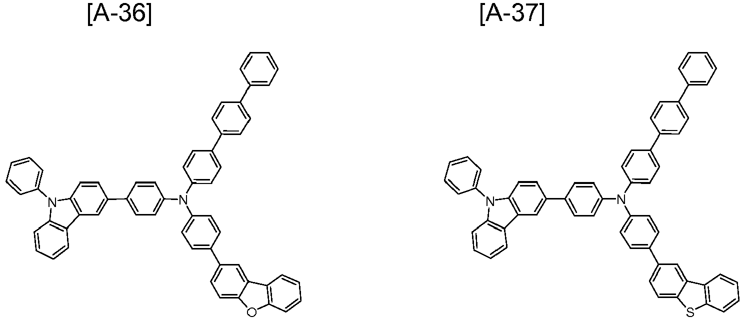

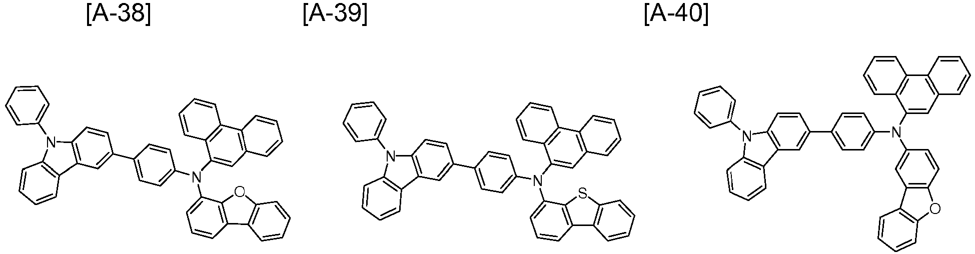

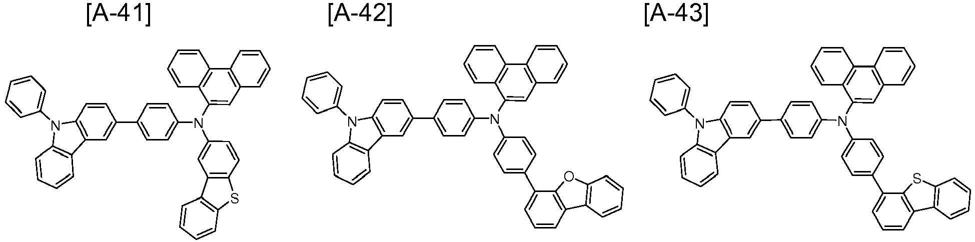

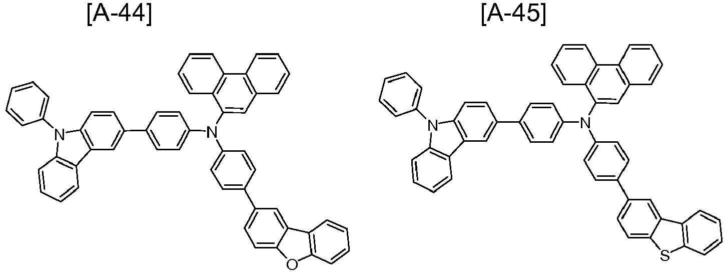

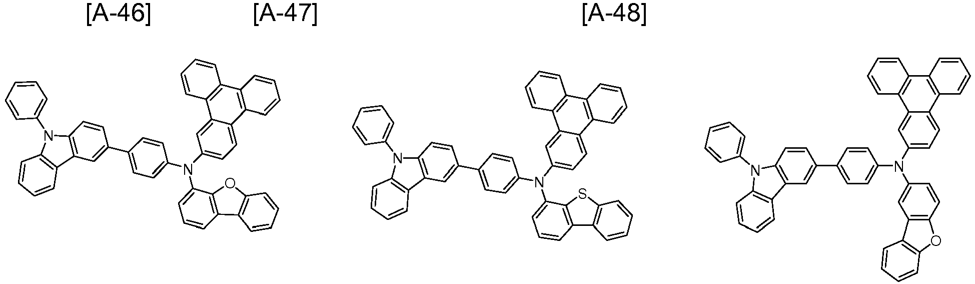

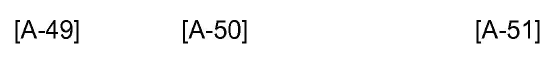

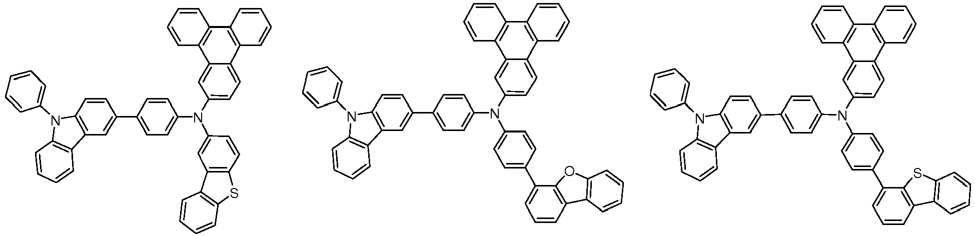

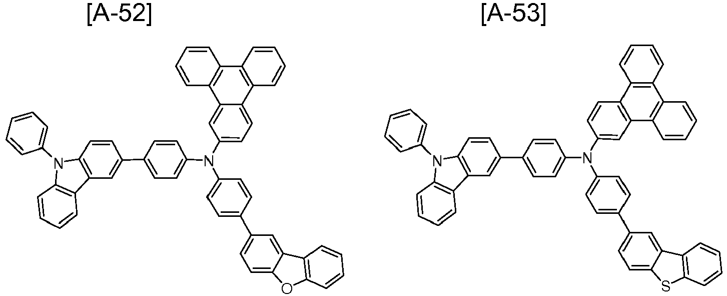

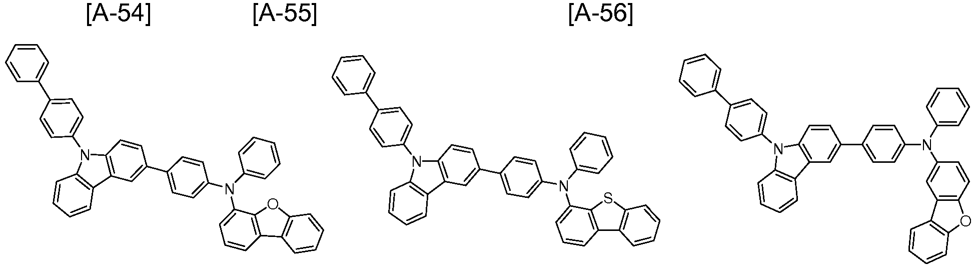

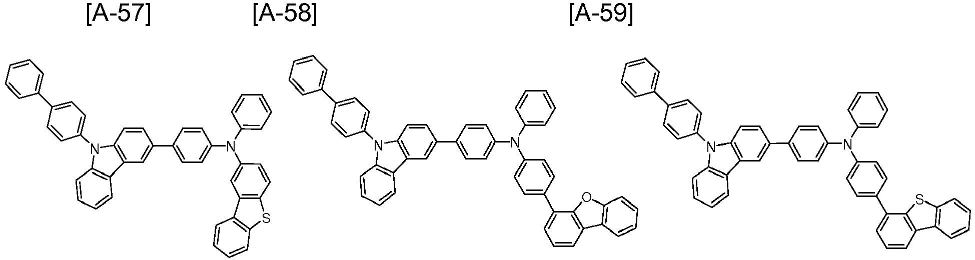

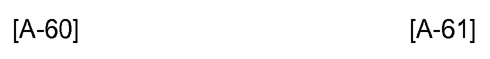

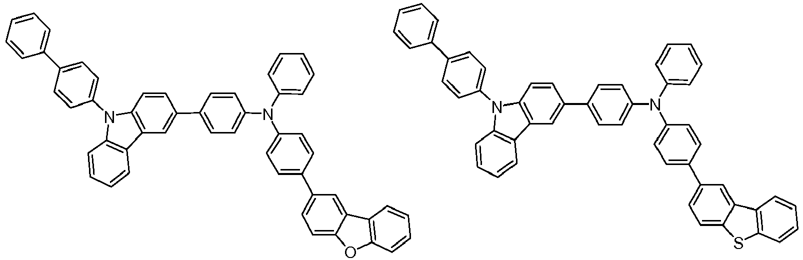

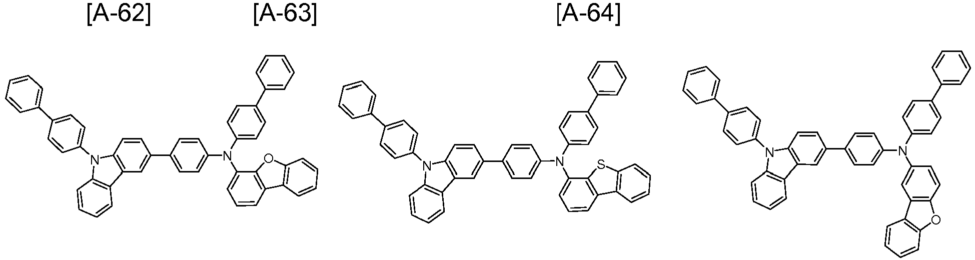

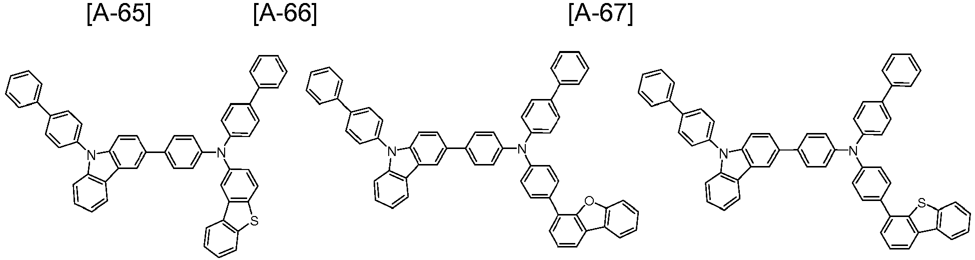

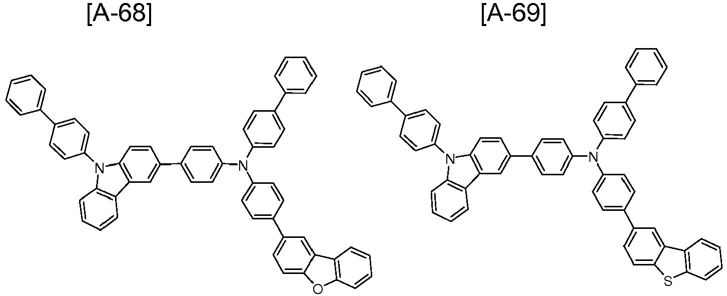

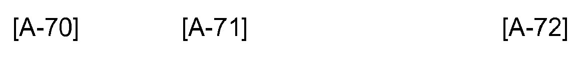

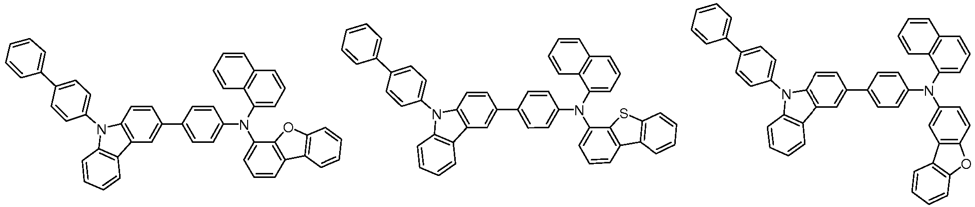

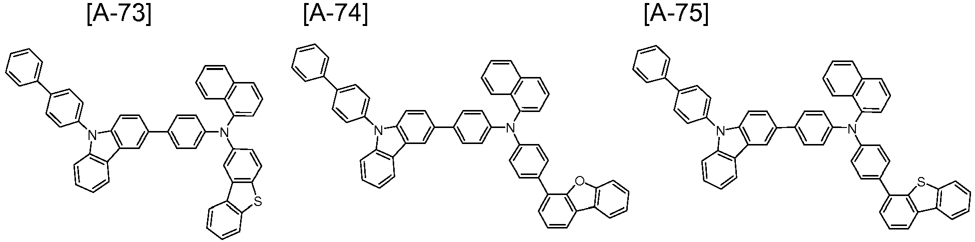

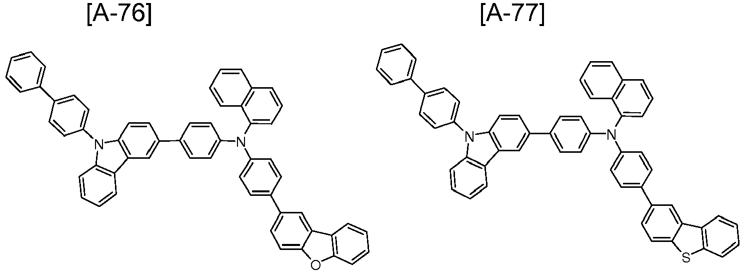

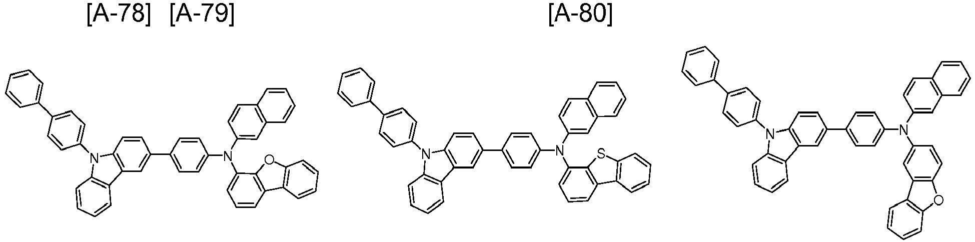

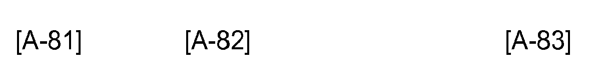

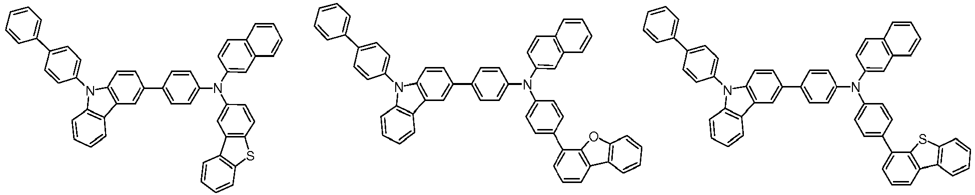

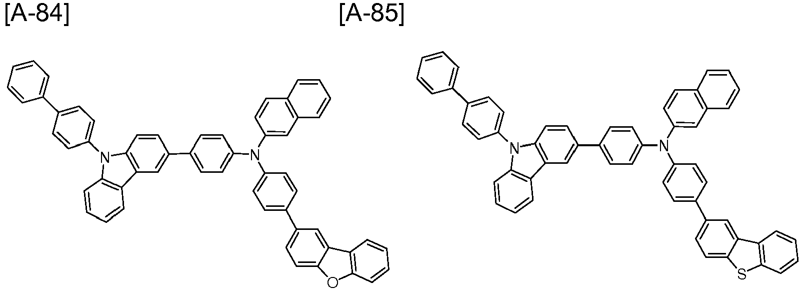

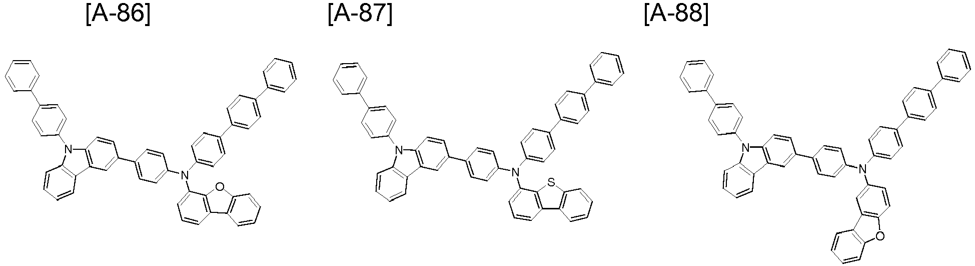

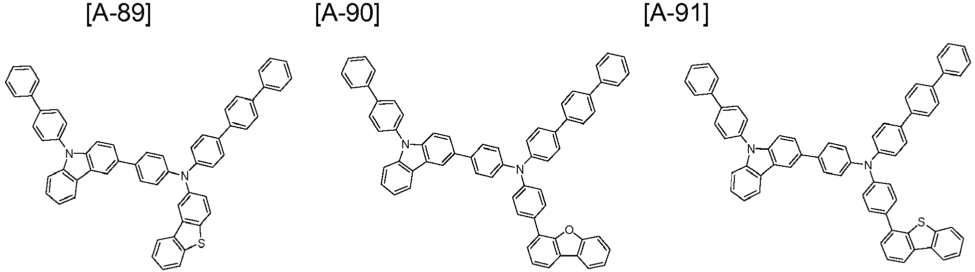

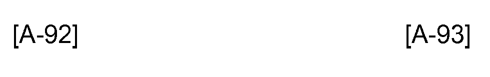

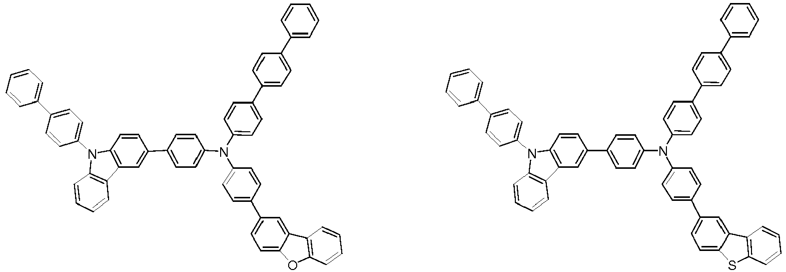

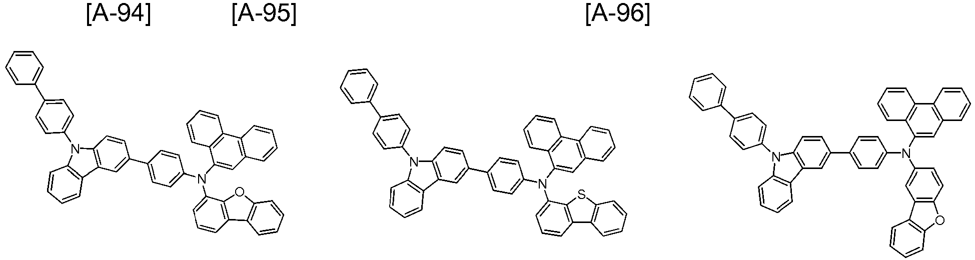

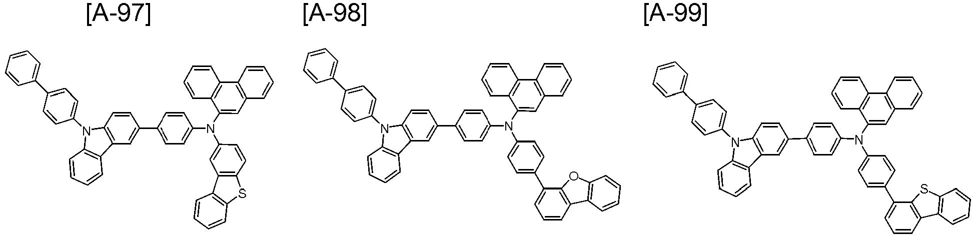

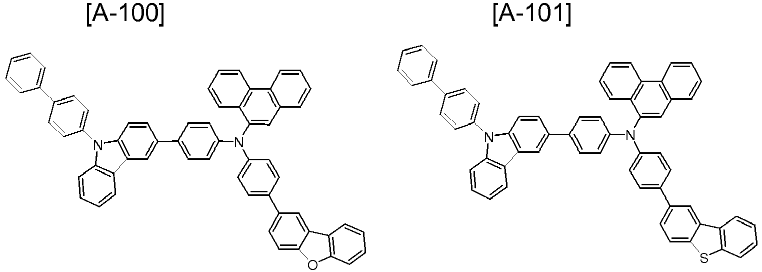

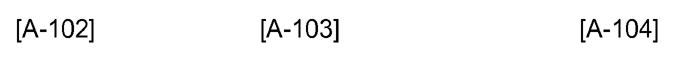

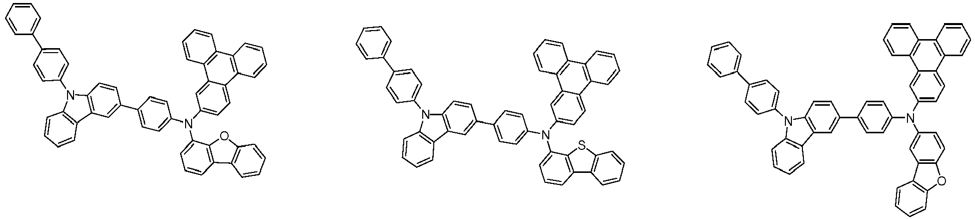

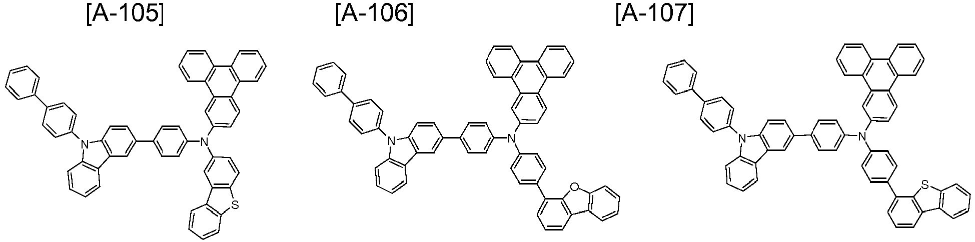

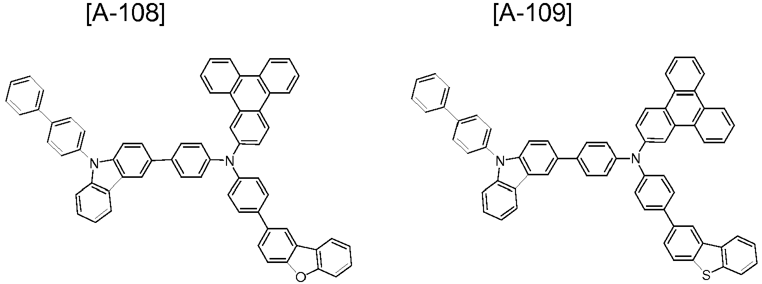

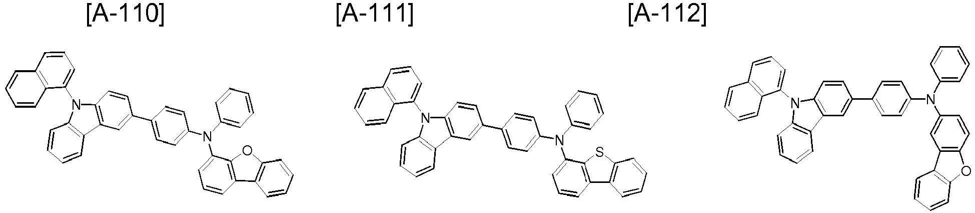

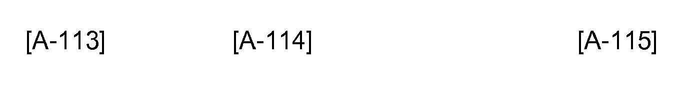

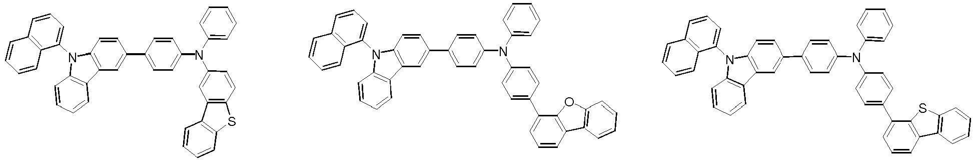

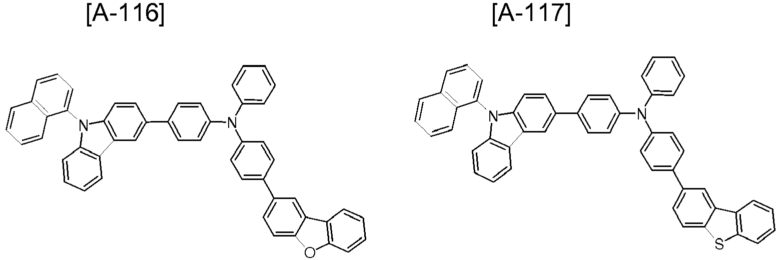

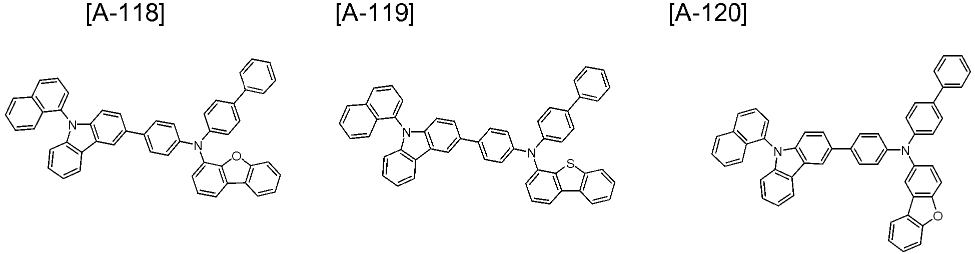

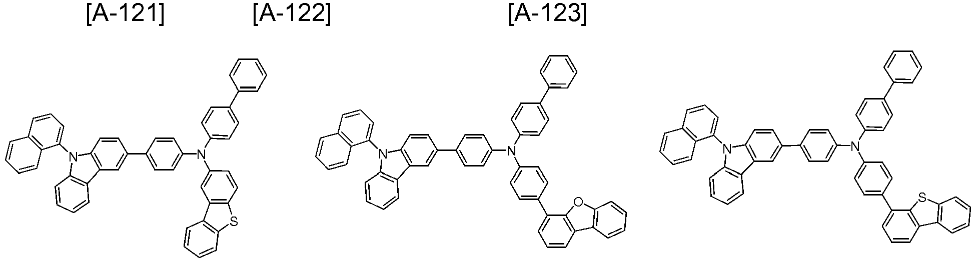

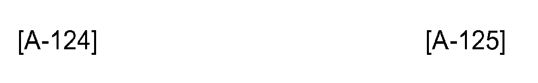

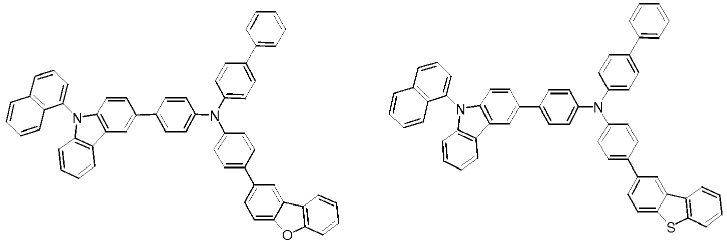

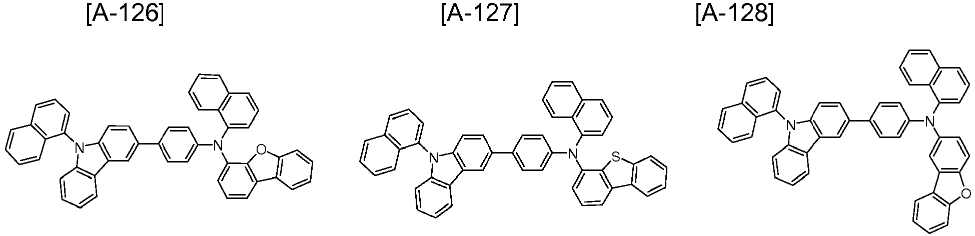

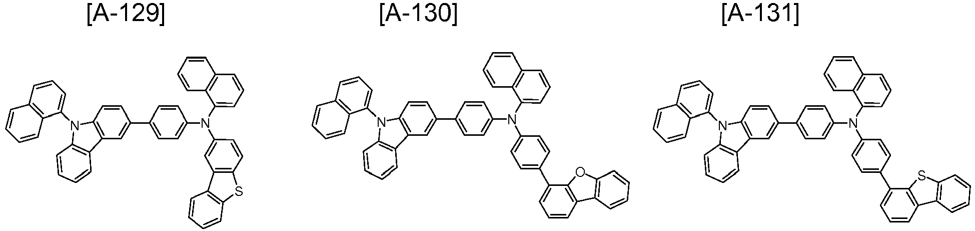

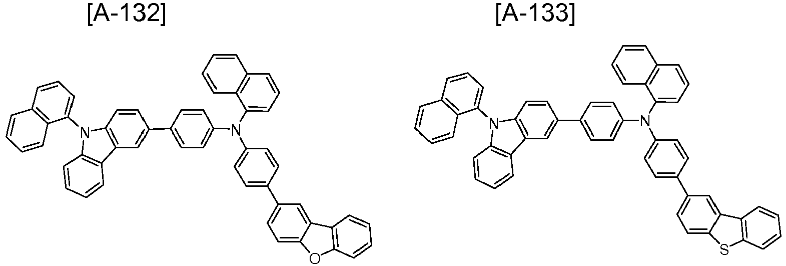

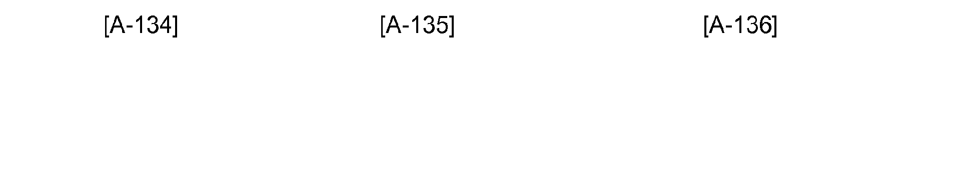

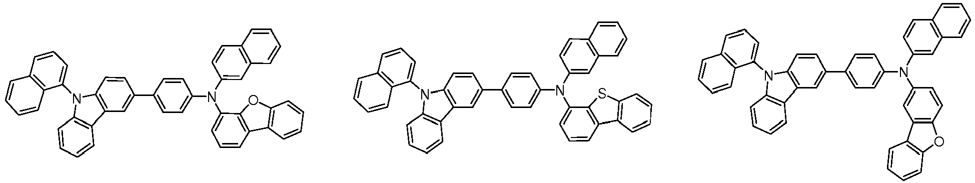

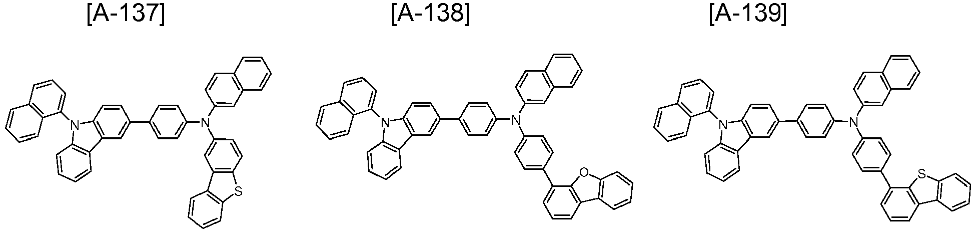

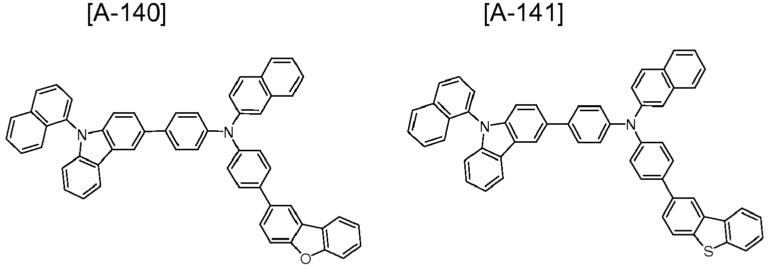

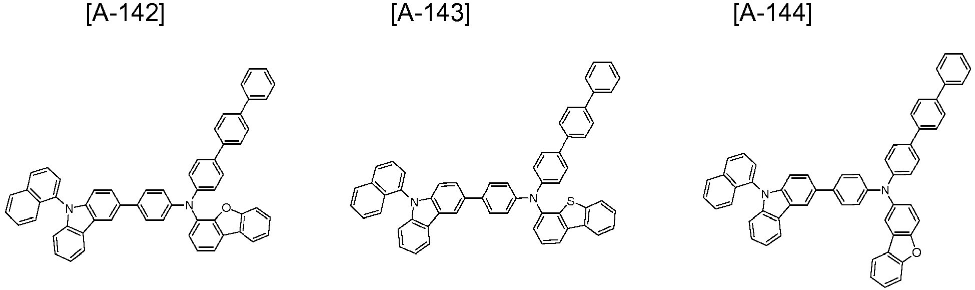

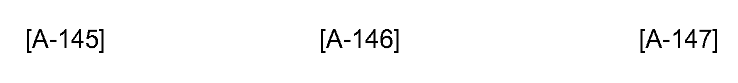

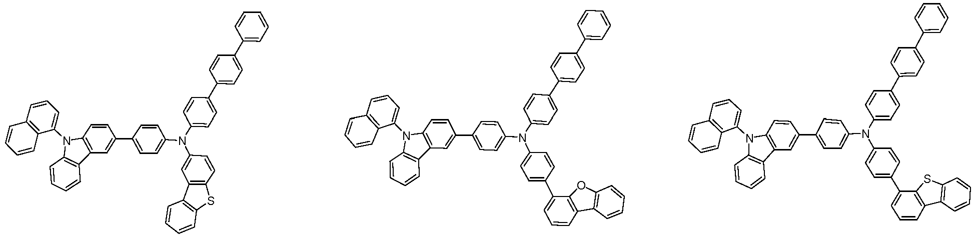

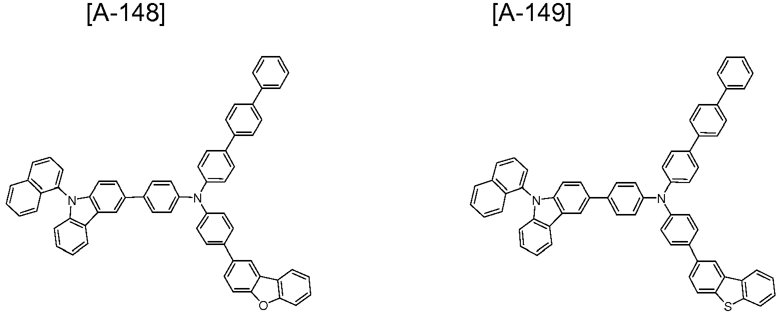

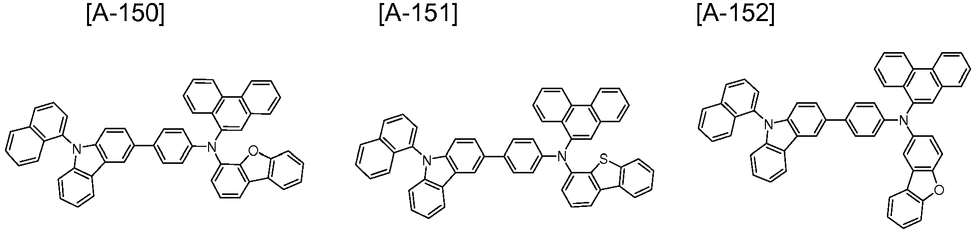

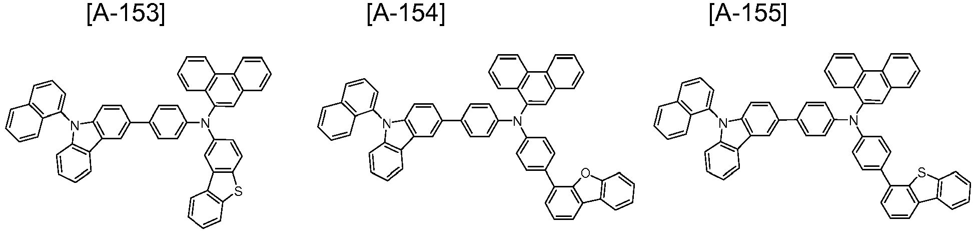

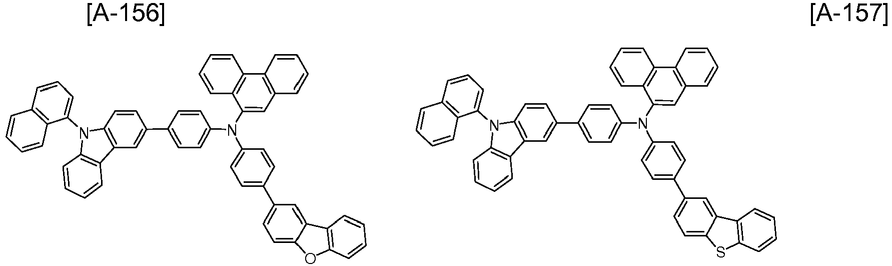

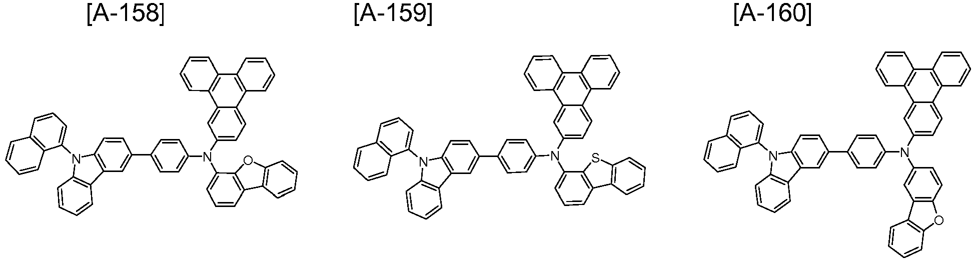

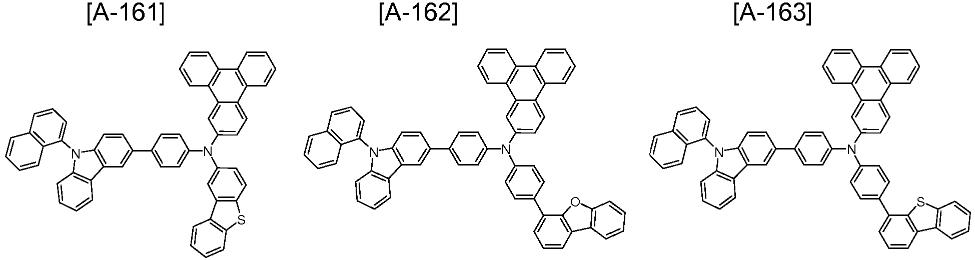

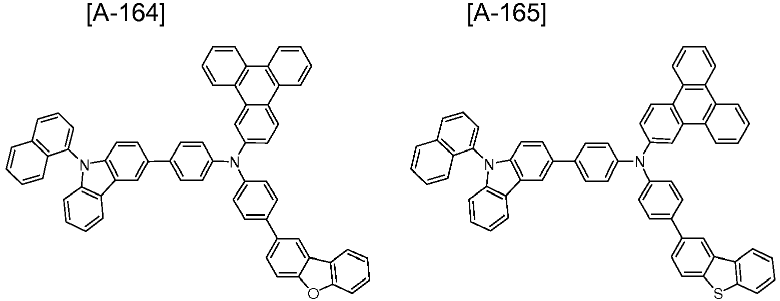

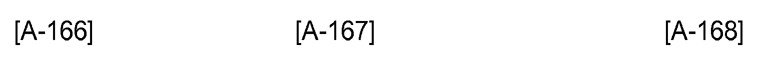

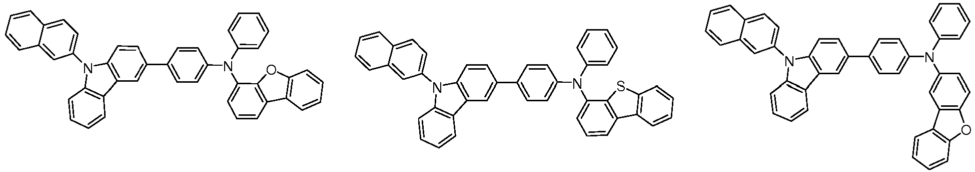

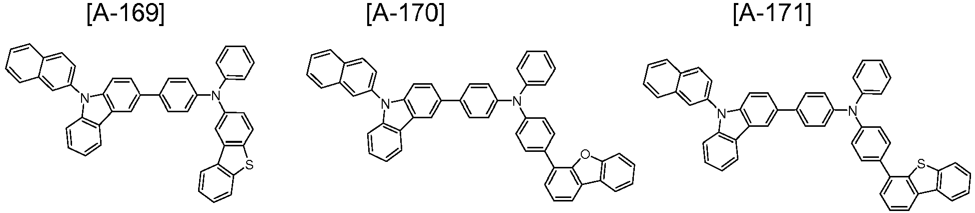

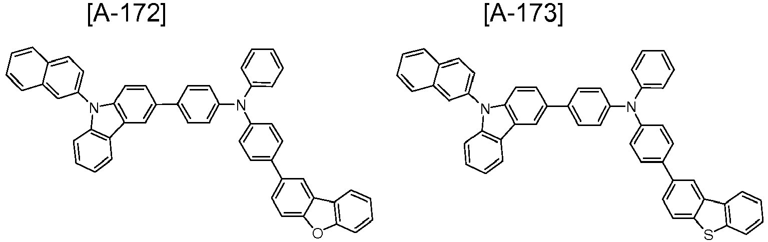

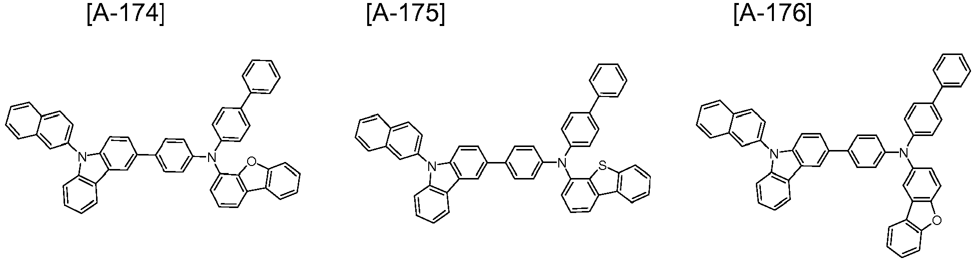

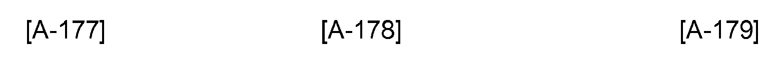

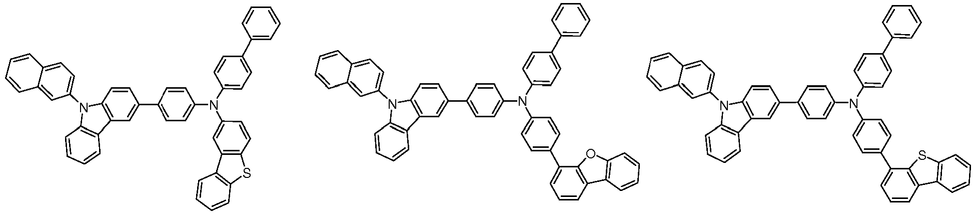

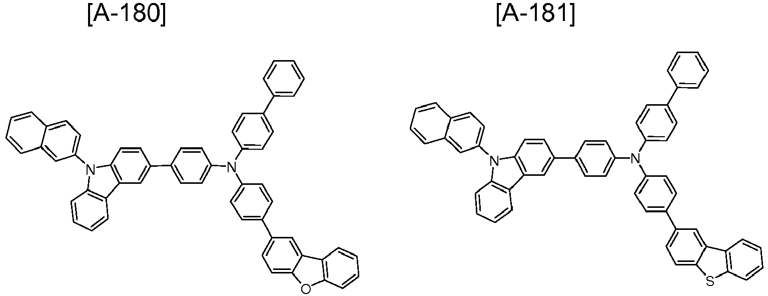

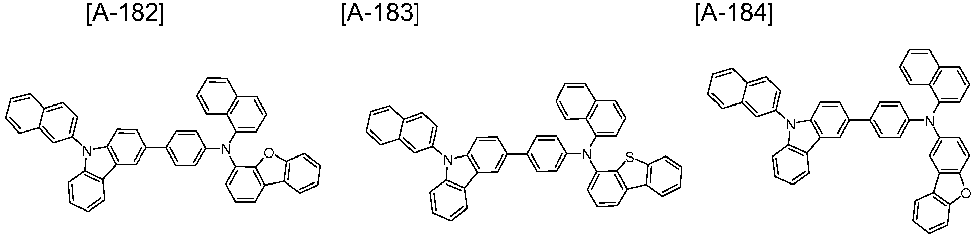

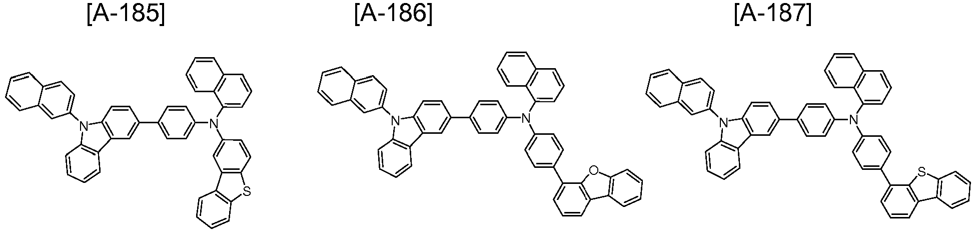

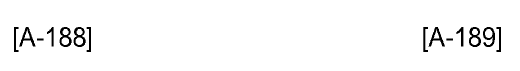

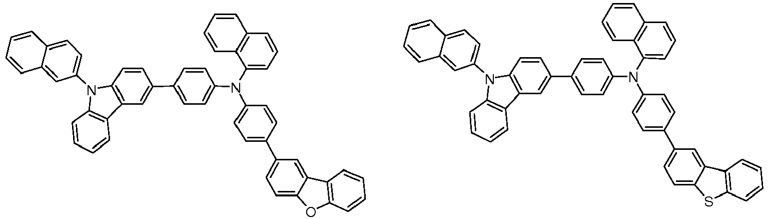

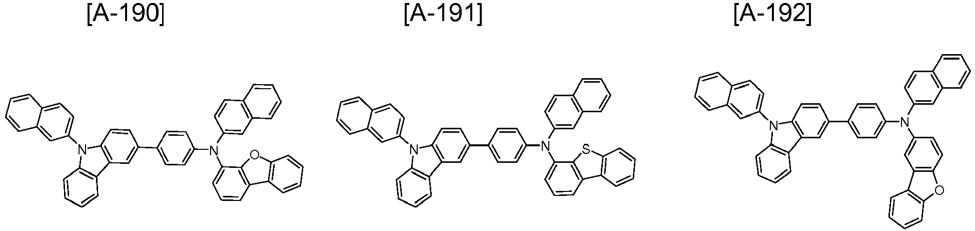

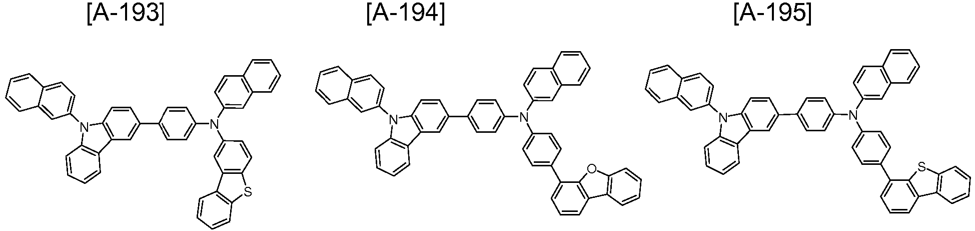

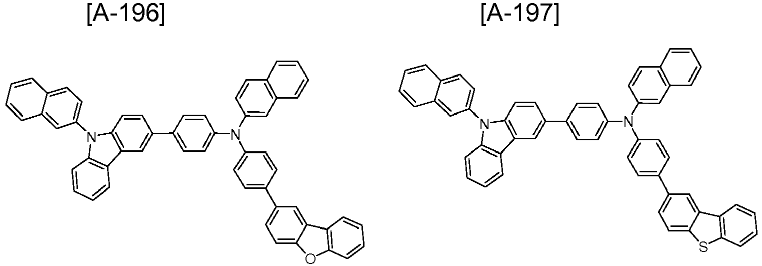

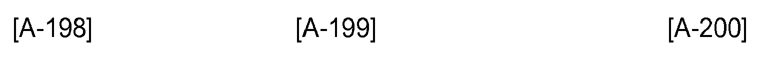

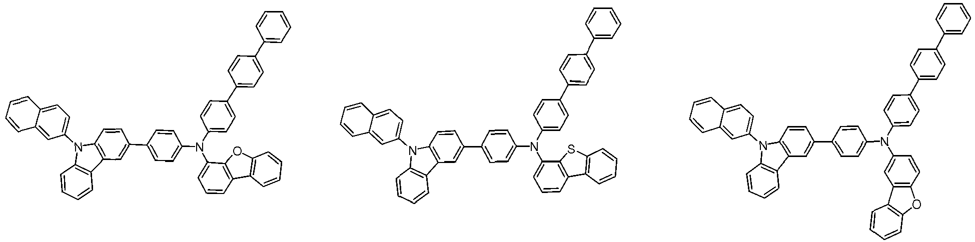

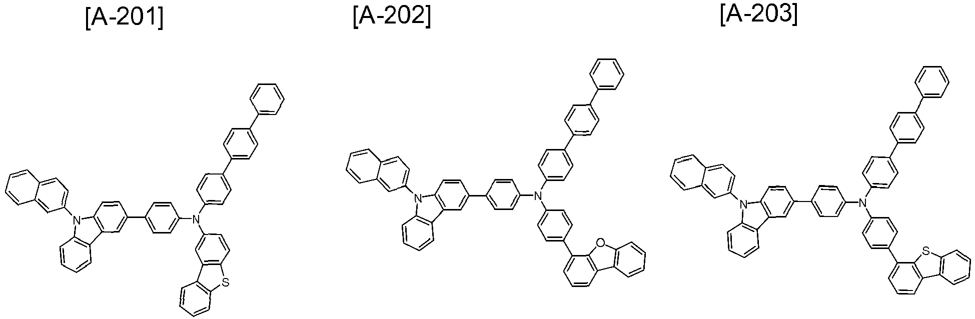

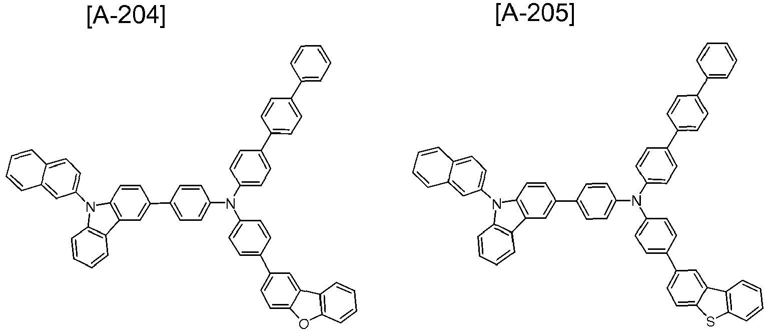

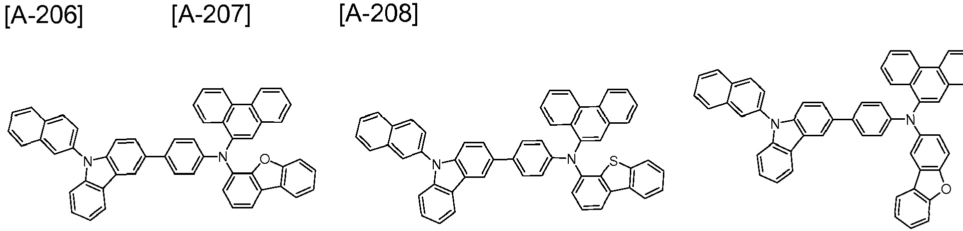

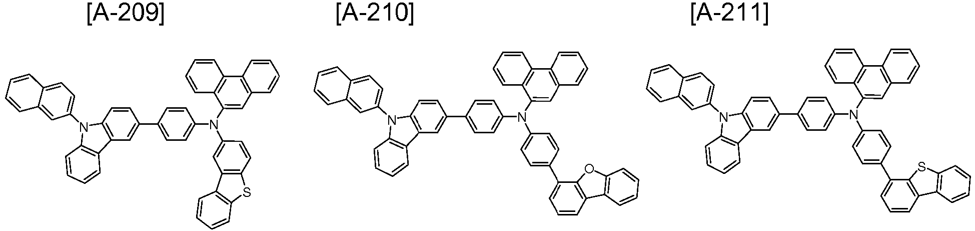

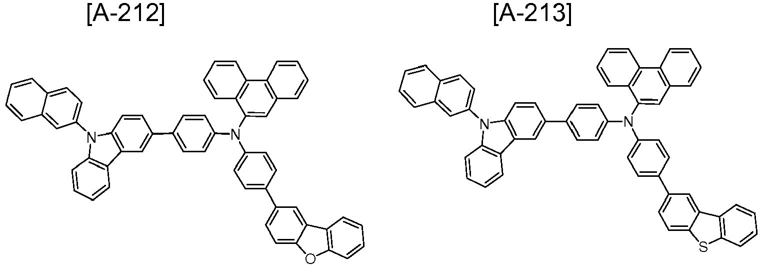

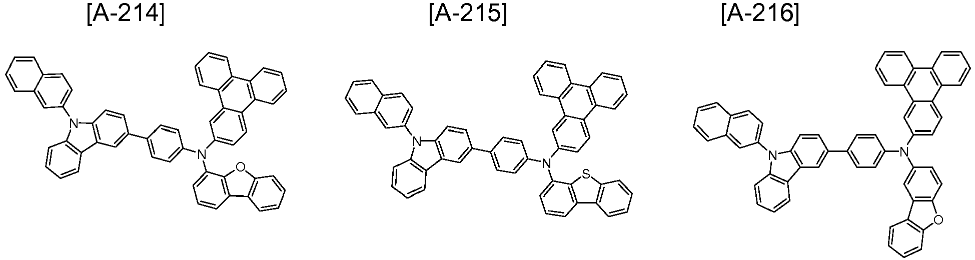

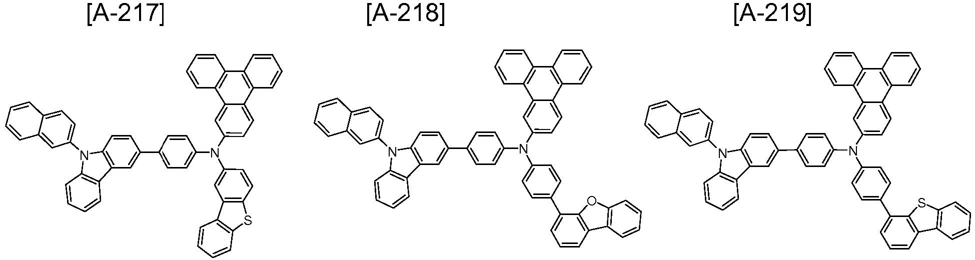

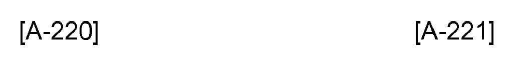

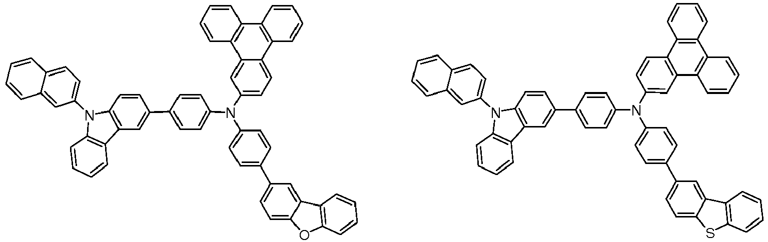

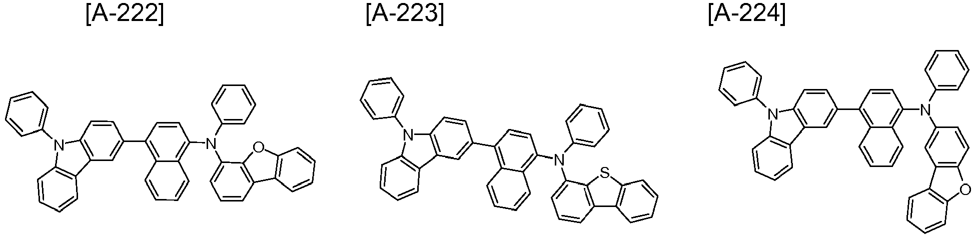

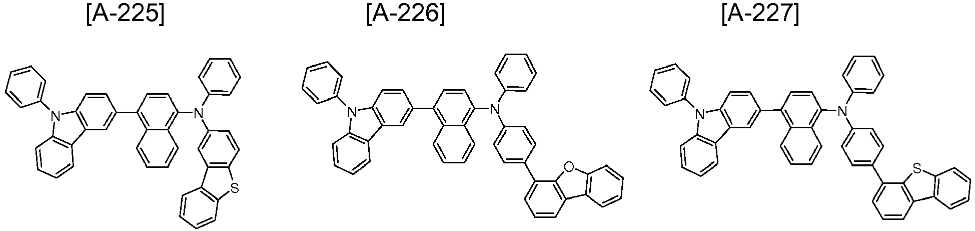

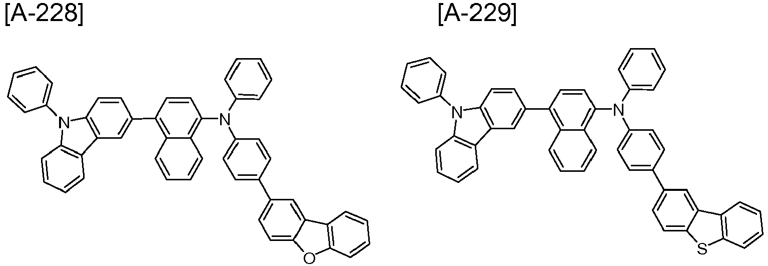

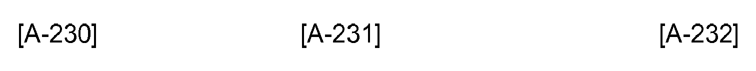

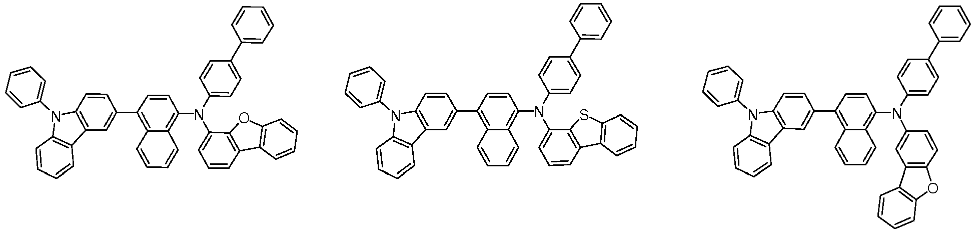

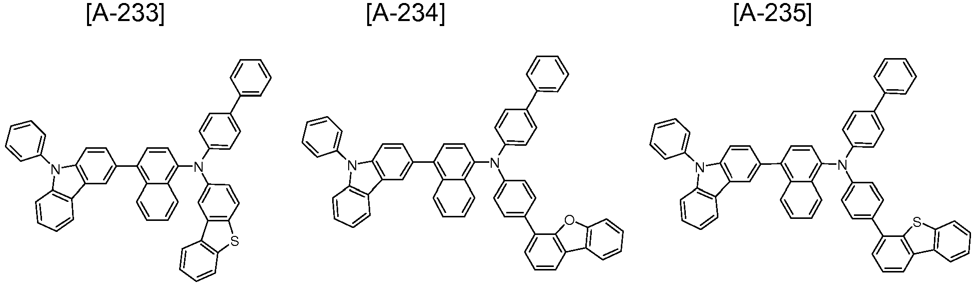

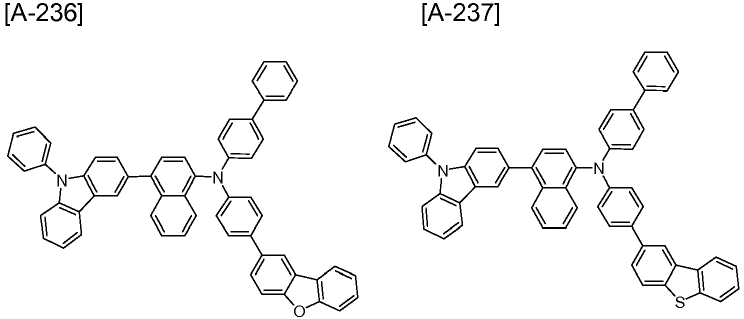

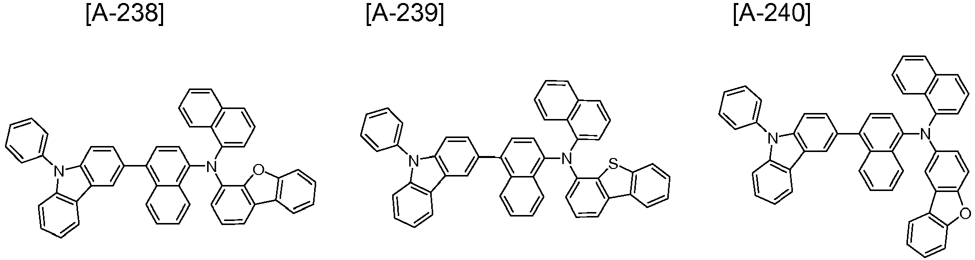

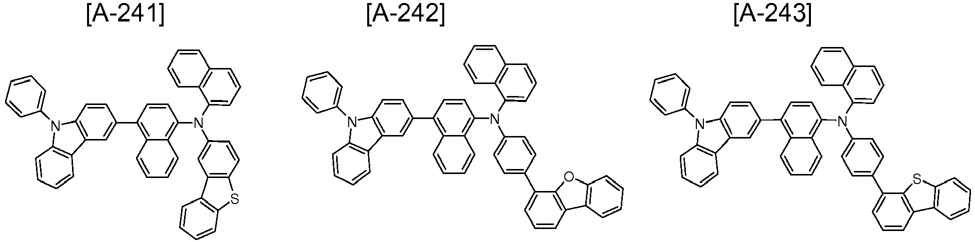

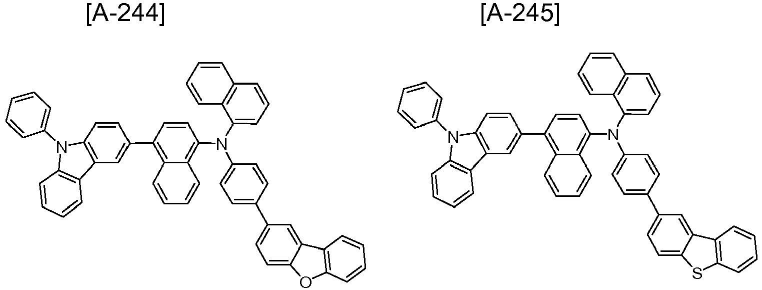

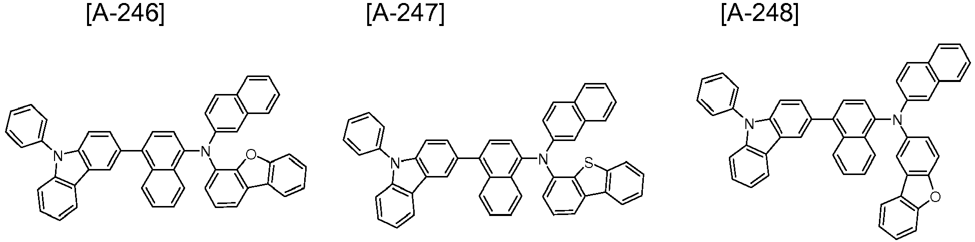

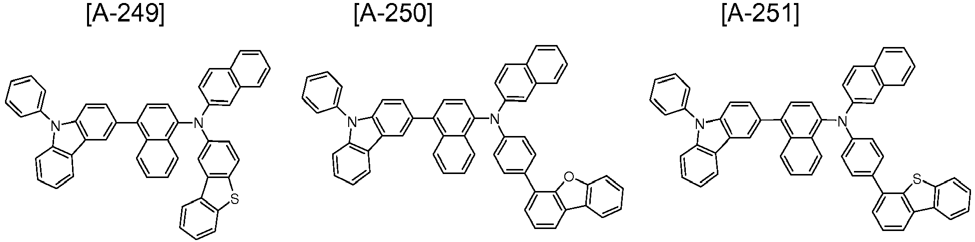

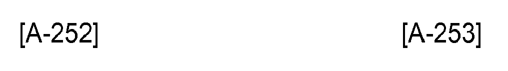

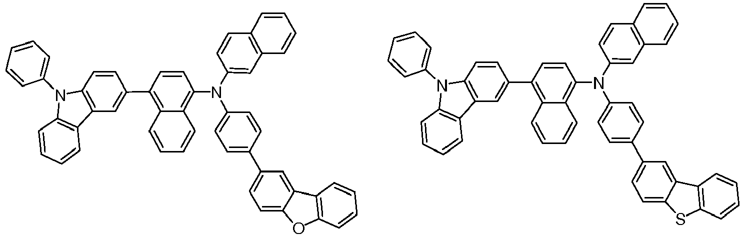

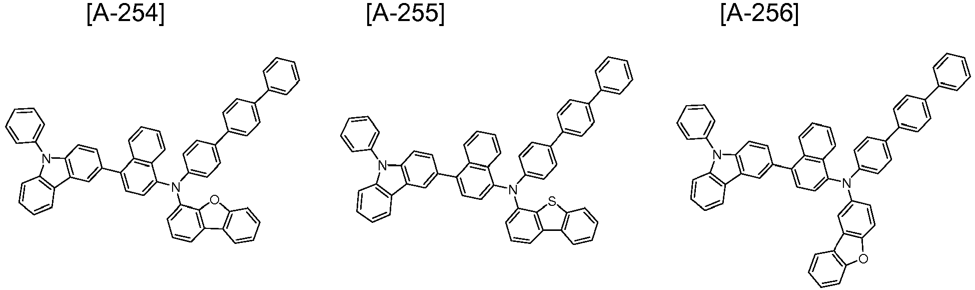

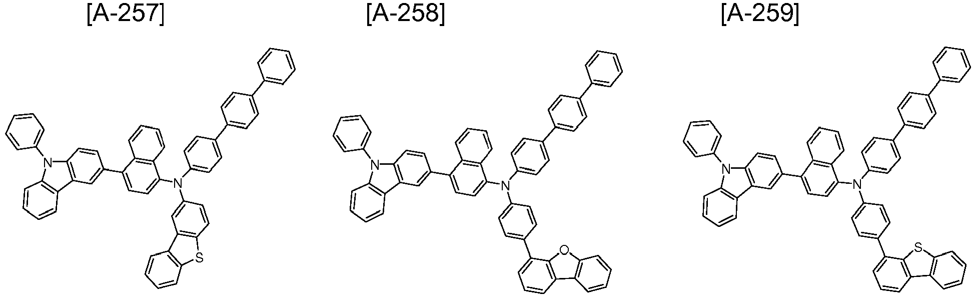

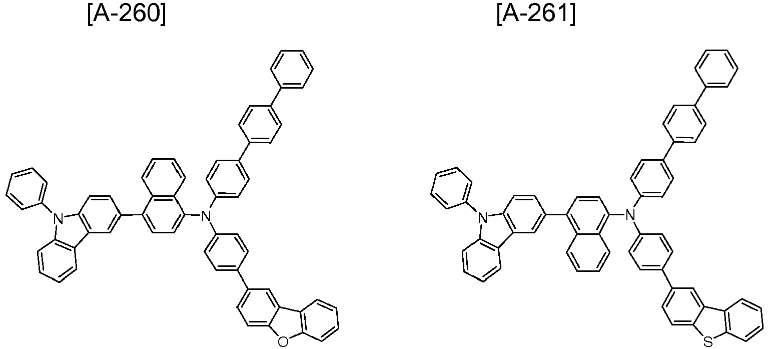

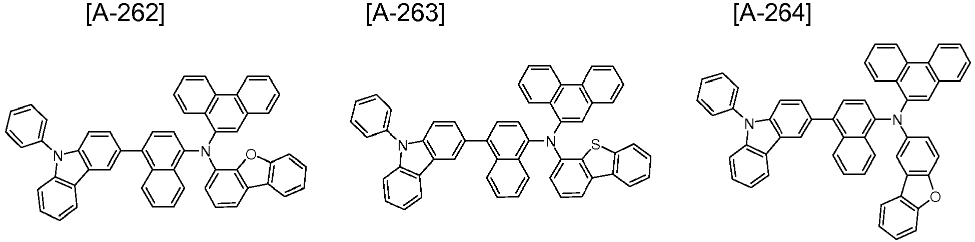

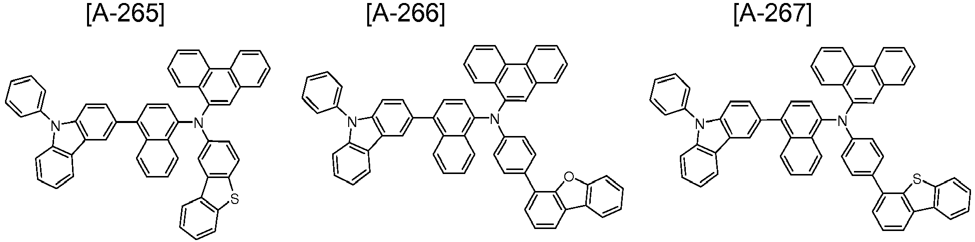

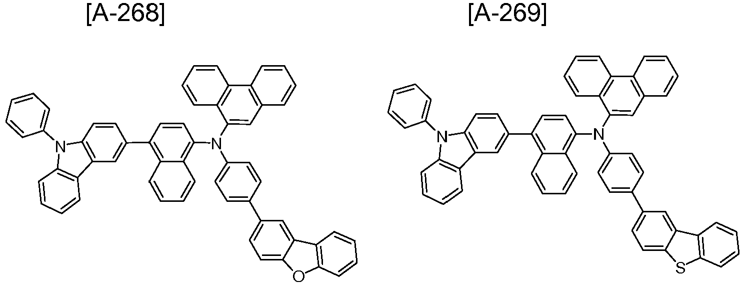

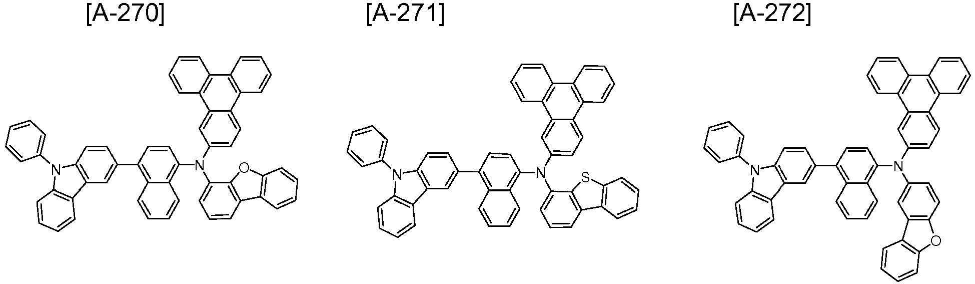

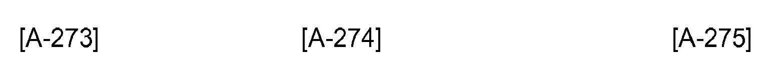

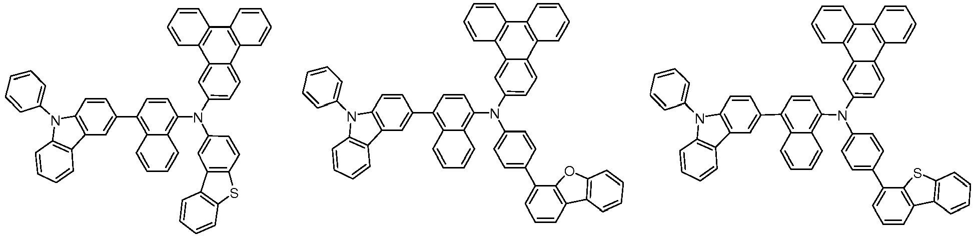

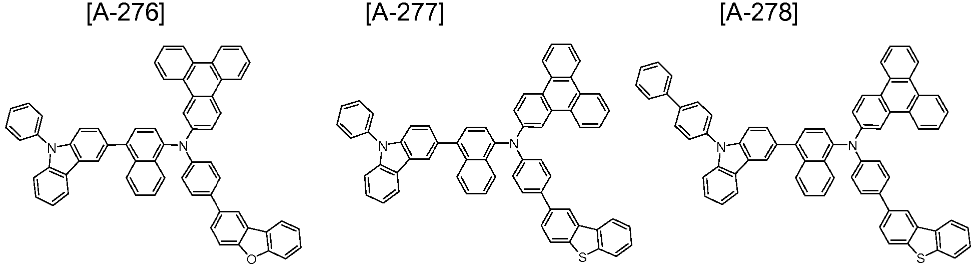

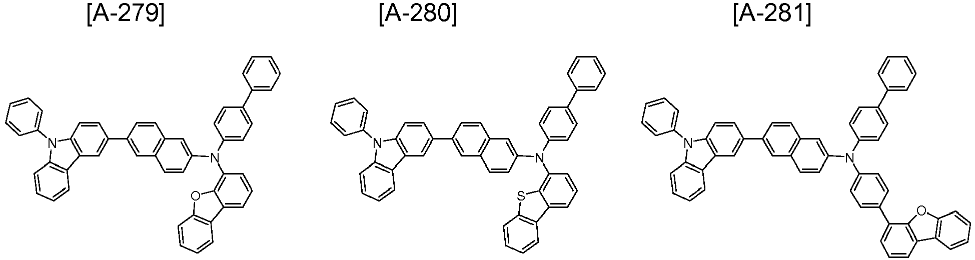

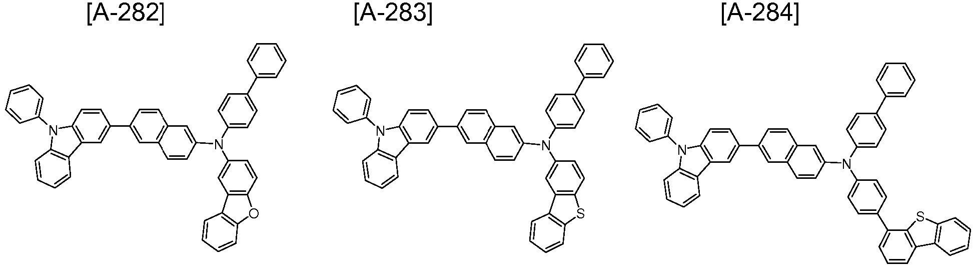

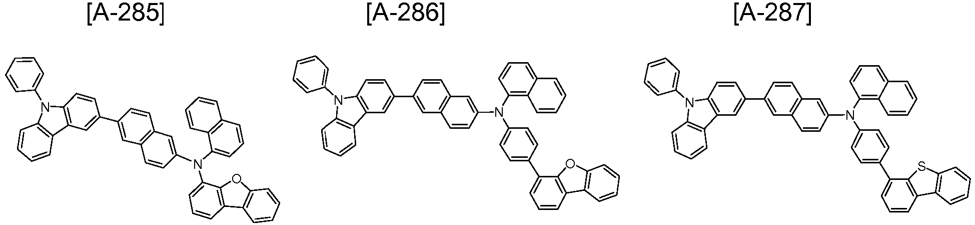

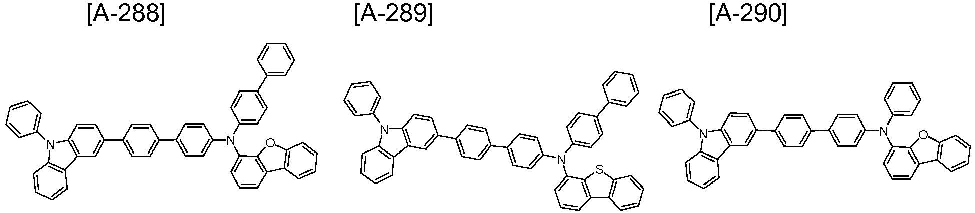

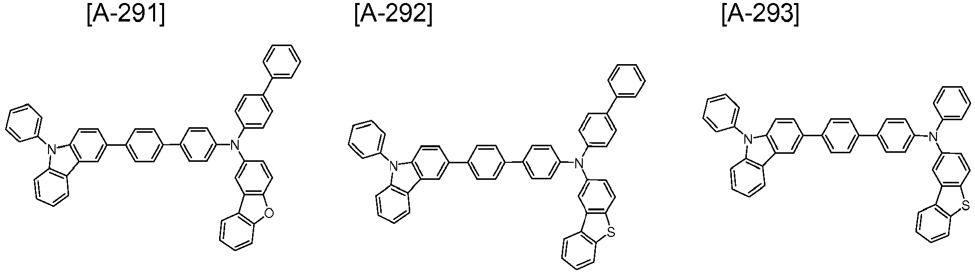

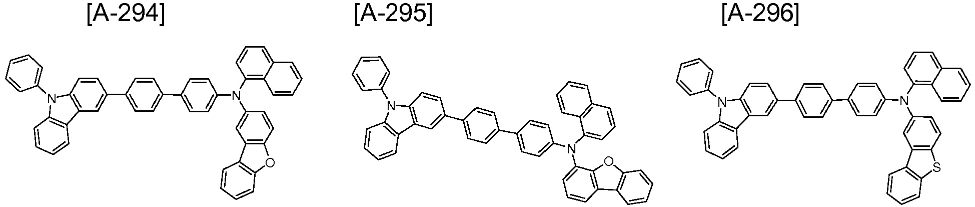

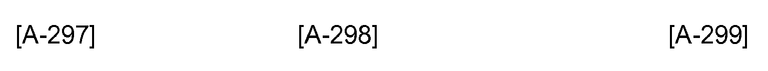

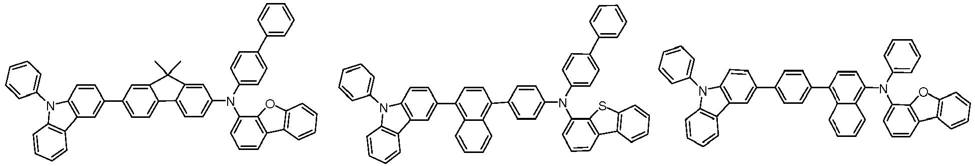

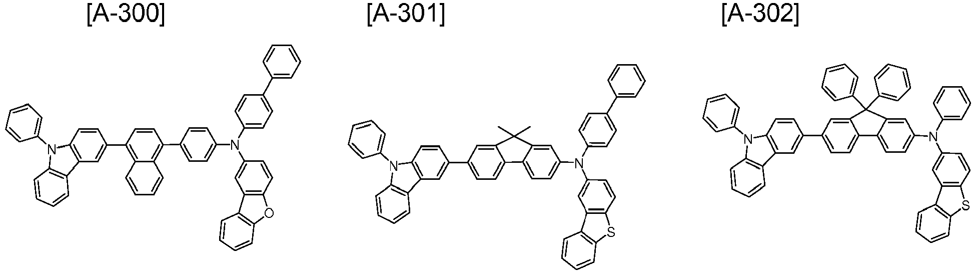

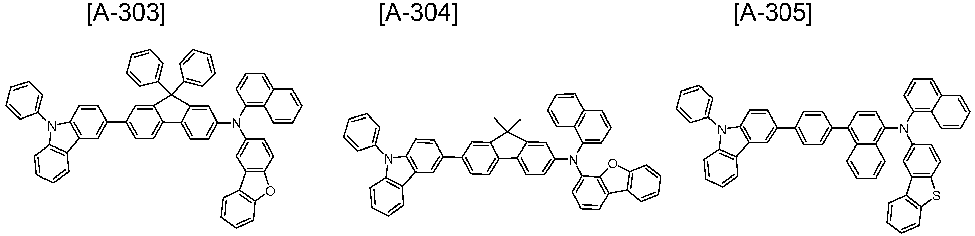

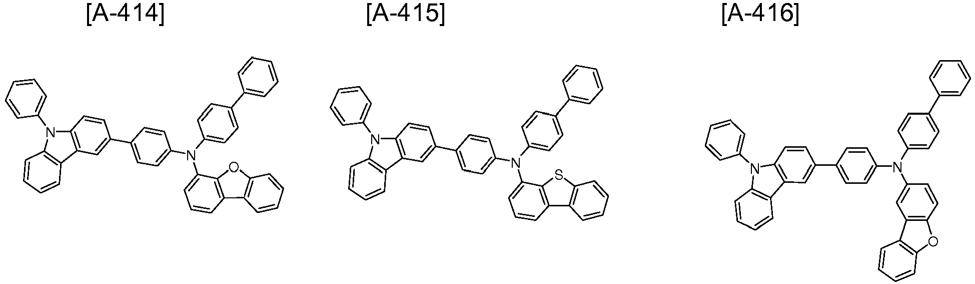

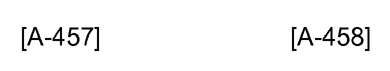

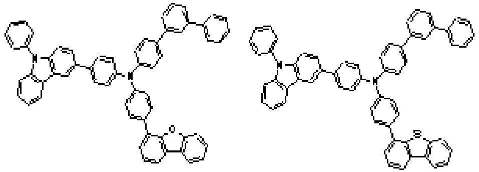

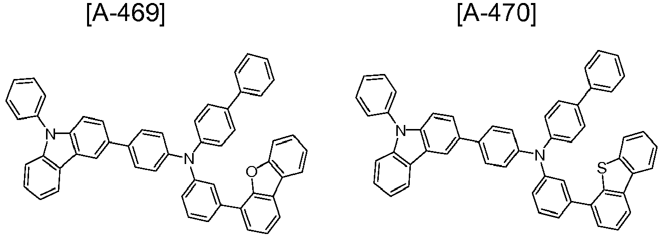

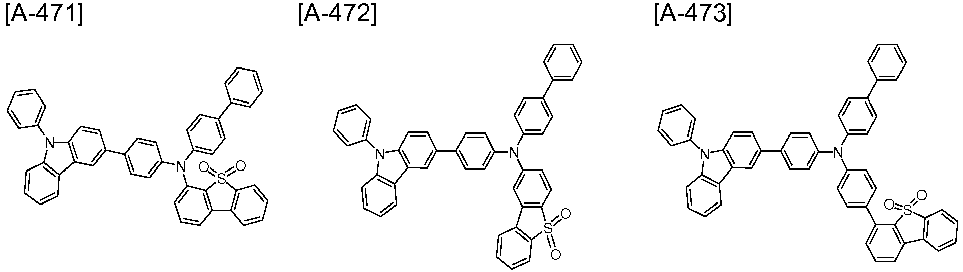

The compound for an optoelectronic device according to one embodiment may include at least one of the compounds represented by the following Chemical Formulae A-417 to A-456, or A-459 to A-468. In the following structure, electro-optical characteristics and thin film characteristics for maximizing the performance of the material for an optoelectronic device may be finely adjusted while maintaining basic characteristics of the compound.

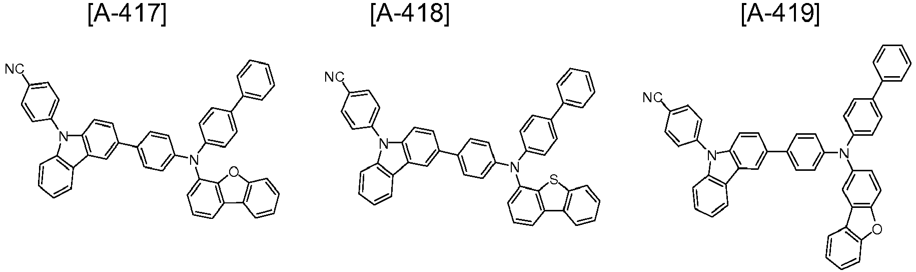

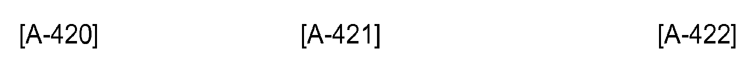

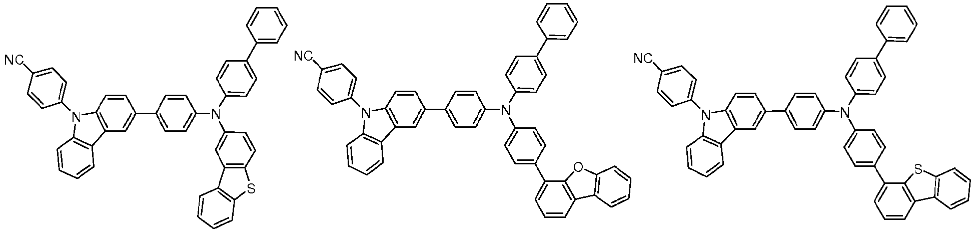

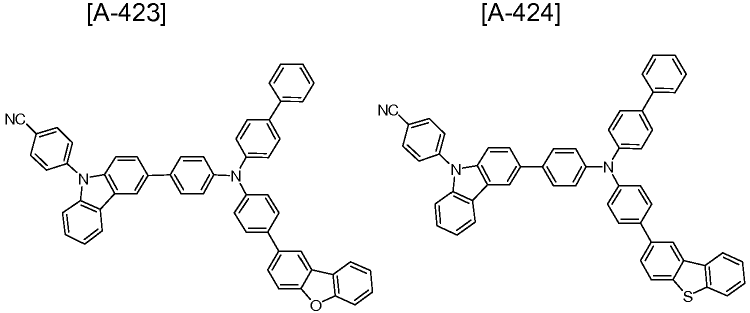

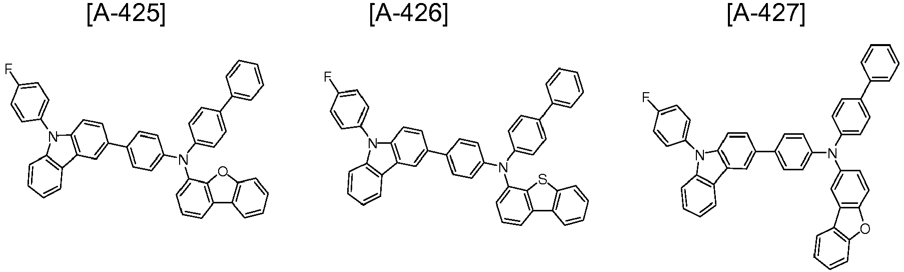

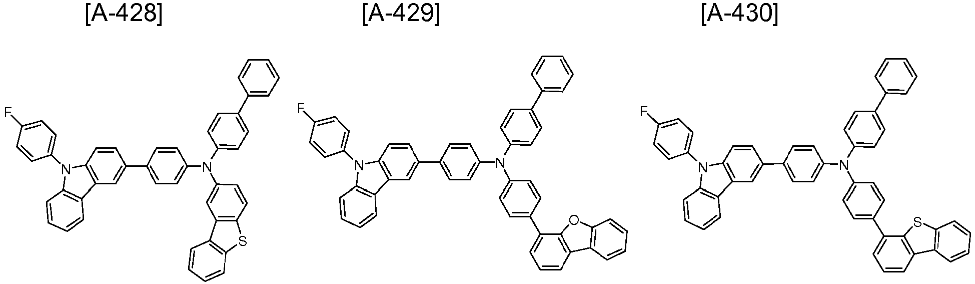

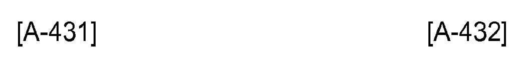

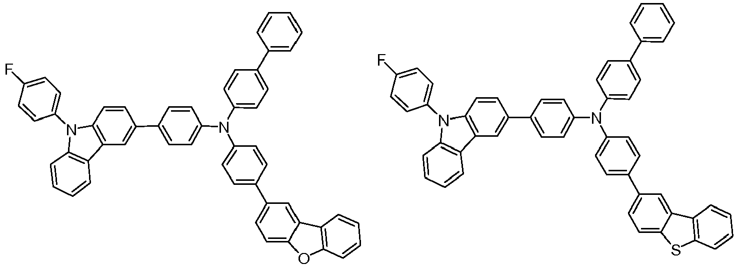

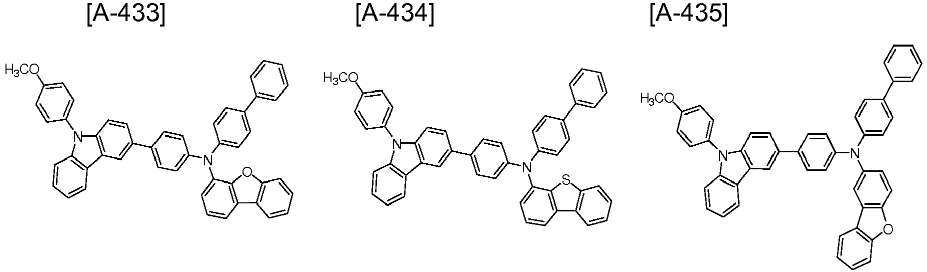

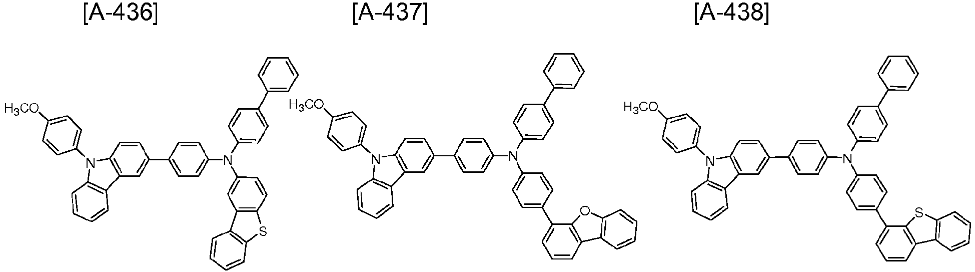

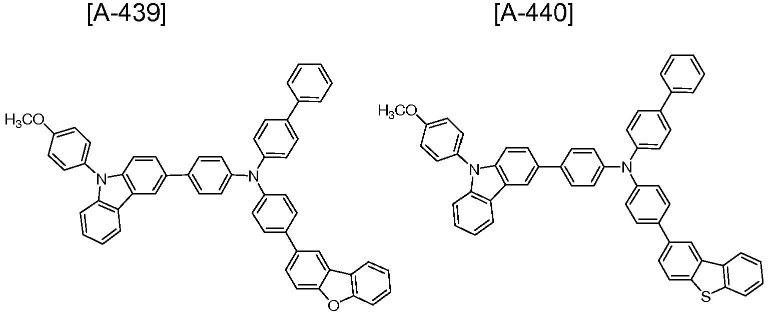

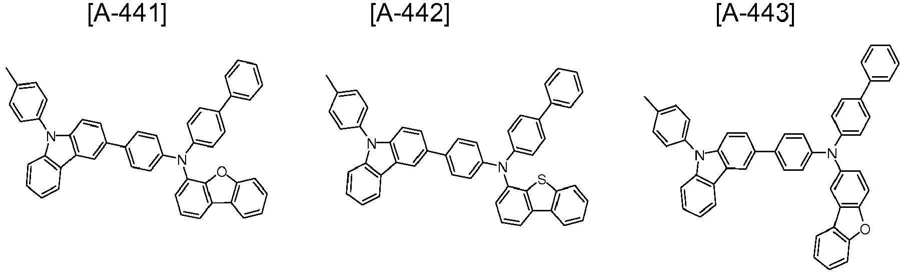

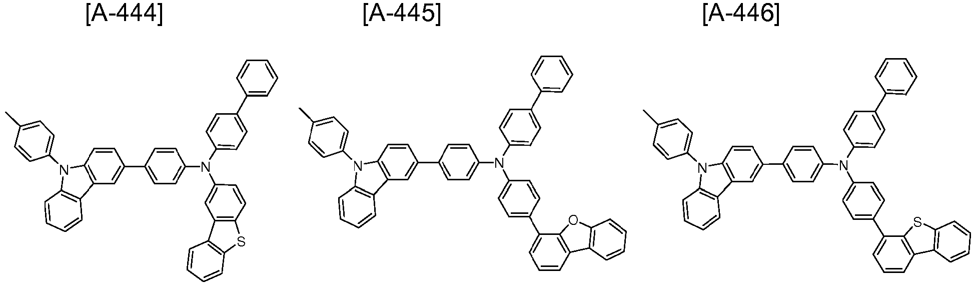

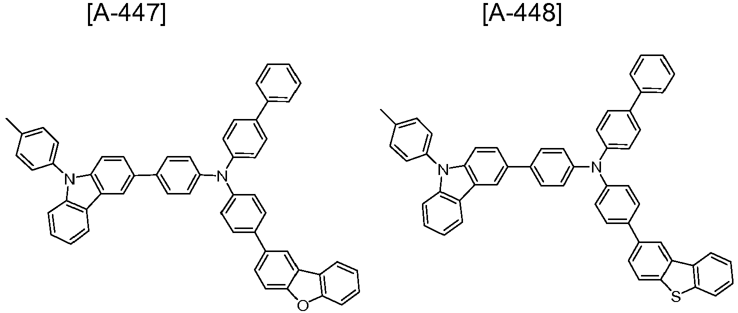

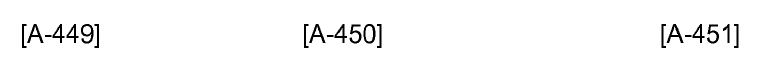

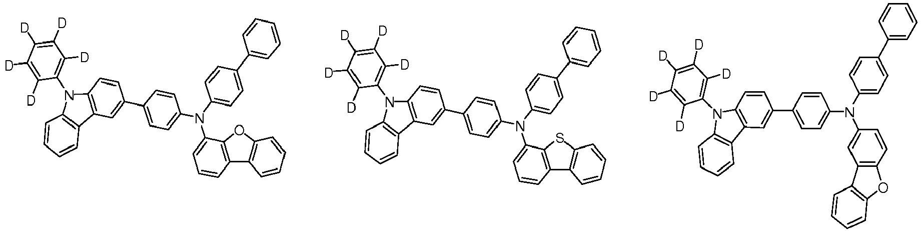

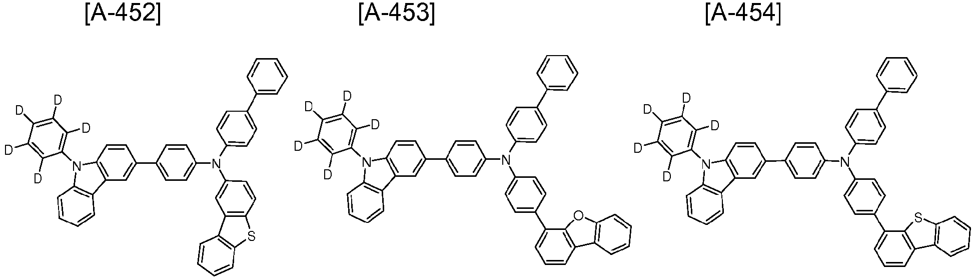

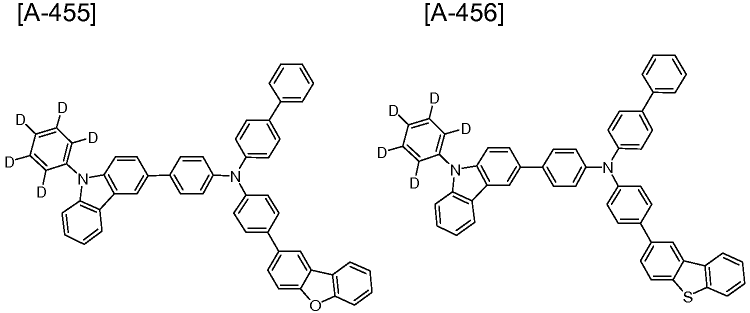

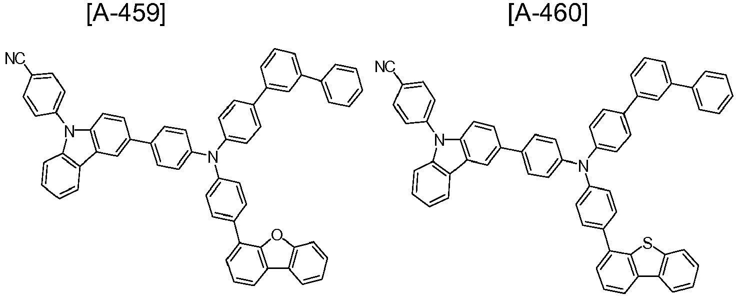

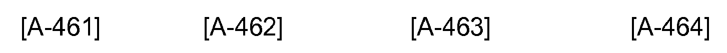

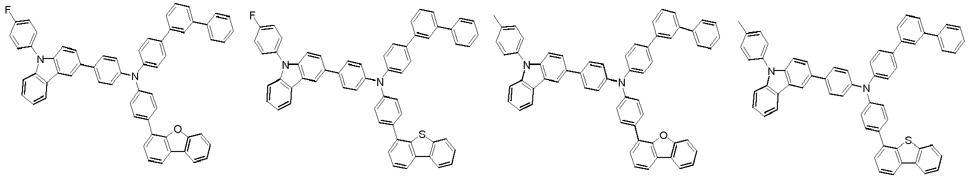

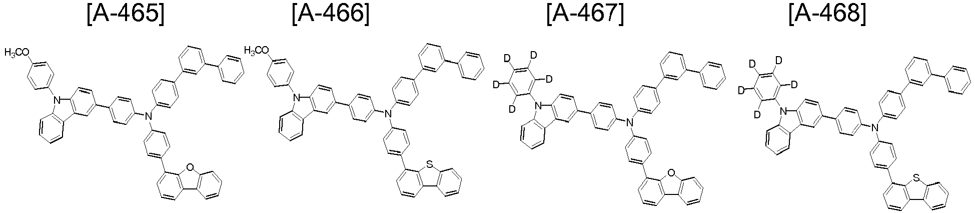

The compound for an optoelectronic device according to one embodiment may include at least one of the compounds represented by the following Chemical Formulae A-324 to A-395. In this structure, since dibenzofuran having a hole transporting property and dibenzothiophene are asymmetrically bound to a tertiary arylamine structure, an excellent hole transporting property and thin film stability may be realized.

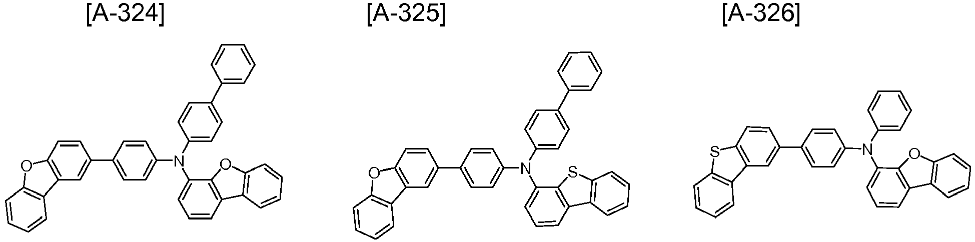

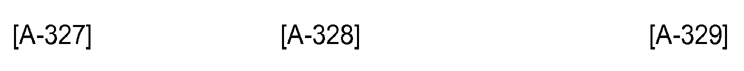

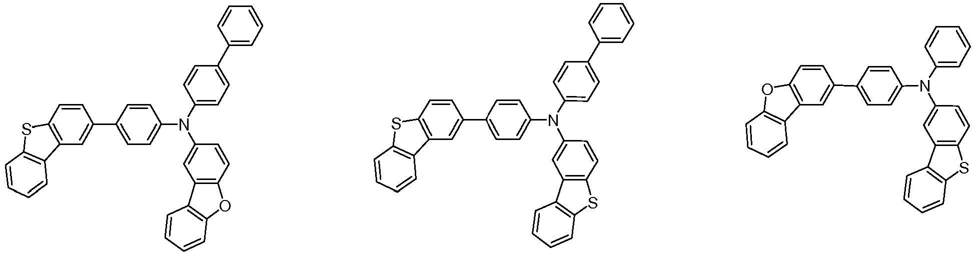

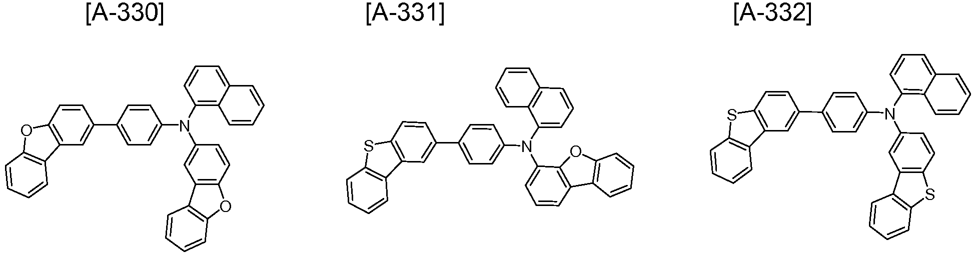

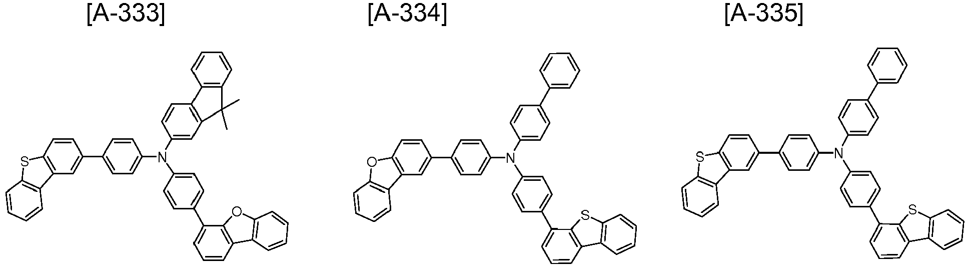

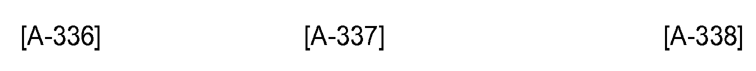

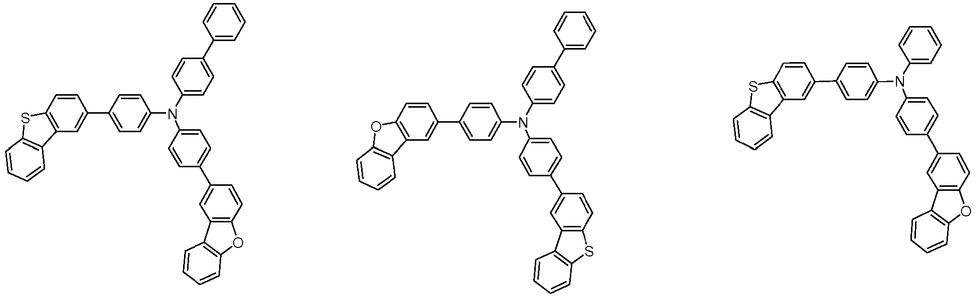

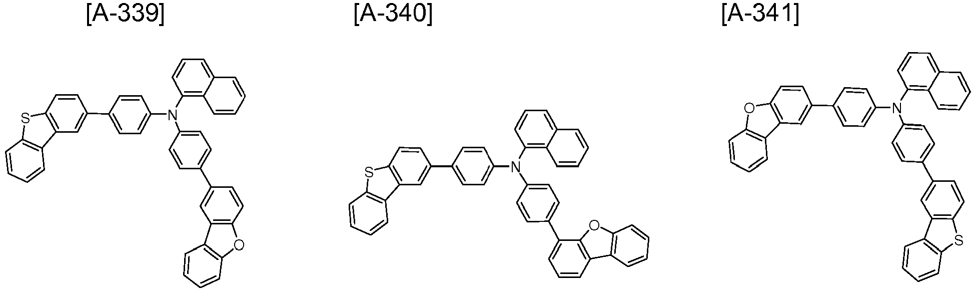

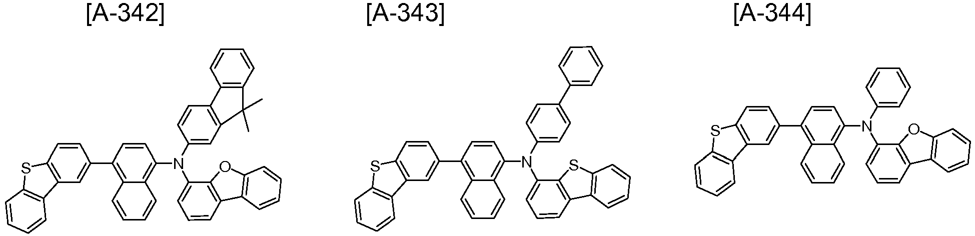

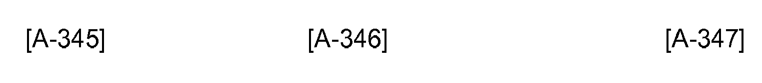

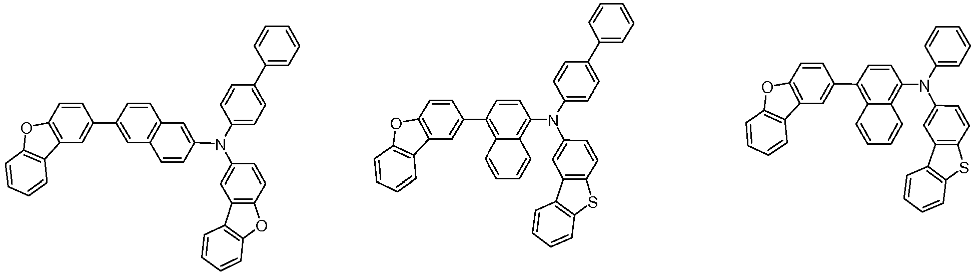

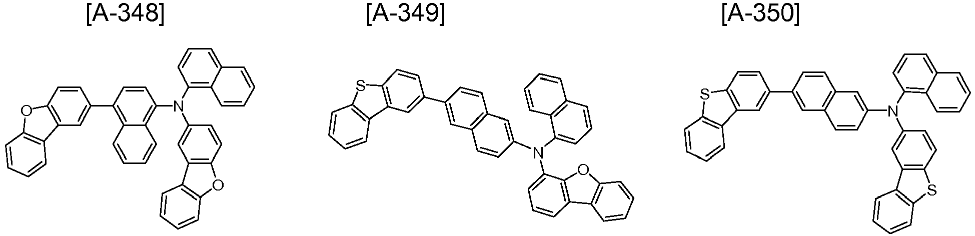

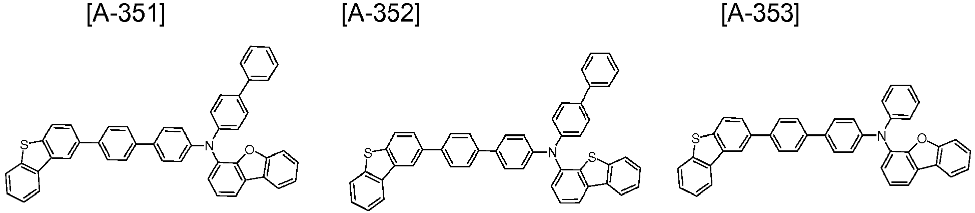

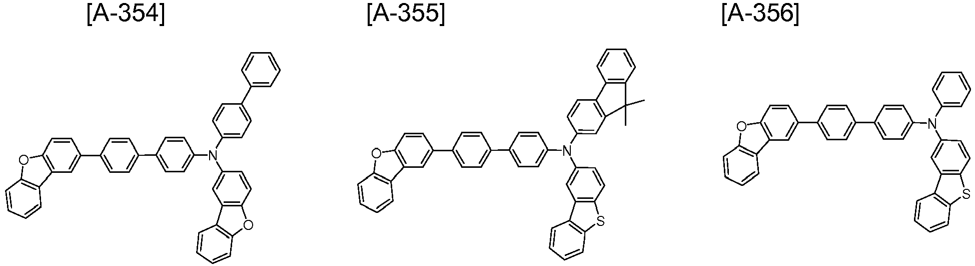

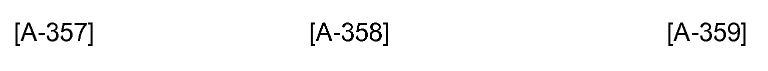

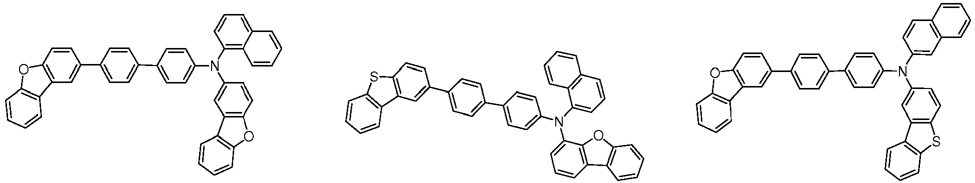

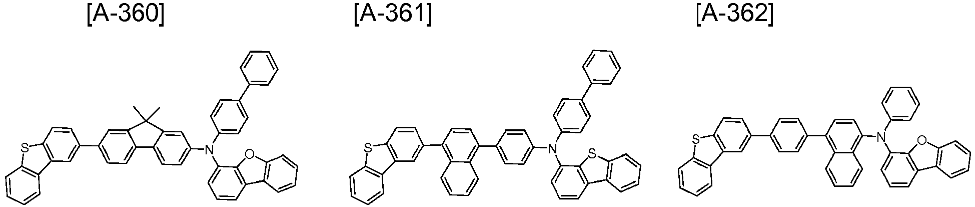

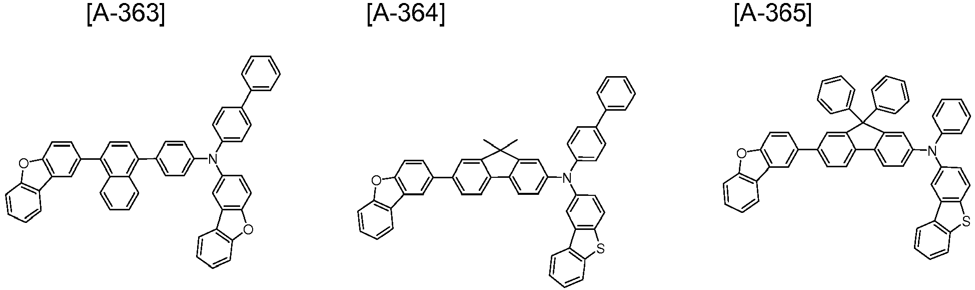

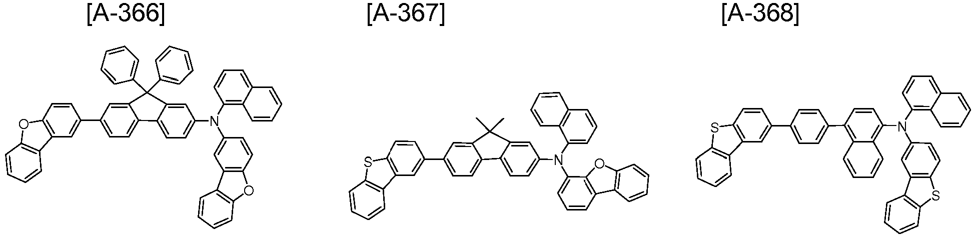

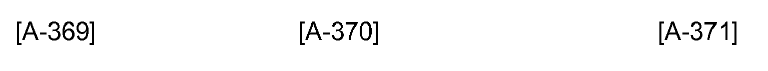

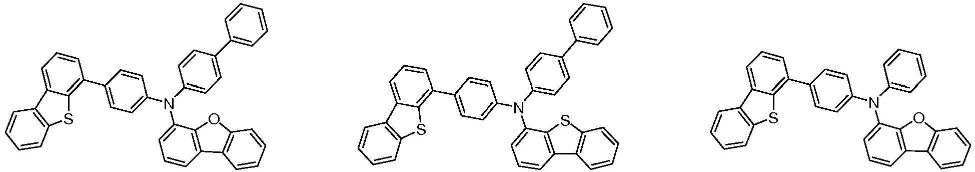

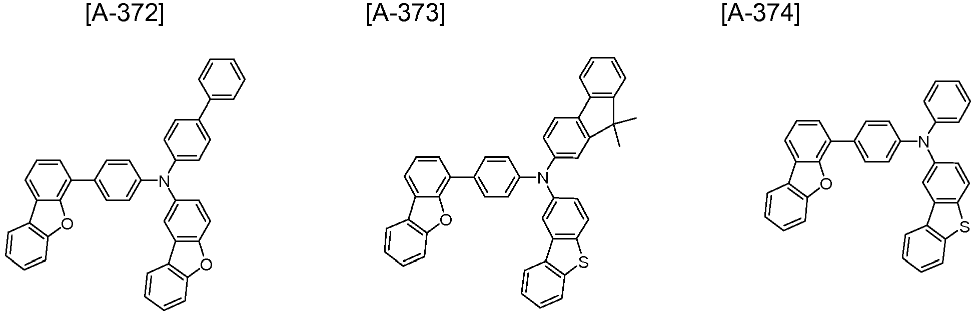

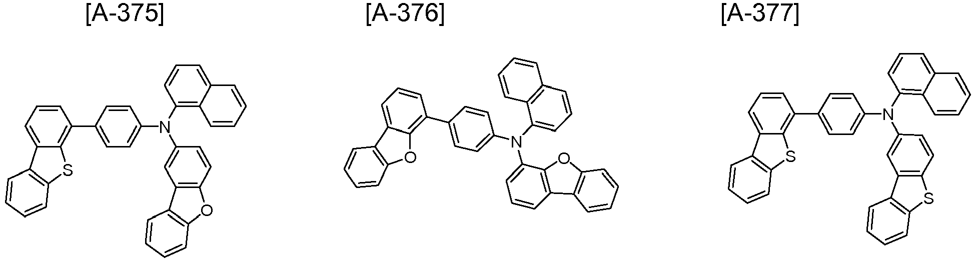

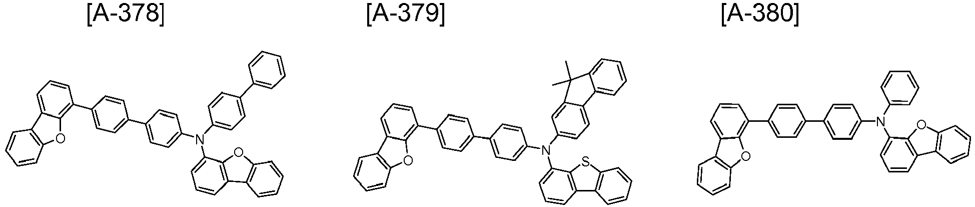

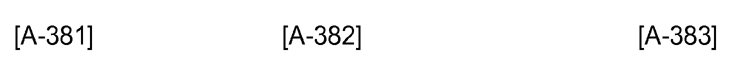

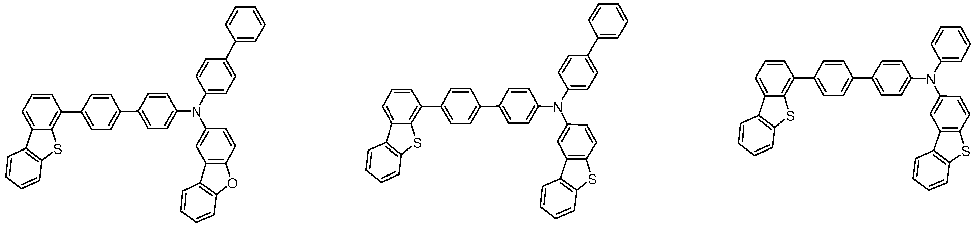

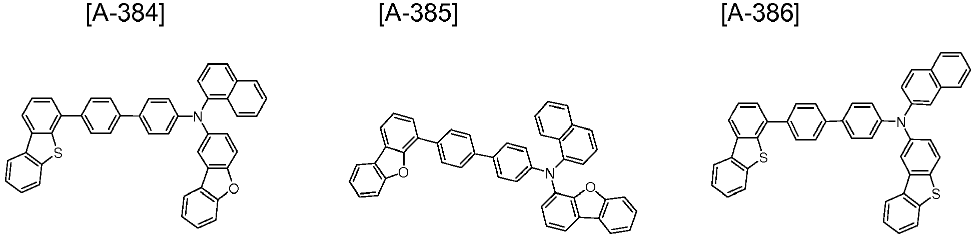

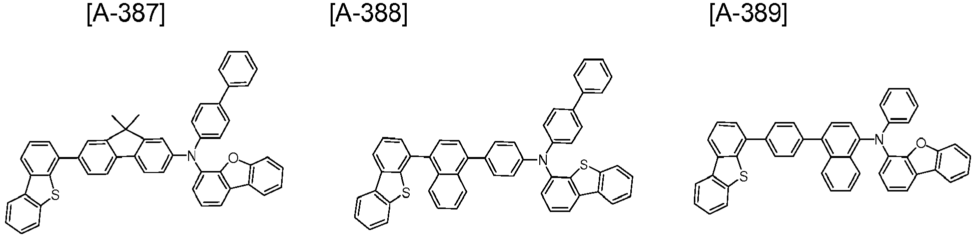

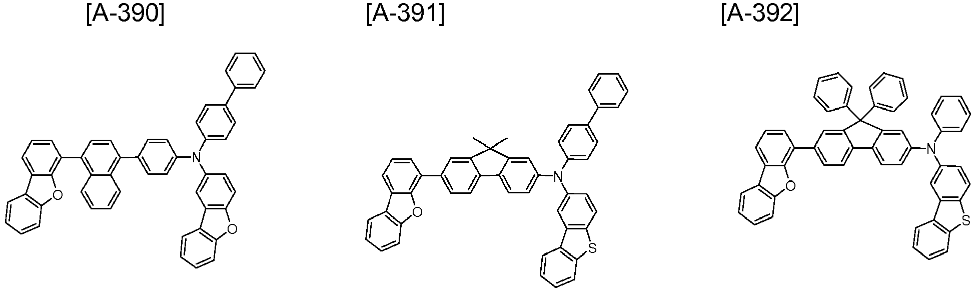

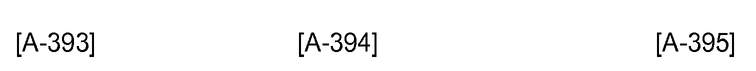

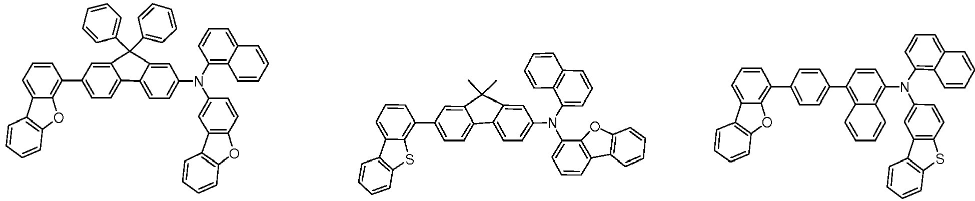

The compound for an optoelectronic device according to one embodiment may include at least one of the compounds represented by the following Chemical Formulae A-306 to A-323. In the following structure, dibenzofuran having a hole transporting property or dibenzothiophene is asymmetrically bound to a carbazole structure to form a tertiary arylamine and includes a hetero aromatic ring group as an electron acceptor, and therefore the structure shows asymmetric bipolar characteristics in its molecular structure. High efficiency may be realized when it is used as a phosphorescent host material and a hole blocking layer material.

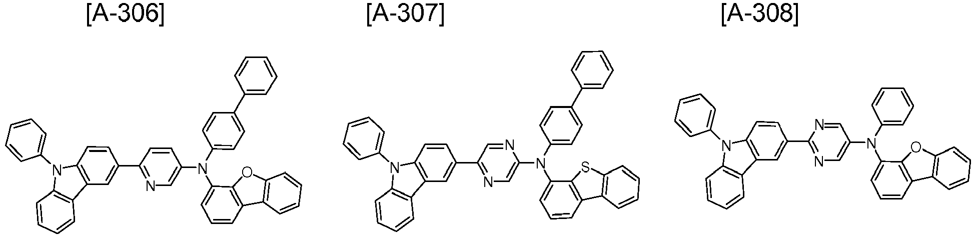

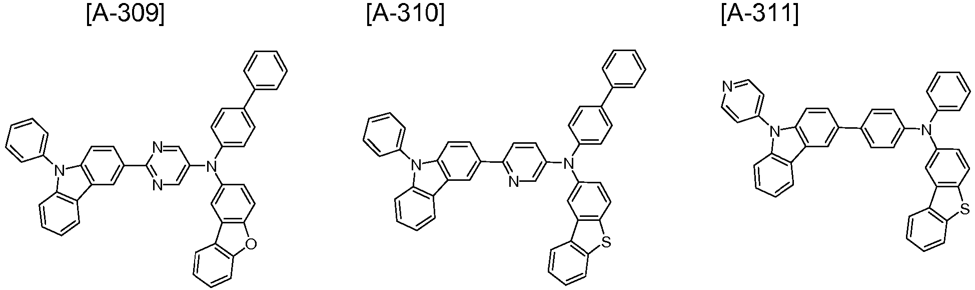

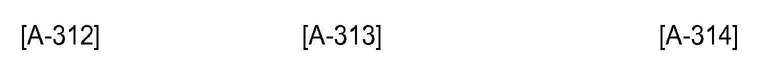

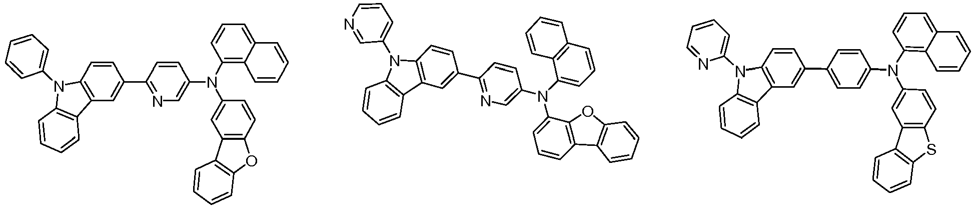

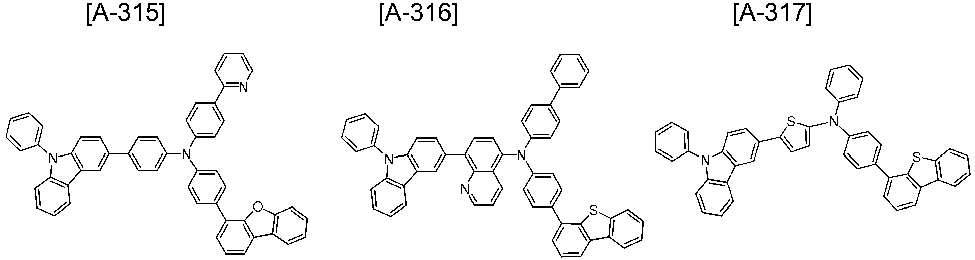

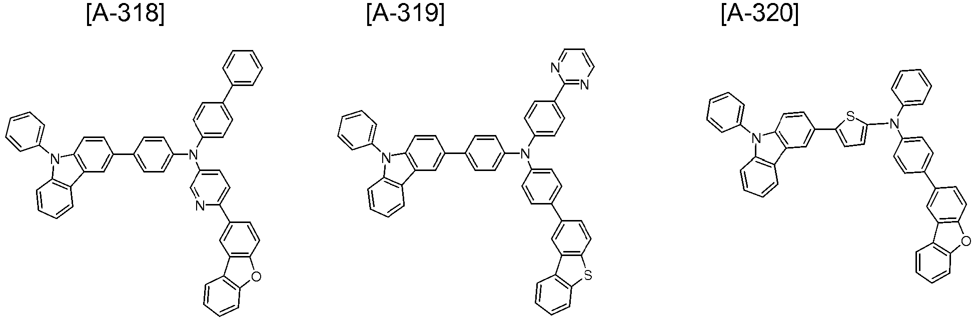

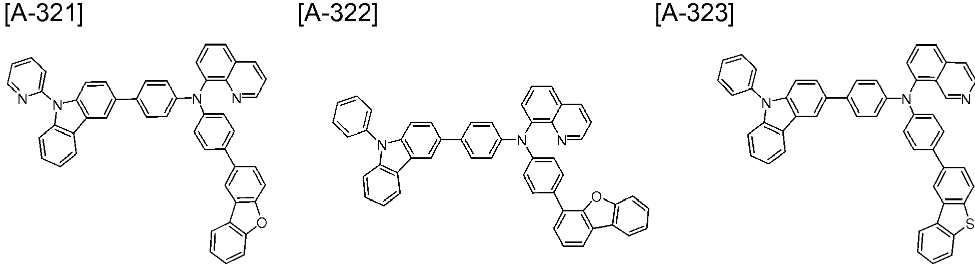

The compound for an optoelectronic device according to one embodiment may include at least one of the compounds represented by the following Chemical Formulae A-396 to A-413. In the following structure, dibenzofuran having a hole transporting property or dibenzothiophene is asymmetrically bound to a carbazole structure to form a tertiary arylamine and includes a hetero aromatic ring group as an electron acceptor, and therefore the structure shows asymmetric bipolar characteristics in its molecular structure. High efficiency may be realized when it is used to be a phosphorescent host material and a hole blocking layer material.

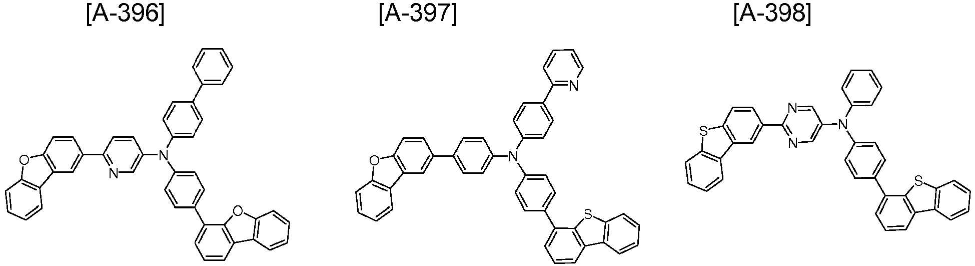

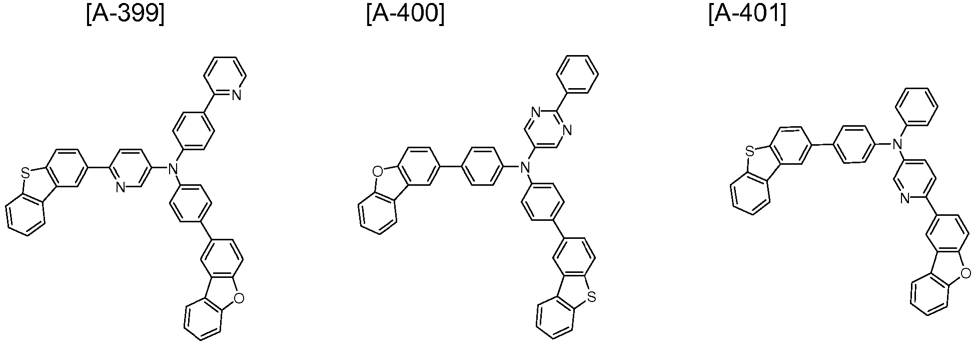

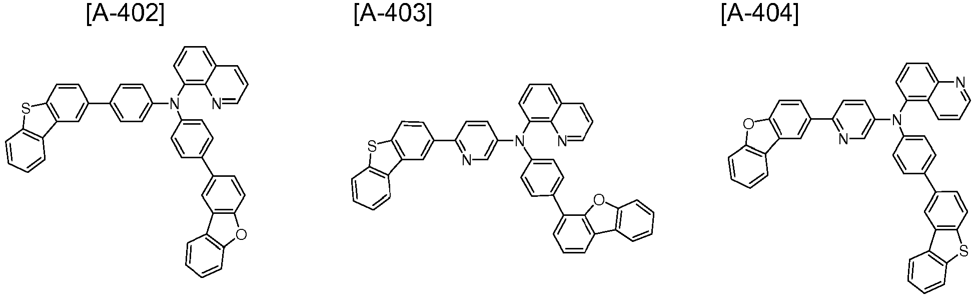

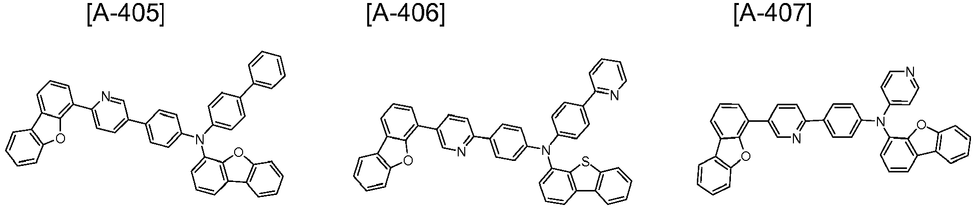

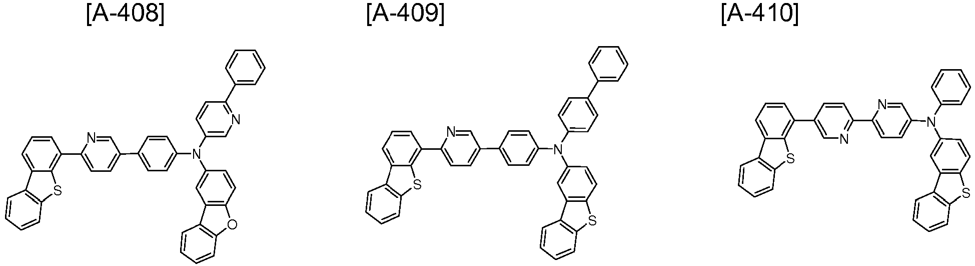

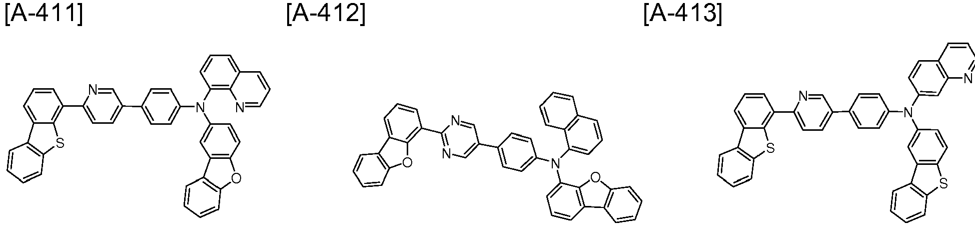

When the compound for an optoelectronic device is applied to an electron blocking layer and a hole transport layer (HTL), its electron blocking properties may be reduced due to a functional group having an electron characteristic in a molecule. Therefore, in order to use the compound as an electron blocking layer, it is preferable that the compound doesn't include a functional group having an electron characteristic. Examples of the functional group having an electron characteristic are benzoimidazole, pyridine, pyrazine, pyrimidine, triazine, quinoline, isoquinoline, or the like. However, the explainations as above are limited to when the compound is used as an electron blocking layer or a hole transport layer (HTL) (or a hole injection layer (HIL)).

When the compound is required to have electron-transporting and hole-transporting properties, a light emitting diode may have improved life-span and reduced driving voltage by introducing the electron transport backbone.

According to the embodiment of the present invention, a compound for an optoelectronic device has a maximum light emitting wavelength ranging from 320 to 500nm and triplet excitation energy of 2.0eV or more (T1), and in particular, ranging from 2.0 to 4.0eV. When it has this high excitation energy, it can transport a charge to a dopant well and improve luminous efficiency of the dopant, and can also decrease the driving voltage by freely regulating HOMO and LUMO energy levels. Accordingly, it can be usefully applied as a host material or a charge-transporting material.

The compound for an optoelectronic device may also be used as a nonlinear optical material, an electrode material, a chromic material, and as a material applicable to an optical switch, a sensor, a module, a waveguide, an organic transistor, a laser, an optical absorber, a dielectric material, and a membrane due to its optical and electrical properties.

The compound for an optoelectronic device including the above compound has a glass transition temperature of 90°C or higher and a thermal decomposition temperature of 400°C or higher, so as to improve thermal stability. Thereby, it is possible to produce an organic photoelectric device having high efficiency.

The compound for an optoelectronic device including the above compound may play a role of emitting light or injecting and/or transporting electrons. In other words, the compound for an optoelectronic device may be used as a phosphorescent or fluorescent host material, a blue light emitting dopant material, or an electron transporting material.

Since the compound for an optoelectronic device according to one embodiment is used for an organic thin layer, it may improve the life-span characteristic, efficiency characteristic, electrochemical stability, and thermal stability of an organic photoelectric device, and decrease the driving voltage.

The optoelectronic device may include an organic photoelectronic device, an organic light emitting diode, an organic solar cell, an organic transistor, an organic photosensitive drum, an organic memory device, or the like. For example, the compound for an optoelectronic device according to one embodiment may be included in an electrode or an electrode buffer layer in the organic solar cell to improve the quantum efficiency, and it may be used as an electrode material for a gate, a source-drain electrode, or the like in the organic transistor.

Hereinafter, an organic light emitting diode will be described in detail.

According to another embodiment of the present invention, an organic light emitting doiode including an anode, a cathode, and at least one or more organic thin layer between the anode and the cathode is provided, and at least one of the organic thin layers may include the compound for an optoelectronic device of the present invention.

The organic thin layer that may include the compound for an optoelectronic device may include a layer selected from the group consisting of an emission layer, a hole transport layer (HTL), a hole injection layer (HIL), an electron transport layer (ETL), an electron injection layer (EIL), a hole blocking film, and a combination thereof. The at least one layer includes the compound for an optoelectronic device according to one embodiment. Particularly, the compound for an optoelectronic device according to one embodiment may be included in a hole transport layer (HTL) or a hole injection layer (HIL). In addition, when the compound for an optoelectronic device is included in the emission layer, the compound for an optoelectronic device may be included as a phosphorescent or fluorescent host, and particularly, as a fluorescent blue dopant material.

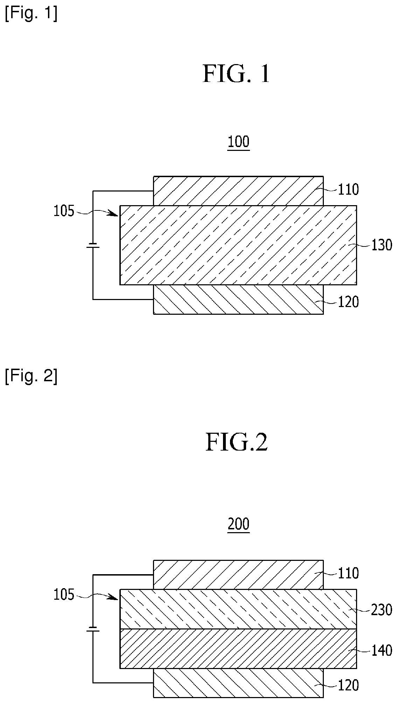

FIGS. 1 to 5 are cross-sectional views showing an organic photoelectric device including the compound for an optoelectronic device according to one embodiment of the present invention.

Referring to FIGS. 1 to 5, organic photoelectric devices 100, 200, 300, 400, and 500 according to one embodiment include at least one organic thin layer 105 interposed between an anode 120 and a cathode 110.

The anode 120 includes an anode material laving a large work function to help hole injection into an organic thin layer. The anode material includes: a metal such as nickel, platinum, vanadium, chromium, copper, zinc, and gold, or alloys thereof; a metal oxide such as zinc oxide, indium oxide, indium tin oxide (ITO), and indium zinc oxide (IZO); a combined metal and oxide such as ZnO:AI or SnO₂:Sb; or a conductive polymer such as poly(3-methylthiophene), poly[3,4-(ethylene-1,2-dioxy)thiophene] (PEDT), polypyrrole, and polyaniline, but is not limited thereto. It is preferable to include a transparent electrode including indium tin oxide (ITO) as an anode.

The cathode 110 includes a cathode material having a small work function to help electron injection into an organic thin layer. The cathode material includes: a metal such as magnesium, calcium, sodium, potassium, titanium, indium, yttrium, lithium, gadolinium, aluminum, silver, tin, and lead, or alloys thereof; or a multi-layered material such as LiF/Al, Liq/Al, LiO₂/Al, LiF/Ca, LiF/Al, and BaF₂/Ca, but is not limited thereto. It is preferable to include a metal electrode including aluminum as a cathode.

Referring to FIG. 1, the organic photoelectric device 100 includes an organic thin layer 105 including only an emission layer 130.

Referring to FIG. 2, a double-layered organic photoelectric device 200 includes an organic thin layer 105 including an emission layer 230 including an electron transport layer (ETL), and a hole transport layer (HTL) 140. The emission layer 130 also functions as an electron transport layer (ETL), and the hole transport layer (HTL) 140 layer has an excellent binding property with a transparent electrode such as ITO or an excellent hole transporting property.

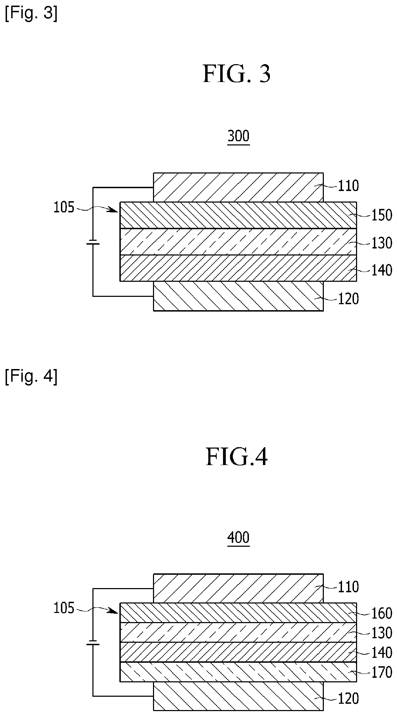

Referring to FIG. 3, a three-layered organic photoelectric device 300 includes an organic thin layer 105 including an electron transport layer (ETL) 150, an emission layer 130, and a hole transport layer (HTL) 140. The emission layer 130 is independently installed, and layers having an excellent electron transporting property or an excellent hole transporting property are separately stacked.

As shown in FIG. 4, a four-layered organic photoelectric device 400 includes an organic thin layer 105 including an electron injection layer (EIL) 160, an emission layer 130, a hole transport layer (HTL) 140, and a hole injection layer (HIL) 170 for binding with the cathode of ITO.

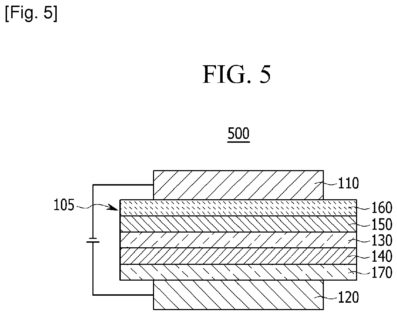

As shown in FIG. 5, a five layered organic photoelectric device 500 includes an organic thin layer 105 including an electron transport layer (ETL) 150, an emission layer 130, a hole transport layer (HTL) 140, and a hole injection layer (HIL) 170, and further includes an electron injection layer (EIL) 160 to achieve a low voltage.

In FIG. 1 to FIG. 5, the organic thin layer 105 including at least one selected from the group consisting of an electron transport layer (ETL) 150, an electron injection layer (EIL) 160, an emission layer 130 or 230, a hole transport layer (HTL) 140, a hole injection layer (HIL) 170, and combinations thereof includes a compound for an optoelectronic device. The material for the organic photoelectric device may be used for an electron transport layer (ETL) 150 including the electron transport layer (ETL) 150 or electron injection layer (EIL) 160. When it is used for the electron transport layer (ETL), it is possible to provide an organic photoelectric device having a simpler structure because it does not require an additional hole blocking layer (not shown).

Furthermore, when the compound for an optoelectronic device is included in the emission layer 130 and 230, the material for the organic photoelectric device may be included as a phosphorescent or fluorescent host or a fluorescent blue dopant.

The organic photoelectric device may be fabricated by: forming an anode on a substrate; forming an organic thin layer in accordance with a dry coating method such as evaporation, sputtering, plasma plating, and ion plating or a wet coating method such as spin coating, dipping, and flow coating; and providing a cathode thereon.

Another embodiment of the present invention provides a display device including the organic photoelectric device according to the above embodiment.

Hereinafter, the embodiments are illustrated in more detail with reference to examples. However, the following are exemplary embodiments and are not limiting.

### Preparation of compound for optoelectronic device

### Synthesizing intermediate porduct

### Synthesis of intermediate product, M-1

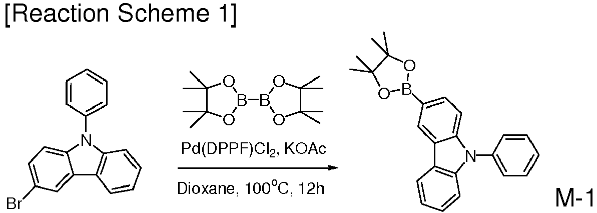

50g (155.18mmol) of 3-bromo-9-phenyl-9H-carbazole, 3.41 g (4.65mmol) of Pd(dppf)Cl₂, 51.32g (201.8mmol) of bis(pinacolato)diboron, and 45.8g (465.5mmol) of potassium acetate were dissolved in 520ml of 1,4-dioxane. The reactants were refluxed and agitated under a nitrogen atmosphere for 12 hours and extracted 3 times with dichloromethane and distilled water. The extract was dried with magnesium sulfite and filtered, and the filtrate was concentrated under reduced pressure. The product was purified with n-hexane/dichloromethane mixed at a volume ratio of 7:3 through silica gel column chromatography, and then 43g of a white solid intermediate M-1 was acquired as a desired compound (yield: 75%).

LC-Mass (theoretical mass: 369.19g/mol, measured mass: M+1 = 370g/mol)

### Synthesis of intermediate product, M-2

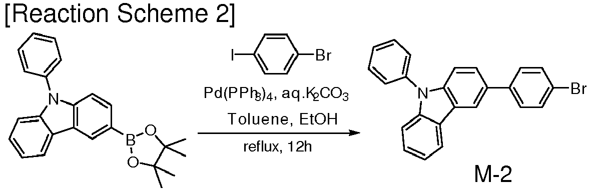

40g (108.3mmol) of the intermediate M-1, 30.6g (108.3mmol) of 1-bromo-4-iodobenzene, and 1.25g (1.08mmol) of tetrakistriphenylphosphine palladium were added to a flask and dissolved in 270ml of toluene and 135mL of ethanol under a nitrogen atmosphere.

Then, 135ml of an aqueous solution including 31.9g (58.9mmol) of potassium carbonate was added to the reactants and then refluxed and agitated for 12 hours. After the reaction, the reactants were extracted with ethylacetate. The extract was dried with magnesium sulfite and filtered. Then, the filtrate was concentrated under reduced pressure. The product was purified with n-hexane/dichloromethane mixed in a volume ratio of 7:3 through silica gel column chromatography, and then 35g of a white solid intermediate M-2 was acquired as a desired compound (yield: 75%).

LC-Mass (theoretical mass: 398.29g/mol, measured mass: M+1 = 399g/mol, M+3 = 401g/mol)

### Synthesis of intermediate product, M-3

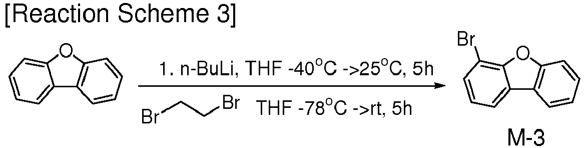

10g (59.5mmol) of a dibenzofuranyl group was added to a two neck round-bottomed flask that was dried under vacuum, and 119mL of anhydrous tetrahydrofuran was added under a nitrogen atmosphere followed by dissolving. Then, the reactants were cooled down to -40°C and agitated.

Then, 26mL of 2.5M n-butyl lithium (in hexane, 65.5mmol) was slowly added to the reactants and the resultant was agitated for 5 hours at room temperature under a nitrogen atmosphere. The reactants were cooled down to -78°C, and 22.4g (119mmol) of 1,2-dibromoethane that was dissolved in 10mL anhydrous tetrahydrofuran was slowly added and then agitated for 5 hours at room temperature.

After the reaction, the solution was concentrated under reduced pressure to remove the solvent. Then the reactants were extracted with distilled water and dichloromethane, and the extract was dried with magnesium sulfite and filtered. The filtrate was concentrated under reduced pressure. The reactants were recrystallized in n-hexane and then 11 g of a white solid intermediate M-3 was acquired as a desired compound (yield: 75%).

GC-Mass (theoretical mass: 245.97g/mol, measured mass: M = 246g/mol, M+2 = 248g/mol)

### Synthesis of intermediate product, M-4

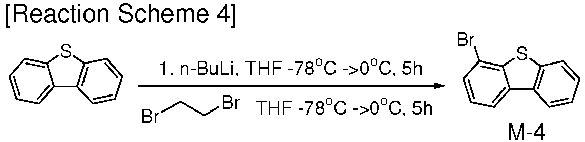

10g (54.3mmol) of dibencothiophene that was dried under a vacuum condition was added to a two neck round-bottomed flask and dissolved with 120mL of anhydrous tetrahydrofuran under a nitrogen atmosphere. Then, the reactant was cooled down to -40 °C and agitated.

Then, 24mL of 2.5M n-butyllithium (in hexane, 59.7mmol) was slowly added to the reactants and agitated for 5 hours at room temperature under a nitrogen atmosphere. The reactants were cooled down to -78 °C, and 20.4g (108.6mmol) of 1,2-dibromoethane that was dissolved in 10mL anhydrous tetrahydrofuran was slowly added and then agitated for 5 hours at room temperature. After the reaction, the solution was concentrated under reduced pressure to remove the solvent. Then the reactant was extracted with distilled water and dichloromethane, and the extract was dried with magnesium sulfite and filtered. The filtrate was concentrated under reduced pressure. The reactant was recrystallized in n-hexane, and then 11 g of a white solid intermediate M-4 was acquired as a desired compound (yield: 77%).

GC-Mass (theoretical mass: 261.95g/mol, measured mass: M = 262g/mol, M+2 = 264g/mol)

### Synthesis of intermediate product, M-5

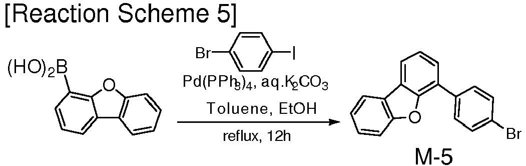

20g (94.4mmol) of 4-dibenzofuranboronic acid, 28g (99.2mmol) of 1-bromo-4-iodobenzene, and 1.08g (0.94mmol) of tetrakistriphenylphosphinepalladium were added to a flask and dissolved in 240ml of toluene and 120mL of ethanol under a nitrogen atmosphere. Then, 120ml of an aqueous solution including 28g (188.8mmol) of potassium carbonate was added to the reactant and then refluxed and agitated for 12 hours. After the reaction, the reactant was extracted with ethylacetate. The extract was dried with magnesium sulfite and filtered. Then, the filtrate was concentrated under reduced pressure. The product was purified with n-hexane/dichloromethane mixed in a volume ratio of 9:1 through silica gel column chromatography, and then 27g of a white solid intermediate M-5 was acquired as a desired compound (yield: 89%).

LC-Mass (theoretical mass: 322.00g/mol, measured mass: M+1 = 323g/mol, M+3 = 325g/mol)

### Synthesis of intermediate product, M-6

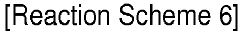

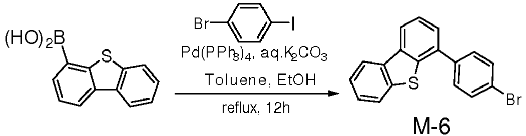

20g (87.69mmol) of 4-dibenzothiopheneboronic acid, 27.3g (96.46mmol) of 1-bromo-4-iodobenzene, and 1.01 g (0.88mmol) of tetrakistriphenylphosphinepalladium were added to a flask and dissolved in 220ml of toluene and 110mL of ethanol under a nitrogen atmosphere. Then, 110ml of an aqueous solution including 25.8g (175.4mmol) of potassium carbonate was added to the reactant and then refluxed and agitated for 12 hours. After the reaction, the reactant was extracted with ethylacetate. The extract was dried with magnesium sulfite and filtered. Then, the filtrate was concentrated under reduced pressure. The product was purified with n-hexane/dichloromethane mixed in a volume ratio of 9:1 through silica gel column chromatography, and then 25g of a white solid intermediate M-6 was acquired as a desired compound (yield: 83%).

LC-Mass (theoretical mass: 337.98g/mol, measured mass: M+1 = 338g/mol, M+3 = 340g/mol)

### Synthesis of intermediate product, M-7

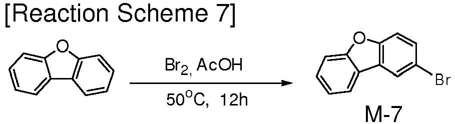

30g (178.4mmol) of a dibenzofuranyl group was added to a round-bottomed flask and dissolved in 270g of acetic acid. Then, 29g (181.5mmol) of bromine that was dissolved in 6g of acetic acid was slowly added to the reactant at 50°C for 4 hours. The reactant was further agitated at 50°C for 8 hours and cooled down, and then the solution was added to distilled water. The orange solid was dissolved in dichloromethane and washed with a sodium thiosulfite aqueous solution, and then the organic layer was dried with magnesium sulfite and filtered. The filtrate was concentrated under reduced pressure. The product was recrystallized in dichloromethane/n-hexane, and then 10.1g of a white solid intermediate M-7 was acquired as a desired compound (yield: 23%).

GC-Mass (theoretical mass: 245.97g/mol, measured mass: M = 246g/mol, M+2 = 248g/mol)

### Synthesis of intermediate product, M-8

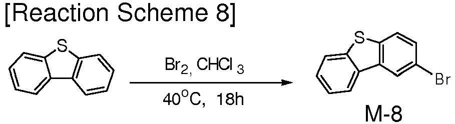

30g (162.8mmol) of a dibenzothiophenyl group was added to a round-bottomed flask and dissolved in 270g of acetic acid. Then, 29g (181.5mmol) of bromine that was dissolved in 6g of acetic acid was slowly added to the reactant for 4 hours. The reactant was further agitated at 40°C for 12 hours and cooled down, and then the solution was added to a sodium thiosulfite aqueous solution. The organic layer was dried with magnesium sulfite and filtered. Then the filtrate was concentrated under reduced pressure. The product was recrystallized with ethylacetate/n-hexane and then 15.4g of a white solid intermediate M-8 was acquired as a desired compound (yield: 36%).

GC-Mass (theoretical mass: 261.95g/mol, measured mass: M = 262g/mol, M+2 = 264g/mol)

### Synthesis of intermediate product, M-9

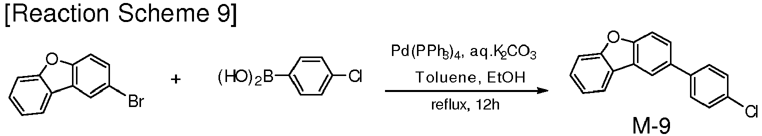

20g (127.9mmol) of 4-chlorophenylboronic acid, 30.0g (121.5mmol) of intermediate M-7, and 1.48g (1.28mmol) of tetrakistriphenylphosphinepalladium were added to a flask and dissolved in 320ml of toluene and 160mL of ethanol under a nitrogen atmosphere. Then, 160ml of an aqueous solution including 37.7g (255.8mmol) of potassium carbonate was added to the reactant and then refluxed and agitated for 12 hours. After the reaction, the reactant was extracted with ethylacetate. The extract was dried with magnesium sulfite and filtered. Then, the filtrate was concentrated under reduced pressure. The product was purified with n-hexane/dichloromethane mixed in a volume ratio of 9:1 through silica gel column chromatography, and then 28.1g of a white solid intermediate M-9 was acquired as a desired compound (yield: 83%).

LC-Mass (theoretical mass: 278.05g/mol, measured mass: M+1 = 279g/mol)

### Synthesis of intermediate product, M-10

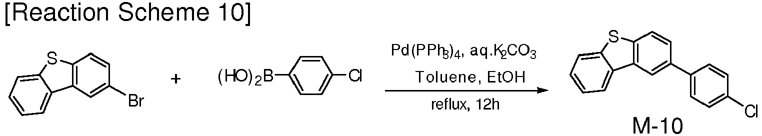

20g (127.9mmol) of 4-chlorophenylboronic acid, 32.0g (121.5mmol) of intermediate M-8, and 1.48g (1.28mmol) of tetrakistriphenylphosphinepalladium were added to a flask and dissolved in 320ml of toluene and 160mL of ethanol under a nitrogen atmosphere. Then, 160ml of an aqueous solution including 37.7g (255.8mmol) of potassium carbonate was added to the reactant and then refluxed and agitated for 12 hours. After the reaction, the reactant was extracted with ethylacetate. The extract was dried with magnesium sulfite and filtered. Then, the filtrate was concentrated under reduced pressure. The product was purified with n-hexane/dichloromethane mixed in a volume ratio of 9:1 through silica gel column chromatography, and then 30.4g of a white solid intermediate M-10 was acquired as a desired compound (yield: 85%).

LC-Mass (theoretical mass: 294.03 g/mol, measured mass: M+1 = 295 g/mol)

### Synthesis of intermediate product, M-11

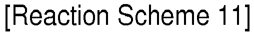

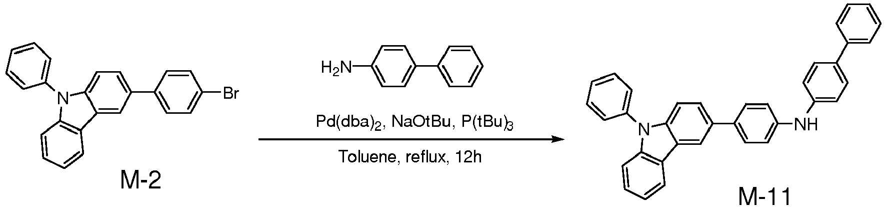

30g (75.3mmol) of intermediate M-2, 14.0g (82.83mmol) of 4-aminobiphenyl, 10.9g (113.0mmol) of sodium t-butoxide, and 0.46g (2.26mmol) of tri-tetra-butylphosphine were added to a flask and dissolved in 750ml of toluene, and 0.43g (0.753mmol) of Pd(dba)₂ was added, and was then refluxed and agitated for 12 hours under a nitrogen atmosphere. After the reaction, the reactant was extracted with ethylacetate and distilled water. The extract was dried with magnesium sulfite and filtered. Then, the filtrate was concentrated under reduced pressure. The product was purified with n-hexane/dichloromethane mixed in a volume ratio of 7:3 through silica gel column chromatography, and then 27.5 g of a white solid intermediate M-11 was acquired as a desired compound (yield: 75%).

LC-Mass (theoretical mass: 486.21 g/mol, measured mass: M+1 = 487g/mol)

### Synthesis of intermediate product, M-12

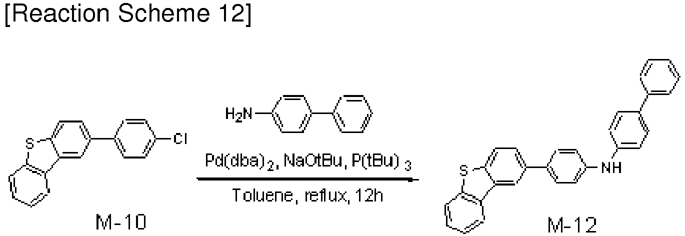

5g (17.0mmol) of intermediate M-10, 3.02g (17.85mmol) of 4-aminobiphenyl, 2.45g (25.5mmol) of sodium t-butoxide, and 0.10g (0.51mmol) of tri-tetra-butylphosphine were added to a flask and dissolved in 170ml of toluene, and 0.098g (0.17mmol) of Pd(dba)₂ was added and then refluxed and agitated for 12 hours under a nitrogen atmosphere. After the reaction, the reactant was extracted with ethylacetate. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 5.23g of a white solid intermediate M-12 was acquired as a desired compound (yield: 72%).

LC-Mass (theoretical mass: 427.14g/mol, measured mass: M+1 = 428g/mol)

### Synthesis of intermediate product, M-13

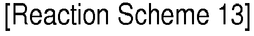

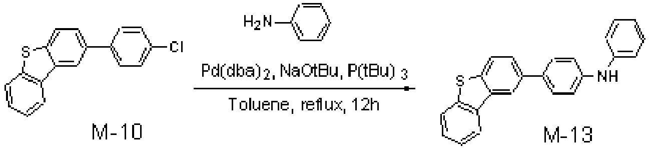

5g (17.0mmol) of intermediate M-10, 1.66g (17.85mmol) of aniline, 2.45g (25.5mmol) of sodium t-butoxide, and 0.10g (0.51mmol) of tri-tetra-butylphosphine were added to a flask and dissolved in 170ml of toluene, and 0.098g (0.17mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 4.66g of a white solid intermediate M-13 was acquired as a desired compound (yield: 78%).

LC-Mass (theoretical mass: 351.11g/mol, measured mass: M+1 = 352g/mol)

### Synthesis of intermediate product, M-14

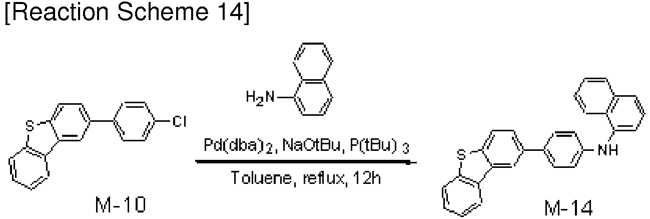

5g (17.0mmol) of intermediate M-10, 2.56g (17.85mmol) of 1-aminonaphthalene, 2.45g (25.5mmol) of sodium t-butoxide, and 0.10g (0.51mmol) of tri-tetra-butylphosphine were added to a flask and dissolved in 170ml of toluene, and 0.098g (0.17mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 4.98g of a white solid intermediate M-14 was acquired as a desired compound (yield: 73%).

LC-Mass (theoretical mass: 401.12g/mol, measured mass: M+1 = 402g/mol)

### Synthesis of intermediate product, M-15

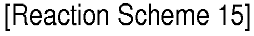

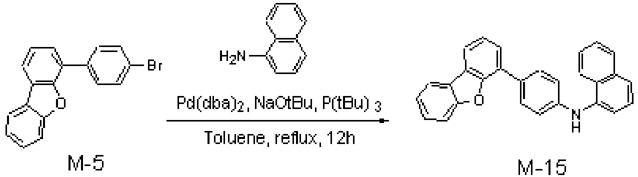

5.49g (17.0mmol) of intermediate M-5, 2.56g (17.85mmol) of 1-aminonaphthalene, 2.45g (25.5mmol) of sodium t-butoxide, and 0.10g (0.51mmol) of tri-tetra-butylphosphine were added to a flask and dissolved in 170ml of toluene, and 0.098g (0.17mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 5.05g of a white solid intermediate M-15 was acquired as a desired compound (yield: 77%).

LC-Mass (theoretical mass: 385.15g/mol, measured mass: M+1 = 386g/mol)

### Synthesis of intermediate product, M-16

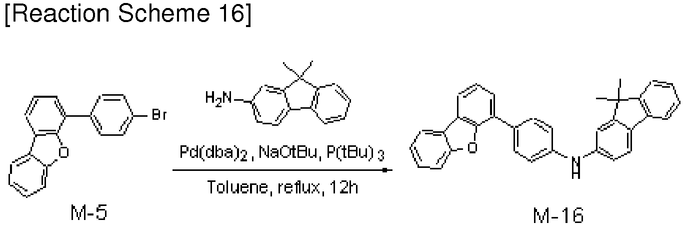

5.49g (17.0mmol) of intermediate M-5, 3.74g (17.85mmol) of (9,9-dimethyl-9H-fluorene-2-yl)amine, 2.45g (25.5mmol) of sodium t-butoxide, and 0.10g (0.51mmol) of tri-tetra-butylphosphine were added to a flask and dissolved in 170ml of toluene, and 0.098g (0.17mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 6.0g of a white solid intermediate M-16 was acquired as a desired compound (yield: 78%).

LC-Mass (theoretical mass: 451.19g/mol, measured mass: M+1 = 452g/mol)

### Synthesis of intermediate product, M-17

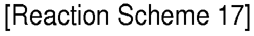

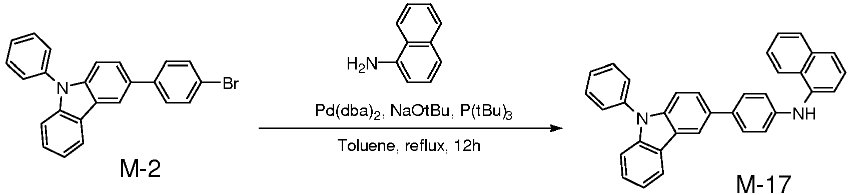

30g (75.3mmol) of intermediate M-2, 11.9g (82.83mmol) of 1-aminonaphthalene, 10.9g (113.0mmol) of sodium t-butoxide, and 0.46g (2.26mmol) of tri-tetra-butylphosphine were added to a flask and dissolved in 750ml of toluene, and 0.43g (0.753mmol) of Pd(dba)₂ was added and then refluxed and agitated for 12 hours under a nitrogen atmosphere. After the reaction, the reactant was extracted with ethylacetate. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 25.7g of a white solid intermediate M-17 was acquired as a desired compound (yield: 74%).

LC-Mass (theoretical mass: 460.19g/mol, measured mass: M+1 = 461 g/mol)

### Synthesis of intermediate product, M-18

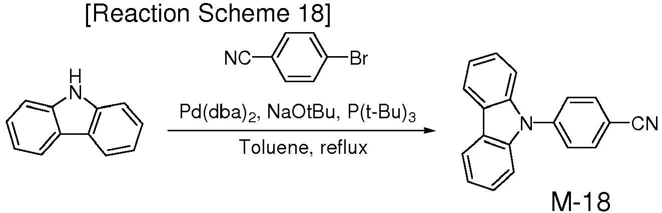

20g (119.6mmol) of carbazole, 23.9g (131.6mmol) of 4-bromobenzonitrile, 23g (239.2mmol) of sodium t-butoxide, and 1.45g (7.18mmol) of tri-tetra-butylphosphine were added to a flask and dissolved in 600ml of toluene, and 1.38g (2.39mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 22.8g of a white solid intermediate M-18 was acquired as a desired compound (yield: 71%).

LC-Mass (theoretical mass: 268.10g/mol, measured mass: M+1 = 269g/mol)

### Synthesis of intermediate product, M-19

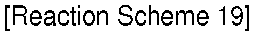

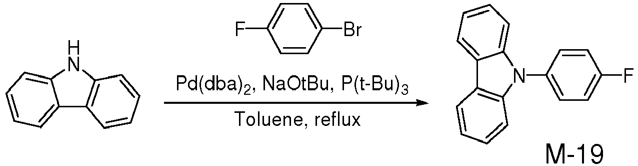

22.8g of a white solid intermediate M-19 was acquired as a desired compound (yield: 73%) in accordance with the same procedure as in the acquiring process of intermediate M-18, except that 1-bromo-4-fluorobenzene was used instead of 4-bromobenzonitrile.

LC-Mass (theoretical mass: 261.10g/mol, measured mass: M+1 = 262g/mol).

### Synthesis of intermediate product, M-20

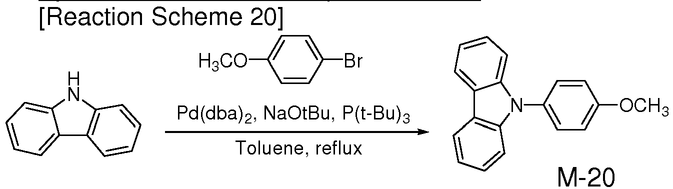

25.5g of a white solid intermediate M-20 was acquired as a desired compound (yield: 78%) in accordance with the same procedure as in the acquiring process of intermediate M-18, except that 4-bromoanisole was used instead of 4-bromobenzonitrile.

LC-Mass (theoretical mass: 273.12g/mol, measured mass: M+1 = 274g/mol).

### Synthesis of intermediate product, M-21

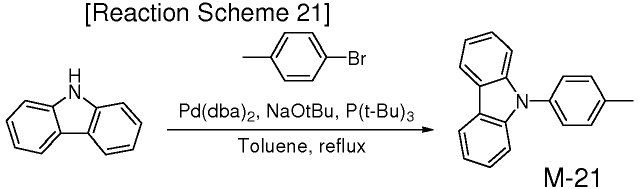

24.3g of a white solid intermediate M-21 was acquired as a desired compound (yield: 79%) in accordance with the same procedure as in the acquiring process of intermediate M-18, except that 4-bromotoluene was used instead of 4-bromobenzonitrile.

LC-Mass (theoretical mass: 257.12g/mol, measured mass: M+1 = 258g/mol).

### Synthesis of intermediate product, M-22

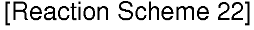

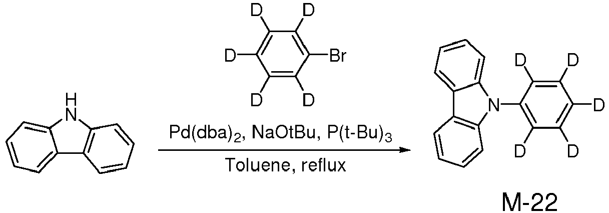

24.1 g of a white solid intermediate M-22 was acquired as a desired compound (yield: 81%) in accordance with the same procedure as in the acquiring process of intermediate M-18, except that bromobenzene-d₅ was used instead of 4-bromobenzonitrile.

LC-Mass (theoretical mass: 248.14g/mol, measured mass: M+1 = 249g/mol).

### Synthesis of intermediate product, M-23

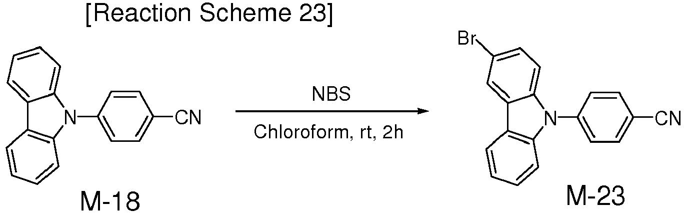

20g (74.5mmol) of intermediate M-18 was dissolved in 370mL of chloroform, and then 13.3g (74.5mmol) of N-bromosuccinimide was added and agitated at room temperature for 2 hours. After the reaction, the reactant was extracted with distilled water and dichloromethane. The organic layer was dried with magnesium sulfite and filtered. Then, the filtrate was concentrated under reduced pressure. The product was recrystallized in n-hexane, and then 21.2g of a white solid intermediate M-23 was acquired as a desired compound (yield: 82%).

LC-Mass (theoretical mass: 346.01 g/mol, measured mass: M+1 = 347g/mol, M+3 = 349g/mol)

### Synthesis of intermediate product, M-24

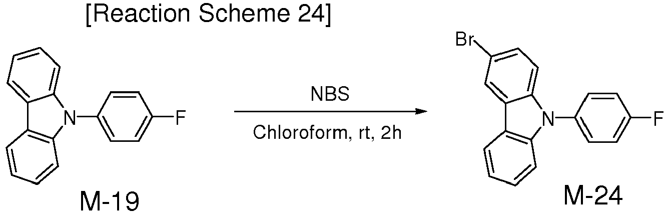

21.0g of a white solid intermediate M-24 was acquired as a desired compound (yield: 83%) in accordance with the same procedure as in the acquiring process of intermediate M-23, except that intermediate M-19 was used instead of intermediate M-18.

LC-Mass (theoretical mass: 339.01g/mol, measured mass: M+1 = 340g/mol, M+3 = 342g/mol).

### Synthesis of intermediate product, M-25

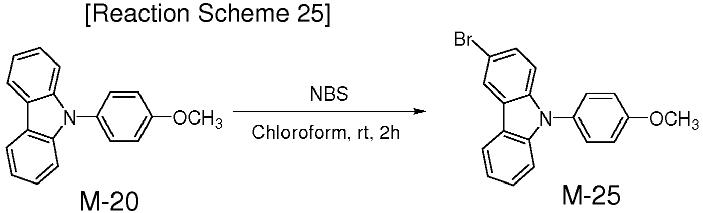

21.8g of a white solid intermediate M-25 was acquired as a desired compound (yield: 83%) in accordance with the same procedure as in the acquiring process of intermediate M-23, except that intermediate M-20 was used instead of intermediate M-18.

LC-Mass (theoretical mass: 351.03g/mol, measured mass: M+1 = 352g/mol, M+3 = 354g/mol).

### Synthesis of intermediate product, M-26

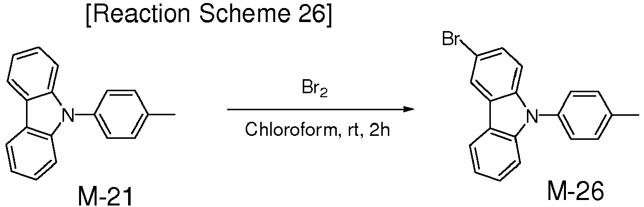

20g (74.5mmol) of intermediate M-21 was dissolved in 370mL of chloroform, and then 11.9g (74.5mmol) of bromine was added and agitated at room temperature for 2 hours. After the reaction, the reactant was extracted with distilled water and dichloromethane. The organic layer was dried with magnesium sulfite and filtered. Then, the filtrate was concentrated under reduced pressure. The product was recrystallized in n-hexane, and then 18.8g of a white solid intermediate M-26 was acquired as a desired compound (yield: 75%).

LC-Mass (theoretical mass: 335.03g/mol, measured mass: M+1 = 336g/mol, M+3 = 338g/mol)

### Synthesis of intermediate product, M-27

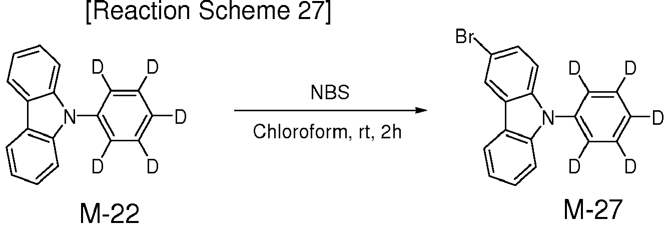

20.7g of a white solid intermediate M-27 was acquired as a desired compound (yield: 85%) in accordance with the same procedure as in the acquiring process of intermediate M-23, except that intermediate M-22 was used instead of intermediate M-18.

LC-Mass (theoretical mass: 326.05g/mol, measured mass: M+1 = 327g/mol, M+3 = 329g/mol).

### Synthesis of intermediate product, M-28

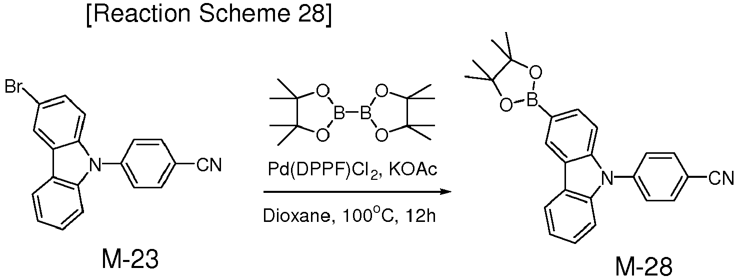

18g (51.8mmol) of intermediate M-23, 0.85g (1.04mmol) of Pd(dppf)Cl₂, 14.5g (57.0mmol) of bis(pinacolato)diboron, and 10.2g (103.6mmol) of potassium acetate were dissolved in 260ml of 1,4-dioxane. The reactant was refluxed and agitated for 12 hours under a nitrogen atmosphere, and then extracted 3 times with dichloromethane and distilled water. The extract was dried with magnesium sulfite and filtered. Then, the filtrate was concentrated under reduced pressure. The product was purified with n-hexane/dichloromethane mixed in a volume ratio of 7:3 through silica gel column chromatography, and then 14.5g of a white solid intermediate M-28 was acquired as a desired compound (yield: 71%).

LC-Mass (theoretical mass: 394.19g/mol, measured mass: M+1 = 395g/mol)

### Synthesis of intermediate product, M-29

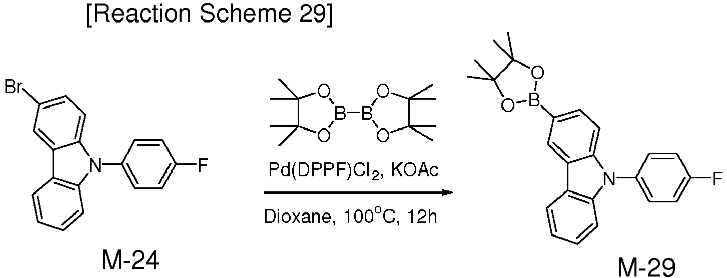

14.2g of a white solid intermediate M-29 was acquired as a desired compound (yield: 75%) in accordance with the same procedure as in the acquiring process of intermediate M-28, except that intermediate M-24 was used instead of intermediate M-23.

LC-Mass (theoretical mass: 387.18g/mol, measured mass: M+1 = 388g/mol).

### Synthesis of intermediate product, M-30

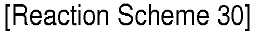

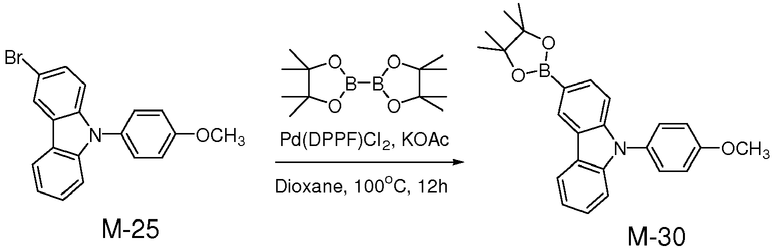

15.9g of a white solid intermediate M-30 was acquired as a desired compound (yield: 77%) in accordance with the same procedure as in the acquiring process of intermediate M-28, except that intermediate M-25 was used instead of intermediate M-24.

LC-Mass (theoretical mass: 399.20g/mol, measured mass: M+1 = 400g/mol).

### Synthesis of intermediate product, M-31

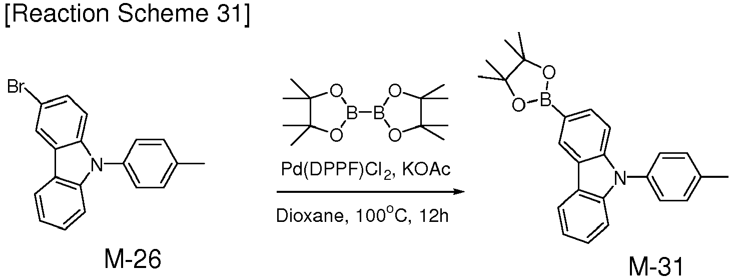

16.1g of a white solid intermediate M-31 was acquired as a desired compound (yield: 81%) in accordance with the same procedure as in the acquiring process of intermediate M-28, except that intermediate M-26 was used instead of intermediate M-23.

LC-Mass (theoretical mass: 383.21g/mol, measured mass: M+1 = 384g/mol).

### Synthesis of intermediate product, M-32

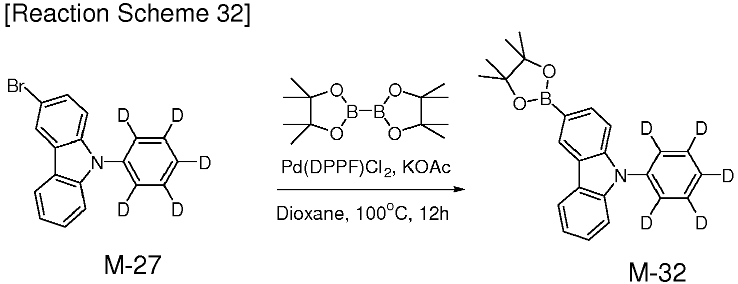

15.1g of a white solid intermediate M-32 was acquired as a desired compound (yield: 81%) in accordance with the same procedure as in the acquiring process of intermediate M-28, except that intermediate M-27 was used instead of intermediate M-23

LC-Mass (theoretical mass: 359.20g/mol, measured mass: M+1 = 360g/mol).

### Synthesis of intermediate product, M-33

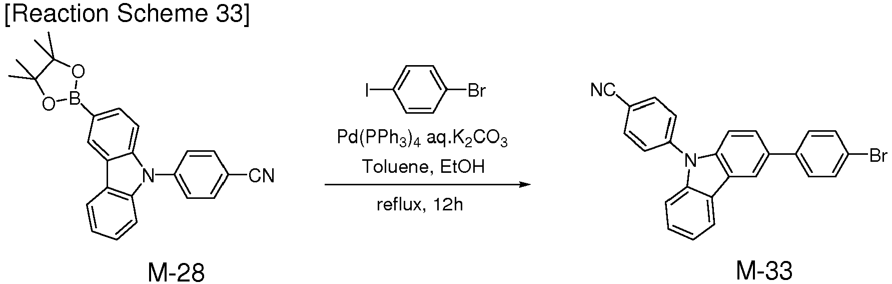

12g (30.4mmol) of intermediate M-28, 8.6g (30.4mmol) of 1-bromo-4-iodobenzene, and 0.35g (0.304mmol) of tetrakistriphenylphosphinepalladium were added to a flask and dissolved in 152ml of toluene and 76mL of ethanol under a nitrogen atmosphere.

76ml of an aqueous solution including 8.95g (60.8mmol) of potassium carbonate was added, and then refluxed and agitated for 12 hours. After the reaction, the reactant was extracted with ethylacetate. The extract was dried with magnesium sulfite and filtered. Then, the filtrate was concentrated under reduced pressure. The product was purified with n-hexane/dichloromethane mixed in a volume ratio of 7:3 through silica gel column chromatography, and then 10.6g of a white solid intermediate M-33 was acquired as a desired compound (yield: 82%).

LC-Mass (theoretical mass: 422.04g/mol, measured mass: M+1 = 423g/mol, M+3 = 425g/mol)

### Synthesis of intermediate product, M-34

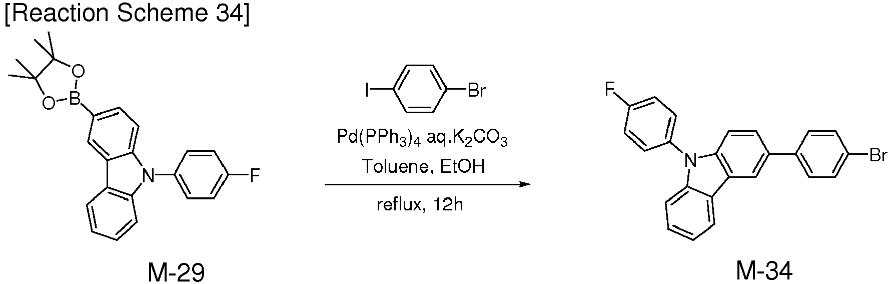

10.8g of white solid intermediate M-34 was acquired as a desired compound (yield: 85%) in accordance with the same procedure as in the acquiring process of intermediate M-33, except that M-9 was used instead of M-28.

LC-Mass (theoretical mass: 415.04g/mol, measured mass: M+1 = 416g/mol, M+3 = 418g/mol).

### Synthesis of intermediate product, M-35

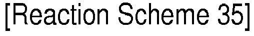

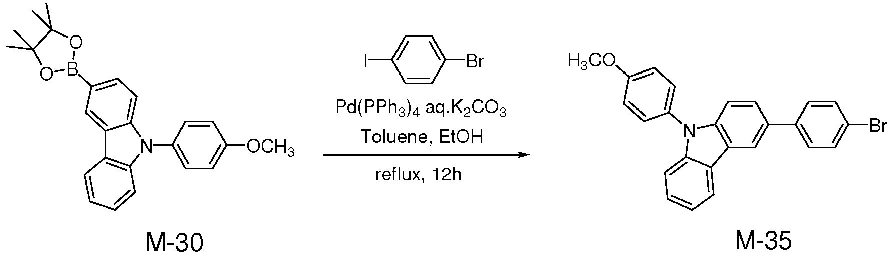

10.9g of a white solid intermediate M-35 was acquired as a desired compound (yield: 84%) in accordance with the same procedure as in the acquiring process of intermediate M-33, except that intermediate M-30 was used instead of intermediate M-28.

LC-Mass (theoretical mass: 428.32g/mol, measured mass: M+1 = 429g/mol, M+3 = 431g/mol).

### Synthesis of intermediate product, M-36

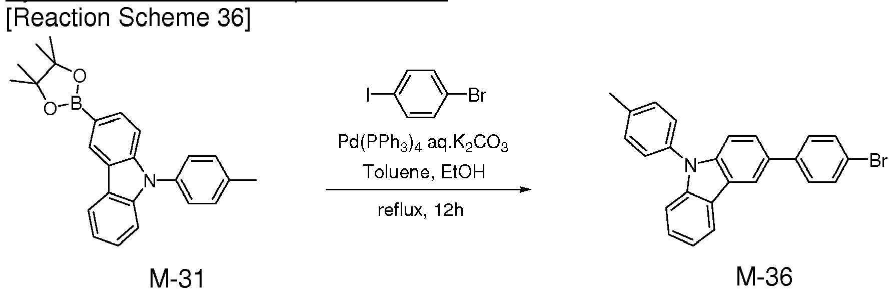

10.9g of white solid intermediate M-36 was acquired as a desired compound (yield: 87%) in accordance with the same procedure as in the acquiring process of intermediate M-33, except that M-31 was used instead of M-28.

LC-Mass (theoretical mass: 411.06g/mol, measured mass: M+1 = 412g/mol, M+3 = 414g/mol).

### Synthesis of intermediate product, M-37

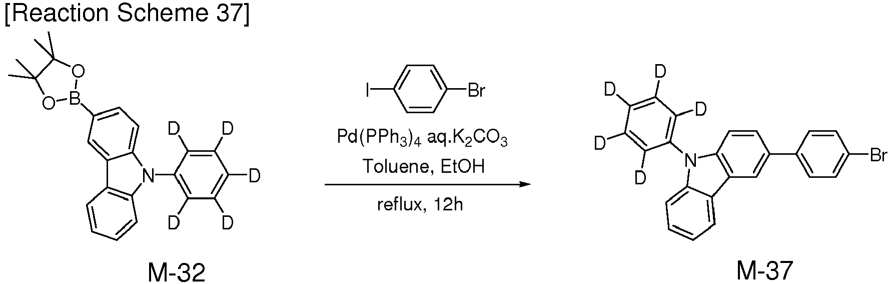

10.9g of white solid intermediate M-36 was acquired as a desired compound (yield: 89%) in accordance with the same procedure as in the acquiring process of intermediate M-33, except that M-31 was used instead of M-28.

LC-Mass (theoretical mass: 402.08g/mol, measured mass: M+1 = 403g/mol, M+3 = 405g/mol).

### Synthesis of intermediate product, M-38

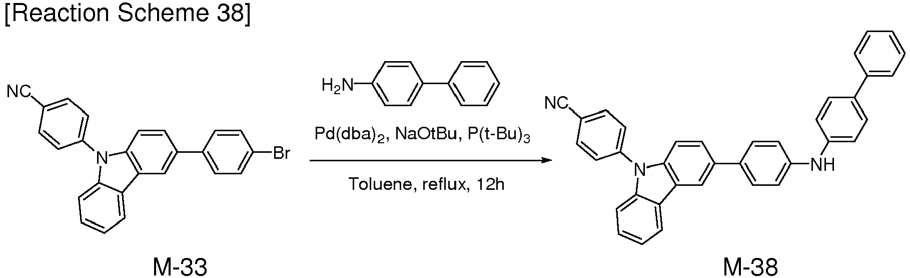

10g (19.5mmol) of intermediate M-33, 3.3g (19.5mmol) of 4-aminobiphenyl, 3.7g (39.0mmol) of sodium t-butoxide, and 0.12g (0.58mmol) of tri-tetra-butylphosphine were added to a flask and dissolved in 195ml of toluene, and 0.11g (0.753mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 7.2g of a white solid intermediate M-38 was acquired as a desired compound (yield: 72%).

LC-Mass (theoretical mass: 511.20g/mol, measured mass: M+1 = 512g/mol)

### Synthesis of intermediate product, M-39

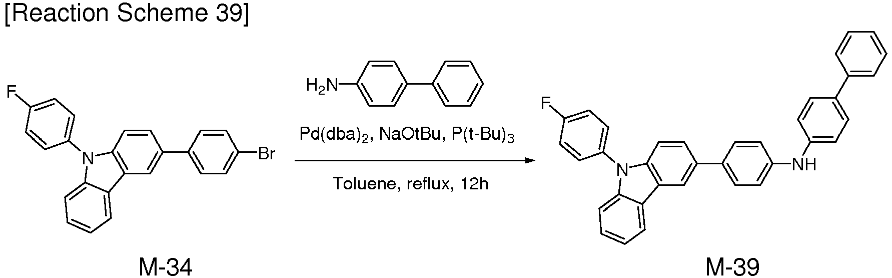

7.4g of a white solid intermediate M-39 was acquired as a desired compound (yield: 75%) in accordance with the same procedure as in the acquiring process of intermediate M-38, except that intermediate M-34 was used instead of intermediate M-33.

LC-Mass (theoretical mass: 504.20g/mol, measured mass: M+1 = 504.60g/mol).

### Synthesis of intermediate product, M-40

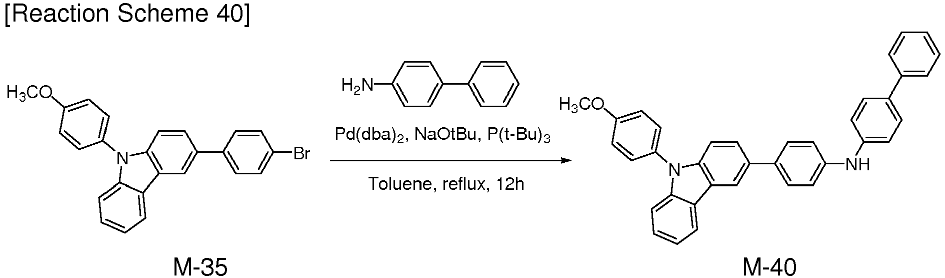

7.7g of a white solid intermediate M-40 was acquired as a desired compound (yield: 76%) in accordance with the same procedure as in the acquiring process of intermediate M-38, except that intermediate M-35 was used instead of intermediate M-33.

LC-Mass (theoretical mass: 516.22/mol, measured mass: M+1 = 517g/mol).

### Synthesis of intermediate product, M-41

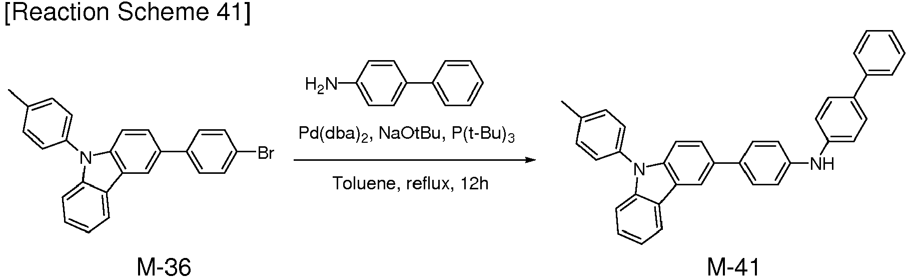

7.7g of a white solid intermediate M-41 was acquired as a desired compound (yield: 79%) in accordance with the same procedure as in the acquiring process of intermediate M-38, except that intermediate M-36 was used instead of intermediate M-33.

LC-Mass (theoretical mass: 500.23/mol, measured mass: M+1 = 501g/mol).

### Synthesis of intermediate product, M-42

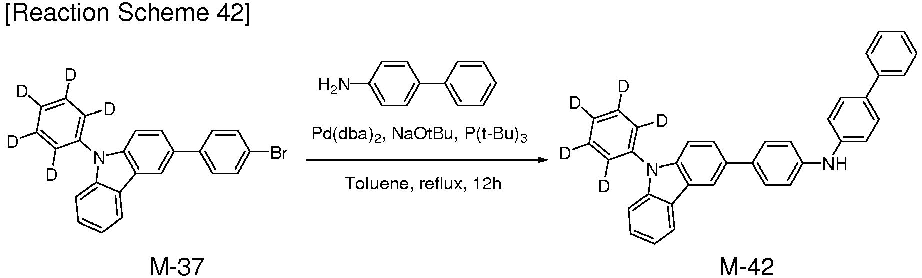

8.0g of a white solid intermediate M-42 was acquired as a desired compound (yield: 83%) in accordance with the same procedure as in the acquiring process of intermediate M-38, except that intermediate M-37 was used instead of intermediate M-33.

LC-Mass (theoretical mass: 491.24/mol, measured mass: M+1 = 492g/mol).

### Example 1: Preparation of Compound represented by Chemical Formula A-414

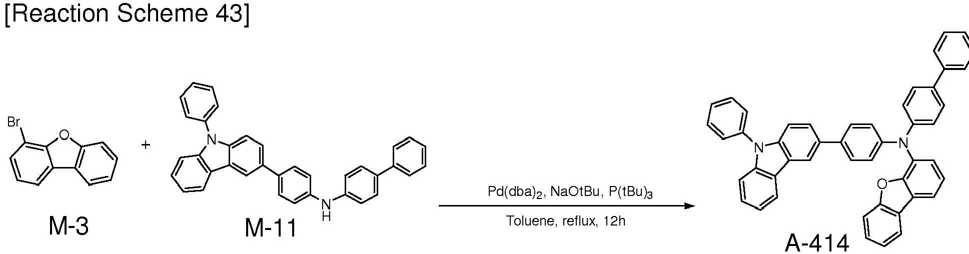

5g (20.2mmol) of intermediate M-3, 9.85g (20.2mmol) of sodium t-butoxide, and 0.12g (2.26mmol) of tri-tetra-butylphosphine were added to a flask and dissolved in 200ml of toluene, and 0.12g (0.202mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 12g of a white solid compound A-414 was acquired as a desired compound (yield: 91 %).

LC-Mass (theoretical mass: 652.25g/mol, measured mass: M+1 = 653g/mol)

### Example 2: Preparation of Compound represented by Chemical Formula A-415

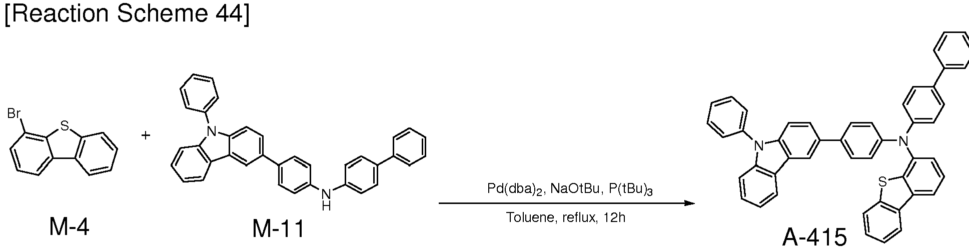

5.3g (20.2mmol) of intermediate M-4, 9.85g (20.2mmol) of M-11, 2.91 g (30.3mmol) of sodium t-butoxide, and 0.12g (2.26mmol) of tri-tetra-butylphosphine were added to a flask and dissolved in 200ml of toluene, and 0.12g (0.202mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 11.8g of a white solid compound A-415 was acquired as a desired compound (yield: 87%).

LC-Mass (theoretical mass: 668.23g/mol, measured mass: M+1 = 669g/mol)

### Example 3: Preparation of Compound represented by Chemical Formula A-9

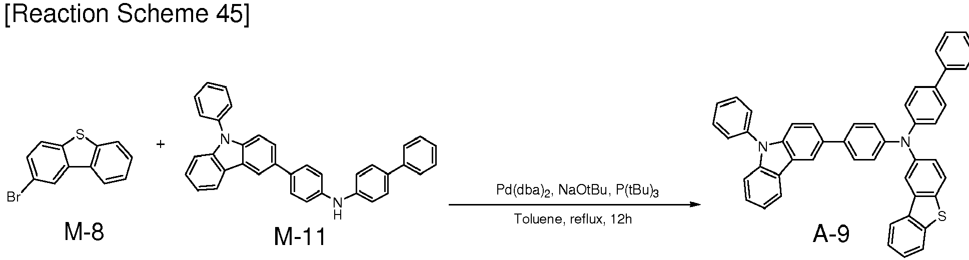

5.3g (20.2mmol) of intermediate M-8, 9.85g (20.2mmol) of M-11, 2.91 g (30.3mmol) of sodium t-butoxide, and 0.12g (2.26mmol) of tri-tetra-butylphosphine were added to a flask and dissolved in 200ml of toluene, and 0.12g (0.202mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 11.8g of a white solid compound A-9 was acquired as a desired compound (yield: 87%).

LC-Mass (theoretical mass: 668.23g/mol, measured mass: M+1 = 669g/mol)

### Example 4: Preparation of Compound represented by Chemical Formula A-10

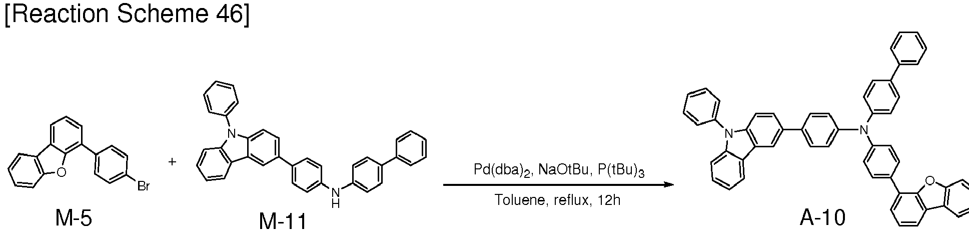

6.5g (20.2mmol) of intermediate M-5, 9.85g (20.2mmol) of intermediate M-11, 2.91 g (30.3mmol) of sodium t-butoxide, and 0.12g (2.26mmol) of tri-tetra-butylphosphine were added to a flask and dissolved in 200ml of toluene, and 0.12g (0.202mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 12.4g of a white solid compound A-10 was acquired as a desired compound (yield: 84%).

LC-Mass (theoretical mass: 728.28g/mol, measured mass: M+1 = 729g/mol)

### Example 5: Preparation of Compound represented by Chemical Formula A-11

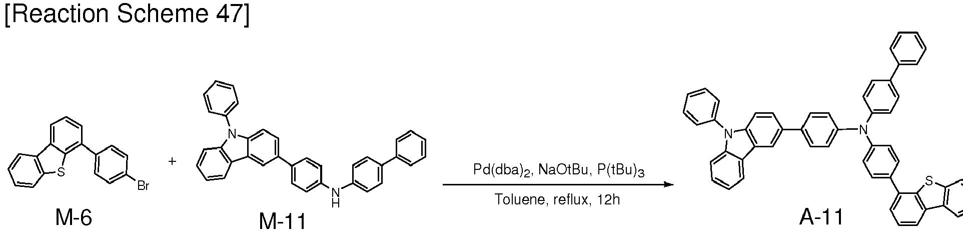

6.85g (20.2mmol) of intermediate M-6, 9.85g (20.2mmol) of M-11, 2.91 g (30.3mmol) of sodium t-butoxide, and 0.12g (2.26mmol) of tri-tetra-butylphosphine were added to a flask and dissolved in 200ml of toluene, and 0.12g (0.202mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 13.2g of a white solid compound A-11 was acquired as a desired compound (yield: 88%).

LC-Mass (theoretical mass: 744.26g/mol, measured mass: M+1 = 745g/mol)

### Example 6: Preparation of Compound represented by Chemical Formula A-18

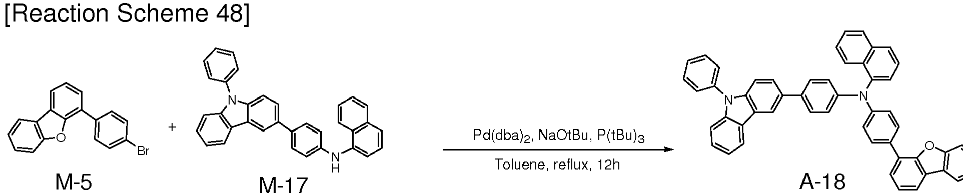

6.53g (20.2mmol) of intermediate M-5, 9.30g (20.2mmol) of M-17, 2.91 g (30.3mmol) of sodium t-butoxide, and 0.12g (2.26mmol) of tri-tetra-butylphosphine were added to a flask and dissolved in 200ml of toluene, and 0.12g (0.202mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 12.5g of a white solid compound A-18 was acquired as a desired compound (yield: 88%).

LC-Mass (theoretical mass: 702.27g/mol, measured mass: M+1 = 703g/mol)

### Example 7: Preparation of Compound represented by Chemical Formula A-19

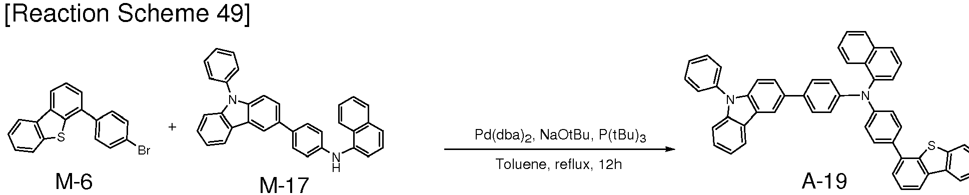

6.85g (20.2mmol) of intermediate M-6, 9.30g (20.2mmol) of M-17, 2.91 g (30.3mmol) of sodium t-butoxide, and 0.12g (2.26mmol) of tri-tetra-butylphosphine were added to a flask and dissolved in 200ml of toluene, and 0.12g (0.202mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 12.3g of a white solid compound A-18 was acquired as a desired compound (yield: 85%).

LC-Mass (theoretical mass: 718.24g/mol, measured mass: M+1= 719g/mol)

### Example 8: Preparation of Compound represented by Chemical Formula A-327

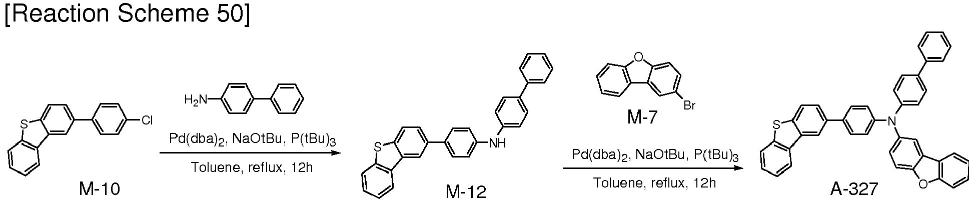

5.2g (12.2mmol) of intermediate M-12, 3.0g (12.2mmol) of intermediate M-7, 1.76g (18.3mmol) of sodium t-butoxide, and 0.074g (0.37mmol) of tri-tetra-butylphosphine were added to a flask and dissolved in 120ml of toluene, and 0.070g (0.122mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 6.2g of a white solid compound A-327 was acquired as a desired compound (yield: 86%).

LC-Mass (theoretical mass: 593.18g/mol, measured mass: M+1= 594g/mol)

### Example 9: Preparation of Compound represented by Chemical Formula A-335

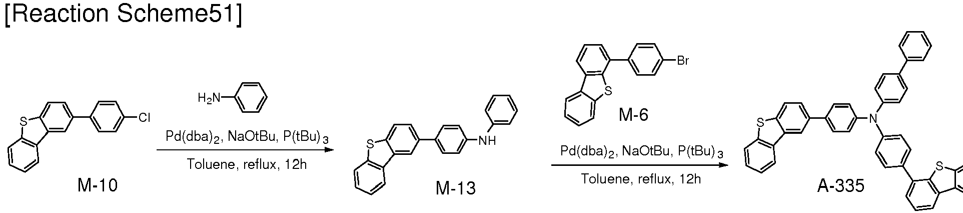

4.3g (12.2mmol) of intermediate M-13, 4.14g (12.2mmol) of intermediate M-6, 1.76g (18.3mmol) of sodium t-butoxide, and 0.074g (0.37mmol) of tri-tetra-butylphosphine were added to a flask and dissolved in 120ml of toluene, and 0.070g (0.122mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 6.8g of a white solid compound A-335 was acquired as a desired compound (yield: 91%).

LC-Mass (theoretical mass: 609.16g/mol, measured mass: M+1= 610g/mol)

### Example 10: Preparation of Compound represented by Chemical Formula A-340

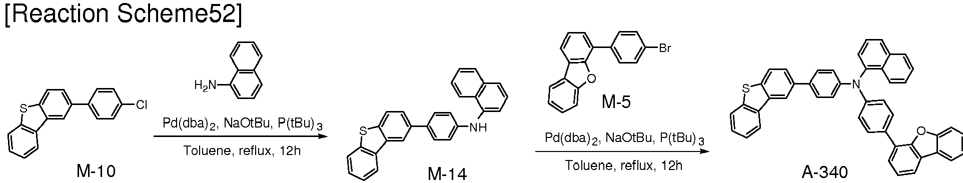

4.9g (12.2mmol) of intermediate M-14, 3.94g (12.2mmol) of intermediate M-5, 1.76g (18.3mmol) of sodium t-butoxide, and 0.074g (0.37mmol) of tri-tetra-butylphosphine were added to a flask and dissolved in 120ml of toluene, and 0.070g (0.122mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 7.2g of a white solid compound A-340 was acquired as a desired compound (yield: 92%).

LC-Mass (theoretical mass: 643.20g/mol, measured mass: M+1= 644g/mol)

### Example 11: Preparation of Compound represented by Chemical Formula A-373

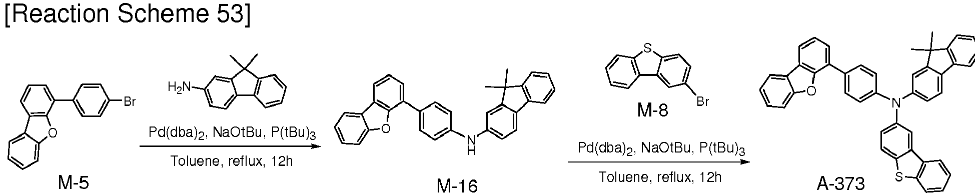

5.51 g (12.2mmol) of intermediate M-16, 3.21 g (12.2 mmol) of intermediate M-8, 1.76g (18.3mmol) of sodium t-butoxide, and 0.074g (0.37mmol) of tri-tetra-butylphosphine were added to a flask and dissolved in 120ml of toluene, and 0.070g (0.122mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 7.0g of a white solid compound A-373 was acquired as a desired compound (yield: 91%).

LC-Mass (theoretical mass: 633.21g/mol, measured mass: M+1 = 634g/mol)

### Example 12: Preparation of Compound represented by Chemical Formula A-376

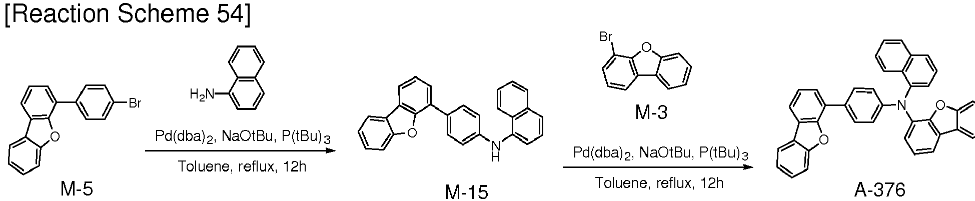

4.7g (12.2mmol) of intermediate M-15, 3.01 g (12.2mmol) of intermediate M-3 , 1.76g (18.3mmol) of sodium t-butoxide, and 0.074g (0.37mmol) of tri-tetra-butylphosphine were added to a flask and dissolved in 120ml of toluene, and 0.070g (0.122mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 6.2g of a white solid compound A-376 was acquired as a desired compound (yield: 92%).

LC-Mass (theoretical mass: 551.19g/mol, measured mass: M+1 = 552g/mol)

### Example 13: Preparation of Compound represented by Chemical Formula A-421

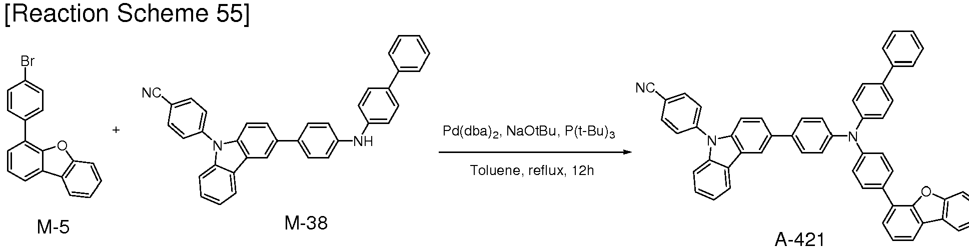

4.4g (13.7mmol) of intermediate M-5, 7.0g (13.7mmol) of intermediate M-38, 2.63g (27.4mmol) of sodium t-butoxide, and 0.08g (0.41 mmol) of tri-tetra-butylphosphine were added to a flask and dissolved in 137ml of toluene, and 0.08g (0.137mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 8.7g of a white solid compound A-421 was acquired as a desired compound (yield: 84%).

LC-Mass (theoretical mass: 753.28g/mol, measured mass: M+1 = 754g/mol)

### Example 14: Preparation of Compound represented by Chemical Formula A-429

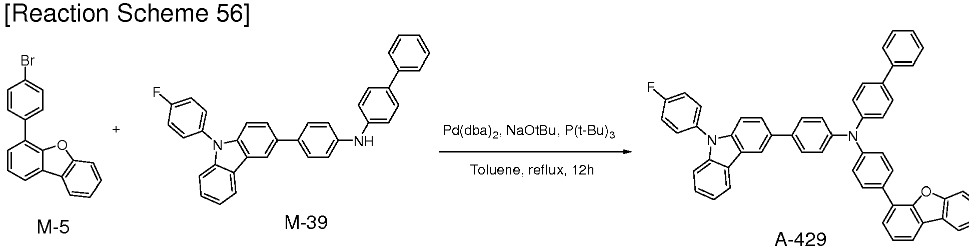

8.3g of a white solid compound A-429 was acquired as a desired compound (yield: 81%) in accordance with the same procedure as in the acquiring process of compound A-421, except that intermediate M-39 was used instead of intermediate M-38.

LC-Mass (theoretical mass: 746.27g/mol, measured mass: M+1= 747g/mol).

### Example 15: Preparation of Compound represented by Chemical Formula A-437

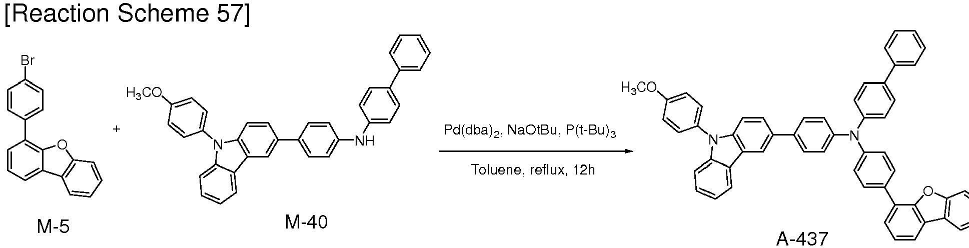

8.8g of a white solid compound A-437 was acquired as a desired compound (yield: 85%) in accordance with the same procedure as in the acquiring process of compound A-421, except that intermediate M-40 was used instead of intermediate M-38.

LC-Mass (theoretical mass: 758.29g/mol, measured mass: M+1 = 759g/mol).

### Example 16: Preparation of Compound represented by Chemical Formula A-445

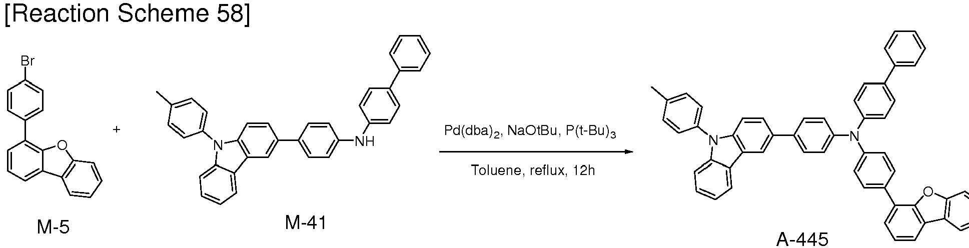

8.9g of a white solid compound A-445 was acquired as a desired compound (yield: 87%) in accordance with the same procedure as in the acquiring process of compound A-421, except that intermediate M-41 was used instead of intermediate M-38.

LC-Mass (theoretical mass: 742.30g/mol, measured mass: M+1 = 743g/mol).

### Example 17: Preparation of Compound represented by Chemical Formula A-453

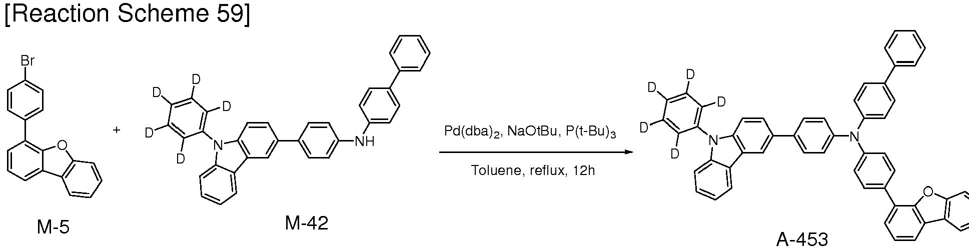

8.3g of a white solid compound A-453 was acquired as a desired compound (yield: 83%) in accordance with the same procedure as in the acquiring process of compound A-421, except that intermediate M-42 was used instead of intermediate M-38.

LC-Mass (theoretical mass: 733.31g/mol, measured mass: M+1 = 734g/mol).

### Example 18: Preparation of Compound represented by Chemical Formula A-422

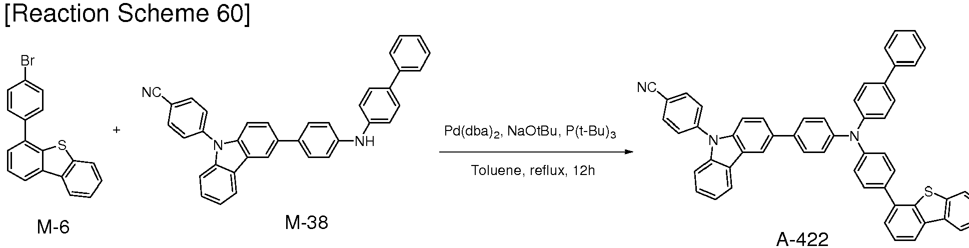

4.6g (13.7mmol) of intermediate M-6, 7.0g (13.7mmol) of intermediate M-38, 2.63g (27.4mmol) of sodium t-butoxide, and 0.08g (0.41 mmol) of tri-tetra-butylphosphine were added to a flask and dissolved in 137ml of toluene, and 0.08g (0.137mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 7:3 through silica gel column chromatography, and then 8.6g of a white solid compound A-422 was acquired as a desired compound (yield: 82%).

LC-Mass (theoretical mass: 769.26g/mol, measured mass: M+1= 770g/mol)

### Example 19: Preparation of Compound represented by Chemical Formula A-430

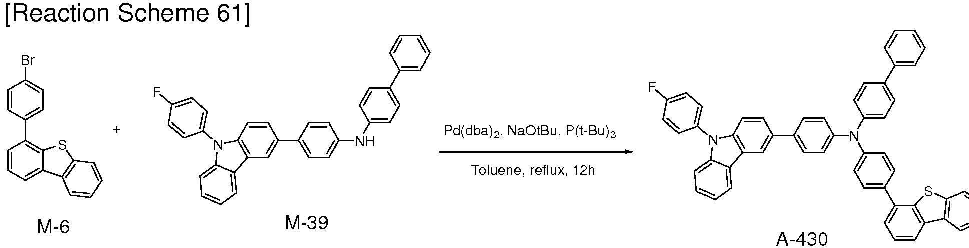

8.8g of a white solid compound A-430 was acquired as a desired compound (yield: 84%) in accordance with the same procedure as in the acquiring process of compound A-422, except that intermediate M-39 was used instead of intermediate M-38.

LC-Mass (theoretical mass: 762.25g/mol, measured mass: M+1 = 763g/mol).

### Example 20: Preparation of Compound represented by Chemical Formula A-438

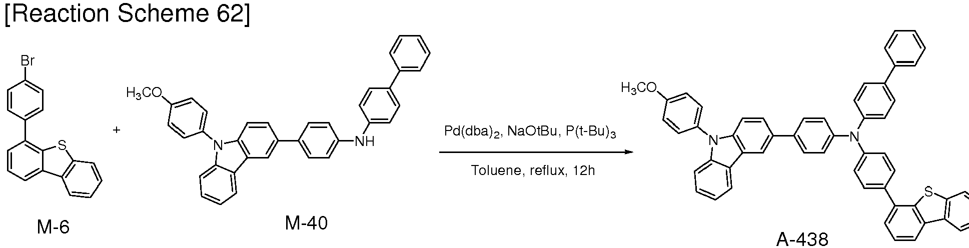

9.1 g of a white solid compound A-438 was acquired as a desired compound (yield: 86%) in accordance with the same procedure as in the acquiring process of compound A-422, except that intermediate M-40 was used instead of intermediate M-38.

LC-Mass (theoretical mass: 774.27g/mol, measured mass: M+1 = 775g/mol).

### Example 21: Preparation of Compound represented by Chemical Formula A-446

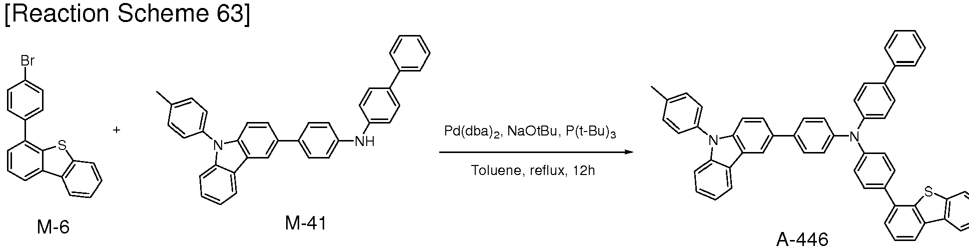

9.2g of a white solid compound A-446 was acquired as a desired compound (yield: 88%) in accordance with the same procedure as in the acquiring process of compound A-422, except that intermediate M-41 was used instead of intermediate M-38.

LC-Mass (theoretical mass: 758.28g/mol, measured mass: M+1 = 759g/mol).

### Example 22: Preparation of Compound represented by Chemical Formula A-454

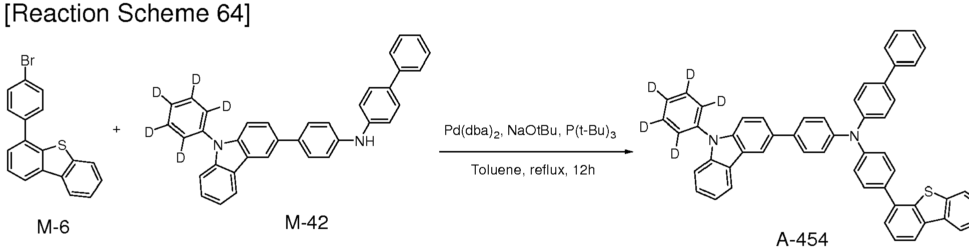

8.8g of a white solid compound A-454 was acquired as a desired compound (yield: 86%) in accordance with the same procedure as in the acquiring process of compound A-422, except that intermediate M-42 was used instead of intermediate M-38.

LC-Mass (theoretical mass: 749.29g/mol, measured mass: M+1 = 750g/mol).

### Example 23: Preparation of Compound represented by Chemical Formula A-42

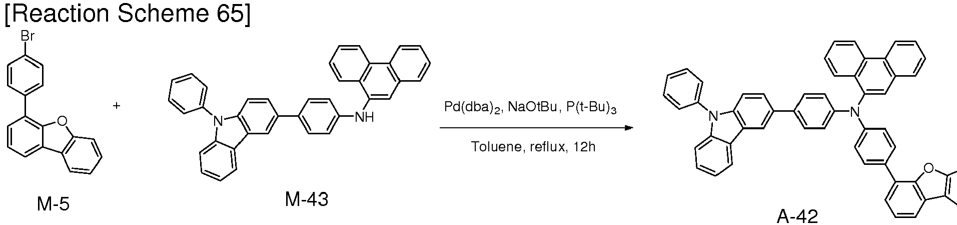

8.4g of a white solid compound A-42 was acquired as a desired compound (yield: 81%) in accordance with the same procedure as in the acquiring process of compound A-421, except that intermediate M-43 was used instead of intermediate M-38.

LC-Mass (theoretical mass: 752.28g/mol, measured mass: M+1= 753g/mol).

### Example 24: Preparation of Compound represented by Chemical Formula A-43

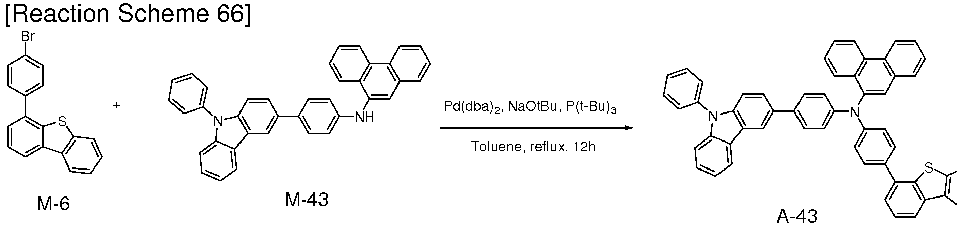

8.7g of a white solid compound A-43 was acquired as a desired compound (yield: 83%) in accordance with the same procedure as in the acquiring process of compound A-422, except that intermediate M-43 was used instead of intermediate M-38.

LC-Mass (theoretical mass: 768.26g/mol, measured mass: M+1 = 769g/mol).

### Example 25: Preparation of Compound represented by Chemical Formula A-234

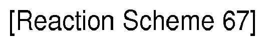

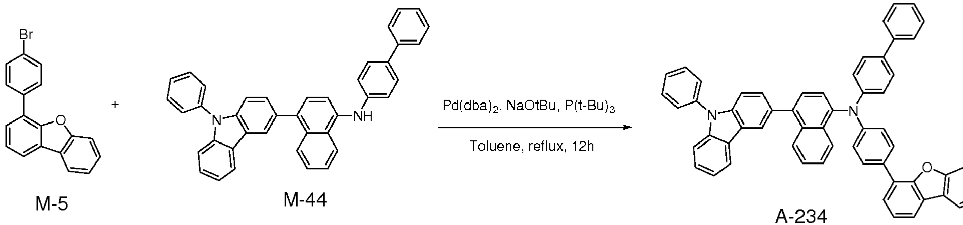

9.0g of a white solid compound A-234 was acquired as a desired compound (yield: 84%) in accordance with the same procedure as in the acquiring process of compound A-421, except that intermediate M-44 was used instead of intermediate M-38.

LC-Mass (theoretical mass: 778.30g/mol, measured mass: M+1= 779g/mol).

### Example 26: Preparation of Compound represented by Chemical Formula A-235

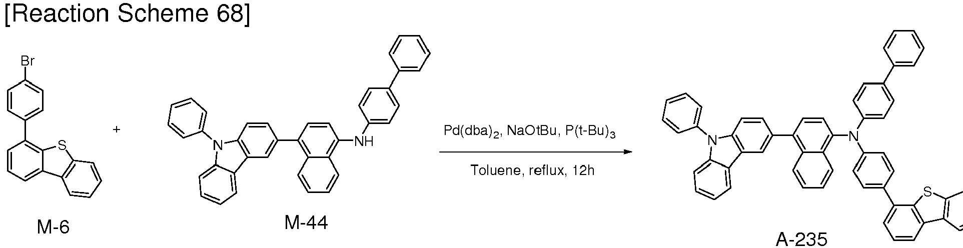

9.0g of a white solid compound A-235 was acquired as a desired compound (yield: 83%) in accordance with the same procedure as in the acquiring process of compound A-422, except that intermediate M-44 was used instead of intermediate M-38.

LC-Mass (theoretical mass: 794.28g/mol, measured mass: M+1 = 795g/mol).

### Example 27: Preparation of Compound represented by Chemical Formula A-469

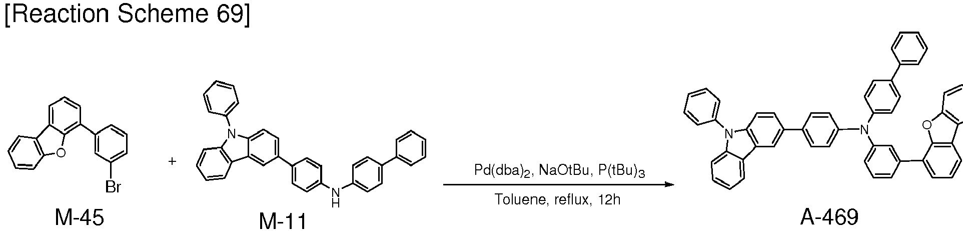

12.8g of a white solid compound A-469 was acquired as a desired compound (yield: 87%) in accordance with the same procedure as in the acquiring process of intermediate compound A-10, except that intermediate M-45 was used instead of intermediate M-5.

LC-Mass (theoretical mass: 728.28g/mol, measured mass: M+1 = 729g/mol).

### Example 28: Preparation of Compound represented by Chemical Formula A-470

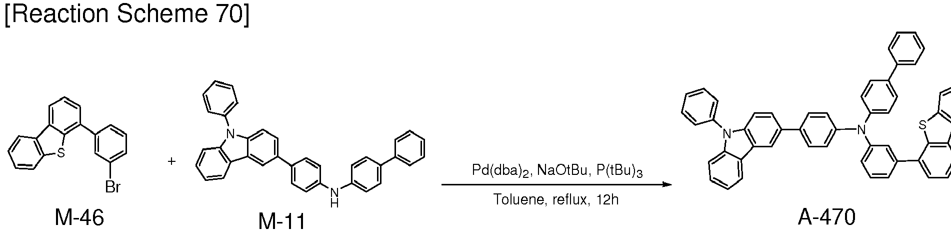

13.4g of a white solid compound A-470 was acquired as a desired compound (yield: 89%) in accordance with the same procedure as in the acquiring process of intermediate compound A-11, except that intermediate M-46 was used instead of intermediate M-6.

LC-Mass (theoretical mass: 744.26g/mol, measured mass: M+1 = 745g/mol).

### Example 29: Preparation of Compound represented by Chemical Formula A-457

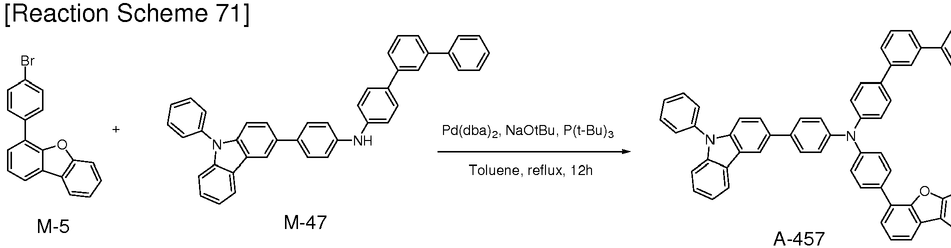

9.4g of a white solid compound A-457 was acquired as a desired compound (yield: 85%) in accordance with the same procedure as in the acquiring process of intermediate compound A-421, except that intermediate M-47 was used instead of intermediate M-38.

LC-Mass (theoretical mass: 804.31 g/mol, measured mass: M+1 = 805g/mol).

### Example 30: Preparation of Compound represented by Chemical Formula A-458

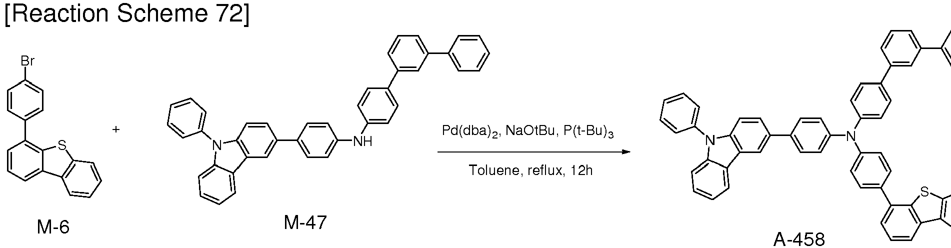

10.01 g of a white solid compound A-458 was acquired as a desired compound (yield: 89%) in accordance with the same procedure as in the acquiring process of intermediate compound A-422, except that intermediate M-47 was used instead of intermediate M-38.

LC-Mass (theoretical mass: 820.29g/mol, measured mass: M+1 = 821 g/mol).

### Example 31: Preparation of Compound represented by Chemical Formula A-463

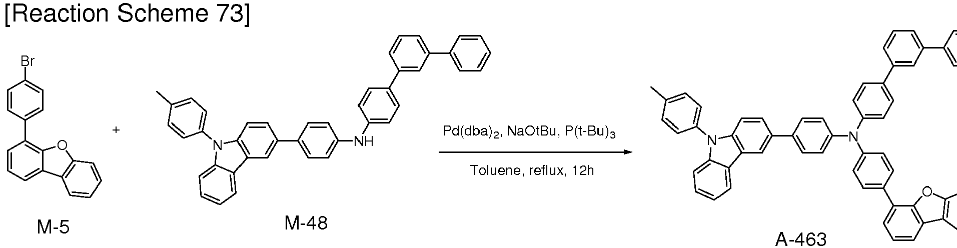

9.5g of a white solid compound A-463 was acquired as a desired compound (yield: 85%) in accordance with the same procedure as in the acquiring process of intermediate compound A-421, except that intermediate M-48 was used instead of intermediate M-38.

LC-Mass (theoretical mass: 818.33g/mol, measured mass: M+1 = 819g/mol).

### Example 32: Preparation of Compound represented by Chemical Formula A-464

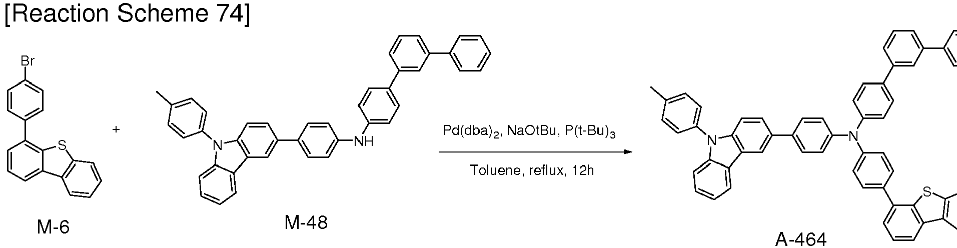

9.8g of a white solid compound A-464 was acquired as a desired compound (yield: 86%) in accordance with the same procedure as in the acquiring process of intermediate compound A-422, except that intermediate M-48 was used instead of intermediate M-38.

LC-Mass (theoretical mass: 834.31 g/mol, measured mass: M+1 = 835g/mol).

### Example 33: Preparation of Compound represented by Chemical Formula A-467

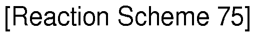

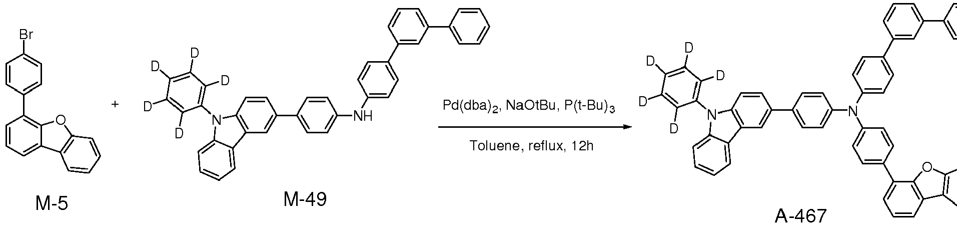

9.8g of a white solid compound A-467 was acquired as a desired compound (yield: 88%) in accordance with the same procedure as in the acquiring process of intermediate compound A-421, except that intermediate M-49 was used instead of intermediate M-38.

LC-Mass (theoretical mass: 809.34g/mol, measured mass: M+1 - 810g/mol).

### Example 34: Preparation of Compound represented by Chemical Formula A-468

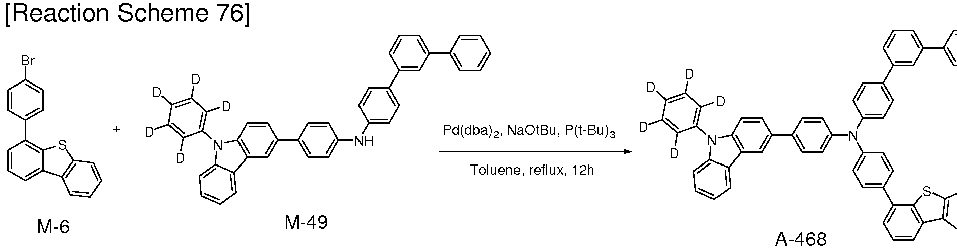

9.3g of a white solid compound A-468 was acquired as a desired compound (yield: 82%) in accordance with the same procedure as in the acquiring process of intermediate compound A-422, except that intermediate M-49 was used instead of intermediate M-38.

LC-Mass (theoretical mass: 825.32g/mol, measured mass: M+1 = 826g/mol).

### Example 35: Preparation of Compound represented by Chemical Formula A-306

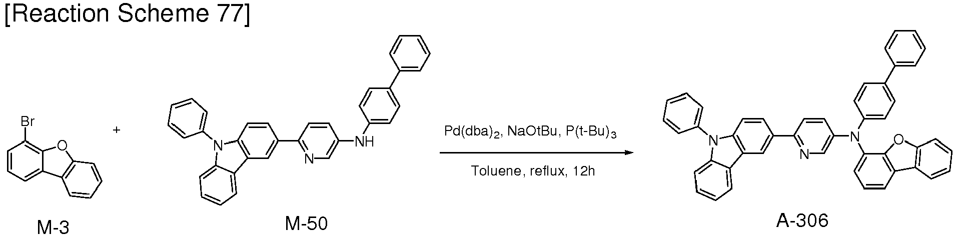

3.4g (13.7mmol) of intermediate M-3, 6.7g (13.7mmol) of intermediate M-50, 2.63g (27.4mmol) of sodium t-butoxide, and 0.08g (0.41 mmol) of tri-tetra-butylphosphine were added to a flask and dissolved in 750ml of toluene, and 0.43g (0.753mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 6:4 through silica gel column chromatography, and then 7.0g of a white solid compound A-306 was acquired as a desired compound (yield: 78%).

LC-Mass (theoretical mass: 653.25g/mol, measured mass: M+1 = 654g/mol)

### Example 36: Preparation of Compound represented by Chemical Formula A-319

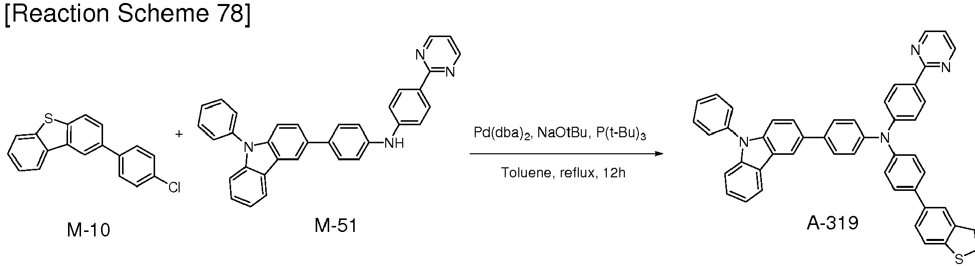

4.0g (13.7mmol) of intermediate M-10, 6.7g (13.7mmol) of intermediate M-51, 2.63g (27.4mmol) of sodium t-butoxide, and 0.08g (0.41 mmol) of tri-tetra-butylphosphine were added to a flask and dissolved in 137ml of toluene, and 0.08g (0.137mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 6:4 through silica gel column chromatography, and then 8.4g of a white solid compound A-306 was acquired as a desired compound (yield: 82%).

LC-Mass (theoretical mass: 746.25g/mol, measured mass: M+1= 747g/mol)

### Example 37: Preparation of Compound represented by Chemical Formula A-416

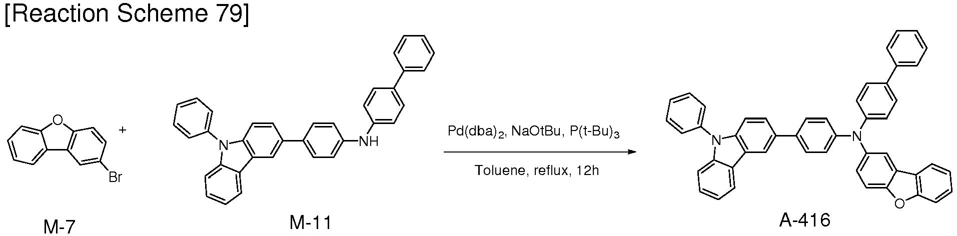

11.2g of a white solid compound A-416 was acquired as a desired compound (yield: 85%) in accordance with the same procedure as in the acquiring process of intermediate compound A-414, except that intermediate M-7 was used instead of intermediate M-3.

LC-Mass (theoretical mass: 652.25g/mol, measured mass: M+1 = 653g/mol).

### Example 38: Preparation of Compound represented by Chemical Formula A-12

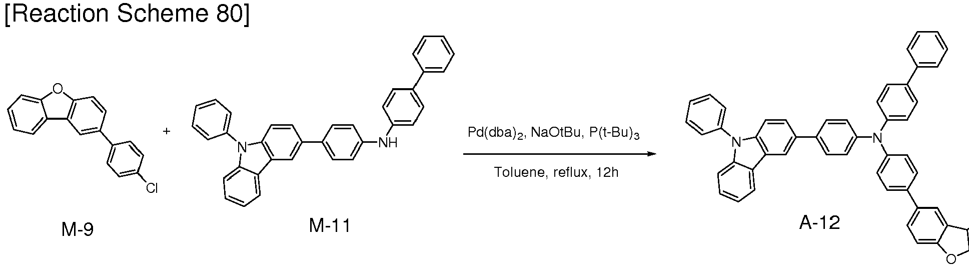

12.2g of a white solid compound A-12 was acquired as a desired compound (yield: 83%) in accordance with the same procedure as in the acquiring process of intermediate compound A-414, except that intermediate M-9 was used instead of intermediate M-3.

LC-Mass (theoretical mass: 728.28g/mol, measured mass: M+1 = 729g/mol).

### Example 39: Preparation of Compound represented by Chemical Formula A-13

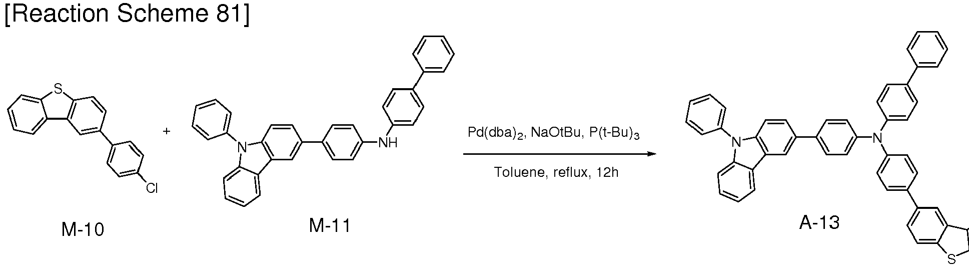

12.8g of a white solid compound A-13 was acquired as a desired compound (yield: 85%) in accordance with the same procedure as in the acquiring process of intermediate compound A-414, except that intermediate M-10 was used instead of intermediate M-3.

LC-Mass (theoretical mass: 744.26g/mol, measured mass: M+1 = 745g/mol).

### Example 40: Preparation of Compound represented by Chemical Formula A-396

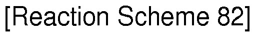

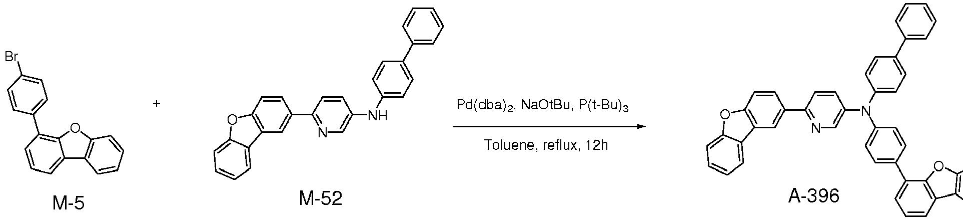

4.4g (13.7mmol) of intermediate M-5, 5.7g (13.7mmol) of intermediate M-52, 2.63g (27.4mmol) of sodium t-butoxide, and 0.08g (0.41 mmol) of tri-tetra-butylphosphine were added to a flask and dissolved in 137ml of toluene, and 0.08g (0.137mmol) of Pd(dba)₂ was added and then refluxed and agitated for 12 hours under a nitrogen atmosphere. After the reaction, the reactant was extracted with ethylacetate and distilled water. The organic layer was dried with magnesium sulfite and filtered. Then, the filtrate was concentrated under reduced pressure. The product was purified with n-hexane/dichloromethane mixed in a volume ratio of 6:4 through silica gel column chromatography, and then 7.2g of a white solid compound A-396 was acquired as a desired compound (yield: 80%).

LC-Mass (theoretical mass: 654.23g/mol, measured mass: M+1= 655g/mol)

### Fabrication of Organic Light Emitting Diode

### Example 41

A glass substrate thin film coated with 1500Å of indium tin oxide (ITO) was ultrasonic-wave cleaned with distilled water. Subsequently, the glass substrate cleaned with distilled water was ultrasonic-wave cleaned with a solvent such as isopropyl alcohol, acetone, methanol, or the like and dried. Then the glass substrate was moved to a plasma cleaner and cleaned for 5 minutes with oxygen plasma, and then the substrate was moved to a vacuum evaporator. 4,4'-bis[N-[4-{N,N-bis(3-methylphenyl)amino}-phenyl]-N-phenylamino]biphenyl (DNTPD) was vacuum deposited on the ITO substrate using an ITO transparent electrode prepared according to the above procedure to provide a 600A thick hole injection layer (HIL). Then the compound prepared according to Example 4 was vacuum deposited to provide a 300Å-thick hole transport layer (HTL). A 250Å-thick emission layer was vacuum deposited on the hole transport layer (HTL) using 9,10-di-(2-naphthyl)anthracene (ADN) as a host and 3wt% of 2,5,8,11-tetra(tert-butyl)perylene (TBPe) as a dopant.

Next, Alq₃ was vacuum-deposited to be 250Å thick on the emission layer, forming an electron transport layer (ETL). On the electron transport layer (ETL), LiF at 10Å and Al at 1000Å were sequentially vacuum-deposited to fabricate a cathode, completing an organic light emitting diode.

The organic light emitting diode had five organic thin layers.

In particular, it had Al (1000Å)/LiF (10Å)/Alq3 (250Å)/EML[ADN:TBPe=97:3] (250 Å)/HTL (300Å)/DNTPD (600Å)/ITO (1500A).

### Example 42

An organic light emitting diode was prepared with the same method as Example 41, except for using Example 5 instead of Example 4.

### Example 43

An organic light emitting diode was prepared with the same method as Example 41, except for using Example 6 instead of Example 4.

### Example 44

An organic light emitting diode was prepared with the same method as Example 41, except for using Example 7 instead of Example 4.

### Example 45

An organic light emitting diode was prepared with the same method as Example 41, except for using Example 9 instead of Example 4.

### Example 46

An organic light emitting diode was prepared with the same method as Example 41, except for using Example 10 instead of Example 4.

### Example 47

An organic light emitting diode was prepared with the same method as Example 41, except for using Example 38 instead of Example 4.

### Example 48

An organic light emitting diode was prepared with the same method as Example 41, except for using Example 39 instead of Example 4.

### Comparative Example 1

An organic light emitting diode was prepared with the same method as Example 41, except for using NPB instead of Example 4. The structure of NPB is shown in the following.

### Comparative Example 2

An organic light emitting diode was prepared with the same method as Example 41, except for using HT1 instead of Example 4. The structure of HT1 is shown in the following.

### Comparative Example 3

An organic light emitting diode was fabricated in accordance with the same procedure as in Example 41, except that HT2 was used instead of the Example 41. The structure of HT2 is described in the following.

The structure of DNTPD, ADN, TBPe, NPB, HT1, and HT2 that are used for preparing the organic light emitting diode is shown as follows.

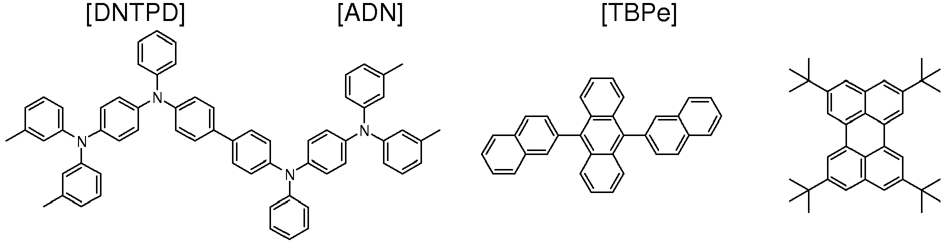

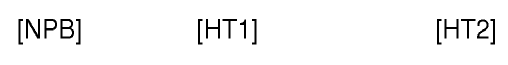

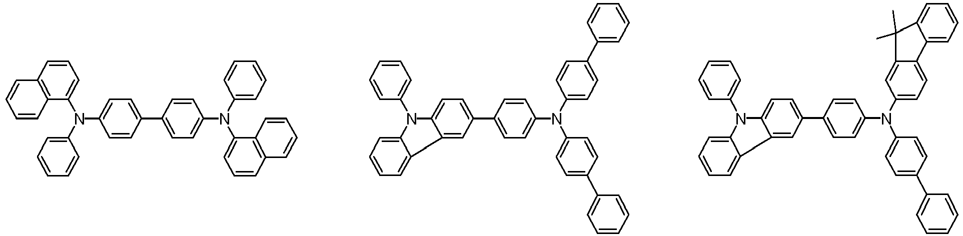

### Analysis and Characteristic Measurement of the Compounds

### Analysis of ¹H-NMR result

In order to structural analyze the intermediate M-1 to M-42 compounds of Examples 1 to 40, the molecular weight was measured using GC-MS or LC-MS, and ¹H-NMR was measured by dissolving the intermediate M-1 to M-42 compounds in a CD₂Cl₂ solvent or a CDCl₃solvent and using 300 MHz NMR equipment.

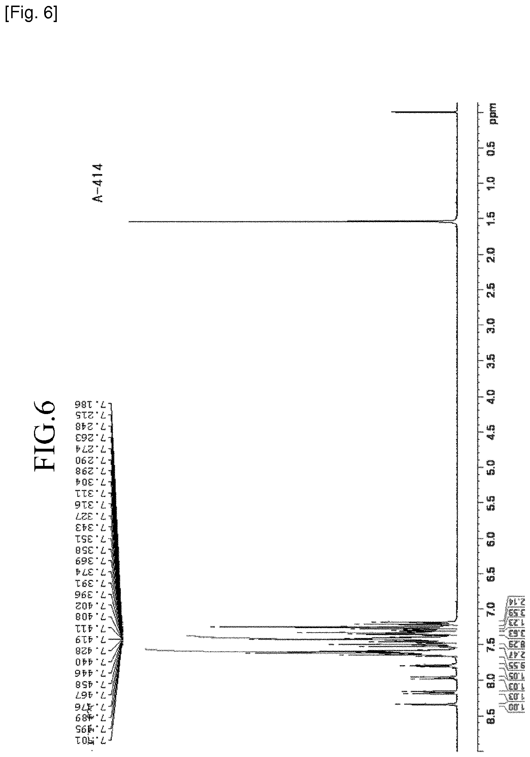

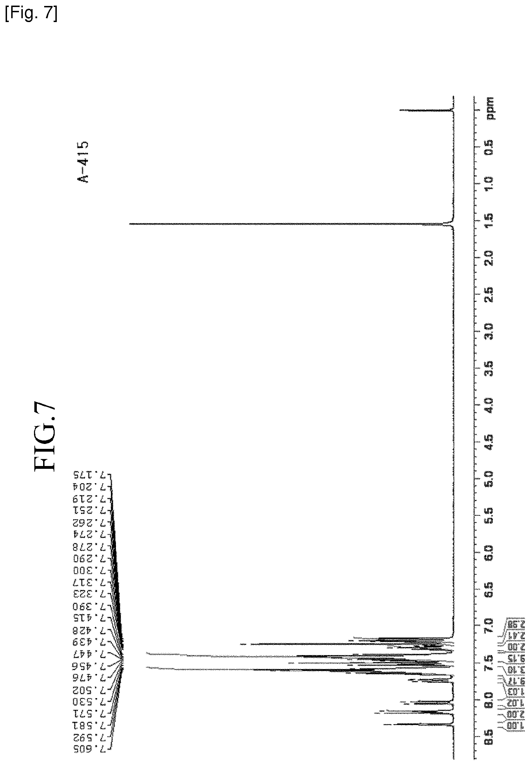

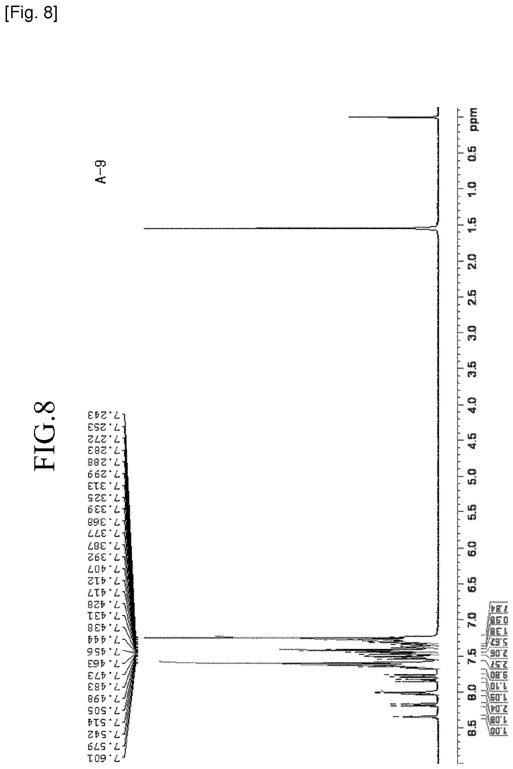

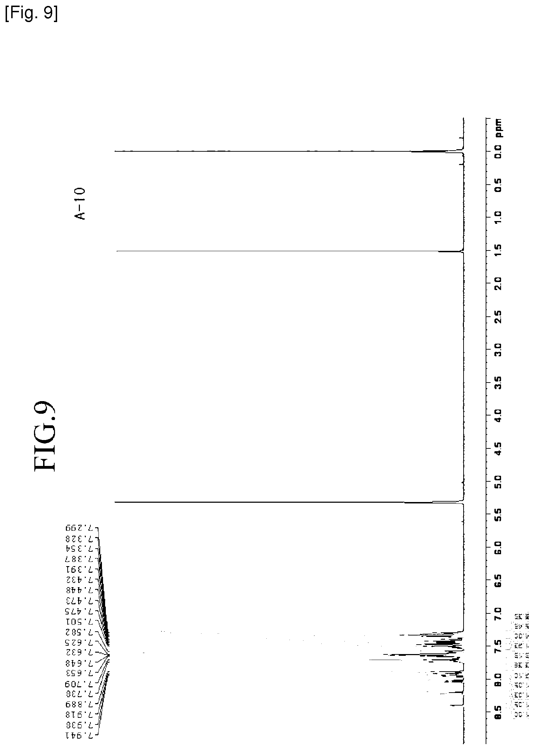

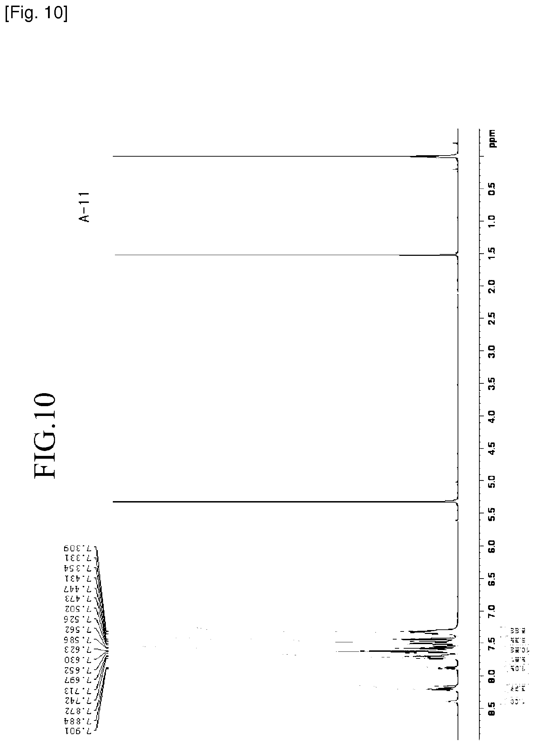

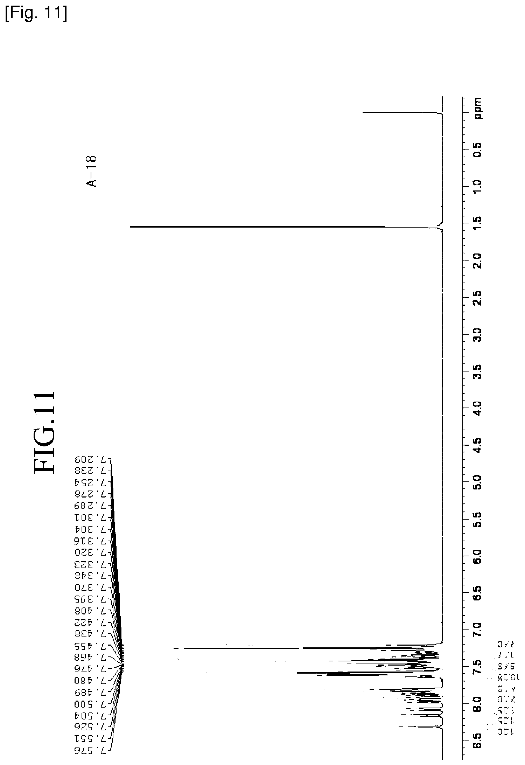

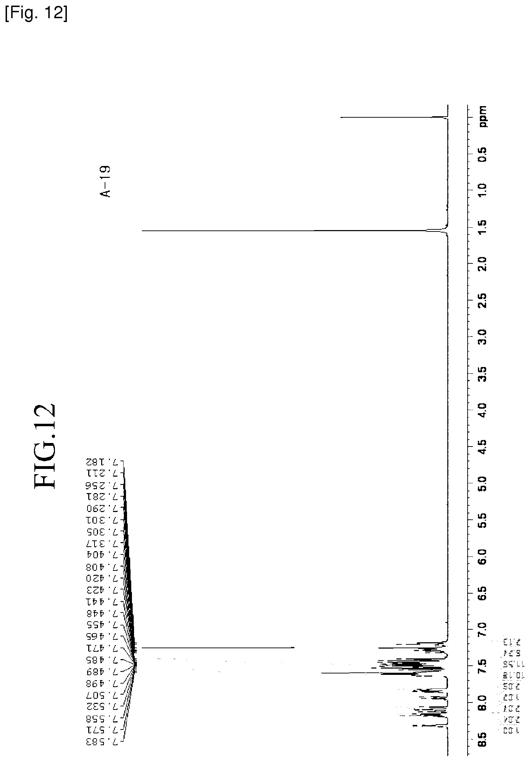

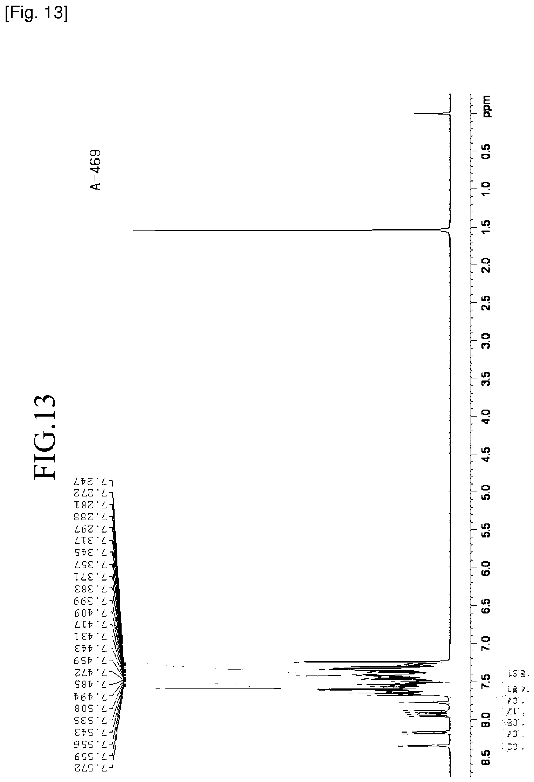

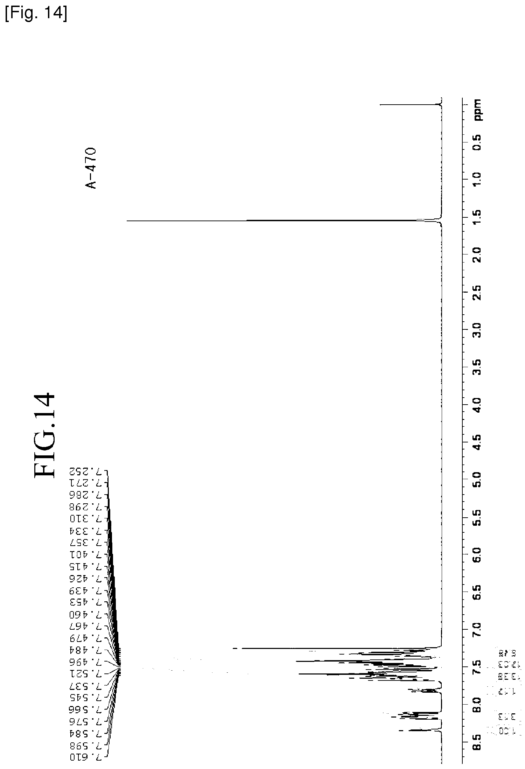

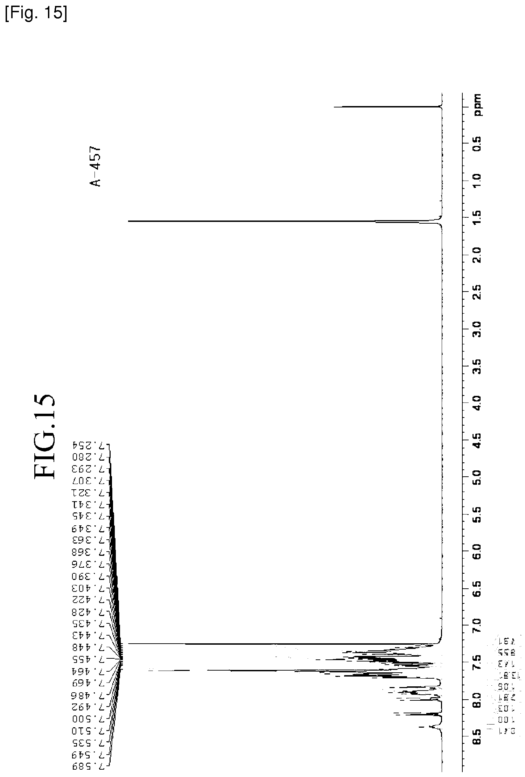

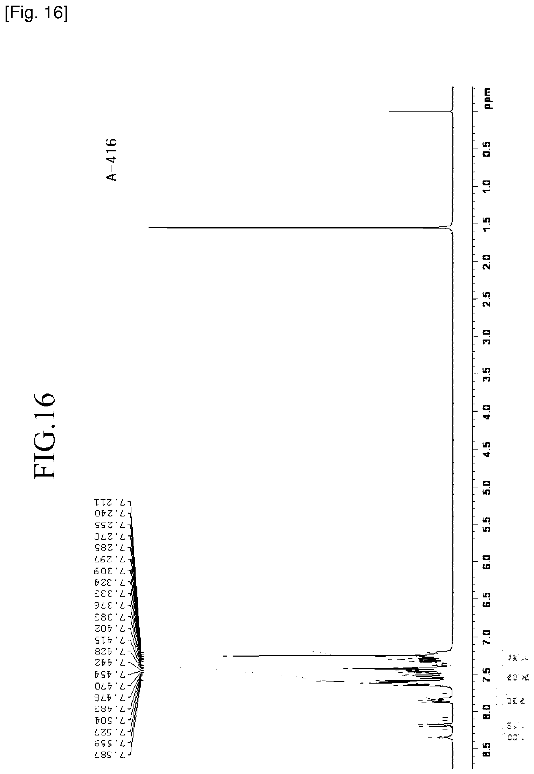

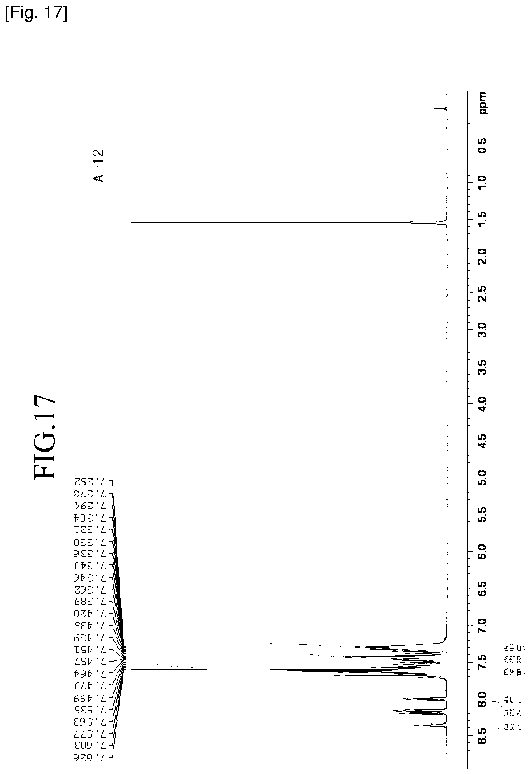

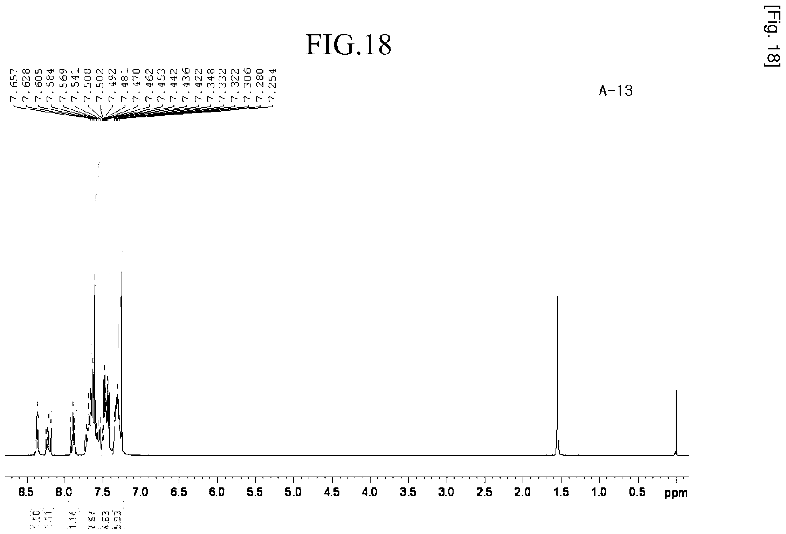

As an example of the analysis, FIG. 6 shows the ¹H-NMR result of A-414 according to Example 1, FIG. 7 shows the result of A-415 according to Example 2, FIG. 8 shows the result of A-9 according to Example 3, FIG. 9 shows the result of A-10 according to Example 4, FIG. 10 shows the result of A-11 according to Example 5, FIG. 11 shows the result of A-18 according to Example 6, FIG. 12 shows the result of A-19 according to Example 7, FIG. 13 shows the result of A-469 according to Example 27, FIG. 14 shows the result fo A-470 according to Example 28, FIG. 15 shows the result of A-457 according to Example 29, FIG. 16 shows the result of A-416 according to Example 37, FIG. 17 shows the result of A-12 according to Example 38, and FIG. 18 shows the result of A-13 according to Example 39.

### Fluorescent Characteristic Analysis

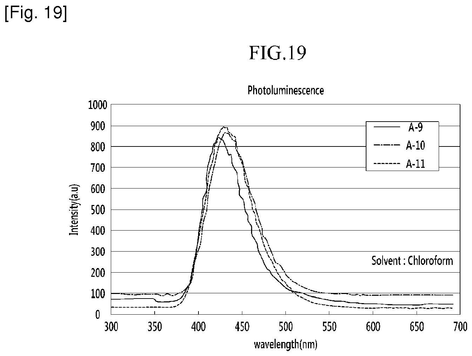

The compounds of the examples were dissolved in THF, and PL (photoluminescence) wavelength was measured using HITACHI F-4500 equipment to measure fluorescent characteristics. FIG. 19 shows the PL wavelength measurement results of Examples 3, 4, and 5.

### Electrochemical Characteristict

The compounds of Examples 1, 2, 3, 4, and 5 were measured regarding electrochemical characteristics by using cyclic voltammetry equipment. The results are provided in Table 1.

**[Table 1]**

| Synthesis Example | Example 1 A-414 | Example 2 A-415 | Example 3 A-9 | Example 4 A-10 | Example 5 A-11 |
|---|---|---|---|---|---|
| HOMO (eV) | 5.24 | 5.23 | 5.23 | 5.22 | 5.27 |
| LUMO (eV) | 2.16 | 2.17 | 2.16 | 2.15 | 2.17 |
| Band gap (eV) | 3.08 | 3.06 | 3.07 | 3.07 | 3.10 |

Referring to Table 1, the compounds according to Examples 1 to 5 turned out to be able to be used as a hole transporting layer and an electron blocking layer.

### Thermal Characteristic

Thermal decomposition temperature of the compounds synthesized according to Examples 1, 2, 3, 4, 5, 6, 7, 27, 28, 29, 37, 38, and 39 were measured by thermogravimetry (TGA) to show the thermal characteristics. The synthesized compounds were measured to determine glass transition temperature (Tg) by differential scanning calorimetry (DSC). The results are shown in the following Table 2.

**[Table 2]**

| Example | Material | Thermal decomposition temperature (°C) | Tg (°C) |
|---|---|---|---|
| Example 1 | A-414 | 485 | 124 |
| Example 2 | A-415 | 460 | 133 |
| Example 3 | A-9 | 475 | 132 |
| Example 4 | A-10 | 522 | 128 |
| Example 5 | A-11 | 532 | 133 |
| Example 6 | A-18 | 506 | 137 |
| Example 7 | A-19 | 520 | 141 |
| Example 27 | A-469 | 503 | 122 |
| Example 28 | A-470 | 511 | 124 |
| Example 29 | A-457 | 546 | 125 |
| Example 37 | A-416 | 449 | 135 |
| Example 38 | A-12 | 516 | 125 |
| Example 39 | A-13 | 531 | 133 |

Referring to Table 2, all of the compounds according to Example 1, 2, 3, 4, 5, 6, 7, 27, 28, 29, 37, 38, and 39 had excellent thermal stability, excellent thermal decomposition temperature of 400°C or higher, and Tg higher than 90°C. When it is used as a material for an organicelectric field light emitting diode (OLED), it may have good life-span characteristics. Also when it is used for preparing an organic light emitting diode with process heat, it may have excellent process stability.

### Performance Measurement of Organic Light Emitting Diode

The organic light emitting emements of Examples 41 to 48 and Comparative Examples 1 to 3 were measured regarding current density and luminance changes depending on voltage change. In particular, the measurements were performed as follows. The results are shown in the following Table 2.

### (1) Corrent density change measurement depending on voltage

The organic light emitting diodes were respectively measured regarding a current in a unit device by using a current-voltage meter (Keithley 2400) while their voltages were increased from 0V. Each current value was divided by area, measuring current density.

### (2) Luminance change measurement depending on voltage change

The prepared organic light emitting diode was measured regarding luminance while its voltage was increased from 0V to 10V by using a luminance meter (Minolta Cs-1000A).

### (3) Luminous efficiency measurement

The organic light emitting diode were measured by using luminance, current density, and voltage measured from (1) and (2) regarding current efficiency (cd/A) at the same current density (10mA/cm²).

**[Table 3]**

| Device | Compound used in hole transport layer (HTL) | Voltage(V) | Color (EL color) | Efficiency (cd/A) | Half-life (h) at 1000cd/m² |
|---|---|---|---|---|---|
| Example 41 | A-10 | 6.3 | Blue | 6.1 | 2170 |
| Example 42 | A-11 | 6.3 | Blue | 6.2 | 2290 |
| Example 43 | A-18 | 6.2 | Blue | 5.9 | 1870 |
| Example 44 | A-19 | 6.2 | Blue | 6.0 | 1910 |
| Example 45 | A-335 | 6.9 | Blue | 5.2 | 1570 |
| Example 46 | A-340 | 6.8 | Blue | 5.7 | 1490 |
| Example 47 | A-12 | 6.1 | Blue | 6.2 | 2150 |
| Example 48 | A-13 | 6.1 | Blue | 6.1 | 2230 |
| Comparative Example 1 | NPB | 7.1 | Blue | 4.9 | 1250 |
| Comparative Example 2 | HT1 | 6.6 | Blue | 5.7 | 1340 |
| Comparative Example 3 | HT2 | 6.4 | Blue | 5.9 | 1350 |

Current density: 10mA/cm²

Referring to the results of [Table 3], the materials that were used for preparing the hole transport layer (HTL) of Examples 41 to 48 turned out to decrease driving voltage of the organic light emitting diode but improved luminance and efficiency.

Further, the half-life of Examples 41 to 48 were remarkably improved compared to the half-life of Comparative Examples 1 to 3, particularly, the organic light emitting diode of Example 42 had a half-life of 2290 hours, which was 1.8 times improved compared to that of Comparative Example 1 of 1250 hours. In terms of commercial selling, the life-span of a device is one of the biggest issues for commercializing a device. Therefore, the devices according to the exemplary embodiments are shown as sufficient to be commercialized.

While this invention has been described in connection with what is presently considered to be practical exemplary embodiments, it is to be understood that the invention is not limited to the disclosed embodiments, but, on the contrary, is intended to cover various modifications and equivalent arrangements included within the scope of the appended claims. Therefore, the aforementioned embodiments should be understood to be exemplary but not limiting the present invention in any way.

### <Description of Symbols>

100: organic photoelectric device 110: cathode
120: anode 105: organic thin film
130: emission layer 140: hole transport layer (HTL)
150: electron transport layer (ETL) 160: electron injection layer (EIL)
170: hole injection layer (HIL) 230: emission layer + electron transport layer (ETL)

## Claims

1. A compound for an optoelectronic device represented by the following Chemical Formula 1: 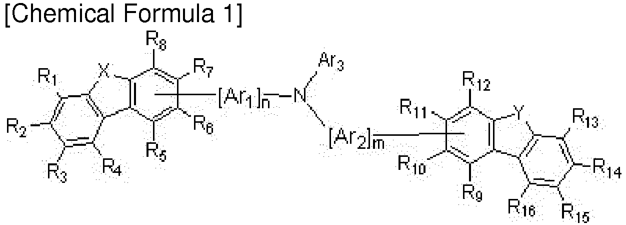 wherein in Chemical Formula 1,
R₁ to R₁₆ are the same or different, and are independently selected from hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonyl amino group, a substituted or unsubstituted C7 to C20 aryloxycarbonyl amino group, a substituted or unsubstituted C1 to C20 sulfamoyl amino group, a substituted or unsubstituted C1 to C20 sulfonyl group, a substituted or unsubstituted C1 to C20 alkylthiol group, a substituted or unsubstituted C6 to C20 arylthiol group, a substituted or unsubstituted C1 to C20 heterocyclothiol group, a substituted or unsubstituted C1 to C20 ureide group, and a substituted or unsubstituted C3 to C40 silyl group,
at least one of R₁ to R₈ represents a bond with Ar₁,
at least one of R₉ to R₁₆ represents a bond with Ar₂,
at least one of R₁ to R₈ is optionally bound to Ar₁ through a sigma bond, at least one of R₉ to R₁₆ is optionally bound to Ar₂ through a sigma bond,
X is selected from NR₁₇, O, S, and SO₂ (O=S=O), wherein R₁₇ is a substituted or unsubstituted C1 to C20 alkyl group, a substituted or unsubstituted C6 to C30 aryl group, or a substituted or unsubstituted C2 to C30 heteroaryl group,
Y is selected from O, S, and SO₂ (O=S=O),
Ar₁ and Ar₂ are the same or different, and are independently a substituted or unsubstituted C6 to C30 aryl group or a substituted or unsubstituted C2 to C30 heteroaryl group,
n is an integer ranging from 1 to 4,
m is an integer ranging from 0 to 4, and
Ar₃ is a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthydinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, or a substituted or unsubstituted phenoxazinyl group..

2. The compound of claim 1, wherein X is selected from NR₁₇, O, S, and SO₂ (O=S=O), wherein R₁₇ is a substituted or unsubstituted C1 to C20 alkyl group, a substituted or unsubstituted C6 to C30 aryl group, or a substituted or unsubstituted C2 to C30 heteroaryl group, and the "substituted" aryl group or heteroaryl group refers to one substituted with at least one substituent selected from deuterium, a halogen, a cyano group, hydroxy group, an amino group, a substituted or unsubstituted C1 to C20 amine group, a nitro group, a substituted or unsubstituted C1 to C20 alkyl group, a substituted or unsubstituted C1 to C20 alkoxy group, a substituted or unsubstituted C3 to C40 silyl group, and a combination thereof.

3. The compound of claim 1, wherein the compound for an optoelectronic device comprises one of the compounds represented by the following Chemical Formulae 2 to 7: 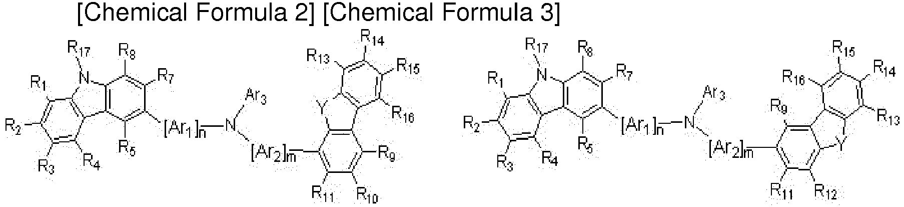 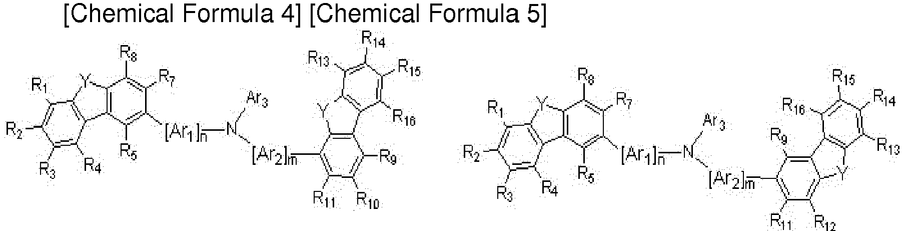 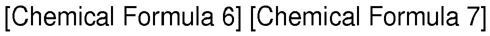 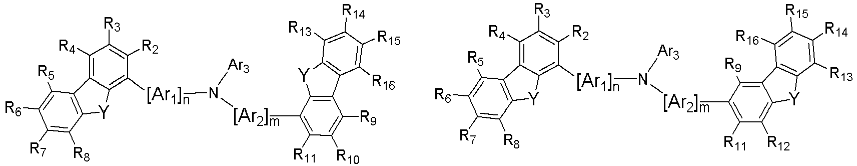 wherein in Chemical Formulae 2 to 7,
R₁ to R₁₆ are the same or different, and are independently selected from hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonyl amino group, a substituted or unsubstituted C7 to C20 aryloxycarbonyl amino group, a substituted or unsubstituted C1 to C20 sulfamoyl amino group, a substituted or unsubstituted C1 to C20 sulfonyl group, a substituted or unsubstituted C1 to C20 alkylthiol group, a substituted or unsubstituted C6 to C20 arylthiol group, a substituted or unsubstituted C1 to C20 heterocyclothiol group, a substituted or unsubstituted C1 to C20 ureide group, and a substituted or unsubstituted C3 to C40 silyl group,
R₁₇ is a substituted or unsubstituted C1 to C20 alkyl group, a substituted or unsubstituted C6 to C30 aryl group, or a substituted or unsubstituted C2 to C30 heteroaryl group,
Y is selected from O, S, and SO₂ (O=S=O),
Ar₁ and Ar₂ are the same or different, and are independently a substituted or unsubstituted C6 to C30 aryl group or a substituted or unsubstituted C2 to C30 heteroaryl group,
n is an integer ranging from 1 to 4,
m is an integer ranging from 0 to 4, and
Ar₃ is a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthydinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, or a substituted or unsubstituted phenoxazinyl group..

4. The compound of claim 1, wherein the compound for an optoelectronic device comprises one of the compounds represented by the following Chemical Formulae 8 or 9: 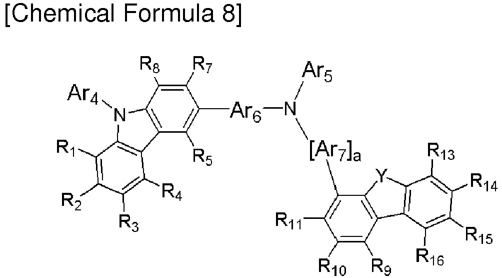 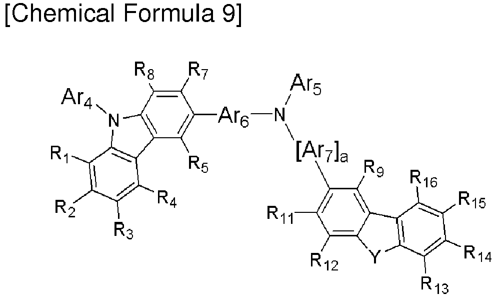 wherein in Chemical Formulae 8 and 9,
Ar₄ is selected from the substituent represented by the following Chemical Formulae 10 to 18, and
Ar₅ is selected from a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthydinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, or a substituted or unsubstituted phenoxazinyl group. 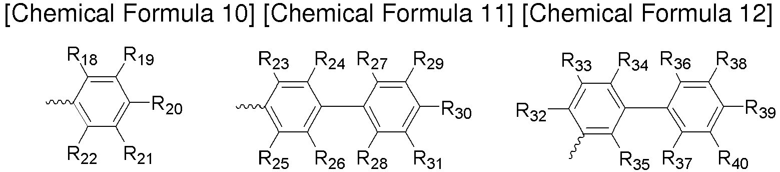 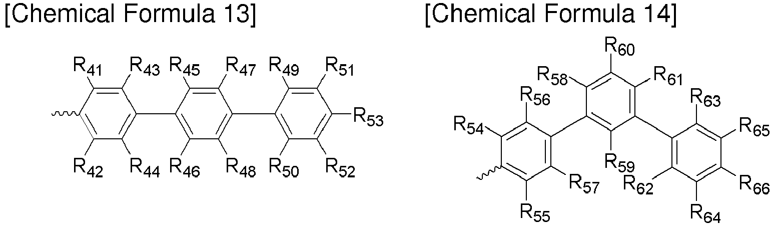 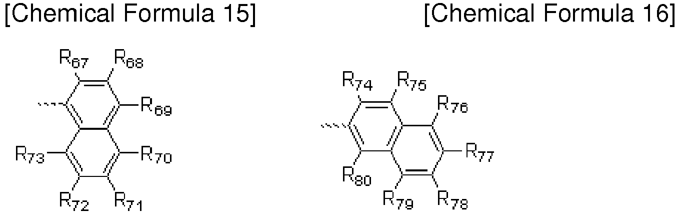 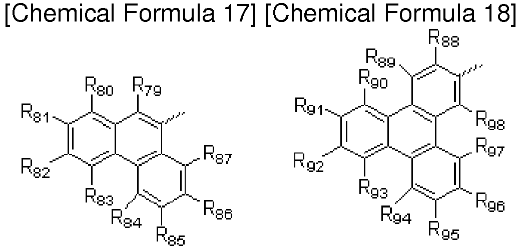
R₁ to R₅, R₇ to R₁₆, and R₁₈ to R₉₈ are the same or different, and are independently selected from hydrogen, deuterium, a halogen, a cyano group, a hydroxyl group, an amino group, a substituted or unsubstituted C1 to C20 amine group, a nitro group, a substituted or unsubstituted C1 to C20 alkyl group, a substituted or unsubstituted C1 to C20 alkoxy group, or a substituted or unsubstituted C3 to C40 silyl group,
Ar₆ and Ar₇ are the same or different, and are independently a substituent selected from the above Chemical Formulae 10 to 18, and at least one of R₁₈ to R₉₈ is bound to an adjacent atom, and
a is 0 or 1.

5. The compound of claim 4, wherein Ar₄ is selected from a substituent represented by the above Formulae 10 to 18, and at least one of the substituents of R₁₈ to R₉₈ that is selected to Ar₄ is not hydrogen.

6. The compound of claim 1, wherein the compound is represented by the following Chemical Formulae A-321 to A-323.

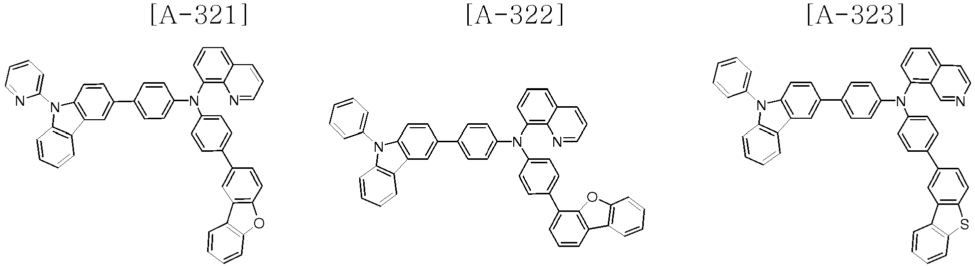

7. The A compound of claim 1, wherein the compound is represented by the following Chemical Formulae A-400, A-402 to A-404, A-407, A-408, A-411 and A-413.

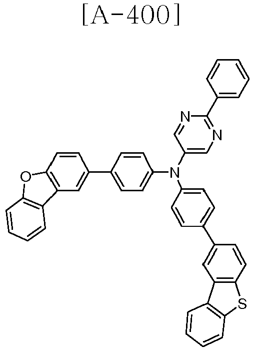

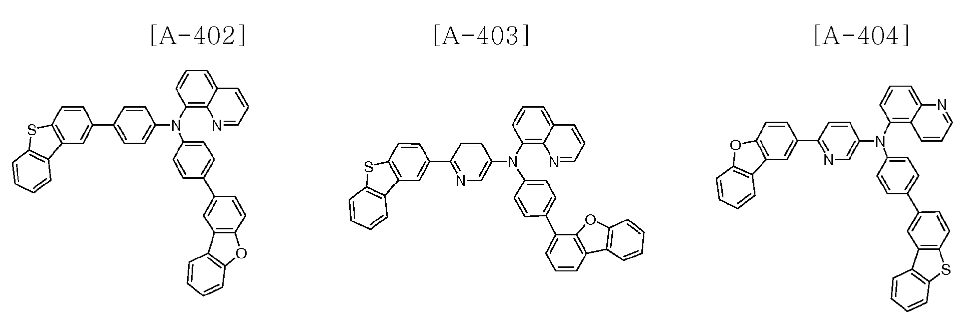

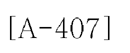

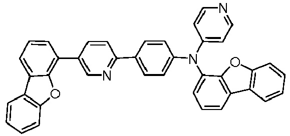

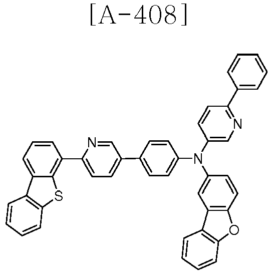

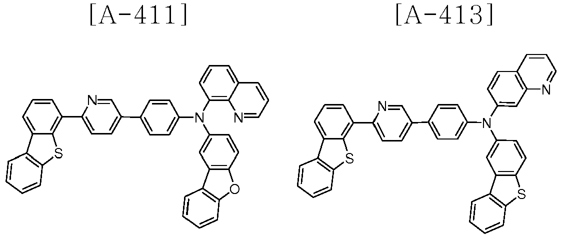

8. The compound of claim 1, wherein the compound for an optoelectronic device comprises a hole transport material or a hole injection material for an organic light emitting diode.

9. The compound of claim 1, wherein the compound for an optoelectronic device comprises triplet exciton energy (T1) of about 2.0eV or higher.

10. The compound of claim 1, wherein the optoelectronic device comprises an organic photoelectronic device, an organic light emitting diode, an organic solar cell, an organic transistor, an organic photo-conductor drum, an organic memory device, or the like.

11. An organic light emitting diode including an anode, a cathode, and at least one or more organic thin film between the anode and the cathode is provided, and at least one of the organic thin films comprises the compound of claim 1.

12. The organic light emitting diode of claim 11, wherein the organic thin film comprises an emission layer, a hole transport layer (HTL), a hole injection layer (HIL), an electron transport layer (ETL), an electron injection layer (EIL), a holeblocking film, or a combination thereof.

13. The organic light emitting diode of claim 11, the compound for an optoelectronic device is included in a hole transport layer (HTL), a hole injection layer (HIL), an electron transport layer (ETL), or an electron injection layer (EIL).

14. The organic light emitting diode of claim 11, wherein the compound for an optoelectronic device is included in an emission layer.

15. The organic light emitting diode of claim 11, wherein the compound for an optoelectronic device is used as a phosphorescent or fluorescent host material in an emission layer.

16. A display device comprising the organic light emitting diode of claim 11.

## Patentansprüche

1. Verbindung für eine optoelektronische Vorrichtung, dargestellt durch die folgende Formel 1: 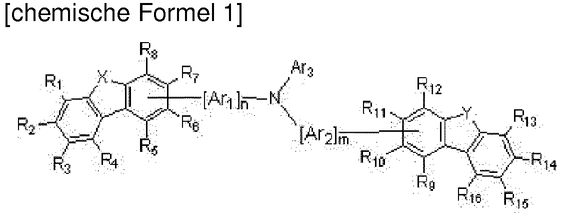 wobei in der chemischen Formel 1:
R₁ bis R₁₆ gleich oder verschieden und unabhängig ausgewählt sind aus Wasserstoff, Deuterium, einem Halogen, einer Cyanogruppe, einer Hydroxylgruppe, einer Aminogruppe, einer substituierten oder unsubstituierten C1- bis C20-Amingruppe, einer Nitrogruppe, einer Carboxylgruppe, einer Ferrocenylgruppe, einer substituierten oder unsubstituierten C1- bis C20-Al-kylgruppe, einer substituierten oder unsubstituierten C6- bis C30-Arylgruppe, einer substituierten oder unsubstituierten C2- bis C30-Heteroarylgruppe, einer substituierten oder unsubstituierten C1- bis C20-Alkoxygruppe, einer substituierten oder unsubstituierten C6- bis C20-Aryloxygruppe, einer substituierten oder unsubstituierten C3- bis C40-Silyloxygruppe, einer substituierten oder unsubstituierten C1- bis C20-Acylgruppe, einer substituierten oder unsubstituierten C2- bis C20-Alkoxycarbonylgruppe, einer substituierten oder unsubstituierten C2- bis C20-Acyloxygruppe, einer substituierten oder unsubstituierten C2- bis C20-Acylaminogruppe, einer substituierten oder unsubstituierten C2- bis C20-Alkoxycarbonylaminogruppe, einer substituierten oder unsubstituierten C7- bis C20-Aryloxycarbonylaminogruppe, einer substituierten oder unsubstituierten C1- bis C20-Sulfamoylaminogruppe, einer substituierten oder unsubstituierten C1- bis C20-Sulfonylgruppe, einer substituierten oder unsubstituierten C1- bis C20-Alkylthiolgruppe, einer substituierten oder unsubstituierten C6- bis C20-Arylthiolgruppe, einer substituierten oder unsubstituierten C1- bis C20-Heterocyclothiolgruppe, einer substituierten oder unsubstituierten C1- bis C20-Ureidgruppe und einer substituierten oder unsubstituierten C3- bis C40-Silylgruppe,
mindestens eine der Komponenten R₁ bis R₈ eine Bindung mit Ar₁ darstellt,
mindestens eine der Komponenten R₉ bis R₁₆ eine Bindung mit Ar₂ darstellt,
mindestens eine der Komponenten R₁ bis R₈ optional über eine Sigma-Bindung mit Ar₁ verbunden ist,
mindestens eine der Komponenten R₉ bis R₁₆ optional über eine Sigma-Bindung mit Ar₂ verbunden ist,
X ausgewählt ist aus NR₁₇, O, S und SO₂ (O=S=O), wobei R₁₇ eine substituierte oder unsubstituierte C1- bis C20-Alkylgruppe, eine substituierte oder unsubstituierte C6- bis C30-Arylgruppe oder eine substituierte oder unsubstituierte C2- bis C30-Heteroarylgruppe ist,
Y ausgewählt ist aus O, S und SO₂ (O=S=O),
Ar₁ und Ar₂ gleich oder verschieden und unabhängig eine substituierte oder unsubstituierte C6- bis C30-Arylgruppe oder eine substituierte oder unsubstituierte C2- bis C30-Heteroarylgruppe sind,
n eine ganze Zahl von 1 bis 4 ist,
m eine ganze Zahl von 0 bis 4 ist, und
Ar₃ eine substituierte oder unsubstituierte Pyrrolylgruppe, eine substituierte oder unsubstituierte Pyrazolylgruppe, eine substituierte oder unsubstituierte Imidazolylgruppe, eine substituierte oder unsubstituierte Triazolylgruppe, eine substituierte oder unsubstituierte Oxazolylgruppe, eine substituierte oder unsubstituierte Thiazolylgruppe, eine substituierte oder unsubstituierte Oxadiazolylgruppe, eine substituierte oder unsubstituierte Thiadiazolylgruppe, eine substituierte oder unsubstituierte Pyridylgruppe, eine substituierte oder unsubstituierte Pyrimidinylgruppe, eine substituierte oder unsubstituierte Pyrazinylgruppe, eine substituierte oder unsubstituierte Triazinylgruppe, eine substituierte oder unsubstituierte Benzimidazolylgruppe, eine substituierte oder unsubstituierte Indolylgruppe, eine substituierte oder unsubstituierte Chinolinylgruppe, eine substituierte oder unsubstituierte Isochinolinylgruppe , eine substituierte oder unsubstituierte Chinazolinylgruppe, eine substituierte oder unsubstituierte Chinoxalinylgruppe, eine substituierte oder unsubstituierte Naphthydinylgruppe, eine substituierte oder unsubstituierte Benzoxazinylgruppe, eine substituierte oder unsubstituierte Benzthiazinylgruppe, eine substituierte oder unsubstituierte Acridinylgruppe, eine substituierte oder unsubstituierte Phenazinylgruppe, eine substituierte oder unsubstituierte Phenothiazinylgruppe oder eine substituierte oder unsubstituierte Phenoxazinylgruppe ist.

2. Verbindung nach Anspruch 1, wobei X ausgewählt ist aus NR₁₇, O, S und SO₂ (O=S=O), wobei R₁₇ eine substituierte oder unsubstituierte C1- bis C20-Alkylgruppe, eine substituierte oder unsubstituierte C6- bis C30-Arylgruppe oder eine substituierte oder unsubstituierte C2- bis C30-Heteroarylgruppe ist, und wobei die "substituierte" Arylgruppe oder Heteroarylgruppe eine Gruppe bezeichnet, die mit mindestens einem Substituienten substituiert ist, der ausgewählt ist aus Deuterium, einem Halogen, einer Cyanogruppe, einer Hydroxygruppe, einer Aminogruppe, einer substituierten oder unsubstituierten C1- bis C20-Amingruppe, einer Nitrogruppe, einer substituierten oder unsubstituierten C1- bis C20-Alkylgruppe, einer substituierten oder unsubstituierten C1- bis C20- Alkoxygruppe, einer substituierten oder unsubstituierten C3- bis C40-Silylgruppe und einer Kombination davon.

3. Verbindung nach Anspruch 1, wobei die Verbindung für eine optoelektronische Vorrichtung eine Verbindung aufweist, die durch die folgenden chemischen Formeln 2 bis 7 dargestellt wird: 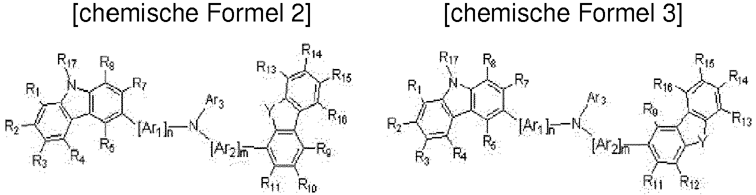 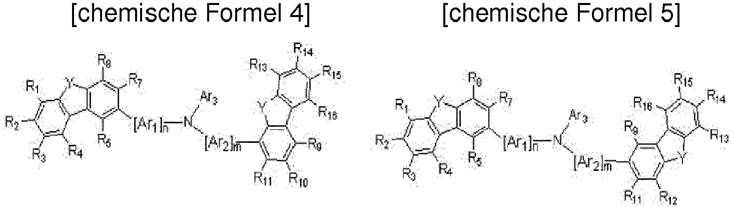 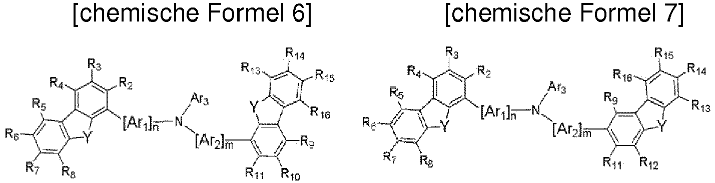 wobei in den chemischen Formeln 2 bis 7:
R₁ bis R₁₆ gleich oder verschieden und unabhängig ausgewählt sind aus Wasserstoff, Deuterium, einem Halogen, einer Cyanogruppe, einer Hydroxylgruppe, einer Aminogruppe, einer substituierten oder unsubstituierten C1- bis C20-Amingruppe, einer Nitrogruppe, einer Carboxylgruppe, einer Ferrocenylgruppe, einer substituierten oder unsubstituierten C1- bis C20-Al-kylgruppe, einer substituierten oder unsubstituierten C6- bis C30-Arylgruppe, einer substituierten oder unsubstituierten C2- bis C30-Heteroarylgruppe, einer substituierten oder unsubstituierten C1- bis C20-Alkoxygruppe, einer substituierten oder unsubstituierten C6- bis C20-Aryloxygruppe, einer substituierten oder unsubstituierten C3- bis C40-Silyloxygruppe, einer substituierten oder unsubstituierten C1- bis C20-Acylgruppe, einer substituierten oder unsubstituierten C2- bis C20-Alkoxycarbonylgruppe, einer substituierten oder unsubstituierten C2- bis C20-Acyloxygruppe, einer substituierten oder unsubstituierten C2- bis C20-Acylaminogruppe, einer substituierten oder unsubstituierten C2- bis C20-Alkoxycarbonylaminogruppe, einer substituierten oder unsubstituierten C7- bis C20-Aryloxycarbonylamino-gruppe, einer substituierten oder unsubstituierten C1- bis C20-Sulfamoylaminogruppe, einer substituierten oder unsubstituierten C1- bis C20-Sulfonylgruppe, einer substituierten oder unsubstituierten C1- bis C20-Alkylthiolgruppe, einer substituierten oder unsubstituierten C6- bis C20-Arylthiolgruppe, einer substituierten oder unsubstituierten C1- bis C20-Heterocyclothiolgruppe, einer substituierten oder unsubstituierten C1- bis C20-Ureidgruppe und einer substituierten oder unsubstituierten C3- bis C40-Silylgruppe,
R₁₇ eine substituierte oder unsubstituierte C1- bis C20-Alkylgruppe, eine substituierte oder unsubstituierte C6- bis C30-Arylgruppe oder eine substituierte oder unsubstituierte C2- bis C30-Heteroarylgruppe ist,
Y ausgewählt ist aus O, S und SO₂ (O=S=O),
Ar₁ und Ar₂ gleich oder verschieden und unabhängig eine substituierte oder unsubstituierte C6- bis C30-Arylgruppe oder eine substituierte oder unsubstituierte C2- bis C30-Heteroarylgruppe sind,
n eine ganze Zahl von 1 bis 4 ist,
m eine ganze Zahl von 0 bis 4 ist, und
Ar₃ eine substituierte oder unsubstituierte Pyrrolylgruppe, eine substituierte oder unsubstituierte Pyrazolylgruppe, eine substituierte oder unsubstituierte Imidazolylgruppe, eine substituierte oder unsubstituierte Triazolylgruppe, eine substituierte oder unsubstituierte Oxazolylgruppe, eine substituierte oder unsubstituierte Thiazolylgruppe, eine substituierte oder unsubstituierte Oxadiazolylgruppe, eine substituierte oder unsubstituierte Thiadiazolylgruppe, eine substituierte ode unsubstituierte Pyridylgruppe, eine substituierte oder unsubstituierte Pyrimidinylgruppe, eine substituierte oder unsubstituierte Pyrazinylgruppe, eine substituierte oder unsubstituierte Triazinylgruppe, eine substituierte oder unsubstituierte Benzimidazolylgruppe, eine substituierte oder unsubstituierte Indolylgruppe, eine substituierte oder unsubstituierte Chinolinylgruppe, eine substituierte oder unsubstituierte Isochinolinylgruppe , eine substituierte oder unsubstituierte Chinazolinylgruppe, eine substituierte oder unsubstituierte Chinoxalinylgruppe, eine substituierte oder unsubstituierte Naphthydinylgruppe, eine substituierte oder unsubstituierte Benzoxazinylgruppe, eine substituierte oder unsubstituierte Benzthiazinylgruppe, eine substituierte oder unsubstituierte Acridinylgruppe, eine substituierte oder unsubstituierte Phenazinylgruppe, eine substituierte oder unsubstituierte Phenothiazinylgruppe oder eine substituierte oder unsubstituierte Phenoxazinylgruppe ist.

4. Verbindung nach Anspruch 1, wobei die Verbindung für eine optoelektronische Vorrichtung eine Verbindung aufweist, die durch die folgenden chemischen Formeln 8 oder 9 dargestellt wird: 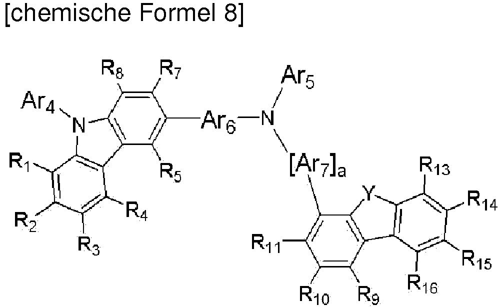 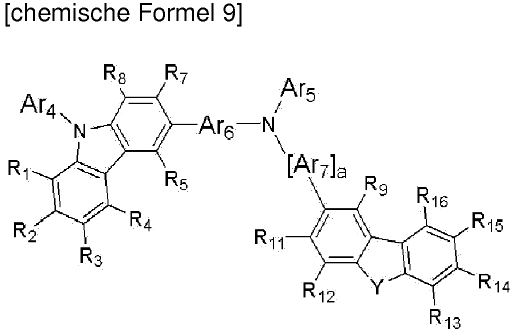 wobei in den chemischen Formeln 8 und 9
Ar₄ ausgewählt ist aus einem Substituenten, der durch die folgenden chemischen Formeln 10 bis 18 dargestellt wird, und
Ar₅ ausgewählt ist aus einer substituierten oder unsubstituierten Pyrrolylgruppe, einer substituierten oder unsubstituierten Pyrazolylgruppe, einer substituierten oder unsubstituierten Imidazolylgruppe, einer substituierten oder unsubstituierten Triazolylgruppe, einer substituierten oder unsubstituierten Oxazolylgruppe, einer substituierten oder unsubstituierten Thiazolylgruppe, einer substituierten oder unsubstituierten Oxadiazolylgruppe, einer substituierten oder unsubstituierten Thiadiazolylgruppe, einer substituierten oder unsubstituierten Pyridylgruppe, einer substituierten oder unsubstituierten Pyrimidinylgruppe, einer substituierten oder unsubstituierten Pyrazinylgruppe, einer substituierten oder unsubstituierten Triazinylgruppe, einer substituierten oder unsubstituierten Benzimidazolylgruppe, einer substituierten oder unsubstituierten Indolylgruppe, einer substituierten oder unsubstituierten Chinolinylgruppe, einer substituierten oder unsubstituierten Isochinolinylgruppe, einer substituierten oder unsubstituierten Chinazolinylgruppe, einer substituierten oder unsubstituierten Chinoxalinylgruppe, einer substituierten oder unsubstituierten Naphthydinylgruppe, einer substituierten oder unsubstituierten Benzoxazinylgruppe, einer substituierten oder unsubstituierten Benzthiazinylgruppe, einer substituierten oder unsubstituierten Acridinylgruppe, einer substituierten oder unsubstituierten Phenazinylgruppe, einer substituierten oder unsubstituierten Phenothiazinylgruppe oder einer substituierten oder unsubstituierten Phenoxazinylgruppe, 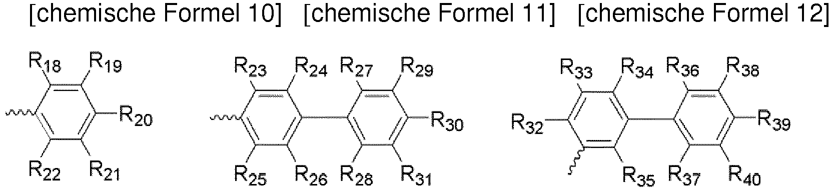 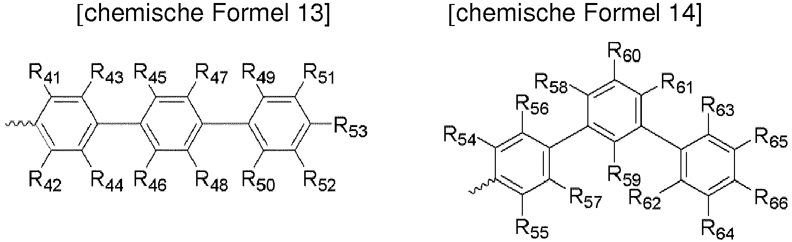 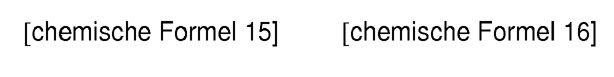 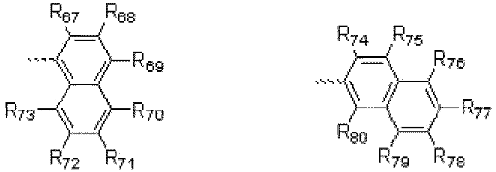 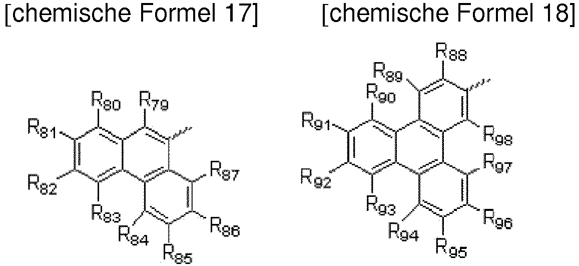
wobei R₁ bis R₅, R₇ bis R₁₆ und R₁₈ bis R₉₈ gleich oder verschieden und unabhängig ausgewählt sind aus Wasserstoff, Deuterium, einem Halogen, einer Cyanogruppe, einer Hydroxylgruppe, einer Aminogruppe, einer substituierten oder unsubstituierten C1- bis C20-Amingruppe, einer Nitrogruppe, einer substituierten oder unsubstituierten C1- bis C20-Alkylgruppe, einer substituierten oder unsubstituierten C1- bis C20-Alkoxygruppe oder substituierten oder unsubstituierten C3- bis C40-Silylgruppe,
Ar₆ und Ar₄ gleich oder verschieden und unabhängig ein Substituent sind, der ausgewählt ist aus den vorstehenden Formeln 10 bis 18, und mindestens eine der Komponenten R₁₈ bis R₉₈ mit einem benachbarten Atom verbunden ist, und
a = 0 oder 1 ist.

5. Verbindung nach Anspruch 4, wobei Ar₄ ausgewählt ist aus einem Substituenten, der durch die folgenden chemischen Formeln 10 bis 18 dargestellt wird, und mindestens einer der Substituenten von R₁₈ bis R₉₈, der als Ar₄ ausgewählt ist, nicht Wasserstoff ist.

6. Verbindung nach Anspruch 1, wobei die Verbindung dargestellt wird durch die folgenden chemischen Formeln A-321 bis A-323:

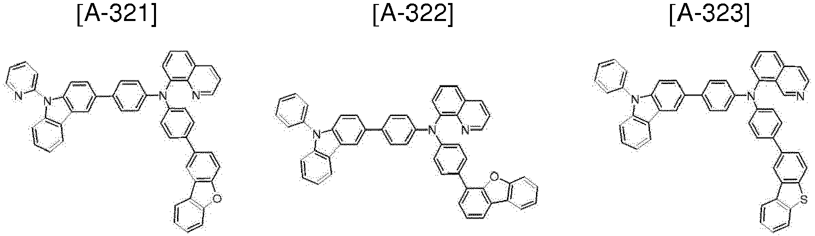

7. Verbindung nach Anspruch 1, wobei die Verbindung dargestellt wird durch die folgenden chemischen Formeln A-400, A-402 bis A-404, A-407, A-408, A-411 und A-413:

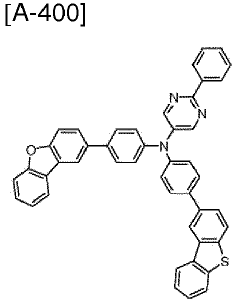

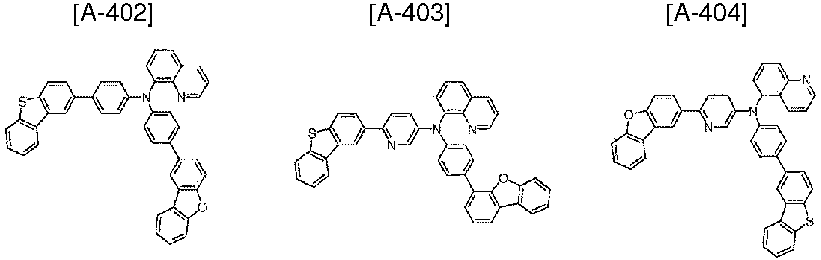

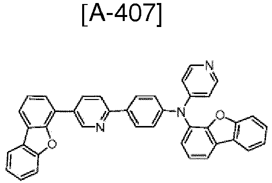

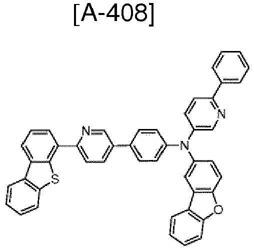

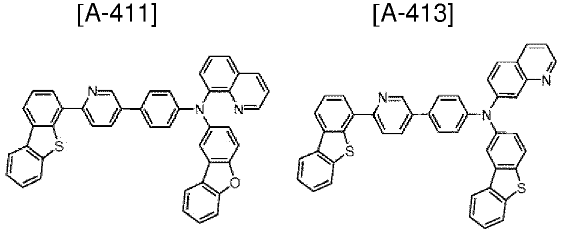

8. Verbindung nach Anspruch 1, wobei die Verbindung für eine optoelektronische Vorrichtung ein Lochtransportmaterial oder ein Lochinjektionsmaterial für eine organische Leuchtdiode aufweist.

9. Verbindung nach Anspruch 1, wobei die Verbindung für eine optoelektronische Vorrichtung eine Triplet-Anregungsenergie (T1) von etwa 2,0 eV oder mehr aufweist.

10. Verbindung nach Anspruch 1, wobei die optoelektronische Vorrichtung eine organische photoelektronische Vorrichtung, eine organische Leuchtdiode, eine organische Solarzelle, einen organischen Transistor, eine organische Photoleitertrommel, eine organische Speichereinrichtung oder dergleichen aufweist.

11. Organische Leuchtdiode mit einer Anode, einer Kathode und mindestens einem oder mehreren organischen Dünnschichten zwischen der Anode und der Kathode, wobei mindestens eine der Dünnschichten die Verbindung nach Anspruch 1 aufweist.

12. Organische Leuchtdiode nach Anspruch 11, wobei die organische Dünnschicht eine Emissionsschicht, eine Lochtransportschicht (HTL), eine Lochinjektionsschicht (HIL), eine Elektronentransportschicht (ETL), eine Elektroneninjektionsschicht (EIL), eine Lochblockierschicht oder eine Kombination davon aufweist.

13. Organische Leuchtdiode nach Anspruch 11, wobei die Verbindung für eine optoelektronische Vorrichtung in einer Lochtransportschicht (HTL), einer Lochinjektionsschicht (HIL), einer Elektronentransportschicht (ETL) oder einer Elektroneninjektionsschicht (EIL) enthalten ist.

14. Organische Leuchtdiode nach Anspruch 11, wobei die Verbindung für eine optoelektronische Vorrichtung in einer Emissionsschicht enthalten ist.

15. Organische Leuchtdiode nach Anspruch 11, wobei die Verbindung für eine optoelektronische Vorrichtung als ein phosphoreszierendes oder fluoreszierendes Wirtsmaterial in einer Emissionsschicht verwendet wird.

16. Display mit der organischen Leuchtdiode nach Anspruch 11.

## Revendications

1. Composé destiné à un dispositif optoélectronique représenté par la formule chimique 1 suivante : 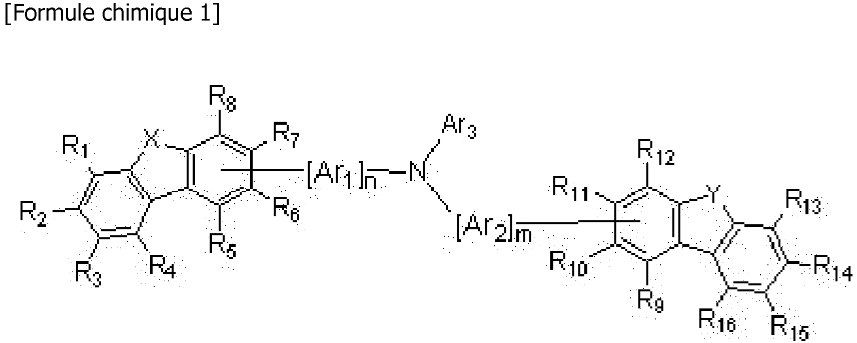 où, dans la formule chimique 1,
les groupes R₁ à R₁₆ sont identiques ou différents et sont indépendamment choisis à partir d'atomes d'hydrogène, de deutérium, d'halogène, d'un groupe cyano, d'un groupe hydroxyle, d'un groupe amine, d'un groupe amine en C1 à C20 substitué ou non substitué, d'un groupe nitro, d'un groupe carboxyle, d'un groupe ferrocényle, d'un groupe alkyle en C1 à C20 substitué ou non substitué, d'un groupe aryle en C6 à C30 substitué ou non substitué, d'un groupe hétéroaryle en C2 à C30 substitué ou non substitué, d'un groupe alcoxy en C1 à C20 substitué ou non substitué, d'un groupe aryloxy en C6 à C20 substitué ou non substitué, d'un groupe silyloxy en C3 à C40 substitué ou non substitué, d'un groupe acyle en C1 à C20 substitué ou non substitué, d'un groupe alcoxycarbonyle en C2 à C20 substitué ou non substitué, d'un groupe acyloxy en C2 à C20 substitué ou non substitué, d'un groupe acylamino en C2 à C20 substitué ou non substitué, d'un groupe alcoxycarbonyl amino en C2 à C20 substitué ou non substitué, d'un groupe aryloxycarbonyl amino en C7 à C20 substitué ou non substitué, d'un groupe sulfamoyl amino en C1 à C20 substitué ou non substitué, d'un groupe sulfonyle en C1 à C20 substitué ou non substitué, d'un groupe alkylthiol en C1 à C20 substitué ou non substitué, d'un groupe arylthiol en C6 à C20 substitué ou non substitué, d'un groupe hétérocyclothiol en C1 à C20 substitué ou non substitué, d'un groupe uréide en C1 à C20 substitué ou non substitué, et d'un groupe silyle en C3 à C40 substitué ou non substitué,
au moins un groupe parmi R₁ à R₈ représente une liaison avec Ar₁,
au moins un groupe parmi R₉ à R₁₆ représente une liaison avec Ar₂,
au moins un groupe parmi R₁ à R₈ est éventuellement lié avec Ar₁ par une liaison sigma, au moins un groupe parmi R₉ à R₁₆ est éventuellement lié avec Ar₂ par une liaison sigma,
X est choisi parmi les groupes NR₁₇, O, S et SO₂ (O=S=O), où R₁₇ est un groupe alkyle en C1 à C20 substitué ou non substitué, un groupe aryle en C6 à C30 substitué ou non substitué, ou un groupe hétéroaryle en C2 à C30 substitué ou non substitué,
Y est choisi parmi les groupes O, S, et SO₂ (O=S=O),
Ar₁ et Ar₂ sont identiques ou différents et sont indépendamment un groupe aryle en C6 à C30 substitué ou non substitué ou un groupe hétéroaryle en C2 à C30 substitué ou non substitué,
n est un nombre entier allant de 1 à 4,
m est un nombre entier allant de 0 à 4, et
Ar₃ est un groupe pyrrolyle substitué ou non substitué, un groupe pyrazolyle substitué ou non substitué, un groupe imidazolyle substitué ou non substitué, un groupe triazolyle substitué ou non substitué, un groupe oxazolyle substitué ou non substitué, un groupe thiazolyle substitué ou non substitué, un groupe oxadiazolyle substitué ou non substitué, un groupe thiadiazolyle substitué ou non substitué, un groupe pyridyle substitué ou non substitué, un groupe pyrimidinyle substitué ou non substitué, un groupe pyrazinyle substitué ou non substitué, un groupe triazinyle substitué ou non substitué, un groupe benzimidazolyle substitué ou non substitué, un groupe indolyle substitué ou non substitué, un groupe quinolinyle substitué ou non substitué, un groupe isoquinolinyle substitué ou non substitué, un groupe quinazolinyle substitué ou non substitué, un groupe quinoxalinyle substitué ou non substitué, un groupe naphthydinyle substitué ou non substitué, un groupe benzoxazinyle substitué ou non substitué, un groupe benzthiazinyle substitué ou non substitué, un groupe acridinyle substitué ou non substitué, un groupe phénazinyle substitué ou non substitué, un groupe phénothiazinyle substitué ou non substitué, ou un groupe phénoxazinyle substitué ou non substitué.

2. Composé selon la revendication 1, dans lequel X est choisi parmi les groupes NR₁₇, O, S et SO₂ (O=S=O), où R₁₇ est un groupe alkyle en C1 à C20 substitué ou non substitué, un groupe aryle en C6 à C30 substitué ou non substitué, ou un groupe hétéroaryle en C2 à C30 substitué ou non substitué, et le groupe aryle ou groupe hétéroaryle « substitué » se rapporte à un groupe substitué avec au moins un substituant choisi parmi un atome de deutérium, d'halogène, un groupe cyano, un groupe hydroxyle, un groupe amine, un groupe amine en C1 à C20 substitué ou non substitué, un groupe nitro, un groupe alkyle en C1 à C20 substitué ou non substitué, un groupe alcoxy en C1 à C20 substitué ou non substitué, un groupe silyle en C3 à C40 substitué ou non substitué, et une combinaison de ceux-ci.

3. Composé selon la revendication 1, dans lequel le composé destiné à un dispositif optoélectronique comprend l'un des composés représenté par les formules chimiques 2 à 7 suivantes : 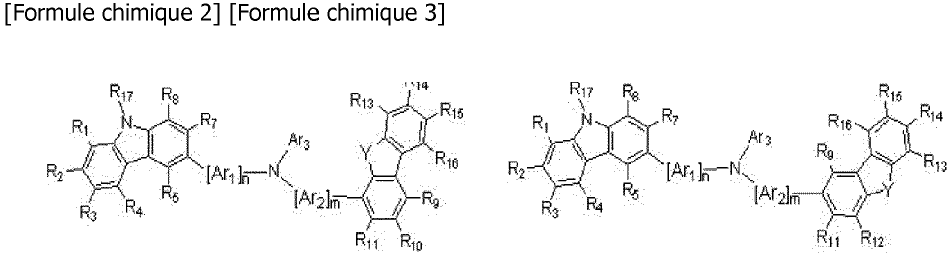 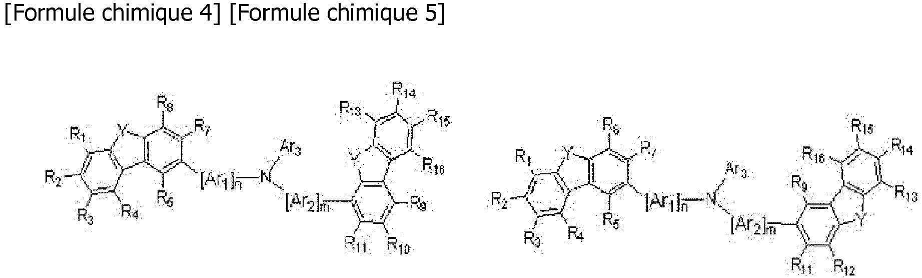 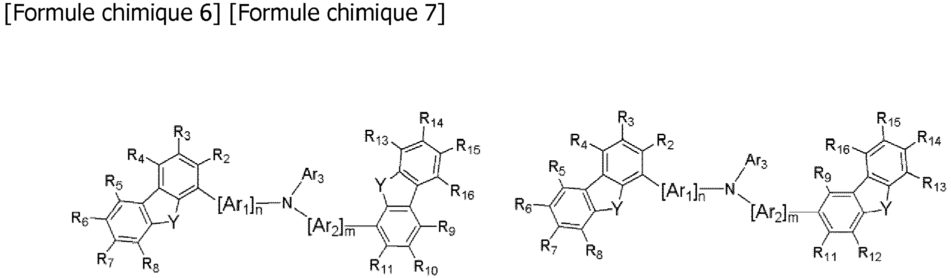 où, dans les formules chimiques 2 à 7,
R₁ à R₁₆ sont identiques ou différents et sont indépendamment choisis à partir d'atomes d'hydrogène, de deutérium, d'halogène, d'un groupe cyano, d'un groupe hydroxyle, d'un groupe amine, d'un groupe amine en C1 à C20 substitué ou non substitué, d'un groupe nitro, d'un groupe carboxyle, d'un groupe ferrocényle, d'un groupe alkyle en C1 à C20 substitué ou non substitué, d'un groupe aryle en C6 à C30 substitué ou non substitué, d'un groupe hétéroaryle en C2 à C30 substitué ou non substitué, d'un groupe alcoxy en C1 à C20 substitué ou non substitué, d'un groupe aryloxy en C6 à C20 substitué ou non substitué, d'un groupe silyloxy en C3 à C40 substitué ou non substitué, d'un groupe acyle en C1 à C20 substitué ou non substitué, d'un groupe alcoxycarbonyle en C2 à C20 substitué ou non substitué, d'un groupe acyloxy en C2 à C20 substitué ou non substitué, d'un groupe acylamino en C2 à C20 substitué ou non substitué, d'un groupe alcoxycarbonyl amino en C2 à C20 substitué ou non substitué, d'un groupe aryloxycarbonyl amino en C7 à C20 substitué ou non substitué, d'un groupe sulfamoyl amino en C1 à C20 substitué ou non substitué, d'un groupe sulfonyle en C1 à C20 substitué ou non substitué, d'un groupe alkylthiol en C1 à C20 substitué ou non substitué, d'un groupe arylthiol en C6 à C20 substitué ou non substitué, d'un groupe hétérocyclothiol en C1 à C20 substitué ou non substitué, d'un groupe uréide en C1 à C20 substitué ou non substitué, et d'un groupe silyle en C3 à C40 substitué ou non substitué,
R₁₇ est un groupe alkyle en C1 à C20 substitué ou non substitué, un groupe aryle en C6 à C30 substitué ou non substitué, ou un groupe hétéroaryle en C2 à C30 substitué ou non substitué,
Y est choisi parmi les groupes O, S, et SO₂ (O=S=O),
Ar₁ et Ar₂ sont identiques ou différents, et sont indépendamment un groupe aryle en C6 à C30 substitué ou non substitué ou un groupe hétéroaryle en C2 à C30 substitué ou non substitué
n est un nombre entier allant de 1 à 4,
m est un nombre entier allant de 0 à 4, et
Ar₃ est un groupe pyrrolyle substitué ou non substitué, un groupe pyrazolyle substitué ou non substitué, un groupe imidazolyle substitué ou non substitué, un groupe triazolyle substitué ou non substitué, un groupe oxazolyle substitué ou non substitué, un groupe thiazolyle substitué ou non substitué, un groupe oxadiazolyle substitué ou non substitué, un groupe thiadiazolyle substitué ou non substitué, un groupe pyridyle substitué ou non substitué, un groupe pyrimidinyle substitué ou non substitué, un groupe pyrazinyle substitué ou non substitué, un groupe triazinyle substitué ou non substitué, un groupe benzimidazolyle substitué ou non substitué, un groupe indolyle substitué ou non substitué, un groupe quinolinyle substitué ou non substitué, un groupe isoquinolinyle substitué ou non substitué, un groupe quinazolinyle substitué ou non substitué, un groupe quinoxalinyle substitué ou non substitué, un groupe naphthydinyle substitué ou non substitué, un groupe benzoxazinyle substitué ou non substitué, un groupe benzthiazinyle substitué ou non substitué, un groupe acridinyle substitué ou non substitué, un groupe phénazinyle substitué ou non substitué, un groupe phénothiazinyle substitué ou non substitué, ou un groupe phénoxazinyle substitué ou non substitué.

4. Composé selon la revendication 1, dans lequel le composé destiné à un dispositif optoélectronique comprend l'un des composés représenté par les formules chimiques 8 ou 9 suivantes : 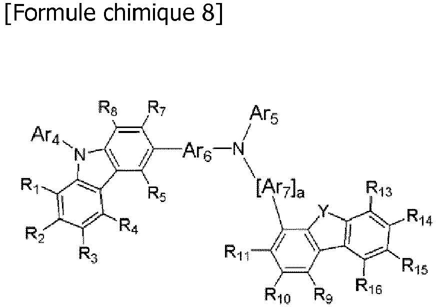 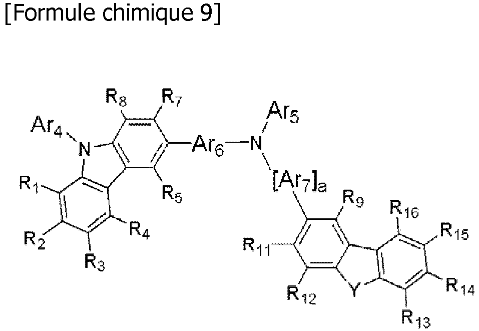 où, dans les formules chimiques 8 et 9,
Ar₄ est choisi à partir du substituant représenté par les formules suivantes 10 à 18, et
Ar₅ est choisi à partir d'un groupe pyrrolyle substitué ou non substitué, d'un groupe pyrazolyle substitué ou non substitué, d'un groupe imidazolyle substitué ou non substitué, d'un groupe triazolyle substitué ou non substitué, d'un groupe oxazolyle substitué ou non substitué, d'un groupe thiazolyle substitué ou non substitué, d'un groupe oxadiazolyle substitué ou non substitué, d'un groupe thiadiazolyle substitué ou non substitué, d'un groupe pyridyle substitué ou non substitué, d'un groupe pyrimidinyle substitué ou non substitué, d'un groupe pyrazinyle substitué ou non substitué, d'un groupe triazinyle substitué ou non substitué, d'un groupe benzimidazolyle substitué ou non substitué, d'un groupe indolyle substitué ou non substitué, d'un groupe quinolinyle substitué ou non substitué, d'un groupe isoquinolinyle substitué ou non substitué, d'un groupe quinazolinyle substitué ou non substitué, d'un groupe quinoxalinyle substitué ou non substitué, d'un groupe naphthydinyle substitué ou non substitué, d'un groupe benzoxazinyle substitué ou non substitué, d'un groupe benzthiazinyle substitué ou non substitué, d'un groupe acridinyle substitué ou non substitué, d'un groupe phénazinyle substitué ou non substitué, d'un groupe phénothiazinyle substitué ou non substitué, ou d'un groupe phénoxazinyle substitué ou non substitué, 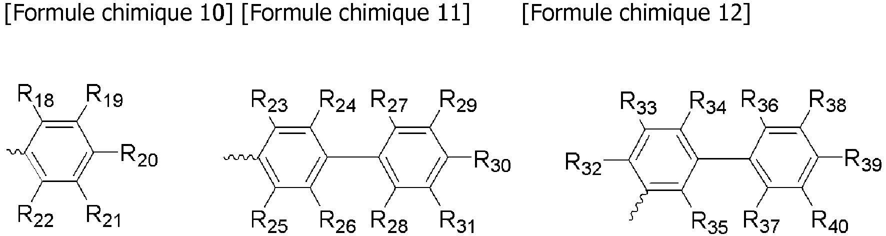 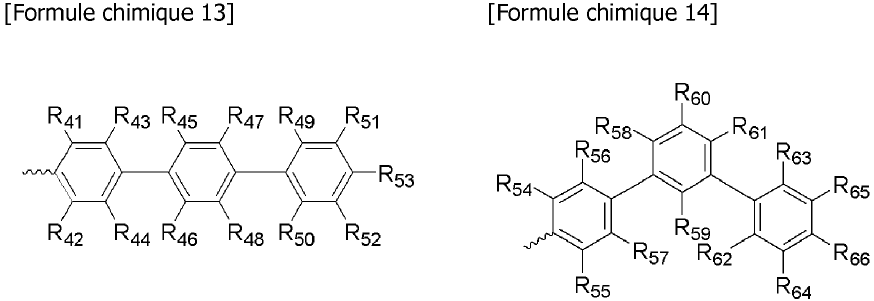 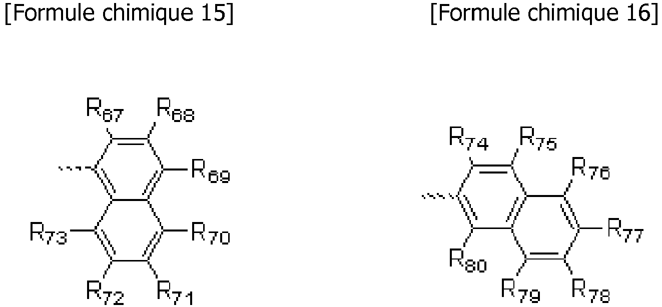 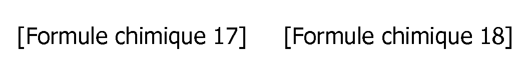 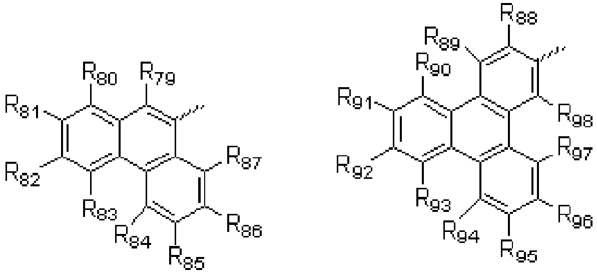
les groupes R₁ à R₅, R₇ à R₁₆ et R₁₈ à R₉₈ sont identiques ou différents, et sont indépendamment choisis parmi à partir d'atomes d'hydrogène, de deutérium, d'halogène, d'un groupe cyano, d'un groupe hydroxyle, d'un groupe amine, d'un groupe amine en C1 à C20 substitué ou non substitué, d'un groupe nitro, d'un groupe alkyle en C1 à C20 substitué ou non substitué, d'un groupe alcoxy en C1 à C20 substitué ou non substitué, ou d'un groupe silyle en C3 à C40 substitué ou non substitué,
Ar₆ et Ar₇ sont identiques ou différents, et sont indépendamment un substituant choisi à partir des formules chimiques 10 à 18 ci-dessus et au moins l'un des groupes R₁₈ à R₉₈ est lié à un atome adjacent, et
a est égal à 0 ou 1.

5. Composé selon la revendication 4, dans lequel Ar₄ est choisi à partir d'un substituant représenté par les formules 10 à 18 ci-dessus, et au moins un des substituants parmi R₁₈ à R₉₈ qui est choisi pour Ar₄ n'est pas un atome d'hydrogène.

6. Composé selon la revendication 1, dans lequel le composé est représenté par les formules chimiques A-321 à A-323 suivantes.

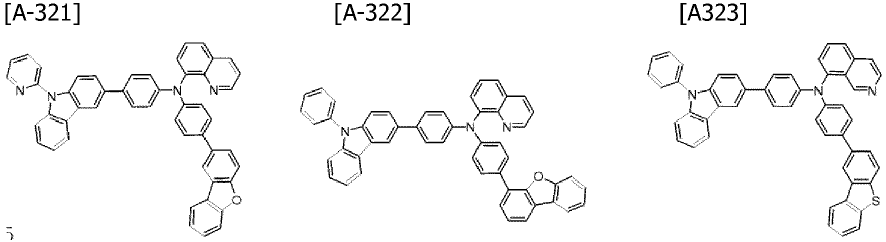

7. Composé A selon la revendication 1, dans lequel le composé est représenté par les formules chimiques A-400, A-402 à A-404, A-407, A-408, A-411 et A-413 suivantes.

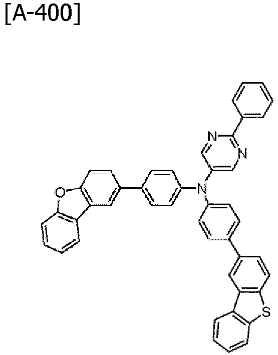

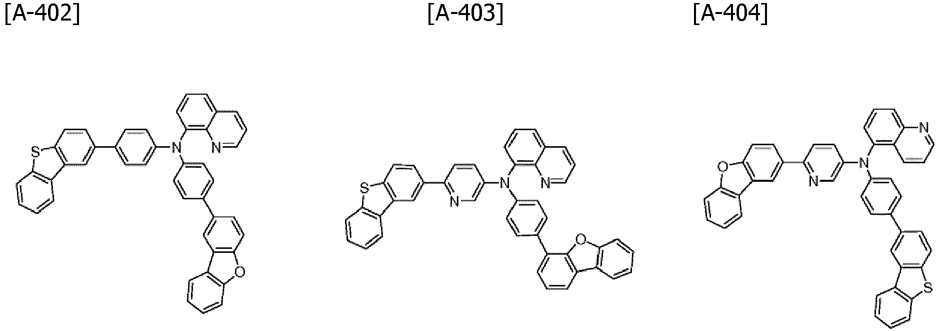

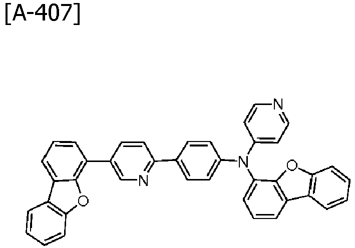

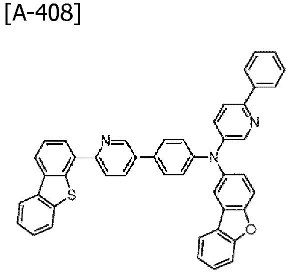

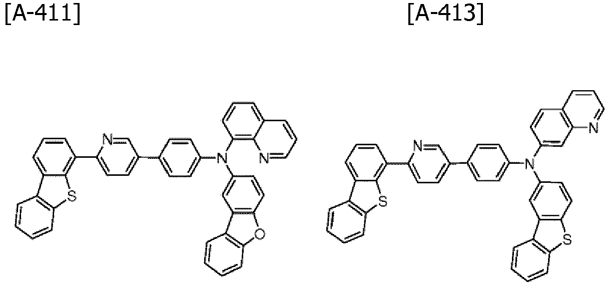

8. Composé selon la revendication 1, dans lequel le composé destiné à un dispositif optoélectronique comprend une matière de transport de trou ou une matière d'injection de trous pour une diode organique émettant de la lumière.

9. Composé selon la revendication 1, dans lequel le composé destiné à un dispositif optoélectronique comprend une énergie des excitons à l'état triplet (T1) d'environ 2,0 eV ou supérieure.

10. Composé selon la revendication 1, dans lequel le dispositif optoélectronique comprend un dispositif photoélectronique organique, une diode organique émettant de la lumière, une cellule organique solaire, un transistor organique, un tambour photoconducteur organique, un dispositif de mémoire organique, ou similaire.

11. Diode organique émettant de la lumière comportant une anode, une cathode, et au moins un ou plusieurs des films minces organiques est mis en place entre l'anode et la cathode, et au moins l'un des films minces organiques comprend le composé selon la revendication 1.

12. Diode organique émettant de la lumière selon la revendication 11, dans laquelle le film mince organique comprend une couche d'émission, une couche de transport de trou (HTL), une couche d'injection de trou (HIL), une couche de transport d'électrons (ETL), une couche d'injection d'électrons (EIL), un film de blocage de trou, ou une combinaison ce ceux-ci.

13. Diode organique émettant de la lumière selon la revendication 11, le composé destiné à un dispositif optoélectronique est compris dans une couche de transport de trou (HTL), une couche d'injection de trou (HIL), une couche de transport d'électrons (ETL) ou une couche d'injection d'électrons (EIL).

14. Diode organique émettant de la lumière selon la revendication 11, dans laquelle le composé destiné à un dispositif optoélectronique est compris dans une couche d'émission.

15. Diode organique émettant de la lumière selon la revendication 11, dans laquelle le composé destiné à un dispositif optoélectronique est utilisé en tant que matière hôte phosphorescente ou fluorescente dans une couche d'émission.

16. Dispositif d'affichage comprenant la diode organique émettant de la lumière selon la revendication 11.
